# EUROPEAN PATENT SPECIFICATION

(11) **EP 0 088 350 B1**
(45) Date of publication and mention of the grant of the patent: **20.02.1985**
(21) Application number: 83102014.4
(22) Date of filing: 02.03.1983
(51) Int. Cl.: C07K 5/06, A61K 37/02

(54) **Carboxyalkyl dipeptides, processes for their production and pharmaceutical compositions containing them**
Carboxyalkyl-Dipeptiden, Verfahren zu deren Herstellung und deren pharmazeutische Zusammensetzungen
Carboxyalkyl dipeptides, leur procédé de préparation et leurs compositions pharmaceutiques les contenant

(30) Priority: 08.03.1982 US 355639; 08.03.1982 US 355638; 22.03.1982 US 360532; 19.01.1983 ZA 830362
(43) Date of publication of application: 14.09.1983
(73) Proprietor: SCHERING CORPORATION, Kenilworth New Jersey 07033 (US)
(72) Inventor: Smith, Elizabeth M., Verona New Jersey 07044 (US); Witkowski, Joseph T., Morristownship New Jersey 07960 (US); Doll, Ronald J., Maplewood New Jersey 07040 (US); Gold, Elijah H., West Orange New Jersey 07052 (US); Neustadt, Bernard R., West Orange New Jersey 07052 (US); Yehaskel, Albert S., Fairlawn New Jersey 07410 (US)
(74) Representative: von Kreisler, Alek, Dipl.-Chem.

## Description

The present invention relates to carboxyalkyl dipeptides substituted with groups containing one sulfamoyl group. The compounds are useful as antihypertensive agents, in the treatment of congestive heart failure and glaucoma.

Carboxyalkyl dipeptides which are useful as inhibitors of angiotensinconverting enzyme and as antihypertensive agents are known from the published European patent applications Nos. 12401 and 50800.

The compounds of the present invention are compounds of the formula 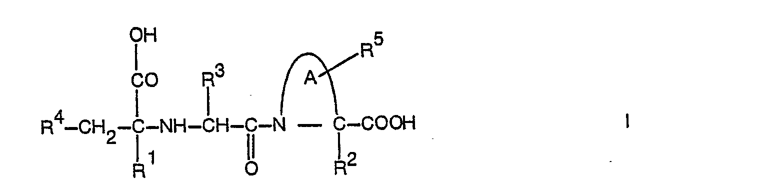 and the pharmaceutically acceptable esters thereof and the pharmaceutically acceptable salts of the free compounds and the esters, wherein;
R¹ and R² independently are hydrogen or lower alkyl; 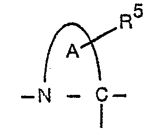 the group is one of the structures II to VIII 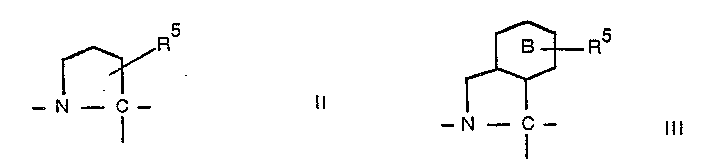 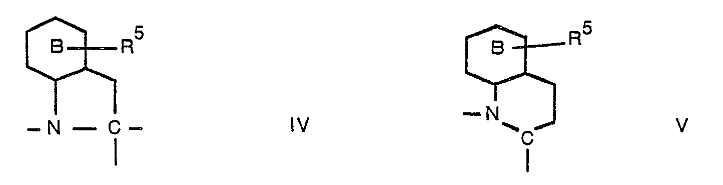 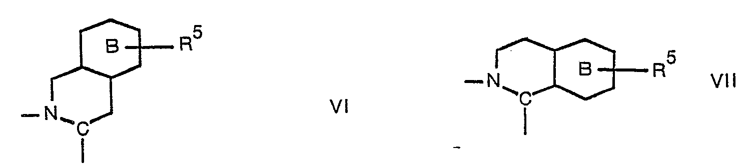 (wherein B is a saturated or aromatic ring) or 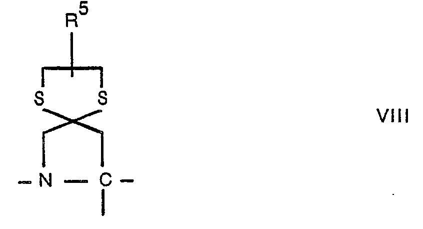 one of R³, R⁴ and R⁵ is a group Z―(CH₂)₀₋₆―. wherein Z has one of the following values Z¹ to Z¹⁰ 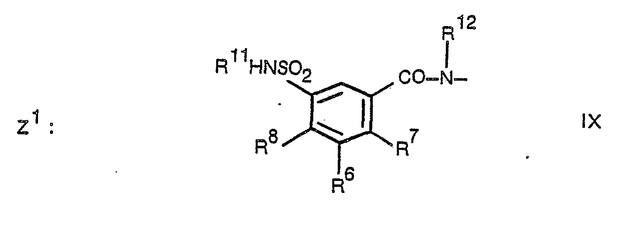 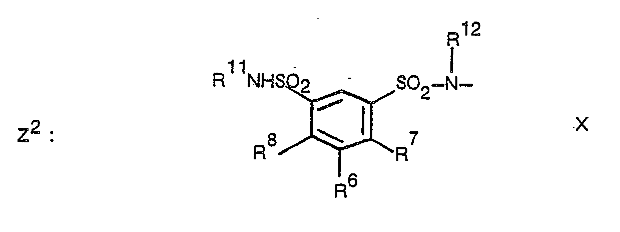 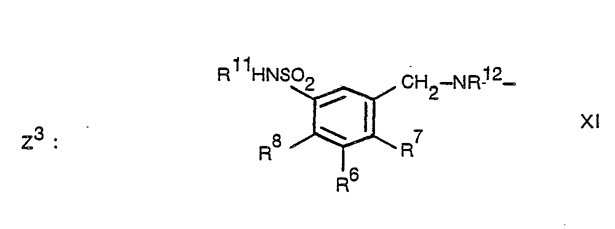 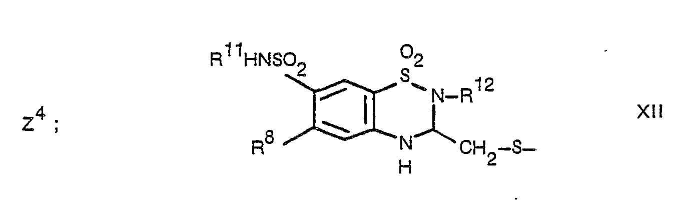 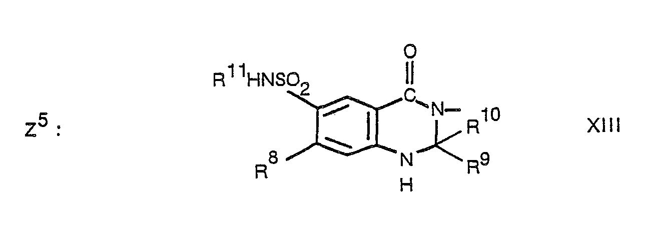 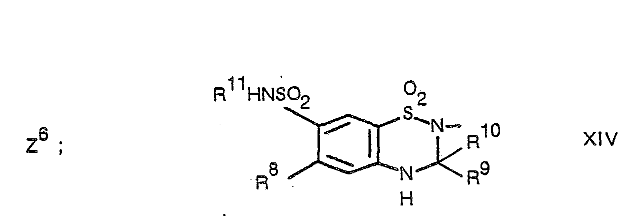 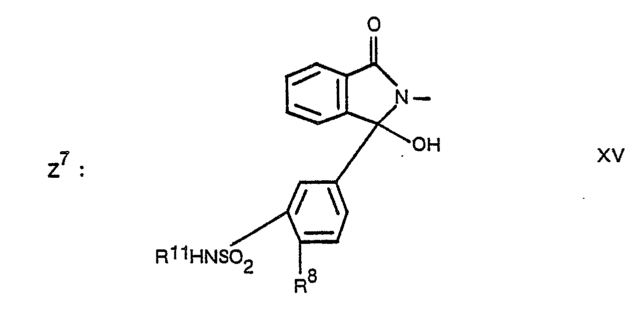 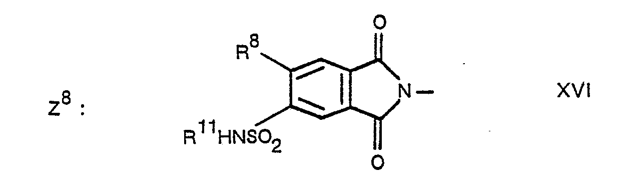 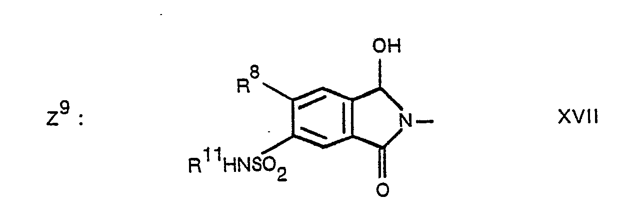 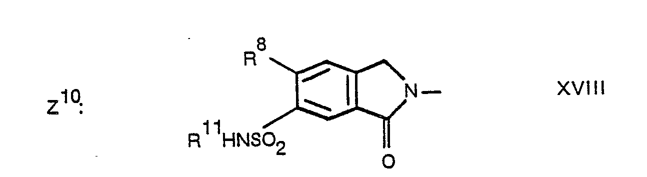 wherein R⁸ is Cl or CF₃;
R⁶ is hydrogen or halogen;
R⁷ is hydrogen, halogen, carboxy, hydroxy or amino;
R⁹ and R¹⁰ are independently hydrogen, lower alkyl or halo-lower alkyl and R⁹ can also be phenyl or phenyl lower alkyl;
R" is hydrogen or lower alkyl;
R¹² is hydrogen, lower alkyl or phenyl lower alkyl;
   whereby when R³ is the group Z―(CH₂)₀₋₆―. then R³ is Z¹―(CH₂)₁₋₆―, Z²―(CH₂)₁₋₆―, Z³―(CH₂)₁₋₆―, Z⁴―CH₂―, Z⁵―(CH₂)₁₋₆―. Z⁶―(CF₂)₁₋₆―, Z⁷-(CH₂)₁₋₆―, Z⁸―(CH₂)₁₋₆―, Z⁹―(CH₂)₁₋₆―, or Z¹⁰―(CH₂)₁₋₆―, R⁴ is lower alkyl, benzyl, benzyloxy, benzylthio, phenoxy, or phenylthio, R⁵ is hydrogen; and the group 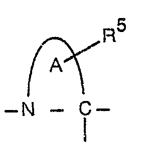 is one of the structures II to VIII;
   and when R⁴ is the group Z―(CH₂)₀₋₆―, then R⁴ is Z¹―(CH₂)₀₋₆―, Z²―(CH₂)₀₋₆―, Z³―(CHJ₂)₀₋₆―, Z⁴―(CH₂)₀₋₆―, Z⁵―(CH₂)₀₋₆―, Z⁶―(CH₂)₀₋₆―, Z⁷―(CH₂)₀₋₆―, Z⁸―(CH₂)₀₋₆―, Z⁹―(CH₂)₀₋₆― or

Z¹⁰―(CH₂)₀₋₆― and R³ is hydrogen, lower alkyl or amino lower alkyl and R⁵ is hydrogen; and the group 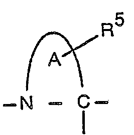 is one of the structures II to VIII; and when R⁵ is the group Z―(CH₂)₀₋₆―, then R⁵ is Z¹, Z², Z³, Z⁴, Z⁵, Z⁶, Z⁷, Z⁸, Z⁹ or Z¹⁰, R³ is hydrogen, lower alkyl or amino lower alkyl and R⁴ is lower alkyl, benzyl, benzyloxy, benzylthio, phenoxy or phenylthio; and the group 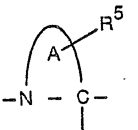 is one of the structures II to VII.

One embodiment of the present invention comprises compounds of formula I, its esters and salts, wherein R⁴ is the group Z―(CH₂)₀₋₆―. Among these compounds certain groups of compounds are preferred:
compounds, wherein the group 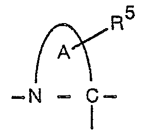 is the group of formula II, IV (wherein B is a saturated ring) or VIII, preferably R⁵ being hydrogen;
compounds, wherein R⁴ is Z―(CH₂)₀₋₆―, Z being Z¹, Z², Z³, Z⁵, Z⁷, Z⁸, Z⁹ or Z¹⁰;
compounds, wherein R⁴ is Z¹―(CH₂)₂ or 3―, Z²―(CH₂)₂ or 3―, Z³―(CH₂)₂ or 3―, Z⁵―(CH₂)₂ or 3―, Z⁷―(CH₂)₂ or 3― Z⁸―(CH₂)₂ or 3―, Z⁹―(CH₂)₂ or 3― or Z¹⁰―(CH₂)₂ or 3―;
compounds, wherein R⁴ is Z⁴;
compounds, wherein R¹ and R² are hydrogen;
compounds, wherein R⁶ is hydrogen and R⁷ is hydrogen or hydroxy;
compounds, wherein R⁹ and R¹⁰ are independently hydrogen or methyl;
compounds, wherein R⁸ is chloro;
compounds, wherein R³ is methyl;
compounds, wherein R¹ and R² are independently hydrogen or lower alkyl (preferably hydrogen), the group 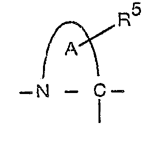 is the group of formula II or IV, wherein B is a saturated ring and R⁵ is hydrogen, R⁴ is Z¹―(GH₂)₃―, Z²―(CH₂)₃― or Z⁴, R⁶ and R⁷ are hydrogen and R⁸ is chloro; and R³ is hydrogen, lower alkyl or amino lower alkyl (preferably methyl);
of particular interest are compounds, wherein R¹ and R² are hydrogen, the group 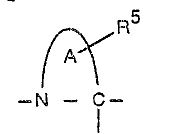 is the group of formula IV wherein B is a saturated ring, and R⁵ is hydrogen, R⁴ is Z¹―(CH₂)₃― or Z²―(CH₂)₃―, wherein R⁶ is hydrogen, R⁷ is hydrogen or hydroxy, and R⁸ is chloro, and R³ is methyl, preferably in the form of its mono-or-di-ethyl ester. Also the analogous compounds, wherein the group 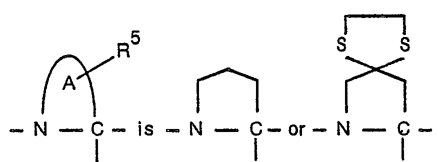 are preferred.

Another embodiment of the present invention comprises compounds of formula I, its esters and salts, wherein R³ is the group Z―(CH₂)₀₋₆―. Among these compounds the following groups of compounds are preferred:
compounds, wherein the group 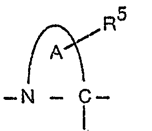 is the group of formula II, IV (wherein B is a saturated ring) or VIII;
compounds, wherein R³ is Z¹―(CH₂)₄―, Z²―(CH₂)₄―, Z³―(CH₂)₄―, Z⁴―CH₂―, Z⁵―(CH₂)₄―, Z⁶―(CH₂)₄―, Z⁷―(CH₂)₄―, Z⁸―(CH₂)₄―, Z⁹―(CH₂)₄― or Z¹⁰―(CH₂)₄―;
compounds, wherein R¹ and R² are hydrogen;
compounds, wherein R⁶ is hydrogen and R⁷ is hydrogen or hydroxy;
compounds, wherein R⁹ and R¹⁰ are independently hydrogen or methyl;
compounds, wherein R⁸ is chloro;
compounds, wherein R⁴ is benzyl or ethyl;
compounds, wherein R¹ and R² are independently hydrogen or lower alkyl (preferably hydrogen), the group 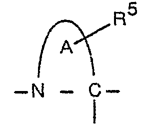 is the group of formula II or IV, wherein B is a saturated ring, and R⁵ is hydrogen, R³ is Z¹―(CH₂)₄―, Z²―(CH₂)₄― or Z⁴―CH₂―, R⁶ and R⁷ are hydrogen and R⁸ is chloro and R⁴ is 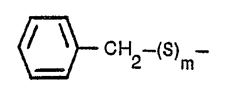 wherein m is zero or 1, of particular interest are compounds, wherein R¹ and R² are hydrogen, the group 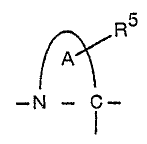 is the group of formula IV, wherein B is a saturated ring and R⁵ is hydrogen, R³ is Z¹―(CH₂)₄― or Z²―(CH₂)₄―, wherein R⁶ and R⁷ are hydrogen, R⁸ is chloro; and R⁴ is benzyl, preferably in the form of its mono-or-di-ethyl ester. Also the analogous compounds, wherein the group 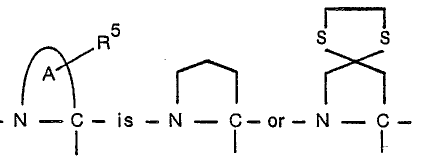 are preferred.

Another embodiment of the present invention comprises compounds of formula I, its esters and salts, wherein R⁵ is the group Z―(CH₂)₀₋₆―. Among these compounds the following groups of compounds are preferred:
compounds, wherein the group 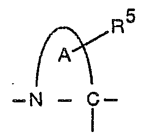 is the group of II, VI (wherein B is an aromatic ring), or IV (wherein B is a saturated ring);
compounds, wherein the group 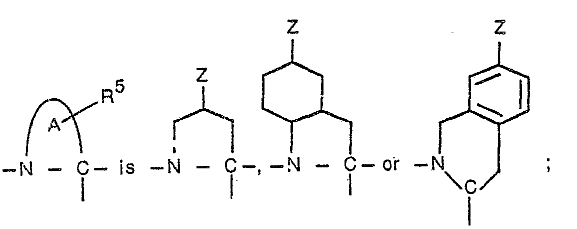 compounds, wherein R⁵ is Z¹, Z², Z³, Z⁵, Z⁷, Z⁸, Z⁹ or Z¹⁰;
compounds, wherein R¹ and R² are hydrogen;
compounds, wherein R⁶ is hydrogen and R⁷ is hydrogen or hydroxy;
compounds, wherein R⁹ and R¹⁰ are independently hydrogen or methyl;
compounds, wherein R⁸ is chloro;
compounds, wherein R³ is methyl;
compounds, wherein R⁴ is benzyl or ethyl;
compounds, wherein R¹ and R² are independently hydrogen or lower alkyl, the group 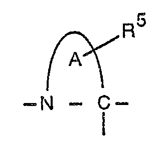 is the group of formula III, IV, V, VI or VII, R⁴ is 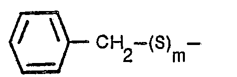 wherein m is zero or 1 and R⁵ is Z¹ or Z², wherein R⁶ and R⁷ are hydrogen and R⁸ is chloro, and R³ is hydrogen or lower alkyl (preferably methyl).

The lower alkyl groups, except where noted otherwise, include straight and branched chain hydrocarbon radicals from one to six carbon atoms, for example, methyl, ethyl, propyl, isopropyl, butyl, isobutyl, t-butyl, pentyl, isopentyl, hexyl, cyclopropyl, cyclohexyl and the like.

The compounds of this invention form esters. In these esters the hydroxy group of the carboxy groups shown in formula I can be replaced by the same or by different groups which are selected from alkoxy having from 1 to 8 carbon atoms, phenoxy, phenylalkyloxy having from 7 to 10 carbon atoms,―OCH₂OCO- alkyl having from 3 to 8 carbon atoms,―OCH₂CO-phenyl,-O(CH₂)ₖ―O―phenyl wherein k is 1 or 2 and the phenyl ring may be substituted by halogen, hydroxy, trifluoromethyl, alkoxy having from 1 to 6 carbon atoms, alkyl having from 1 to 6 carbon atoms (the phenyl group preferably containing one substituent) and ―OlCH₂)ₖ―O―naphthyl wherein k is 1 or 2.

Preferred are alkyl esters (the alkyl group being defined as above) and aryl esters, especially the ethyl and benzyl esters. Of particular interest are the mono-esters, wherein the carboxy group attached to the group 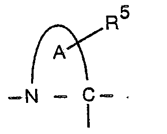 is in the free form.

The compounds of this invention form salts with various inorganic and organic acids and bases which are also within the scope of the invention. Such salts include ammonium salts, alkali metal salts like sodium and potassium salts (which are preferred), alkaline earth metal salts like the calcium and magnesium salts, salts with organic bases e.g., dicyclohexylamine salts, N-methyl-D-glucamine, salts with amino acids like arginine, lysine and the like. Also, salts with organic and inorganic acids may be prepared, e.g., HCI, HBr, H₂SO₄, H₃PO₄, methanesulfonic acid, toluenesulfonic acid, maleic acid, fumaric acid and camphorsulfonic acid. The non-toxic physiologically acceptable salts are preferred, although other salts are also useful, e.g., in isolating of purifying the product.

This invention includes all possible stereoisomers of the compounds. Preferred stereoisomers are those in which the absolute configurations at each of the three carbon atoms bonded to both a nitrogen and a carbonyl group corresponds most closely to the absolute configuration of L-aminoacids. The preferred compounds contain a cis,syn-octahydro-1H-indole-2(S)-carboxylic acid moiety or a 1,4-dithia-7- azaspiro[4.4]nonane-8(S)-carboxylic acid moiety.

Non limiting examples of preferred compounds of the present invention are:
1 - {N - [1(S) - ethoxycarbonyl - 5 - (4 - chloro - 3 - sulfamoyl) - benzenesulfonaminopentyl] - (S) - alanyl}- cis,syn - octahydro - 1H - indole - 2(S) - carboxylic acid.
1 - {N - [1 (S) - ethoxycarbonyl - 5 - (4 - chioro - 3 - sulfamoyl) - benzamidopentyl] - (S) - alanyl} - cissyn - octahydro - 1H - indole - 2(S) - carboxylic acid,
1 - {N - [1(S) - ethoxycarbonyl - 5 - (4 - chloro - 2[hydroxy - 5 - sulfamoyl) - benzamidopentyl] - (S) - alanyl} - cis,syn - octahydro - 1H- indole - 2(S) - carboxylic acid,
1 - {Na - [1 (S) - ethoxycarbonyl - 3 - phenylpropyl] - Ne - [(4 - chloro - 3 - sulfamoyl)benzenesulfonyl] - (S) - lysyl} - cissyn - octahydro - 1H - indole - 2(S) - carboxylic acid,
1 - {Na - [1(S) - ethoxycarbonyl -3 - phenylpropyl] - Nε - [(4 - chloro - 3 - sulfamoyl)benzoyl] - (S) - lysyl}- cis,syn - octahydro - 1H - indole - 2(S) - carboxylic acid,
7 - (4 - chloro - 3 - sulfamoylbenzamidol - 2 - {N - [1(S) - ethoxycarbonyl - 3 - phenylpropyl] - (S) - alanyl}-1,2,3,4 - tetrahydroisoquinoline - 3(S) - carboxylic acid,
1 - {N - [1(S) - carboxy - 5 - [[(4 - chloro - 2 - Hydroxy - 5 - sulfamoylphenyl)carbonyl]amino]pentyl] - (S) - alanyl} - cis,syn - octahydro - 1 H - indole - 2(S) - carboxylic acid,
1 - {N - [1(S) - carboxy - 5 - [[(4- chloro - 3 - (N - methyl sulfamoyl)phenyl]carbonyl]amino]pentyl] - (S) - alanyl} - cis,syn - octahydro - 1 H - indole - 2(S) - carboxylic acid,
1 - {N - [1(S) - carboxy - 5 - [[(4 - chloro - 3 - sulfamoyl phenyl)carbonyl]amino]pentyl] - (S) - alanyl} - cis,syn - octahydro - 1H - indole - 2(S) - carboxylic acid,
1 - [N - [1 (S) - ethoxycarbonyl - 5[[(4 - chloro - 2 - amino - 5 - sulfamylphenyl) - carbonyl]amino]pentyl] - (S)alanyl] - cis,syn - octahydro - 1H - indole 2(S)carboxylic acid,
1 - {N - [1(S) - ethoxycarbonyl - 5 - [7 - chloro - 4 - oxo - 6 - sulfamyl - 2 - phenyl - 1,2,3,4 tetrahydro quinazolin - 3 - yl]pentyl] - (S) - alanyl} - cis,syn - octahydro - 1H - indole - 2(S)carboxylic acid, or the corresponding free acids or esters, respectively.

The compounds of the present invention can be produced by one or more of the methods and subroutes depicted in the following equations. Reactive groups not involved in the reactions described below such as amino and carboxy groups may be protected by methods standard in peptide chemistry prior to the coupling reactions and subsequently deprotected to obtain the desired products. Racemates, if obtained by these processes, can be resolved by standard techniques such as column chromatography or fractional crystallization.

A. For the preparaton of compounds of formula I, wherein R¹ is hydrogen a ketocompound (XIX) is condensed with a dipeptide (XX) under reduction. 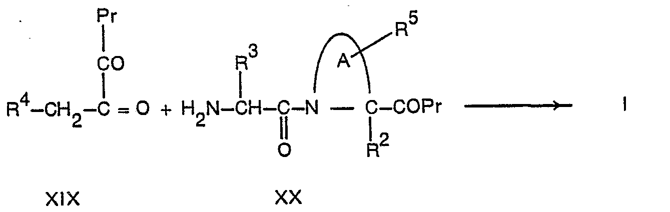 In these compounds A, R², R³, R⁴ and R⁵ are as defined above and Pr stands for a free or a protected (e.g. by esterification) hydroxy group.

The ketocompound (XIX) can be condensed with the dipeptide (XX) in aqueous solution, optimally near neutrality, or in a suitable organic solvent (for example CH₃0H) in the presence of a reducing agent such as for example sodium cyanoborohydride to give directly the desired compound I (wherein R' is hydrogen). Alternatively, the intermediate Schiff base, enamine, or aminol may be catalytically reduced to yield product I, for example, by hydrogen in the presence of palladium on carbon (e.g. 10% palladium on carbon) or of Raney nickel. The ratio of diasteriomeric products formed may be altered by the choice of catalyst.

B. Alkylation of a dipeptide (XX) by means of a compound of formula (XXI) 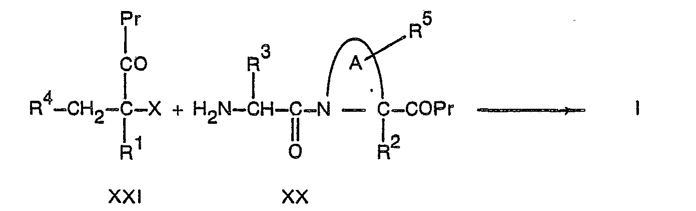 wherein X is chloro, bromo, iodo, alkanesulfonyloxy or arenesulfonyloxy, R¹, R², R³, R⁴, R⁵ are as defined above for compounds of formula I and Pr stands for a free or protected (e.g. by esterification) hydroxy group. The reaction can be carried out under basic conditions in water or in an organic solvent.

C. Condensation of an aminoacid (XXII) with an aminoacid (XXIII) 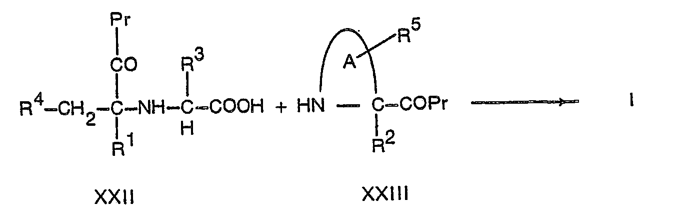 A, R¹, R², R³, R⁴, R⁵ are as defined above for compounds formula I and Pr stands for a free or protected (e.g. esterification) hydroxy group.

The reaction is well known from peptide chemistry. the reaction can be carried out in the presence of a condensing agent such as for example dicyclohexylcarbodiimide (DCC), diphenylphosphoryl azide (DPPA) and N,N-disuccinimidyl carbonate in CH₃CN. While, as mentioned above, reactive groupe (e.g. hydroxy groups) are protected before the coupling reaction is carried out, the amino group of compound (XXIII) can be activated, e.g. by means of tetraethyldiphosphit and/or the carboxy group of compound (XXII) can be activated via the intermediacy of active esters such as that derived from 1-hydroxybenzotriazole, its mixed anhydride (derived from a chlorocarbonic acid ester), its azide or dicyclohexylcarbodiimide.

This process is of particular use for the preparation of compounds wherein the R³, R⁴ or R⁵ contains or is Z', especially wherein R⁴ is or contains Z'.

D. Condensation of an amino compound (XXIV) with a keto-compound (XXV). 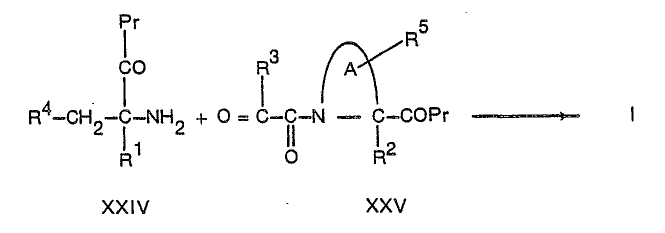 under the conditions described for process A. A, R¹, R², R³, R⁴, R⁵ are as defined above for compounds formula I and Pr stands for a free or protected (e.g. by esterification) hydroxy group.

E. Alkylation of an amino compound (XXIV) by means of a compound (XXVI) 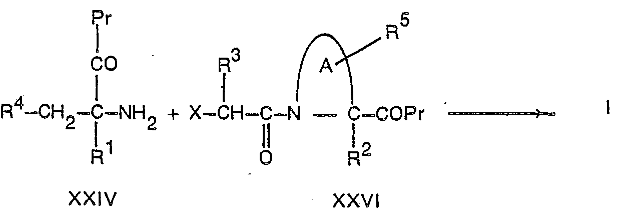 wherein X is chloro, bromo, iodo, alkanesulfonyloxy or arenesulfonyloxy, A, R¹, R², R⁴, R⁵ are as defined above for compounds of formula I, and Pr stands for a free or protected (e.g. by esterification) hydroxy group. The reaction can be carried out under the conditions described for process B.

F. For the preparation of compounds of formula I, wherein one of R³, R⁴ and R⁵ is a group Z―(CH₂)₀₋₆―, wherein Z is Z⁵, Z⁶, Z⁷, Z⁸, Z⁹ or Z¹⁰ preferably Z⁷, Z⁸ or Z⁹:
Condensation of a peptide of the general formula (XXX) with a compound containing the desired group (XXXI) 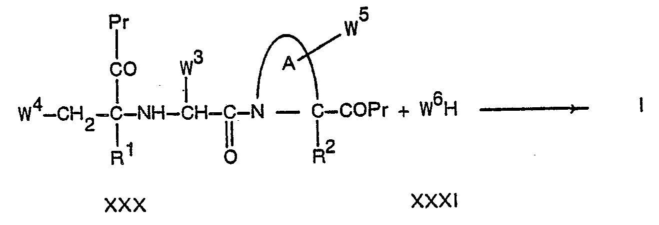 wherein R¹, R² and A are as defined for formula I, Pr is a protected hydroxy group, W³, W⁴ and W⁵ are defined like ^{R3,} R⁴ and R⁵ respectively with the difference that one of W³, W⁴ and W⁵ contains an NH₂-group instead of the respective Z⁵ to Z¹⁰-group; and W⁶ is Z⁵, Z⁶, Z⁷, Z⁸, Z⁹ or Z¹⁰. The reaction can be carried out in an inert organic solvent, e.g. an alcohol, (preferably ethanol) at reflux temperature.

This reaction may be exemplified by the following Reaction Scheme:

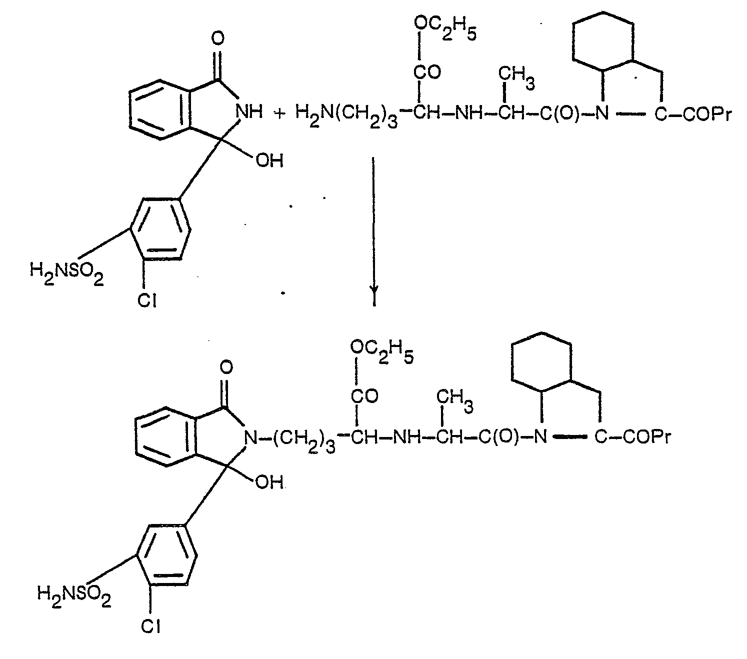

G. For the preparation of compounds of formula I wherein one of R³, R⁴ and R⁵ is a group Z-(CH₂)₀₋₆―, wherein Z is Z¹, Z² or Z³:
condensation of a peptide of formula XXXII with an appropriately substituted compound of formula XXXIII 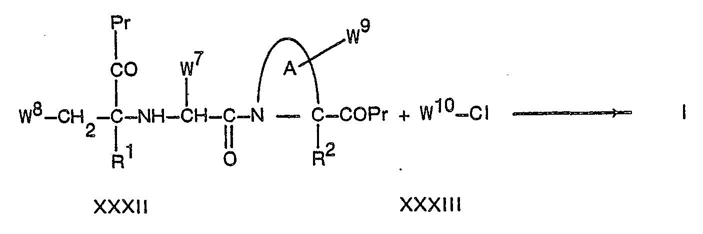 wherein R¹, R² and A are as defined for formula I, Pr is a protected hydroxy group, W⁷, W⁸ and W⁹ are defined like R³, R⁴ and R⁵ respectively, with the difference that one of W⁷, W⁸ and W⁹ contains an NH₂-group instead of the respective Z¹, Z² or Z³ group, and W¹⁰ is

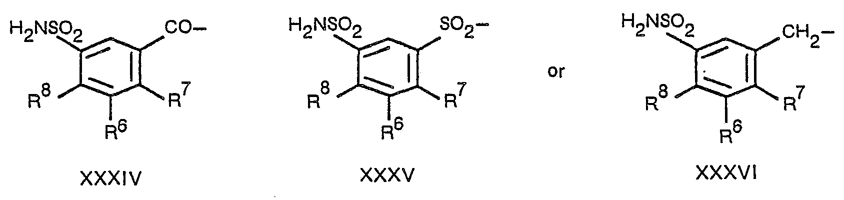

This condensation can be exemplified by the following Reaction Scheme: 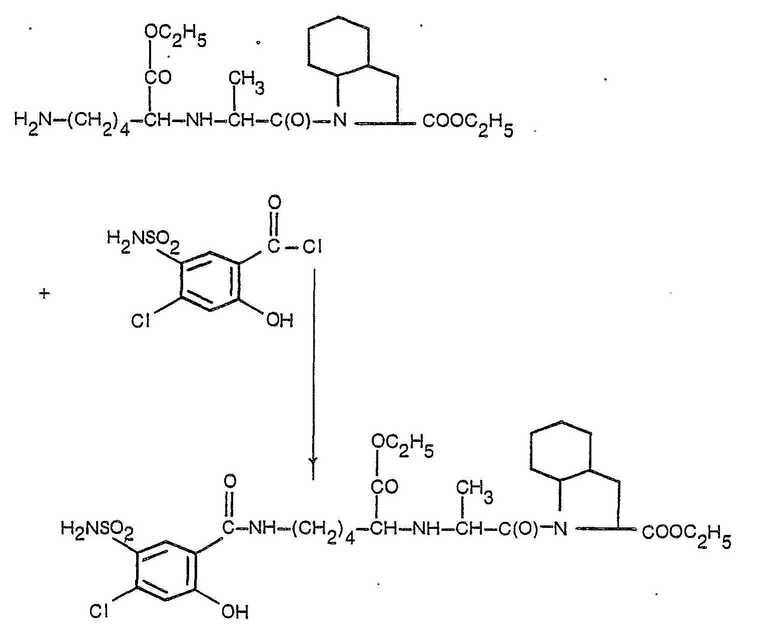 The reaction can be carried out in a suitable solvent (e.g. THE, pyridine or mixture of THF and triethylamine), usually between 0°C and room temperature.

H. For the preparation of compounds of formula I, wherein one of R³, R⁴ and R⁵ is a group Z―(CH₂)₀₋₆―, wherein Z is Z⁴: condensation of a peptide of formula (XXXVII) with a 3- halomethylbenzothiadiazine (XXXVIII) 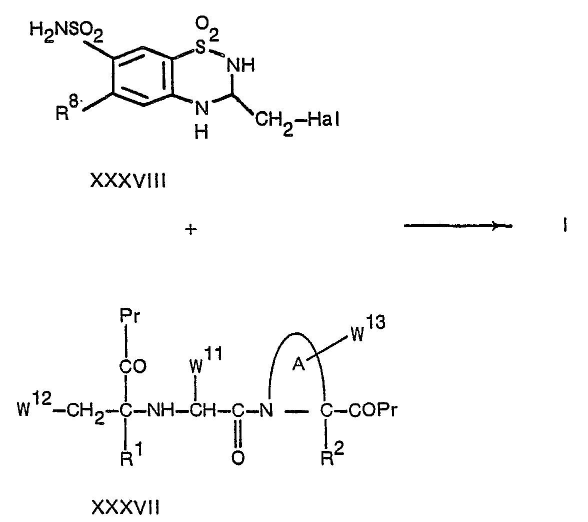 wherein R¹, R² and A are as defined for formula I, Pr is a protected hydroxy group, W¹¹, W¹² and W¹³ are defined like R³, R⁴ and R⁵ respectively with the difference that one of W¹¹, W¹² and W¹³ contains a ―SH- group instead of the respective Z⁴-group. and Hal is halogen, preferably chloro. The reaction is carried out in a suitable solvent (e.g. DMF), preferably in the presence of triethylamine.

I. For the preparation of compounds of formula 1, wherein one of R³, R⁴ and R⁵ is a group Z―(CH₂)₀₋₆―, wherein Z is Z⁵ or Z⁶: condensation of a peptide of formula XXXIX with a compound of formuls XXXX 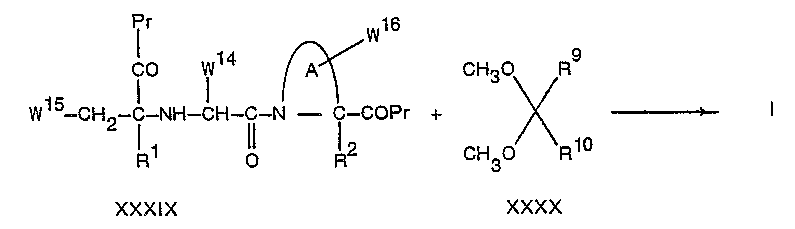 wherein R¹, R², R⁹, R¹⁰ and A are as defined for formula I, Pr is a protected hydroxy group, W¹⁴, W¹⁵ and W¹⁶ are defined like R³, R⁴ and R⁵ respectively with the difference that one of W¹⁴, W¹⁵ and W¹⁶ contains the group 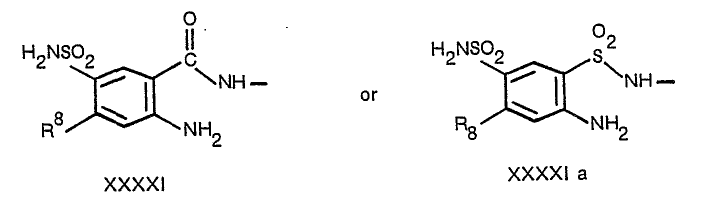 respectively .instead of the group Z⁵ or Z⁶ respectively. The reaction can be carried out in an inert organic solvent, e.g. an alcohol, preferably ethanol, under acidic conditions (e.g. by addition of a hydrochloric acid) at reflux temperature. The starting compounds in these reactions can be prepared according to known methods.

The compound of formula XXII, wherein R^{I} is hydrogen can for example be prepared by reacting a keto compound (XIX) with an aminoacid (XXVII) 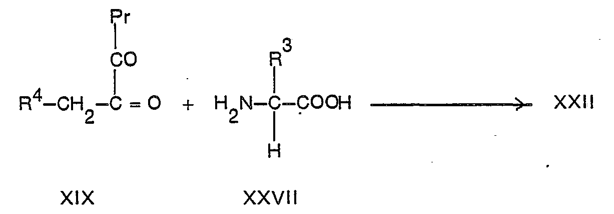 according to the condition described in process A.

Alternatively, the compound of formula XXII can be prepared by condensing XXIV with a keto acid (XXVIII) 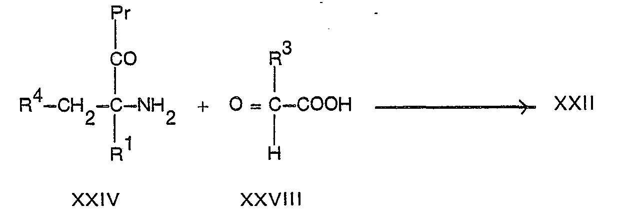 or by condensing 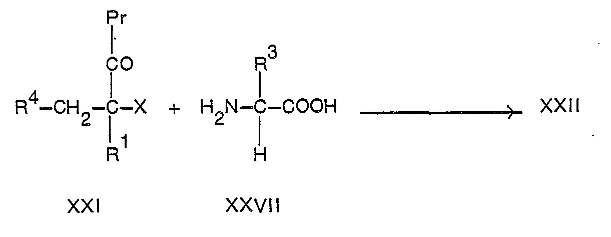 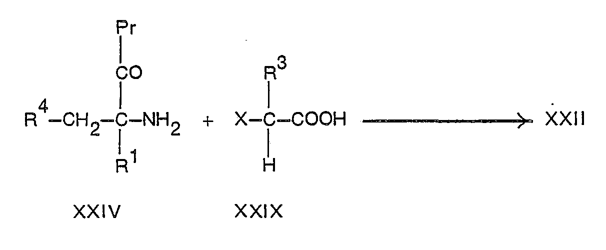 under the conditions described for process B (X being as defined in process B).

The compound XXII can also be prepared analogously to process G described above.

The starting compound (XXXVII) of process H can for example be prepared from a corresponding compound wherein the respective group W¹¹, W¹² or W¹³ is―SCHC₆H₅ by reduction with sodium in liquid ammonia.

The above processes are followed by setting free protected groups by known methods. Protected carboxy groups, e.g. when, for example, - protected by removable ester groups (e.g. Pr being alkoxy, (methoxy, ethoxy, tert. butyloxy), nitrobenzyloxy or bezyloxy,) are set free by hydrolysis or hydrogenation. (Reductive cleavage of a compound, wherein one of the protecting groups (Pr) is benzyloxy and the other protecting group is alkoxy will yield a compound, wherein the benzyloxy group has been replaced by hydroxy but the alkoxy group has not been replaced). Hydrolysis can be carried out under acidic conditions (using e.g. a halogen hydracid or trifluoroacetic acid), under basic conditions or by means of photochemical hydrolysis. The amino group(s) can be protected by protecting groups such as for example formyl, t-butoxycarbonyl, carbobenzyloxy, triphenylmethyl and nitrophenylsulfenyl. These groups can be removed under acidic conditions, e.g. by means of a halogenhydroacid and/or trifluoroacetic acid.

Esters obtained by the above processes can also be transesterified. For example, ethyl esters can be converted to the corresponding benzyl ester with benzyl alcohol under acidic conditions.

As mentioned before the compounds of this invention exist in diastereoisomeric forms or in mixtures thereof. The above described synthesis can utilize racemates, enantiomers - or diastereomers as starting mateials. Enantiomeric intermediates may be obtained by resolution methods known in the art. When diastereomeric products result from the synthetic procedures, the diastereomeric products can be separated by conventional chromatographic or franctional crystallization methods (e.g. described in the European published application No. 12401).

The compounds of this invention form salts with various inorganic or organic acids and bases which are also within the scope of the invention. Such salts include ammonium salts, alkali metal salts like sodium and potassium salts (which are preferred), alkaline earth metal salts like the calcium and magnesium salts, salts with organic bases e.g., dicyclohexylamine salts, N-methyl-D-glucamine, salts with amino acids like arginine, lysine and the like. Also, salts with organic and inorganic acids may be prepared, e.g., HCI, HBr, H₂SO₄, H₂PO₄, methanesulfonic acid, toluenesulfonic acid, maleic acid, fumaric acid and camphorsulfonic acid. The non-toxic physiological acceptable salts are preferred, although other salts are also useful, e.g., in isolating or purifying the product.

The salts may be formed by conventional means, as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

The compounds of this invention are useful as antihypertensive agents in mammals, including humans, in which the blood pressure has become abnormally elevated.

The compounds of the present invention can be combined with pharmaceutical carriers and administered in a variety of well known pharmaceitucal forms suitable for oral or parenteral administration to provide compositions useful in the treatment of cardiovascular disorders and particularly mammalian hypertension.

The effective dose (ED₅₀) 'of the compounds of this invention will typically be in the range of about 0.01 to about 30 mg/kg, preferable of about 0.1 to about 10 mg/kg, of mammalian weight, administered in single or divided doses. The exact dose to be administered is dependent upon where the particular compound lies within the above quoted range, as well as upon the age, weight and condition of the individual.

Generally, in treating humans, the compounds of this invention may be administered to patients in need of such treatment in a dosage range of 5 to 500 mg per patient generally given several times, thus giving a total daily dose of from 5 to 2000 mg per day.

The composition containing the compounds of this invention will preferably contain from about 5 to 250 mg of the active compound per dosage unit. These compositions. are most preferably administered orally. Typical formulations for oral administration are those such as tablets, capsules, syrups, elixirs or suspensions. Typical injectable formulations include solutions and suspensions.

The typical acceptable pharmaceutical carriers for use in the formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate, polyvinylpyrrolidone, polyvinyl alcohol; stearic acid; alkaline earth metal stearates such as magnesium stearate and calcium stearate, stearic acid, vegetable oils such as peanut oil, cottonseed oil, sesame oil, olive oil and corn oil; non-ionic, cationic and anionic surfactants; ethylene glycol polymers; beta-cyclodextrin; fatty alcohols and hydrolyzed cereal solids; as well as other non-toxic compatible fillers, binders, disintegrants, buffers, preservatives, antioxidants, lubricants, flavoring agents, and the like commonly used in pharmaceutical formlations.

The following examples illustrate the preparation of the compounds of the present invention. The diasteromers prepared as setforth below may be isolated by column chromatography or by fractional crystallization.

In the examples below, octahydroindole-2(S)-carboxylic acid refers to cis,syn-octahydroindole-2(S)-carboxylic acid, also named 3a(S), 7a(S)-octahydroindole-2(S)-carboxylic acid.

### Example 1

### 1{Nα-[1(S)-Ethoxycarbonyl-3-phenylpropyl-Nε-[(4-ohloro-3-sulfamoyl)benzenesulfonyl]-(S)-lysyl}-cis,syn-octahydro-1H-indole-2(S)-carboxylic acid

Stir a suspension of 24.0 g of Ne-benzoxycarbonyl-(S)-lysine and 36.0 g of ethyl 2-oxo-4-phenylbutanoate acid in 2500 ml of absolute ethanol at room temperature for 24 hours. Add 16.0 g of sodium cyanoborohydride and stir the resulting mixture at room temperature for 48 hours. Add 80 ml of water and stir the resulting mixture at room temperature for 72 hours. Concentrate this mixture in vacuo at 30°C to give a white residue. Suspend the residue in 1200 ml of ice water, add concentrated hydrochloric acid to maintain pH 2-4, and stir this mixture for 2 hours. Absorb this aqueous solution on 2000 ml of XAD-2 (Rohm & Haas Co.) resin. Elute the resin with 16,000 ml of water and then with 8000 ml of absolute ethanol. Concentrate the ethanol solution and chromatograph the residue on a column of silica gel (3000 ml, 60-200 mesh) eluting with chloroform:isopropanol: 7% ammonium hydroxide 1:1:1 (organic layer) to give a white residue. Chromatograph this residue on a column of silica gel (3000 ml), eluting with chloroform:isopropanol: 7% ammonium hydroxide 1:1:1 (organic layer) to give fractions A, B, C, and D. Absorb fraction B on a column of silica gel (1500 ml), eluting with chloroform:isopropanol: 7% ammonium hydroxide 1:1:1 (organic layer) to give N-benzyloxycarbonyl-Na-[1(S1-carboethoxy-3-(phenyl)propyl]-(S)-lysine, a white solid, [a]2⁶ + 6.1° (ethanol), m.p. 114-115°C.

B. Cool a solution of 1.9 g of the product of part A and 1.3 g of cis,syn-octahydro-1H-indole-2(S)-carboxylic acid benzyl ester in 24 ml of dimethylformamide to 0°C under nitrogen. Add dropwise a solution of 0.9 of diphenylphosphorylazide in 6 ml of dimethylformamide, followed by a solution of 0.7 ml of N-methylmorpholine in 6 ml of dimethylformamide, also added dropwise, and stir at room temperature for 18 hours. Pour the reaction solution into water, adjust to pH 8 with 1N NaOH, and extract with ether. Dry the ether layer over magnesium sulfate, and concentrate under vacuum to a yellow oil. Chromatograph the oil on silica gel (1000 ml, 60-200 mesh), eluting with hexane:ethyl acetate (1:2) to give 1-{Na-1(S)-carboethoxy-3-(phenyl)propyl-Nε-benzyloxy-ca rbonyl-(S)-lysyl}-cis,syn-octahydro-1H-indole-2(S)-carboxylic acid, benzyl ester, a yellow oil.

C. Dissolve 1.60 g of the product of part B in 150 ml of absolute ethanol. Add 0.75 g of 10% palladium-on-charcoal and hydrogenate the mixture at 50 psi at room temperature. Filter the reaction mixture and concentrate the filtrate in vacuo to give 1-{Nα-[1(S)-ethoxycarbonyl-3-(phenyl)propyl-(S)-lysyl}-cis,syn-octahydro-1H-indole-(S)-carboxylic acid hydrate, a white foam, 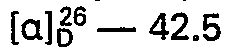 (ethanol).

D. To 4.9 g of 1-{Na-[1(S)-[ethoxycarbonyl-3-phenylpropyl]-(S)-lysyl}-cis,syn-octahydro-1H-indole-2(S)-carboxylic acid in 200 ml of tetrahydrofuran and 2 g of triethylamine at 0―5°C, add 2.9 g of 4-chloro-3-sulfamoylbenzenesulfonyl chloride and stir the resulting mixture at room temperature. Concentrate the resulting mixture in vacuo and chromatograph the residue on an Lobar RP-8, size B column (E. Merck) using acetonitrile: water as eluant to give the title compound.

### Example 2

### 1-{Nα-[1(S)-Ethoxycarbonyl-3-phenylpropyl]-Nε-[(4-chloro-3-sulfarnoyl)benzoyl]-(S)-lysyl}-cis-syn-octahydro-1H-indole-2(S)-carboxylic acid

Treat 4.9 g of 1-{NαR1(S)-ethoxycarbonyl-3-phenylpropyl-(S)-lysyl}-cis,syn-octahydro-1H-indole-2(S)-carboxylic acid (obtainable as described in Example 1A to 1C) in 200 ml of tetrahydrofuran and 2.0 g of triethylamine at 0-5°C with 2.2 g of 4-chloro-3-sulfamoyl-benzoyl chloride and stir the resulting mixture at room temperature. Concentrate the resulting mixture in vacuo and chromatograph the residue on a Labor RP-8, size B column (E. Merck) using acetonitrile: water as eluant to give the title compound.

### Example 3

### 1-{Nα[1(S)-Ethoxycarbonyl-3-phenylpropyl]-Nε-[(4-chloro-3-sufamoyl)benzenesulfonyl]-S)-lysyl}-(S)-proline

Substitute 2,17 g of 1-{Nα-[1(S)-ethoxycarbonyl-3-phenylpropyl]-(S)-lysyl}-(S)-proline for the respectively substituted octahydro-1H-indole-2(S)-carboxylic acid in Example 1D to obtain the title compound.

### Example 4

### 1-{Nα-[1(S)-Ethoxycarbonyl-3-phenylpropyl]-Nε-[(4-chloro-3-sulfamoyl)benzoyl]-(S)-lysyl}-(S)-proline

Substitute 2,17 g of 1-{Na-[1(S)-ethoxycarbonyl-3-phenylpropyl]-(S)-lysyl}-(S)-proline for the respectively substituted octahydro-lH-indole-2(S)-carboxylic acid in Example 2 to obtain the title compound.

### Example 5

### 1-{Nα-[1(S)-Carboxy-3-phenylpropyl)-Nε-[(4-chloro=3-sulfamoyl)benzenesulfonyl]-(S)-lysyl}-cis,syn-octahydro-1H-indole-2(S)-carboxylic acid

A. To a solution of 1.10 g of 1-{Na[1(S)-ethoxycarbonyl-3-phenylpropyl]-(S)-lysyl}-cis,syn-octahydro-1H-indole-2(S)-carboxylic acid (prepared as in Example 1) in 100 ml of methanol at 0-5°C, add 2.0 ml of 2.5 N sodium hydroxide solution and stir at room temperature for 24 hours. Add 20 ml of water, concentrate to one-half volume, and stir 24 hours. Concentrate this solution in vacuo and absorb on AG 50W-X2 (100-200 mesh, hydrogen form, Bio-Rad resin) (50 ml). Place said 50 ml of resin on an additional 300 ml of resin, elute the resin with 1200 ml of water, and then elute with 4% pyridine in water to yield 1- {Nα-[1(S)-carboxy-3-phenylpropyl]-(S)-lysyl}-cis,syn-octahydro-1H-indole-2(S)-carboxylic acid, a white solid, m.p. 165-166° [α]²⁶_{D}- 8-2 (ethanol).

B. Treat 2.45 g of the product of Step A with 1.45 g of 4-chloro-3-sulfamoylbenzenesulfonyl chloride as described in Example 1D to give the title compound.

### Example 6

### 1-{Nα-[1(S)-Carboxy-3-phenylpropyl]-Ns-((4-chloro-3-sulfamoyl)benzoyl-(S)-lysyl}-cis,syn-octahydro-1H-indole-2(S)-carboxylic acid

Treat 2.45 g of the product from Example 5A with 1.1 g of 4-chloro-3-sulfamoylbenzoyl chloride as described in Example 2 to give the title compound.

### Example 7

### 1-{N-[1 (S)-Carboxy-3-phenylpropyl]-S-(3-(6-chloro-3,4-dihydro-7-sulfamoyl-1,2,4-benzothiadiazinyl-1,1-dioxide)methyl]-(R)-cysteinyl}-cis,syn-octahydro-1H-indole-2(S)-carboxylic acid.

A. Stir 10.5 g of S-benzyl-L-cysteine and 11.0 g of 2-oxo-4-phenylbutyric acid, ethyl ester in 1000 ml of absolute ethanol at room temperature for 24 hours. Add. 5.28 g of sodium cyanoborohydride and stir the resulting mixture at room temperature for 48 hours. Concentrate this mixture in vacuo at 30°C to give a white residue. Suspend the residue in ice-water, add concentrated hydrochloric acid to maintain pH 2-4, and stir this mixture for 1 1/2-2 hours. Absorb this aqueous solution on XAD-2 (Rohm & Haas Co.) resin. Elute the resin with water and then with absolute ethanol. Concentrate the ethanol solution and chromatograph the residue on a column of silica gel using chloroform:isopropanol: 7% ammonium hydroxide 1:1:1 (organic layer) to give N-[1(S)-carboethoxy-3-phenylpropyl]-S-benzyl-(R)-cysteine.

B. Treat 4.0 g of the product of part A and 2.6 g of cis,syn-octahydro-1H indole-2(S)-carboxylic acid, benzyl ester in 10 ml of dimethylformamide at 0°C under nitrogen with a solution of 2.75 g of diphenylphosporylazide in 1.0 g of N-methylmorpholine in 10 ml of dimethylformamide, and stir at room temperature for 18 hours. Pour the reaction solution into water, adjust to pH 8 with 1 N, NaOH, and extract with ether. Wash the combined ether layers with aqueous sodium chloride solution, dry the ether layer over magnesium sulfate, filter, and concentrate in vacuo to give a residue. Chromatograph this residue on silica gel (60-200 mesh) using hexane:ethylacetate to give 1-{N-[1(S)-ethoxycarbonyl-3-phenylpropyl]-S-benzyl-(R)-cysteinyl}-cis,syn-octahydro-1H-indole-2-(S)-carboxylic acid, benzyl ester.

C. Stir the product of part B in 50 ml of a 15-20% solution of hydrobromic in acetic acid under nitrogen for 2 hours, then concentrate to dryness under vacuum at room temperature. Triturate the resultant residue with ether to obtain 1-{N-[1(S)-(ethoxycarbonyt-3-phenytpropyt]-S-benzyt-(R)-cysteinyt}-cis,syn-octahydro-1H-indole-2(S)-carboxylic acid, hydrobromide.

D. React 1.5 g of the product from part C in methanol with 3.0 ml of 2.5 N sodium hydroxide at room temperature for 24 hours and concentrate the resulting mixture in vacuo at room temperature. Absorb the residue on AG 50W-X2 (100-200 mesh, hydrogen form, Bio-Rad) resin. Elute the resin with water and then elute with 4% pyridine in water to yield 1-{N-[1(S)-carboxy-3-phenyipropyt]-S-benzy)-(R)-cysteinyl}-cis,syn-octahydro-lH-indole-2(S)-carboxylic acid, hydrobromide.

E. Treat 1.0 g of product form part D with 0.05 g of sodium in 100 ml of liquid ammonia. Evaporate and concentrate the resulting mixture to give 1-{N-1 (S)-carboxy-3-phenylpropyl-(R)-cysteinyl}-cis,syn-octahydro-lH-indole-2(S)-carboxylic acid as the sodium salt.

F. React 0.4 g of the product from Step E in 20 ml of dimethylformamide with 0.36 g of 2-bromomethyl-6-chloro-3,4-dihydro-7-sulfamoyl-1,2,4-benzothiadiazine-1,1-dioxide and triethylamine. Concentrate the resulting mixture and chromatograph on an AG 50W-X2 column eluting with 4% pyridine in water to give the title compound.

### Example 8

### 1-{N-[(R)-Carboxy-2-[S-( (3-(6-chloro-3,4-dihydro-7-sulfamoyl-1,2,4-benzothiadiazinyl-1,1-dioxide)methyl))thio]-ethyl]]-(S)-alanyl}-cis,syn-octahydro-1H-indole-2(S)-carboxylic acid

A. Stir 100 g of S-benzyl-L-cysteine ethyl ester hydrochloride, 132 g of benzyl pyruvate, and 10 g of 3 A molecular sieves in 8 liters of ethanol for 18 hours under nitrogen. Add dropwise a solution of 52 g of sodium cyanoborohydride in 100 ml of ethanol, stir at room temperature for 24 hours, filter, then concentrate the filtrate at room temperature under vacuum. Suspend the resultant residue in 100 ml of water and 500 ml of ether and adjust the mixture to pH 8 with 1 N HCI. Wash the organic layer with saturated sodium chloride solution, dry over sodium sulfate, and filter. Adjust the filtrate to pH 2 with 3 M ethereal HCI, decant the supernatant, wash the resulting oily precipitate with 200 ml of ether, and mix with saturated aqueous sodium bicarbonate to obtain a solution of pH 8. Extract the mixture with 1 liter of ether, dry the ether layer over sodium sulfate and concentrate at room temperature to give N-[1 (R)-carboethoxy-2-(benzylthio)ethyl]-(R,S)-alanine, benzyl ester, an amber oil. Thin layer chromatography in ethyl acetate: hexane (15:85) may be used to separate the two isomers (isomer A at Rf = 0.36, and isomer B at Rf = 0.28), or the procedure may be continued on the mixture.

B. Add 50 g of the product of part A to 1800 ml of a 15-20% solution of hydrobromic-acetic acid and heat at 50°C for 20 hours. Concentrate the resultant mixture to dryness under vacuum, and wash the resultant oily residue with ether until free of acetic acid to produce N-[1(R)-carboxyethyt-2(benzylthio)ethyl]-(R,S)-alanine hydrobromide, an amber oil.

C. Cool a solution of 50.5 g of the product of part B and 33.4 g of cis,syn-octahydroindole-2(S)-carboxylic acid benzyl ester in 1 liter of dimethylformamide to 0°C under nitrogen, add dropwise a solution of 35.5 g of diphenylphosphorylazide in 1 liter of dimethylformamide, followed by a solution of 33.4 g of N-methylmorpholine in 200 ml of dimethylformamide, also added dropwise, and stir at room temperature for 18 hours. Pour the reaction solution into 3 liters of water, adjust to pH 8 with 1 N NaOH, and extract with 4 x 1 liter of ether. Wash the combined ether layers with 1 liter of aqueous sodium chloride, dry the ether layer over magnesium sulfate, filter, and concentrate under vacuum to an amber oil.

Chromatograph the resultant oil on 2 kg of silica gel (60-200 mesh) using ether:hexane (90:10). Collect components having Rf 0.38 and Rf 0.61 as indicated by thin layer chromatography on silica gel eluted with ether. The isomer with Rf 0.61 is 1-{N-[1(R)-carboethoxy-2-(benzylthio)ethyl]-(S)-alanyl}- cis,syn-octahydro-1H-indole-2(S)-carboxylic acid, benzyl ester.

D. Stir 0.70 g of the (S)-alanyl product of part C and 25 ml of a 15-20% solution of hydrobromic-acetic acid under nitrogen for 2 hours, then concentrate to dryness under vacuum at room temperature. Triturate the resultant residue with ether and filter to obtain 1-{N-[1 (R)-carboethoxy-2-(benzylthio)-ethyl]-(S)-alanyl}- cis,syn-octahydro-lH-indole-2(S)-carboxyllc acid, hydrobromide as a tan solid, m.p. 124―125°C.

E. To a solution of 10.6 g of the product of Step D in 500ml of methanol, add 24 ml of 2.5 N sodium hydroxide solution and stir at room temperature for 24 hours. Concentrate this solution in vacuo and absorb on AG 50W-2 (Bio-Rad) resin (100-200 mesh, hydrogen form). Elute the resin with water and then elute with 4% pyridine in water to yield 1-{N-[1(R)-carboxy-2-(benzylthio)ethyl]-(S)-alanyl}-cis,syn-octahydro-lH-indole-2(S)-carboxylic acid.

F. Treat 4.22 g of the product of Step E with 0.23 g of sodium in 200 ml of liquid ammonia. Evaporate the ammonia and absorb the residue on AG 50W-2 (Bio-Rad) resin (100-200 mesh, hydrogen form). Elute the resin with water and then elute with 4% pyridine in water to yield 1-{N-[1(R)-carboxy-2-(mercapto)ethyl]-(S)-alanyl}-cis,syn-octahydro-1H-indole-2(S)-carboxylic acid.

G. React 2.2 g of the product of Step F in 20 ml of dimethylformamide with 2.4 g of 3-bromomethyl-6-chloro-3,4-dihydro-7-sulamoyl-1,2,4-benzothiadiazine-1,1-dioxide and triethylamine. Concentrate the resulting mixture to give the title compound.

### Example 9

### 1-{N-[1(S)-Ethoxycarbonyl-5-(4-chloro-3-sulfamoyl)-benzenesulfonaminopentyl]-(S)-alanyl}-cis,syn-octahydro-1 H-indole-2(S)-carboxylic acid

A. Dissolve 27.0 g of ethyl indole-2-carboxylate in 250 ml of trifluoroacetic acid. Add 2.05 g of platinium oxide, hydrogenate the mixture at 50 Ib/in² at room temperature. Filter the mixture and concentrate the filtrate in vacuo to give a residue. Suspend the residue in ether and treat with cold dilute sodium hydroxide solution. Dry the organic layer over magnesium sulfate and concentrate it to give ethyl octahydroindole-2-carboxylate, a pale yellow oil.

B. Dissolve 116 g of 10-d-camphorsulfonic acid in 1 liter of warm ethyl acetate and add a solution of 86 g of the product of part A in 1 liter of ethyl acetate. Allow the mixture to crystallize, heat to reflux, cool to room temperature, and filter. Recrystallize the filter cake from a mixture of 500 ml of isopropanol and 1800 ml ethyl acetate, filter and dry the crystals to obtain 2-(S)-carboethoxy-cis,syn-octahydro-1H-indole, d-10-camphorsulfonate, m.p. 192-193°C.

C. Slurry 10 g of the product of part B in 1 liter of ether, adjust to pH 11 with aqueous sodium hydroxide, and stir for 5 minutes. Wash the organic layer with sodium chloride solution, dry over magnesium sulfate, filter, and evaporate in vacuo at room temperature to obtain 2(S)-carboethoxy-cissyn- octahydro-1H-indole as a colorless oil. Dissolve the resultant oil in 50 ml of methynol containing 23 ml of ' N sodium hydroxide, stir at 25°C for 30 minutes, adjust to pH 7 with 1 N hydrochloric acid, and evaporate the solvent to give cis,syn-octahydro-1H indole-2(S)-carboxylic acid.

D. Cool 23 ml of benzyl alcohol to 0°C under nitrogen and add 5.95 g of thionyl chloride dropwise over 15 minutes, maintaining the temperature at 0°C. Add the product of part C, stir for 1 hour at 0°C, then stir for 24 hours at room temperature. Pour the resulting mixture into 500 ml of ether, stir 1 hour under nitrogen, then allow to stand under nitrogen until the solution is clear. Decant the supernatant, wash the precipitate with 25 ml ether, then slurry the precipitate in 200 ml ether and adjust to pH 8-9 with 1 N sodium hydroxide. Stir 5 minutes, wash the organic layer with sodium chloride solution, dry over magnesium sulfate, filter and evaporate in vacuo at room temperature to obtain cis,syn-octahydroindole-2(S)-carboxylic acid, benzyl ester as a colorless oil (TLC in ether: one spot, Rf 0.3).

E. To. 26 g of the product of Step D in 100 ml of dichloromethane and 7.8 ml of pyridine add 11.0 g of pyruvoyl chloride and stir the resulting mixture at room temperature. Extract the reaction mixture with water and dry the organic layer over magnesium sulfate. Concentrate the dichloromethane solution in vacuo and distill the residue to give 1-pyruvoyl-cis,syn-octahydro-1H-indole-2(S)-carboxylic acid, benzyl ester.

F. To 20 g of the product from Step E in 400 ml of ethanol, add 2.0 g of 10% palladium-on-charcoal and hydrogenate at 50 psi at room temperature. Filter the resulting mixture and concentrate the filtrate in vacuo to give 1-pyruvoyl-cis,syn-octahydro-1H-indole-2(S)carboxylic acid.

G. React 6.20 g of Ne-(benzyloxycarbonyl)-L-lysine ethyl ester i 20 ml of tetrahydrofuran with 4.8 g of 1- pyruvoyl-cis,syn-octahydro-1H-indole-2(S)-carboxylic acid and add 20 ml of molecular sieves 4A (Rohm and Haas). Stir the resulting mixture for 4 hours, add 12 g of sodium cyanoborohydride in 20 ml of methanol and stir the reaction mixture 20 hours. Filter, concentrate to dryness, and partition the residue between water and dichloromethane. Absorb the aqueous phase on strong acidic ion-exchange resin and elute with 4% pyridine in water to give 1-{N-[1(S)-ethoxycarbonyl-5-benzyloxycarbonylaminopentyl]-(R,S)-alanyl}-cis,syn-octahydro-1H-indole-2(S)-carboxylic acid. Separate the isomers on a column of silica gel using CHCI₃: isopropanol: 7% ammonium hydroxide 1:1:1 (organic) as eluant to give 1-N-[1(S)-ethoxycarbonyl-5-benzyloxycarbonylaminopentyl]-cis,syn-octahydro-1H indole-2(S)-carboxylic acid.

H. Hydrogenate the product from Step G in 300 ml of ethanol using 1 g of 10% palladium-on-charcoal at 50 psi at room temperature. Filter the mixture and concentrate the filtrate to give 1-{N-[1(S)-ethoxycaronyl-5-aminopentyl]-(S)-alanyl}-cis,syn-octahydro-1H indole-2(S)-carboxylic acid.

I. React 1.01 g of the product of Step H in 20 ml of tetrahydrofuran and 0.25 g of triethylamine with 0.75 g of 4-chloro-3-sulfamoylbenzensulfonyl chloride and stir the resulting mixture at room temperature. Concentrate the resulting mixture in vacuo and chromatograph the residue on a Lobar RP-8 (E. Merck) size B column using acetonitrile: water as eluant to give the title compound.

### Example 10

### 1-{N-[1(S)-Ethoxycarbonyl-5-(4-chioro-3-sulfamoyl-benzamido-pentyl]-(S)-alanyl}-cis,syn-octahydro-1H-indole-2(S)-carboxylic acid. -

Treat 1.01 g of 1-{N-[1(S)-ethoxycarbonyl-5-aminopentyl]-(S1-alanyl}-cis,syn-octahydro-1H-indole-2(S)-carboxylic acid, obtained according to Example 9H, in 20 ml of tetrahydrofuran and 0.25 g of triethylamine with 0.55 g of 4-chloro-3-sulfamoylbenzoyl chloride and stir the resulting mixture at room temperture. Concentrate the resulting mixture in vacuo and chromatograph the residue on a Lobar RP-8 (E. Merck) size B column using acetonitrile: water as eluant to give the title compound.

### Example 11

### 1-{N-[ (S)-Carboxy-5-(4-chloro-3-sulfamoyl)benzamidopentyl]-(S)-alanyl}-cis,syn-octahydro-1H-indole-2(S)-carboxylic acid

A. To 4.04 g of 1-{N-[1(S)-ethoxycarbonyl-5-aminopentyl]-(S)-alanyl}-cis,syn-octahydro-1H-indole-2(S)-carboxylic acid in 100 ml of methanol: water 1:1 add 8 ml of 2.5 N NaOH at 0-5°C and then stir the resulting mixture at room temperature for 24 hours. Concentrate the resulting mixture and absorb on AG 50W-2 (Bio-Rad) resin (100-200 mesh, hydrogen form). Elute the resin with water, and then elute with 4% pyridine in water to yield 1-{N-1(S)-carboxy-5-aminopentyl]-(S)-alanyl}-cis,syn-octahydro-1H indole-2(S)-carboxylic acid.

B. React 0.95 g of the product from Step 11A with 0.55 g of 4-chloro-3-sulfamoylbenzoyl chloride as described in Example 10 to give the title compound.

### Example 12

### 1-{N-[1(S)-Carboxy-5-(4-chloro-3-sulfamoyl)benzenesulfamidopentyl]-(S)-alanyl}-cis,syn-octahydro-1H-indole-2(S)-carboxylic acid

Treat 0.95 g of the product from Example 11A with 0.75 g of 4-chloro-3-sulfamoylbenzenesulfonyl chloride as described in Example 91 to give the title compound.

### Example 13

### 1-{N-[1(S)-Ethoxycarbonyl-5-(4-chloro-3-sulfamoyl)-benzenesulfonamidopentyl-(R,S)-alanyl}-(S)-proline

A. Make a solution of Ne-benzyloxycarbonyl-L-lysine ethyl ester hydrochloride (2.94 g) in water (10 ml) basic with 15 ml of saturated aqueous potassium bicarbonate and extract with CH₂CI₂. Dry the extract over MgS0₄ and concentrate to dryness. Dissolve the residue, Ns-benzyloxycarbonyl-L-lysine ethyl ester, in tetrahydrofuran (20 ml) and pyruvoylproline (555 mg) and add powdered No. 4A molecular sieves (1.0 g). Stir the mixture at room temperature for 4 hours. Add sodium cyanoborohydride (630 mg) in 1 ml of methanol over 2 hours and stir the mixture overnight. Filter the mixture, concentrate to dryness, and partition the residue between water (10 ml) and CH₂Cl₂ (15 mi). Absorb the aqueous phase on strong acid ion-exchange resin and elute with 4% pyridine in water to yield 470 mg of 1-{N-[1 (S)-ethoxycarbonyl-5-benzyloxycarbonylaminopentyl]-(R,S)-alanyl}-S-proline. Remove the protecting group by hydrogenation in ethanol: water 1:1 over 10% Pd/C catalyst at 40 psi. Filter the mxiture and take the filtrate to dryness. Chromatograph the residue in methanol on an LH-20 column to give the desired 1-{N-[1(S)-ethoxycarbonyl-5-aminopentyl]-(R,S)-alanyl)-(S)-proline.

B. Condense 0.90 g of the product from Step A with 0.75 g of 4-chloro-3-sulfamoylbenzenesulfonyl chloride as described in Example 91 to give the title compound.

### Example 14

### 1-{N-[1(S)-Ethoxycarbonyl-5-(4-chloro-3-sulfamoyl)-benzamidopentyl]-(R,S)-alanyl}-(S)-proline

React 0.90 g of the product of Example 13A with 0.55 g of 4-chloro-3-sulfamoylbenzoylchloride as described in Example 10 to give the title compound.

### Example 15

### 1-{N-[1(S)-Carboxy-5-(4-chforo-3-sulfamoyl)benzenesulfonamidopentyl]-R,S)-alanyl}-(S)-proline

A. Treat 3.50 g of the product of Example 13A in 100 ml of methanol: water 1:1 with 8.0 ml of 2.5 N NaOH as described in Example 11A to give 1-{N-[1(S)-carboxy-5-aminopentyl]-(R,S)-alanyl}-(S)-proline.

B. Condense 0.80 g of the product of Step A with 0.75 g of 4-chloro-3-sulfamoylbenzenesulfonyl chloride as described in Example 91 to give the title compound.

### Example 16

### 1-{N-[(S)-Carboxy-5-(4-chloro-3-sulfamoyl)benzamidopentyl]-(R,S)-alanyl}-(S)-proline

React 0.80 g of the product of Example 15A with 0.55 g of 4-chloro-3-sulfamoylbenzoyl chloride as described in Example 10 to give the title compound.

### Example 17

### 7-(4-Chloro-3-sulfamoylbenzamido)-2-{N-[1 (S)-carboxy-3-phenylpropyl]-(S)-alanyl}-1,2,3,4-tetrahydroisoquinoline-3(S)-carboxylic acid

A. Dissolve 1,2,3,4-tetrahydro-7-nitroisoquinoline-3(S)-carboxylic acid ethyl ester (0.1 mole) in ethanol and add the solution of 10% palladium on carbon (1.0 g) in a hydrogenation bottle. Hydrogenate the mixture at 30 psi, at room temperature until the reduction is complete as indicated by thin layer chromatography. Remove the catalyst by filtration and evaporate the solvent under reduced pressure to obtain 7-amino-1,2,3,4-tetrahydroisoquinoline-3-(S)-carboxylic acid ethyl ester.

(The preparation of 1,2,3,4-tetrahydro-7-nitroisoquinoline-3(S)-carboxylic acid is described in US patent 4,064,274).

B. Treat 7-amino-1,2,3,4-tetrahydroisoquinoline-3(S)-carboxylic acid ethyl ester (Step A) (0.1 mole) with benzyl alcohol (0.5 mole) and p-toluenesulfonic acid (0.22 mole) in toluene at reflux overnight. Evaporate the solvent under reduced pressure to obtain the p-toluenesulfonic acid salt of the product. Add this salt to aqueous sodium bicarbonate solution with stirring. Extract the mixture with chloroform, dry the extract with magnesium sulfate and evaporate the solvent under reduced pressure to obtain 7-amino-1,2,3,4-tetrahydroisoquinoline-3(S)-carboxylic acid benzyl ester.

C. Add a solution of 4-chloro-3-sulfamoylbenzoyl chloride (0.1 mole) in tetrahydrofuran to a solution of product of Step B in tetrahydrofuran containing triethylamine (0.1 mole). When the reaction is complete as indicated by thin layer chromatography, remove the triethylamine hydrochloride by filtration and evaporate the solvent at reduced pressure. Purify the residue by chromatrography to obtain 7-(4-chioro-3-sulfamoylbenzamido)-1,2,3,4-tetrahydroisoquinoline-3(S)-carboxylic acid benzyl ester.

D. Cool a solution of N-[1 (S)-carboethoxy-3-phenylpropyl]-(S)-alanine (0.01 mole) and the benzyl ester (0.01 mole) from Step C in dry dimethylformamide to 0°C. Add N-methylmorpholine (0.01 mole) with stirring, then add dropwise, with stirring, a solution of diphenylphosphoryl azide (0.01 mole) in dry dimethylformamide while maintaining the temperature at 0°C. Stir the reaction for one hour at 0°C and overnight at room temperature. Dilute the mixture with ethyl acetate and wash with aqueous sodium bicarbonate. Dry the organic solution with magnesium sulfate and evaporate the solvent under reduced pressure. Purify the residue by chromatography to give 7-(4-chloro-3-sulfamoylbenzamido)-2-{N-[1(S)-carboethoxy-3-phenylpropyl]-(S)-alanyl}-1,2,3,4-tetrahydroisoquinoline-3-(S)-carboxylic acid benzyl ester.

E. Add a solution of 0.01 mole of the benzyl ester from Step D in ethanol to 10% palladium on charcoal (0.5 g) in a hydrogenation bottle. Hydrogenate the mixture at 60 psi, at room temperature until removal of benzyl group is complete as indicated by thin layer chromatography. Remove the catalyst by filtration and evaporate the solvent under reduced pressure to obtain 7-(4-chloro-3-sulfamoylbenzamido)-2-{N-[1(S)-carboethoxy-3-phenylpropyl]-(S)-alanyl}-1,2,3,4-tetrahydroisoquinoline-3(S)-carboxylic acid.

F. Stir a solution of 0.01 mole of the product from Step E in water containing sodium hydroxide (0.022 mole) at room temperature until the reaction is complete as indicated by thin layer chromatography. Add methanol to the reaction and then add 0.022 equivalents of Dowex-50(H+) with stirring. Remove the resin by filtration and evaporate the solvent under reduced pressure. Purify the product by chromatography to obtain the title compound.

### Example 18

### 7-(4-Chloro-3-sulfamoylbenzenesulfonamido)-2-{N-[1 (S)-carboxy-3-phenylpropyl]-(S)-alanyl}-1,2,3,4-tetrahydroisoquinoline-3(S)-carboxylic acid

A. Follow the procedure of Example 17C using 4-chloro-3-sulfamoylbenzenesulfonyl chloride in place of 4-chloro-3-sulfamoylbenzoyl chloride to obtain 7-(4-chloro-3-sulfamoylbenzoyl chloride to obtain 7-(4-chloro-3-sulfamoylbenzenesulfoamido)-1,2,3,4-tetrahydroisoquinoline-3(S)-carboxylic acid benzyl ester.

B. Couple the product from Step A with N-[1 (S)-carboethoxy-3-phenylpropyl]-(S)-alanine following the procedure of Example 17D. Chromatograph the crude product to obtain 7-(4-chloro-3-sulfamoylbenzenesulfonamido)-2-{N-[1 (S)-carboethoxy-3-phenylpropyl]-(S)-alanyl}-1,2,3,4-tetrahydroisoquinoline-3(S)-carboxylic acid benzyl ester.

C. Subject the benzyl ester from Step B to hydrogenolysis as described in Example 17E to obtain 7-(4-chloro-3-sulfamoylbenzenesulfonamido)-2-{N-[1 (S)-carboethoxy-3-phenylpropyl]-(S)-alanyl}-1,2,3,4-tetrahydroisoquinoline-3(S)-carboxylic acid.

D. Treat the product from Step C with aqueous sodium hydroxide followed by Dowex-50(H+) as described in Example 17F to obtain the title compound.

### Example 19

### 5-(4-Chloro-3-sulfamoylbenzamido)-1-{N-[1(S)-carboxy-2-phenylpropyl]-(S)-alanyl}-octahydro-1H-indole-2-carboxylic acid

A. Following the procedure of Example 17A, substitute 5-nitroindole-2-carboxylic acid ethyl ester for 1,2,3,4-tetrahydro-7-nitroisoquinoline-3(S)-carboxylic acid ethyl ester to obtain 5-aminoindole-2-carboxylic acid ethyl ester.

B. Dissolve 5-aminoindole-2-carboxylic acid ethyl ester in trifluoroacetic acid containing Pt0₂. Hydrogenate at 60 psi on a Parr shaker for 24 hours. Distill the trifluoroacetic acid at reduced pressure and dissolve the residue in ethyl acetate. Filter and adjust to pH 9 with 1 N NaOH. Dry the organic layer over MgS0₄ and distill the solvent at reduced pressure to obtain 5-aminooctahydro-1H-indole-2-carboxylic acid ethyl ester.

C. Following the procedure of Example 17B, substitute 5-aminooctahydro-1H-indole-2-carboxylic acid ethyl ester for 7-amino-1,2,3,4-tetrahydroisoquinoline-3(S)-carboxylic acid ethyl ester to obtain 5-aminooctahydro-1H-indole-2-carboxylic acid benzyl ester.

Analogously following the procedures of Examples 17C, 17D 17E and 17F obtain 5-(4-chloro-3-sulfamoylbenzamido)octahydro-lH-indole-2-carboxylic acid benzyl ester, 5 - (4 - chloro - 3 - sulfamoylbenzamido) - 1 - {N - [1 (S) - carboethoxy - 3 - phenylpropyl] - (S) - alanyl} - octahydro - -1H- indole - 2 - carboxylic acid benzyl ester, 5 - (4 - chloro - 3 - sulfamoylbenzamido) - 1 - {N - [1(S) - carboethoxy - 3 - phenylpropyl] - (S) - alanyl} - octahydro - 1H - indole - 2 - carboxylic acid and the title compound.

### Example 20

### 5-(4-Chloro-3-sulfamoylbenzenesulfonamido)-1-{N-[1 (S)-carboxy-3-phenylpropyl]-(S)-alanyl}-octahydro-1H-indole-2-carboxylic acid

A. Following the procedures of Example 18A, substitute 5-aminooctahydro-lH-indole-2-carboxylic acid benzyl ester for 7-amino-1,2,3,4-tetrahydroisoquinoline-3(S)-carboxylic acid benzyl ester and 4-chloro-3-sulfamoylbenzenesulfonyl chloride for 4-chloro-2-sulfamoylbenzoyl chloride to obtain 5-(4-chloro-3-sulfamoylbenzenesulfonamido)-octahydro-lH-indole-2-carboxylic acid benzyl ester.

B. Following the procedures of Examples 17D, 17E and 17F obtain 5 - (4 - chloro - 3 - sulfamoylbenzenesulfonamido) - 1 - {N - [1 (S) - carboethoxy - 3 - phenylpropyl] - (S) - alanyl} - octahydro - 1H- indole - 2 - carboxylic acid benzyl ester, 5 - (4 - chloro - 3 - sulfamoylbenzenesulfonamido) -1 - {N - [1 (S) - carboethoxy - 3 - phenylpropyl] - (S) - alanyl} - octahydro - 1H - indole - 3 - carboxylic acid and the title compound.

### Example 21

### 1-{N-[1(S)-Ethoxycarbonyl-5-(4-chloro-3-sulfamoyl-benzamido)pentyl]-(S)-alanyl}-cis,syn-octahydro-1H-indole-2(S)-carboxylic acid hydrochloride

A. N-[1(S)-ethoxycarbonyl-5-(benzyloxycarbonylamino) pentyl]-(S(-alanine.

Dissolve 17 g of N-[1(S)-ethoxycarbonyl-5-(benzyloxy-carbonylamino)pentyl]-(S)--alanine, t-butyl ester in 150 ml of trifluoroacetic acid at 5°C. Stir at room temperature for 5 hr. and then concentrate at room temperature in vacuo. Triturate the residue with ether and dry under vacuum to give the title compound.

B. 1 - {N - [1(S) - ethoxycarbonyl - 5 - (benzyloxycarbonylamino) - pentyl] - (S) - alanyl} - cis,syn - octahydro - 1H - indole - 2(S) - carboxylic acid benzyl ester.

In 5 ml of DMF, dissolve 0.7 g of N-[1 (S)-ethoxycarbonyl-5-(benzyloxycarbonylamino)pentyl]-(S)-alanine, 0.325 g of cis,syn-octahydro-1H-indole-2(S)-carboxylic acid benzyl ester, 0.24 g of 1-(3-dimethylaminopropyl)-3-ethyl-carbodiimide hydrochloride, and 0.20 g of 1-hydroxybenzotriazole. Stir this solution under nitrogen for 18 hr. and then concentrate at room temperature in vacuo. Partition between ether and water. Dry the organic layer (MgS0₄) and concentrate at room temperature in vacuo to give the title compound.

C. 1-{N-[1 (S)-ethoxycarbonyl-5-aminopentyl]-(S)-alanyl}-cis,syn-octahydro-1H-indole-2(S)-carboxylic acid. in 80 ml of ethanol dissolve 3.7 g of 1 - {N - [1(S) - ethoxycarbonyl - 5 - (benzyloxycarbonylamino)pentyl] - (S) - alanyl} - cis,syn - octahydro - 1H - indole - 2(S) - carboxylic acid benzyl ester. To this add 1 g of 20% palladium hydroxide on carbon. Hydrogenate this mixture at 60 psi for 18 hr. Filter and concentrate at room temperature in vacuo. Triturate the oily residue with ether and filter to give the title compound, m.p. 160°C (decomp.), [α]²⁶_{D} - 40.7° (MEOH).

D. 1 - {N - [1 (S) - ethoxycarbonyl - 5 - (4 - chloro - 3 - sulfamoylbenzamido)pentyl] - (S) - alanyl} - cis,syn - octahydro - 1H- indole - 2(S) - carboxylic acid hydrochloride.

Dissolve 0.7 g of 1 - {N - [1 (S) - ethoxycarbonyl - 5 - aminopentyl] - (S) - alanyl} - cis,syn - octahydro -1H - indole-2(S) carboxylic acid and 0.4 g of triethyl amine in 40 ml of tetrahydrofuran and cool to 5°C. To this solution add dropwise a solution of 0.48 g of 4-chloro-3-sulfamoylbenzoyl chloride in 20 ml of tetrahydrofuran. Stir for 1 hr. at 5°C and 1 hr. at 25°C. Filter and concentrate in vacuo. Dissolve the residue in 200 ml of dichloromethane and acidify with HCI-gas. Decant and triturate the residue several times with dichloromethane to give the title compound, m.p. 185°C, [α]²⁶_{D} - 20° (MEOH).

### Example 22

### 1-{N-[1(S)-Ethoxycarbonyl-5-(4-chloro-3-sulfamoylbenzamido)pentyl]-(S)-alanyl}-cis,syn-octahydro-1H-indole-2(S)-carboxylic acid

A. N-[1(S)-ethoxycarbonyl-5-(benzyloxycarbonylamino) pentyl]-(S)-alanine, t-butyl ester.

Dissolve 60 g of Ns-benzyloxycarbonyl-(S)-lysine, ethyl ester and 90 g of t-butyl bromopropionate and 22 g of triethylamine in 200 ml of DMF. Heat thise solution at 70°C for 18 hr. Concentrate in vacuo and dissolve the residue in ethyl acetate. Wash organic layer with water and brine. Dry organic layer (MgS0₄) and concentrate in vacuo. Chromatograph the residue on silica gel (100-200 mesh) using ether:hexane (1:1) as solvent. Elute SR-isomer and then the title compound. Thin layer chromatography in ether:hexane 1:1 shows the SR-isomer at 0.2 and the SS-isomer at 0.1.

B. N-[1(S)-ethoxycarbonyl-5-aminopentyl]-(S)-alanine t-butyl ester.

Dissolve 10 g of N-[1(S)-ethoxycarbonyl-5-(benzyloxycarbonylamino)pentyl]-(S)-alanine, t-butyl ester in 40 ml of ethanol. Add 3.0 g of 20% palladium hydroxide on carbon. Hydrogenate at 60 psi for 18 hr. Filter and concentrate in vacuo to give title compound.

C. N-[1(S)-ethoxycarbonyl-5-(4-chloro-3-sulfamoylbenzamido)pentyl]-(S)-alanine, t-butyl ester. Dissolve 1.0 g of N-[1 (S)-ethoxycarbonyl-5-aminopentyl]-(S)-alanine, t-butyl ester in 50 ml of THF. Add 0.3 g of triethylamine. Cool to 5°C under nitrogen. Add, dropwise, with stirring a solution of 0.8 g of 4-chloro,3-sulfamoyl benzoyl chloride in 30 ml of THF. Warm to room temperature and stir for 10 hr. Filter and concentrate in vacuo. Dissolve residue in ethyl acetate and wash with water and brine. Dry organic layer (MgS0₄) and concentrate in vacuo to give the title compound. Purify by chromatography on silica gel using ethyl acetate as eluant.

D. N[1(S)-ethoxycarbonyl-5-(4-chloro-3-sulfamoylbenzamido)pentyl]-(S)-alanine, hydrochloride.

Dissolve 0.75 g of N-[1(S)-ethoxycarbonyl-5-(4-chloro-3-sulfamoylbenzamido)pentyl]-(S)-alanine, t-butyl ester in 10 ml of dioxane saturated with HCI gas. Keep at room temperature 18 hr. Concentrate in vacuo and triturate the residue with ether to give title compound.

E. 1 - {N - [1 (S) - ethoxycarbonyl - 5 - (4 - chloro - 3 - sulfamoylbenzamido)pentyl] - (S) - alanyl} - cis,syn - octahydro - 1H - indole - 2(S) - carboxylic acid, benzyl ester.

In 20 ml of DMF dissolve 0.75 g of N - [1(s) - ethoxycarbonyl - 5 - (4 - chloro - 3 - sulfamoylbenzamido)pentyl - (S) - alanine HCI, 0.39 g of cis,syn-octahydro-1H-indole-2(S)-carboxylic acid benzyl ester, 0.40 g of 1-(3-dimethylaminopropyl)-3-ethyl-carbodiimide HCl, 0.23 g of 1-hydroxybenzotriazole and 0.15 g of N-methyl morpholine. Stir under nitrogen for 18 hr., concentrate at room temperature (0.03 mm) and partition residue between ethyl acetate and water. Dry organic layer (MgS0₄) and concentrate in vacuo to give an oil. Chromatograph the oil on silica gel using ethyl acetate as eluant to give the title compound.

F. 1 - {N - [1 (S) - ethoxycarbonyl - 5 - (4 - chloro - 3 - sulfamoylbenzamido)pentyl] - (S) - alanyl} - cis,syn - octahydro - 1H - indole - 2(S) - crboxylic acid.

Dissolve 0.05 g of 1 - {N - [1 (S) - ethoxycarbonyl - 5 - (4 - chloro - 3 - sulfamoylbenzamido]pentyl - (S) - alanyl} - cissyn - octahydro - 1H - indole - 2(S) - carboxylic acid benzyl ester in 5 ml of 20% HBr in glacial acetic acid and allow to stand for 4 hr. Concentrate at room temperature (0.03 mm) and triturate with ether to yield product in the form of its hydrobromide salt. Generate the free base by dissolving the hydrobromide salt in 20% aqueous ethanol and absorbing on a strongly acidic ion exchange column (Bio-Rad AG 50w-XZ). Elute with pyridine:water 4:96 and concentrate eluant in vacuo to yield title compound. Generate HCI salt of title compound by adding title compound to dichloromethane and HCI gas to give title compound in the form of its hydrochloride. m.p. 185°C [α]²⁶_{D} - 20° (MEOH).

### Example 23

Treat the benzyl ester from Example 17C with N-carbobenzoxy-alonine-N-hydroxysuccinimide ester (0.10 mole) in cimethylformamide at room temperature. When the reaction is complete as indicated by thin layer chromatography, evaporate the solvent at reduced pressure. Add ethyl acetate and wash with aqueous sodium bicarbonate solution. Dry the organic solution with magnesium sulfate and evaporate the solvent at reduced pressure. Purify the residue by chromatography. Dissolve this product in ethanol and add the solution to 10% palladium on charcoal (1.0 g) in a hydrogenation bottle. Hydrogenate the mixture at 30 psi, until the reaction is complete as indicated by thin layer chromatography. Filter the mixture and evaporate the solvent at reduced pressure to give 7 - (4 - chloro - 3 - sulfamoyl - benzamido) - 2 - [(S) - alanyl] - 1,2,3,4 - tetrahydroisoquinoline - 3(S) - carboxylic acid. Dissolve 7 - (4 - chloro - 3 - sulfamoyl - benzamido) - 2 - [(S) - alanyl] - 1,2,3,4-tetrahydroisoquinoline - 3(S) - carboxylic acid (0.10 mole) in 100 ml of absolute ethanol and add 2-oxo-4-phenylbutyric acid, ethyl ester (0.30 mole). Add 50 ml of 3 Angstrom molecular sieve pellets and stir the resulting mixture at room temperature for 18 hrs. Filter the mixture and treat the filtrate with sodium cyanoborohydride (0.30 mole) at room temperature for 2 hrs. Concentrate the mixture under reduced pressure, dilute the oil with dilute hydrochloric acid and stir at room temperature for one hour. Absorb the aqueous solution on XAD-2 resin (Rohm & Haas Co.). Elute the resin with water and then with methanol. Concentrate the methanol solution and purify the residue by chromatography to obtain 7 - (4 - Chlora - 3 - Sulamoylbenzamido) - 2 - N - [1 (S) - Carboethoxy - 3 - Phenylpropyl] - (S) - Alanyl - 1,2,3,4 - Tetrahydroisoquinoline - 3 - (S) - Carboxylic Acid.

### Example 24

Add ethyl 2-bromo-4-phenylbutanoate (0.10 mole) to a solution of 7 - (4 - chloro - 3 - sulfamoylbenzasmido - 2 - [(S) - analyl] - 1,2,3,4 - tetrahydrosiquinoline - 3(S) - carboxylic acid (0.10 mole) and triethylamine (0.20 mole) in 200 ml of dimethylformamide and heat the mixture at 70°C for 18 hrs. Remove the solvent at reduced pressure and purify the residue by ion-exchange chromatography to give the product as a mixture of diostereoisomers. Purify this mixture by chromatography to obtain 7 - (4 - Chloro - 3 - Sulfamoylbenzamido) - 2 - N - [1(S) - Carboethoxy - 3 - Phenylpropyl] - (S) - Alanyl - 1,2,3,4-Tetrahydroisoquinoline - 3 - (S) - Carboxylic Acid.

### Example 25

### 1 - {N - [1(S) - ethoxycarbonyl - 5 - [3 - hydroxy - 3 - (4 - chloro - 3 - sulfamoylphenyl)phthalimidine - 2 - yl]pentyl] - (S) - alanyl} - cis,syn - octahydro - 1H - indole - 2(S) carboxylic acid.

Dissolve 3.4 g (0.01 mole) of 3-hydroxy-3-(4-chloro-3-sulfamoylphthalilimidine, 4 g (0.01 mole) of 1 - {N - [1 (S) - ethoxycarbonyl - 5 - amino-pentyl] - (S) - alanyl} - cis,syn - octahydro - 1H- indole - 2 - (S) carboxylic acid and 2 g (0.01 g) of p-toluenesulfonic acid monohydrate in 10 ml of N,N-dimethylformamdie. Stir at 25°C for 2 days then concentrate at room temperature under vacuum. Chromatograph the crude product on an acid ion exchange column (Dowex-50) using water followed by 4% aqueous pyridine followed by chromatography on Sephadep LH-20 (methanol) to obtain the product.

### Example 26

### 1 - {N - [1(S) - ethoxycarbonyl - 5 - [7 - chloro - 4 - oxo - 6 - sulfamyl - 2 - phenyl - 1,2,3,4 - tetrahydro quinazolin -3-yl]pentyl] - (S) -alanyl} -cis,syn -octahydro 1H- indole-2(S) carboxylic acid hydrochloride.

Dissolve 3.8 g (0.01 mole) of 6-chloro-7-sulfamylisotoic anhydride and 4 g (0.01 mole) of 1 - {N - [1 (S) - ethoxycarbonyl - 5 - aminopentyl] - (S) - alanyl} - cis,syn - octahydro - 1H- indole - 2 - (S) - carboxylic acid in 20 ml of pyridine. Stir mixture until gas evolution stops (3 hrs). Concentrate at room temperature under vacuum. Chromatograph the crude product on an acid ion exchange column (Dowex-50) using water followed by 4% aqueous pyridine to obtain 1 - {N - [1(S) - ethoxycarbonyl - 5 - [[4 - chloro - 2 - amino - 5 - sulfamoylphenyl)carbonyl]amino]pehtyl] - (S)alanyl} - cis,syn - octahydro - 1H - indole - 2(S) carboxylic acid. HCI salt m.p. 180°C(d) [α]²⁶_{D} = - 16.6° (methanol C = 0.7)

Dissolve the above intermediate in 20 ml of acetic acid and add 2 g (0.02 mole) of benzaldehyde. Stir for 3 days and concentrate at room temperature under vacuum. Chromatograph the residue on a strong acid on exchange column (Dowex-50) using water followed by 4% aqueous pyridine. Concentrate under vacuum and dissolve the residue in ethanol ether. Crudify with HCI gas and dilute with ether to cause the product to precipitate as a white solid. m.p. 180°C(d).

The following compounds exemplify the compounds of formula I, which can be prepared according to the described processes and the examples. Other esters and the corresponding free acids are equally important.

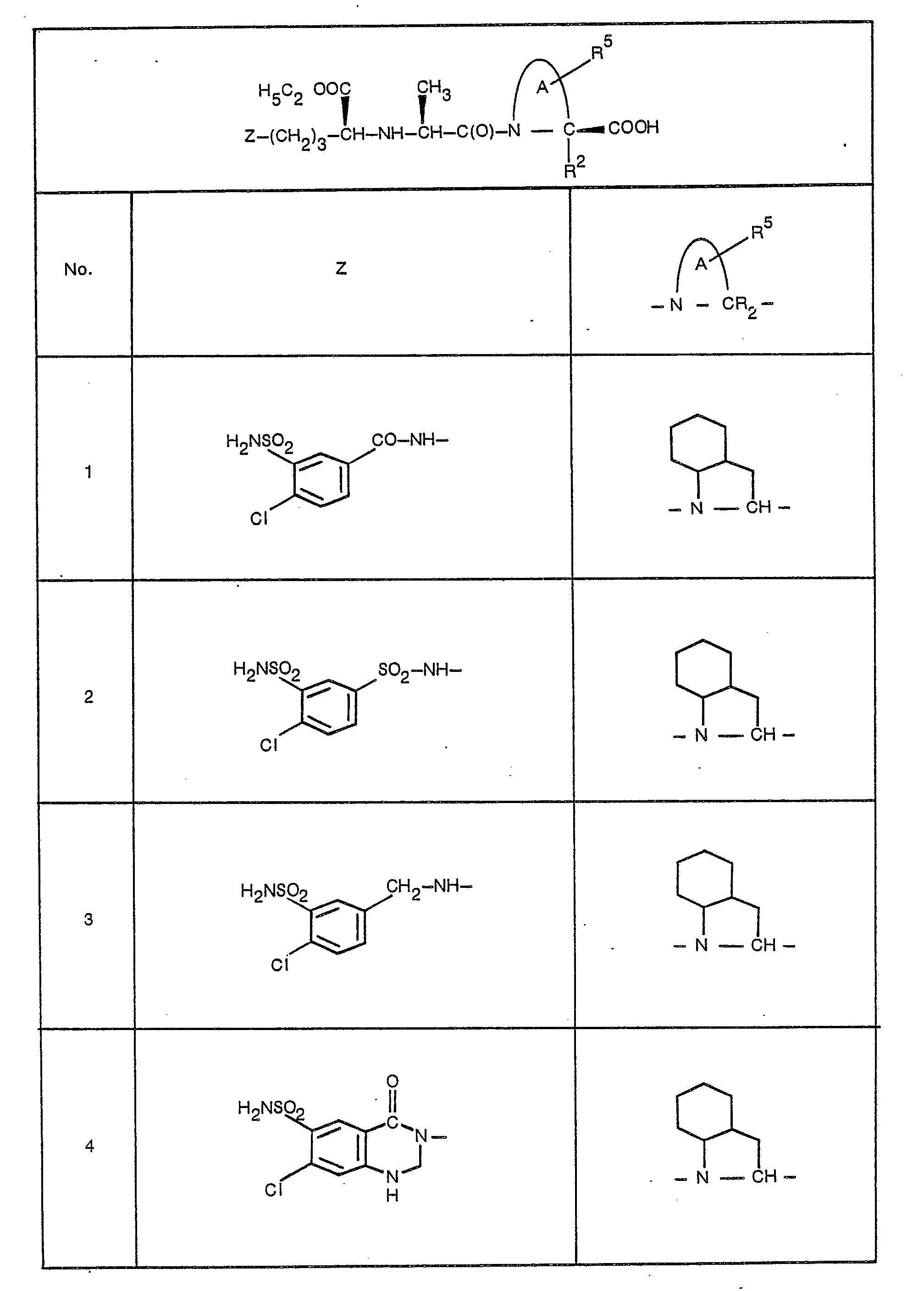

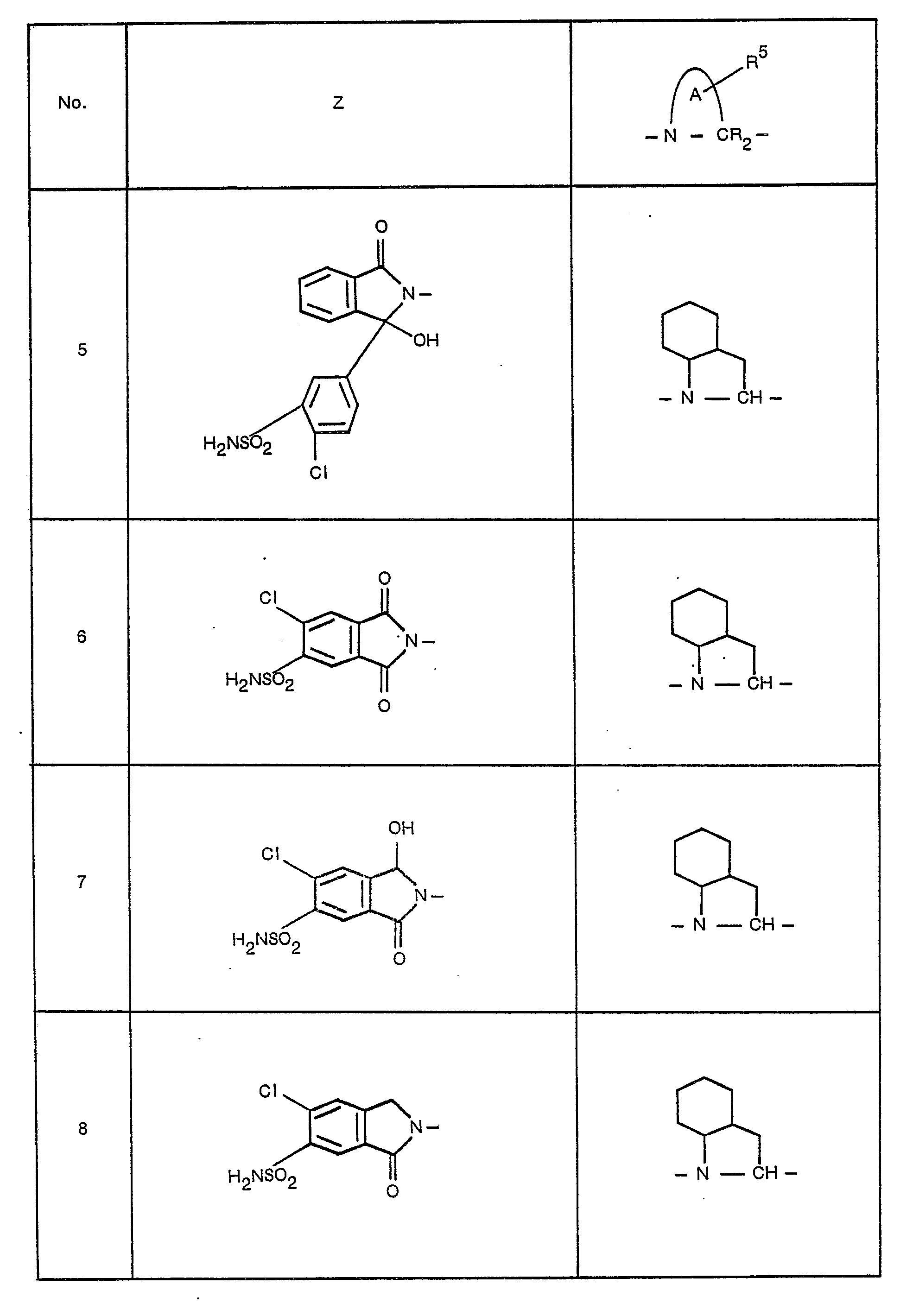

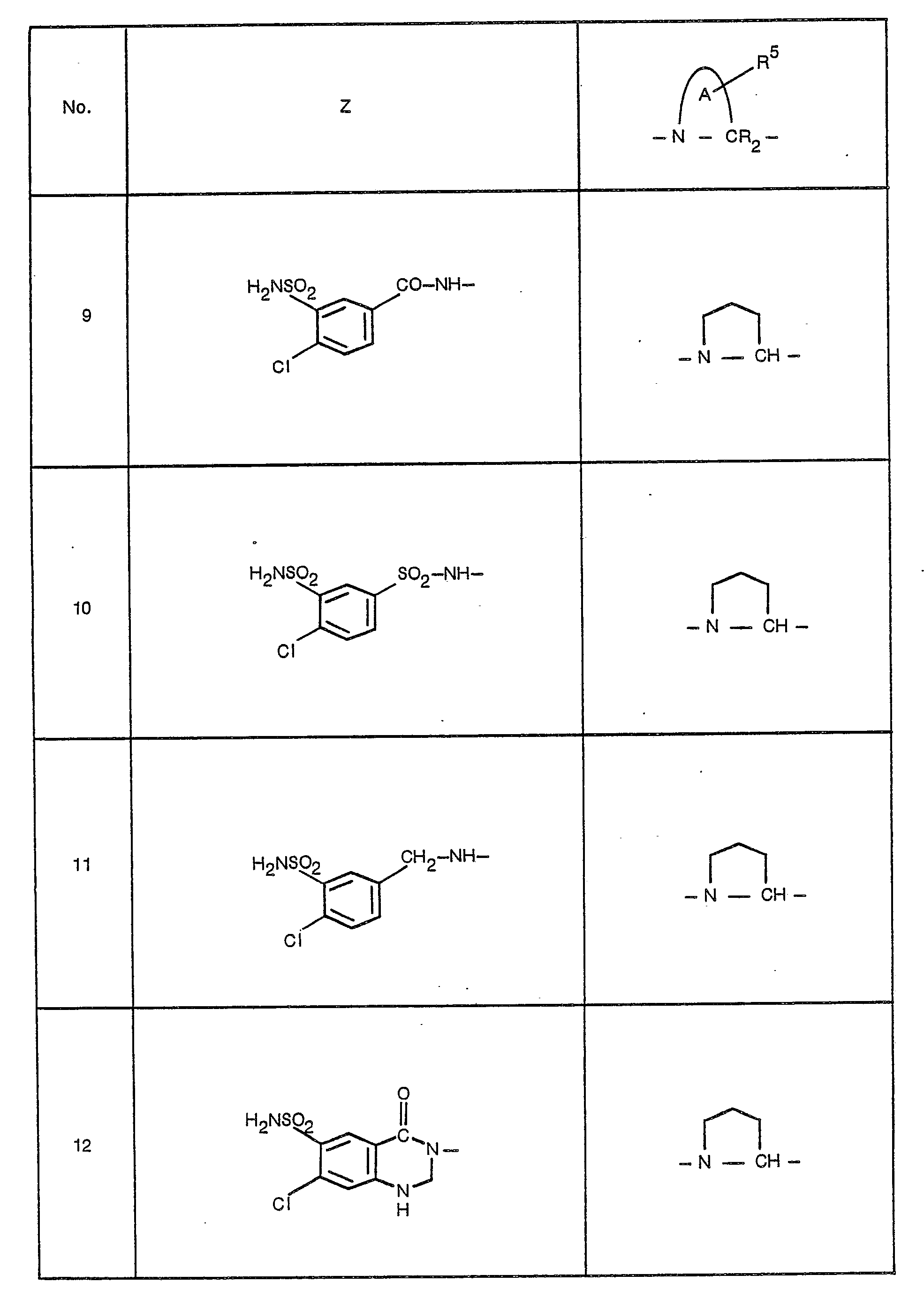

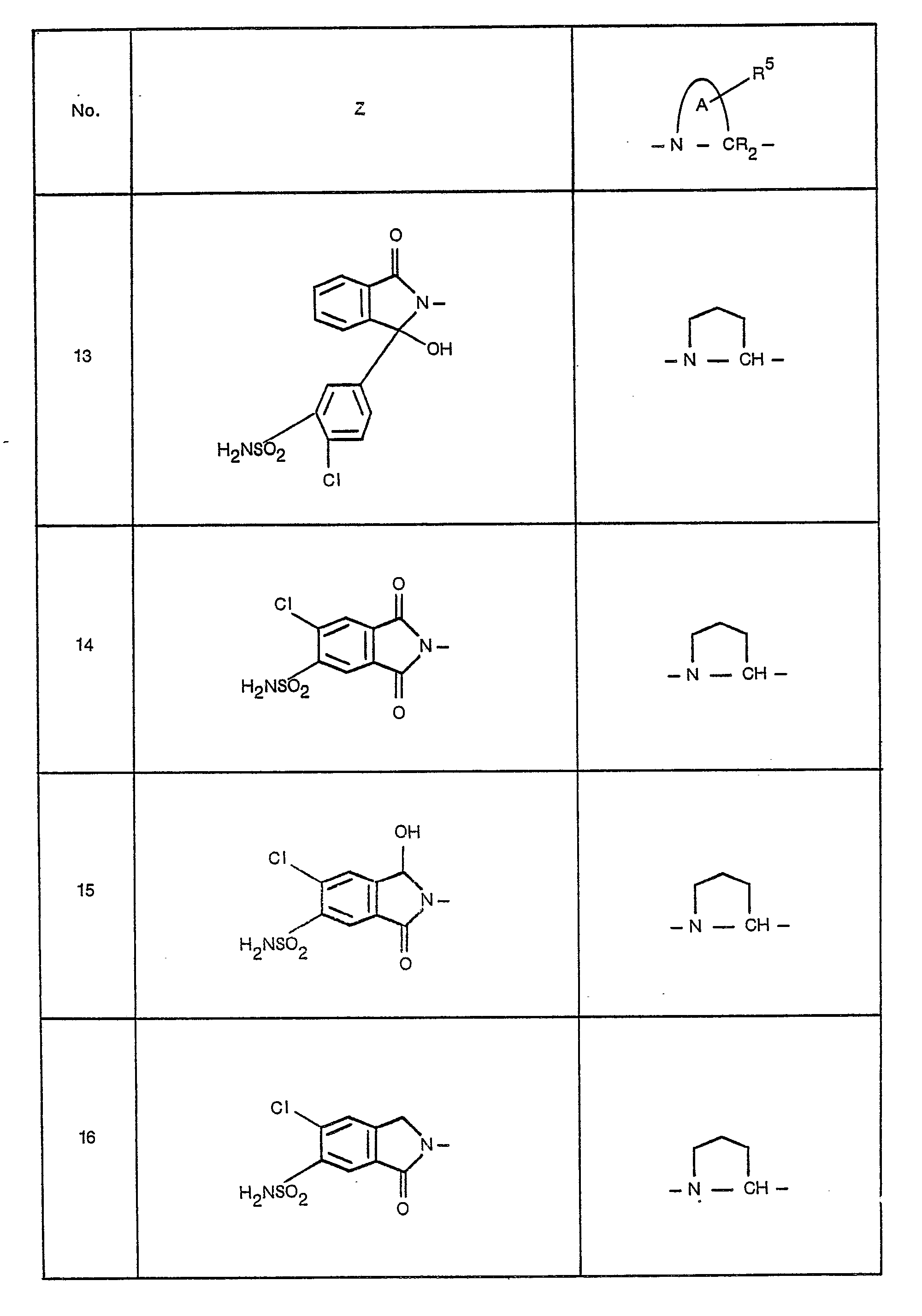

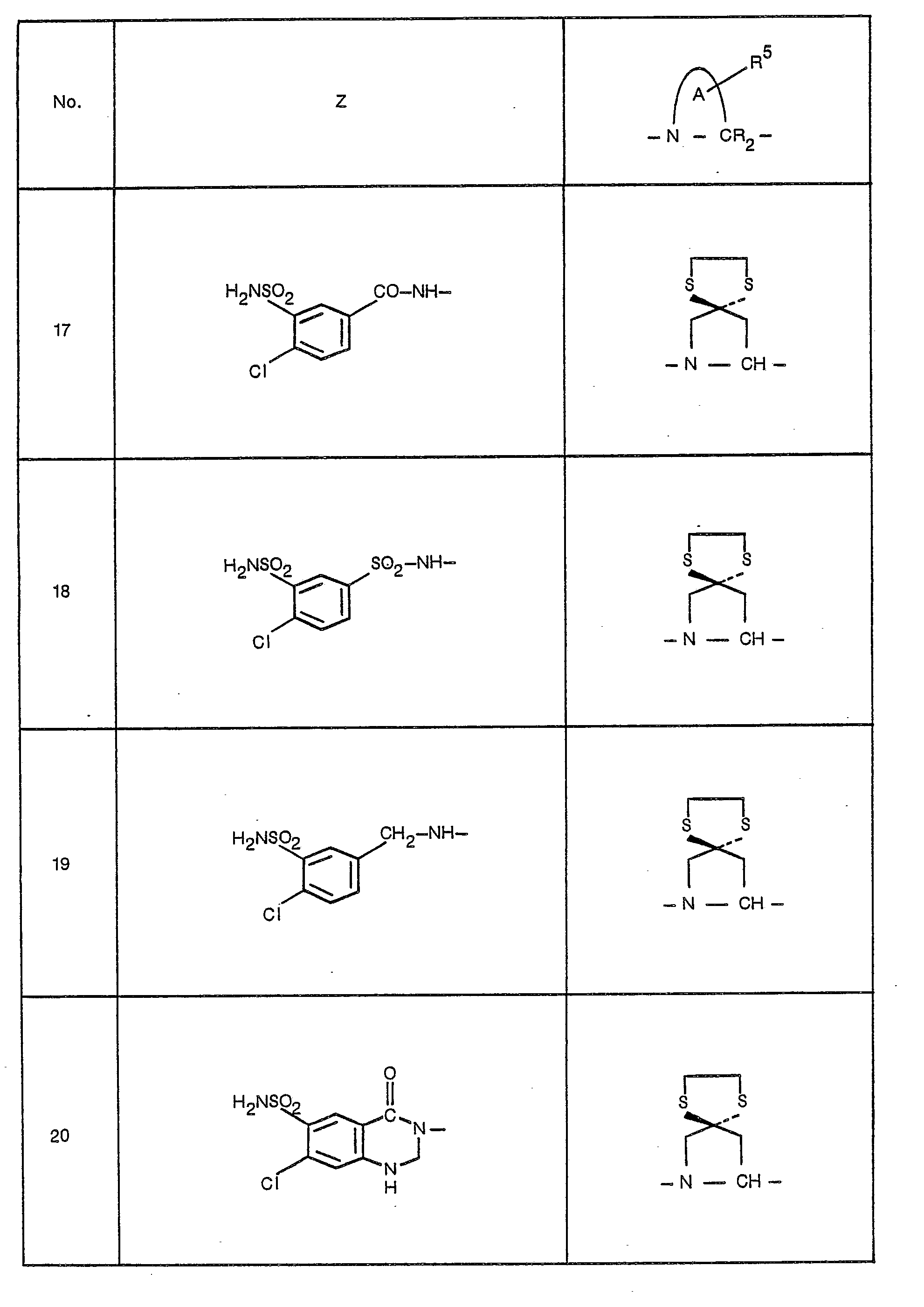

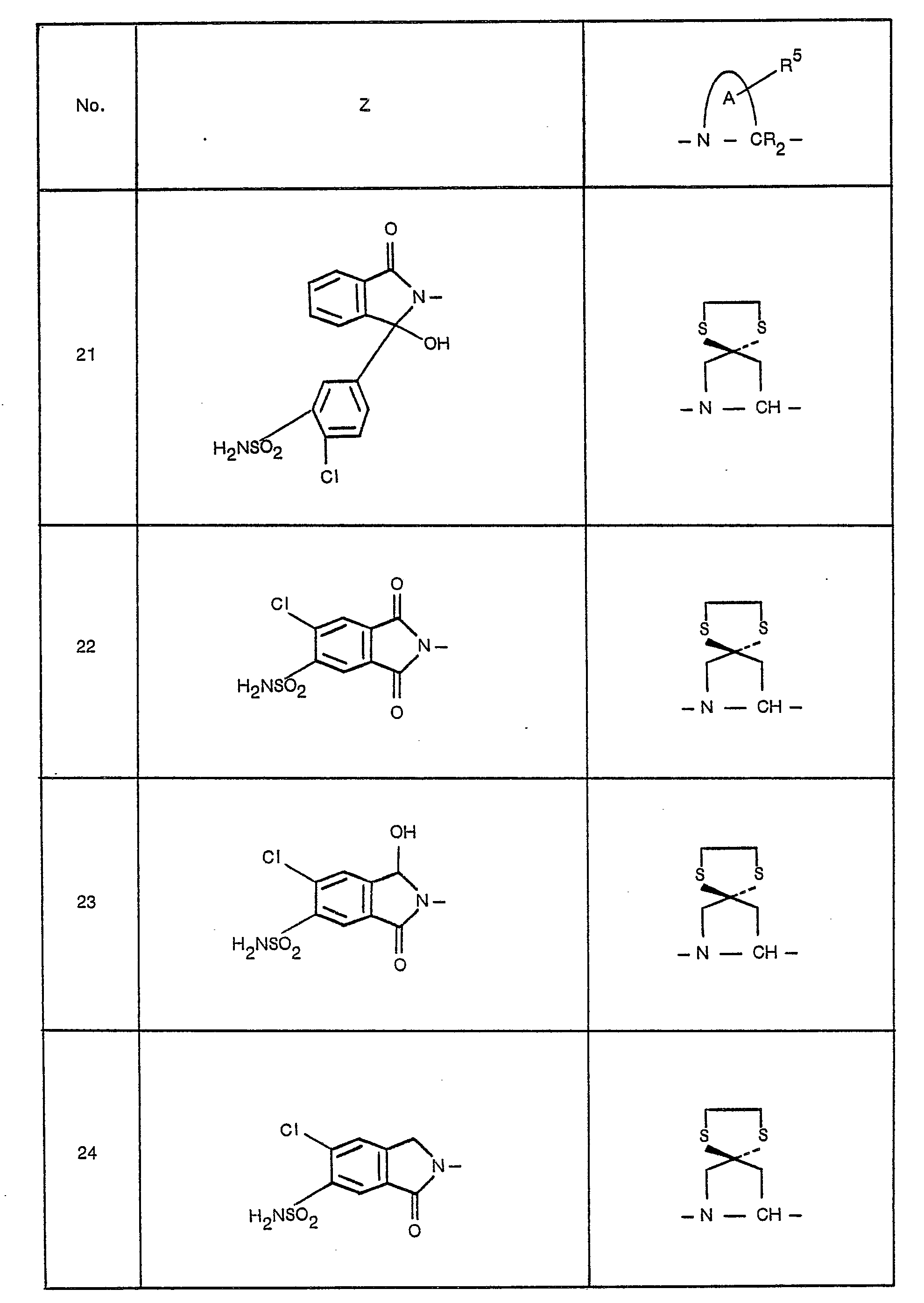

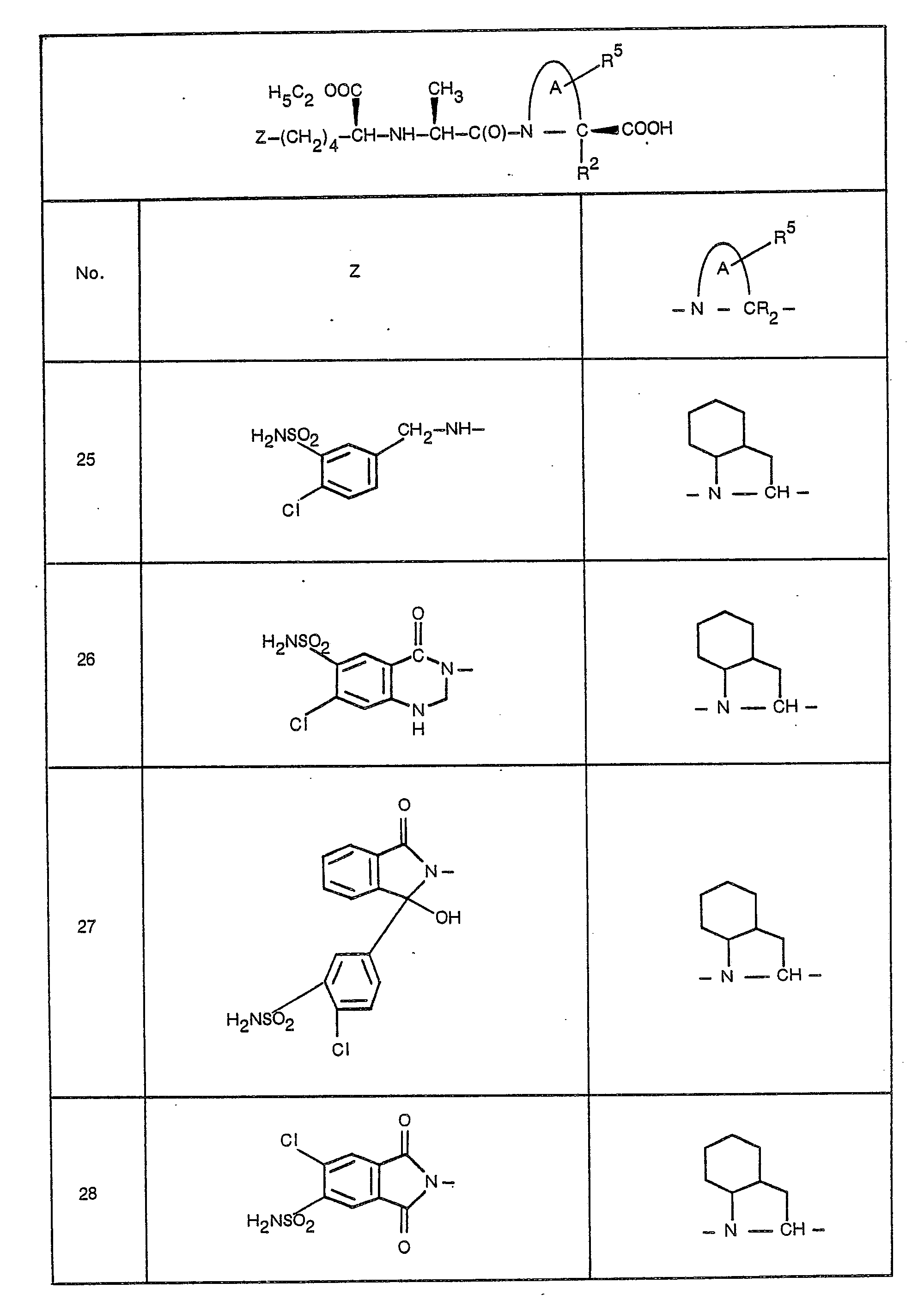

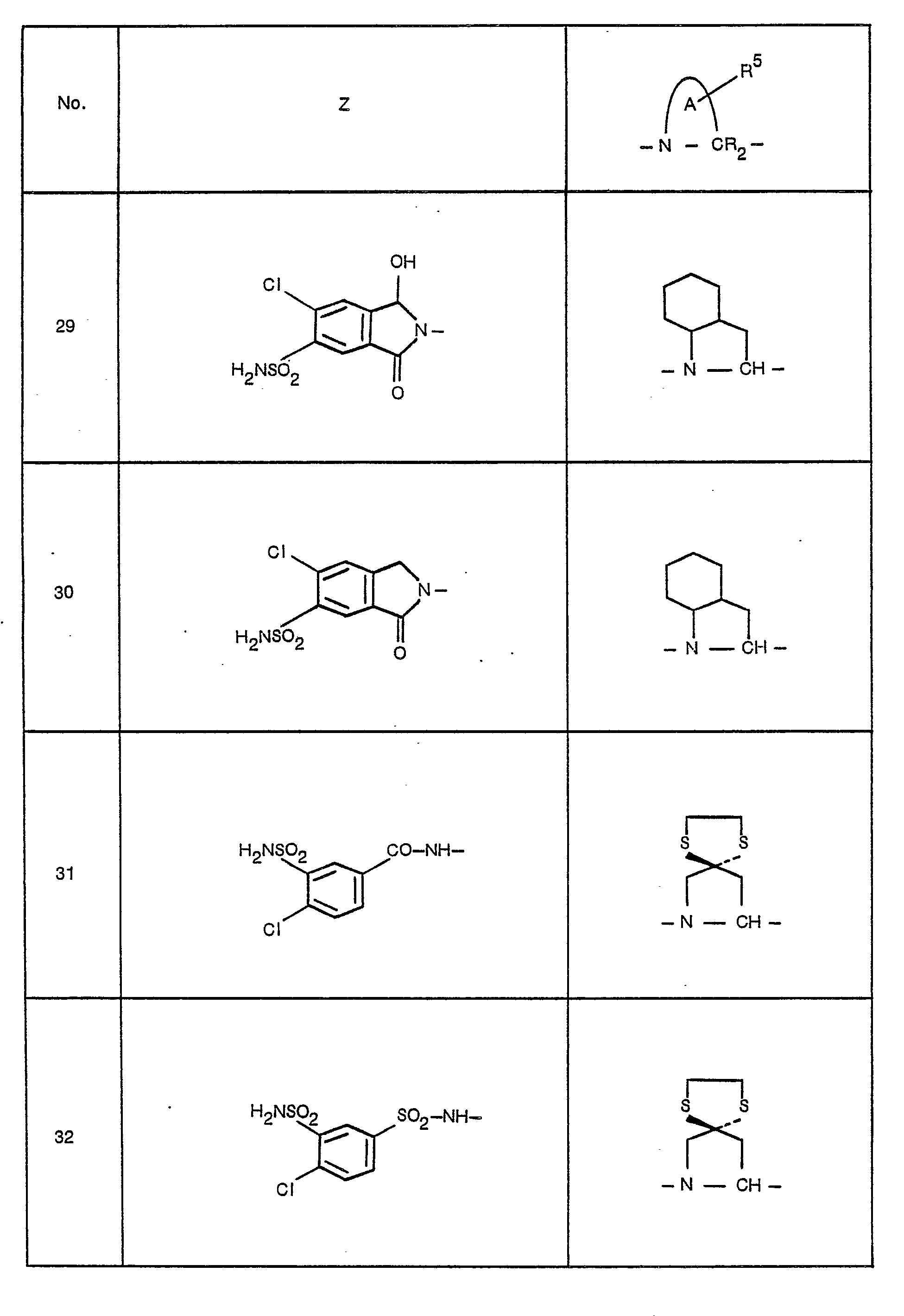

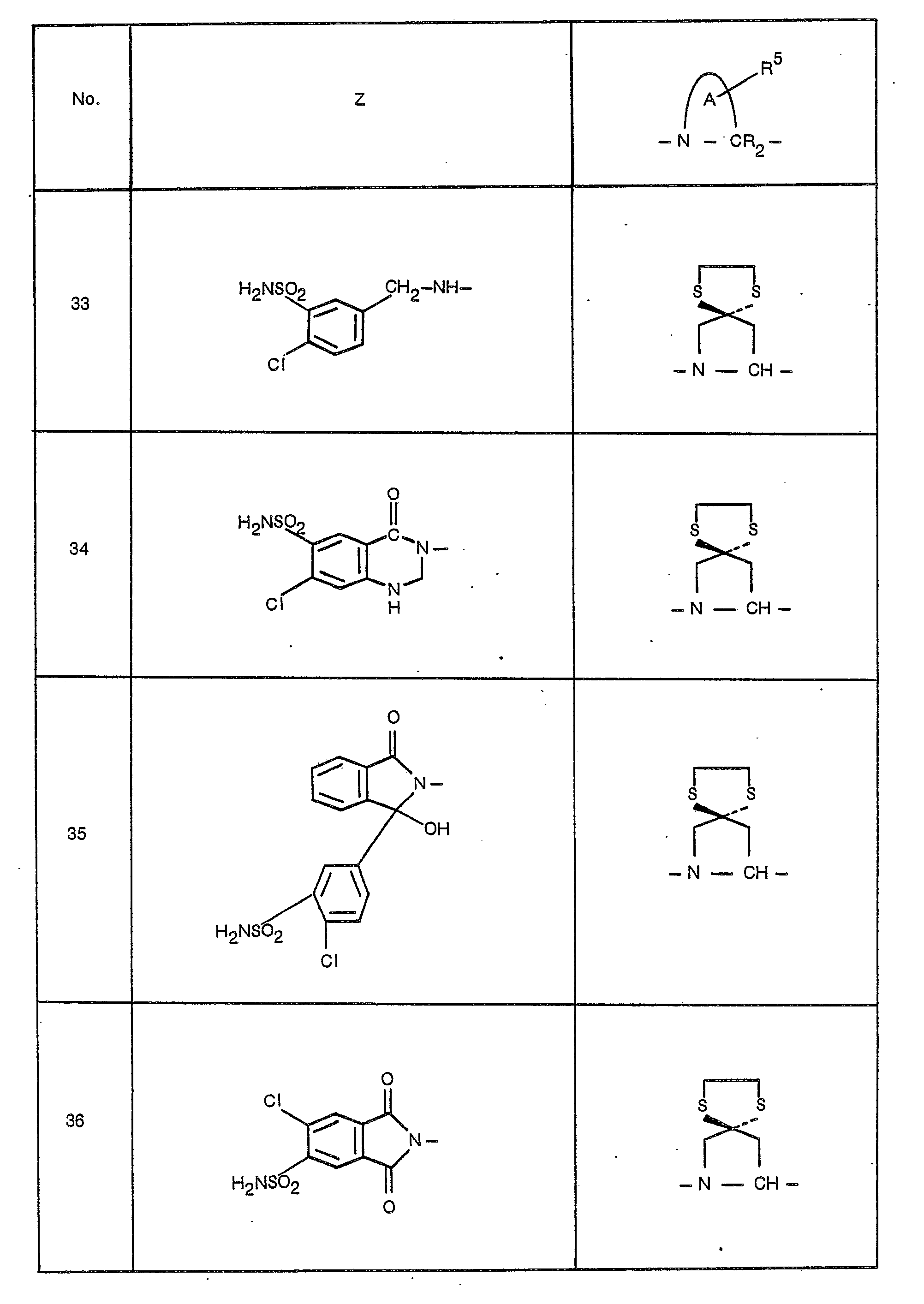

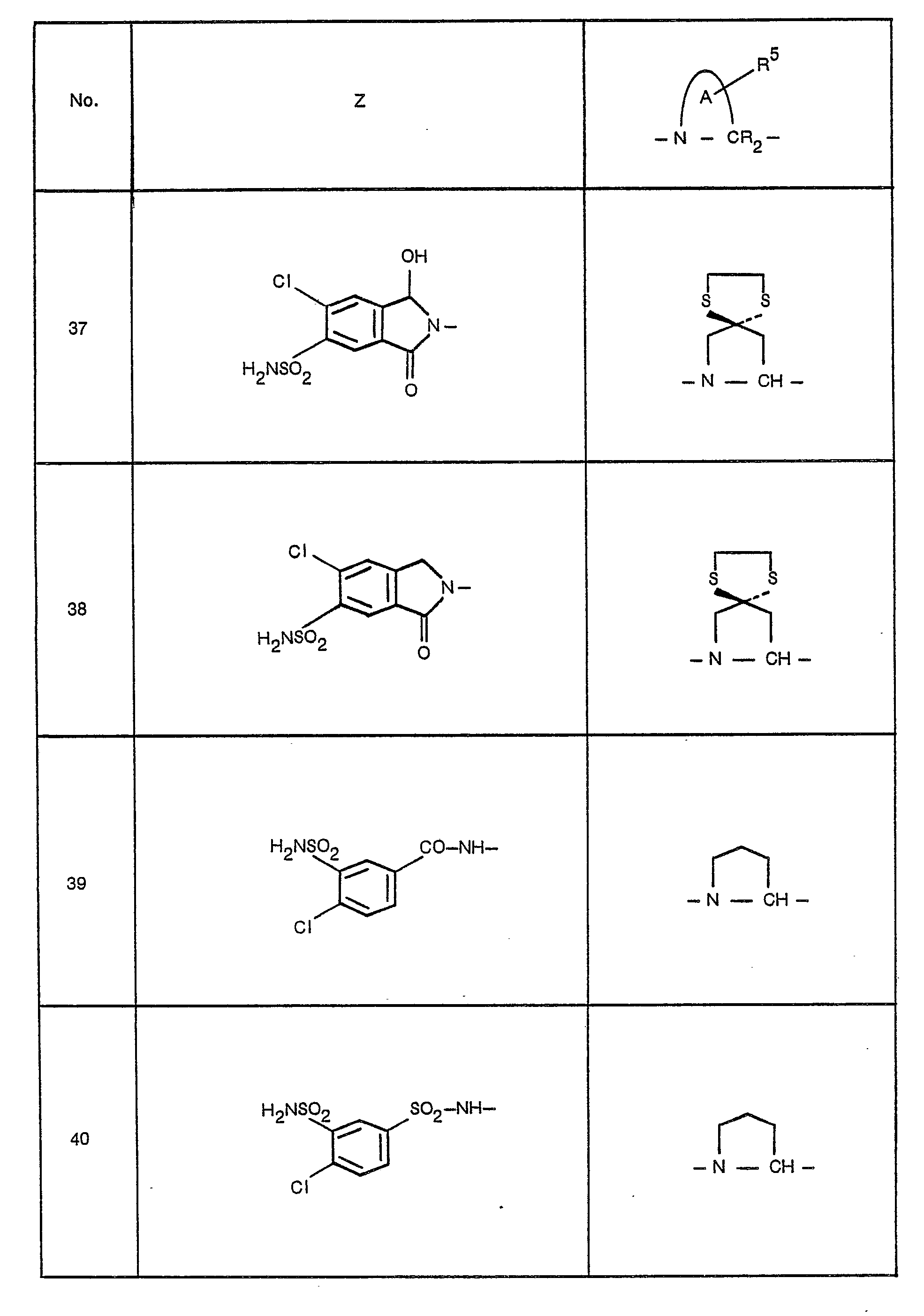

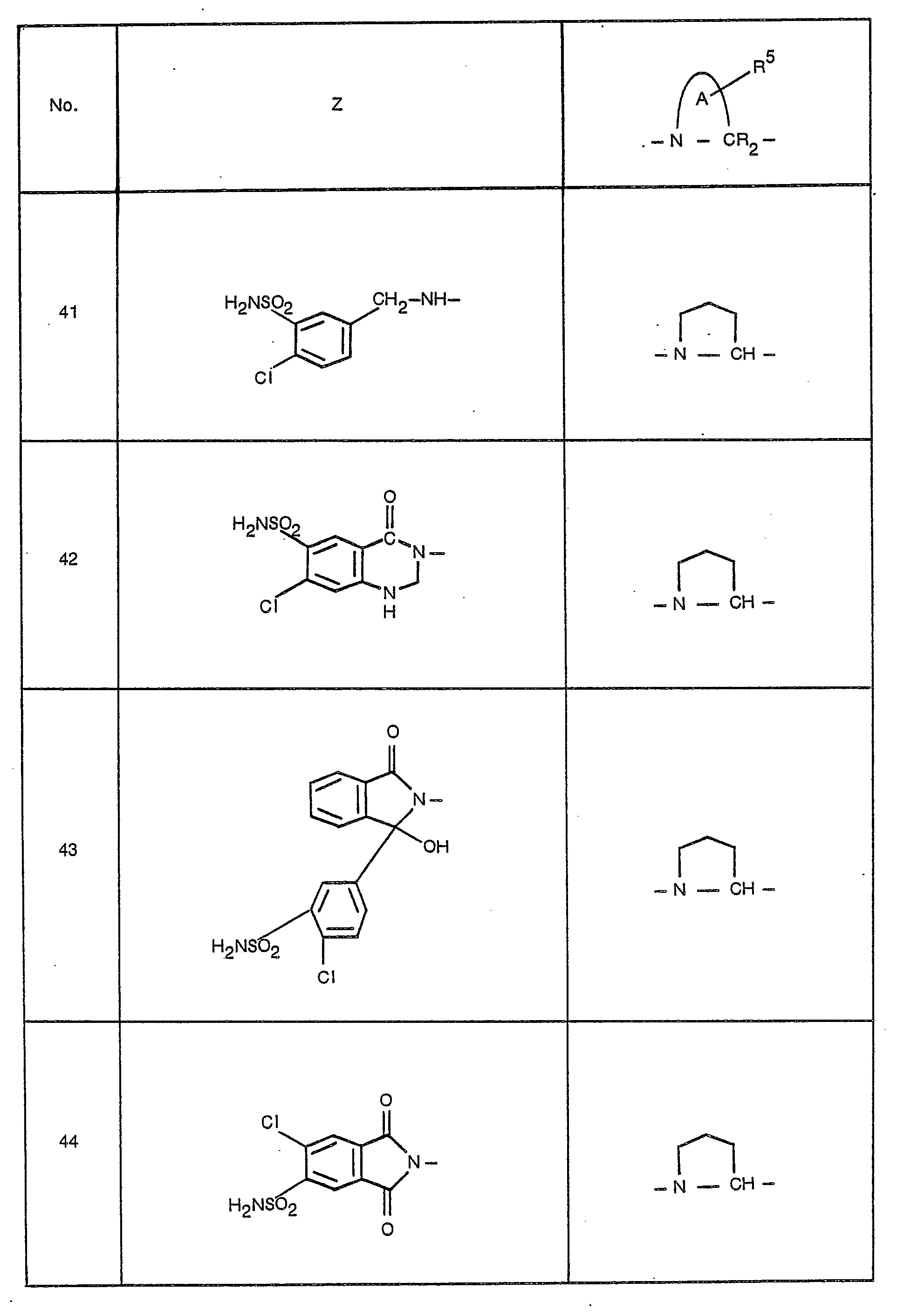

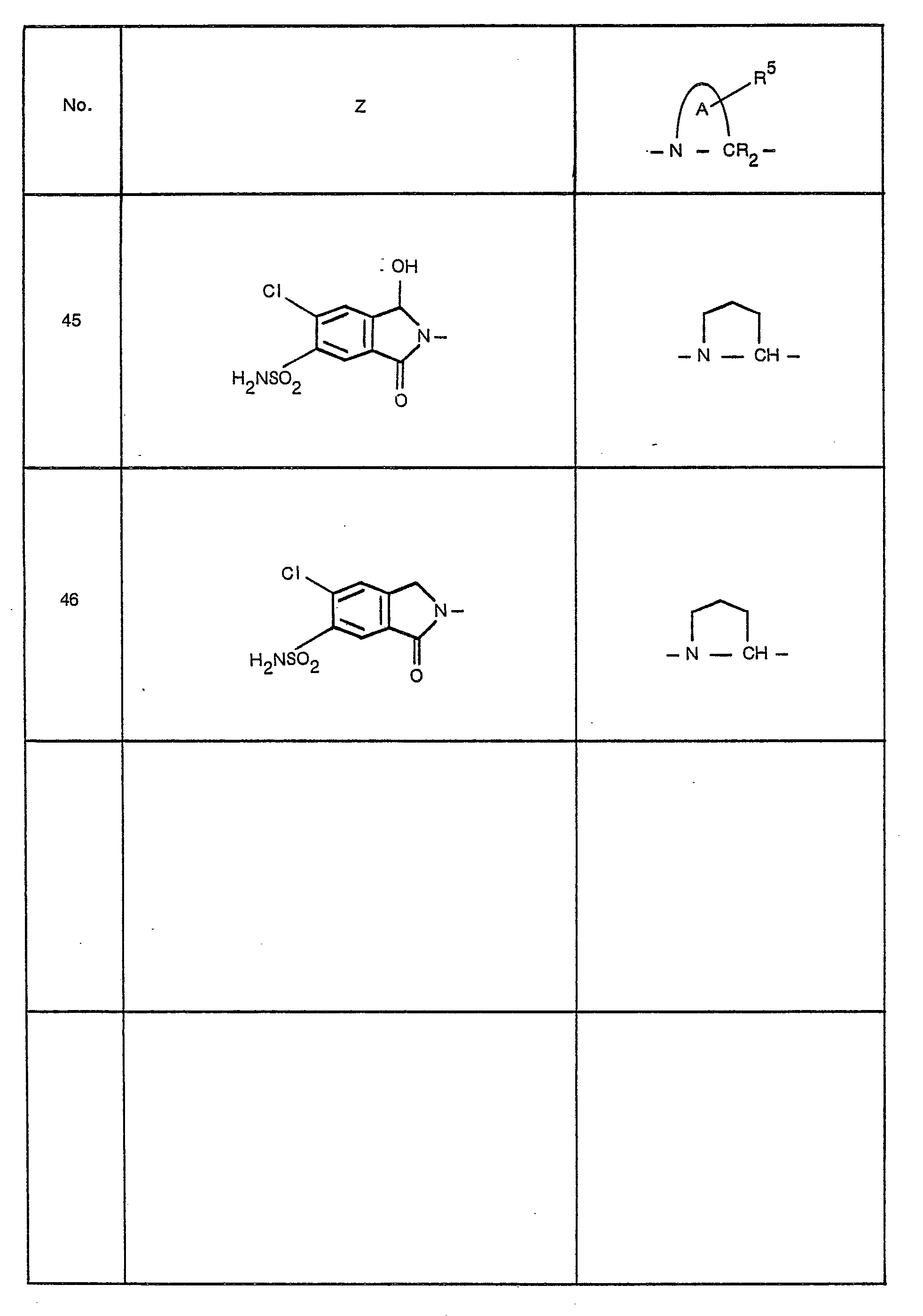

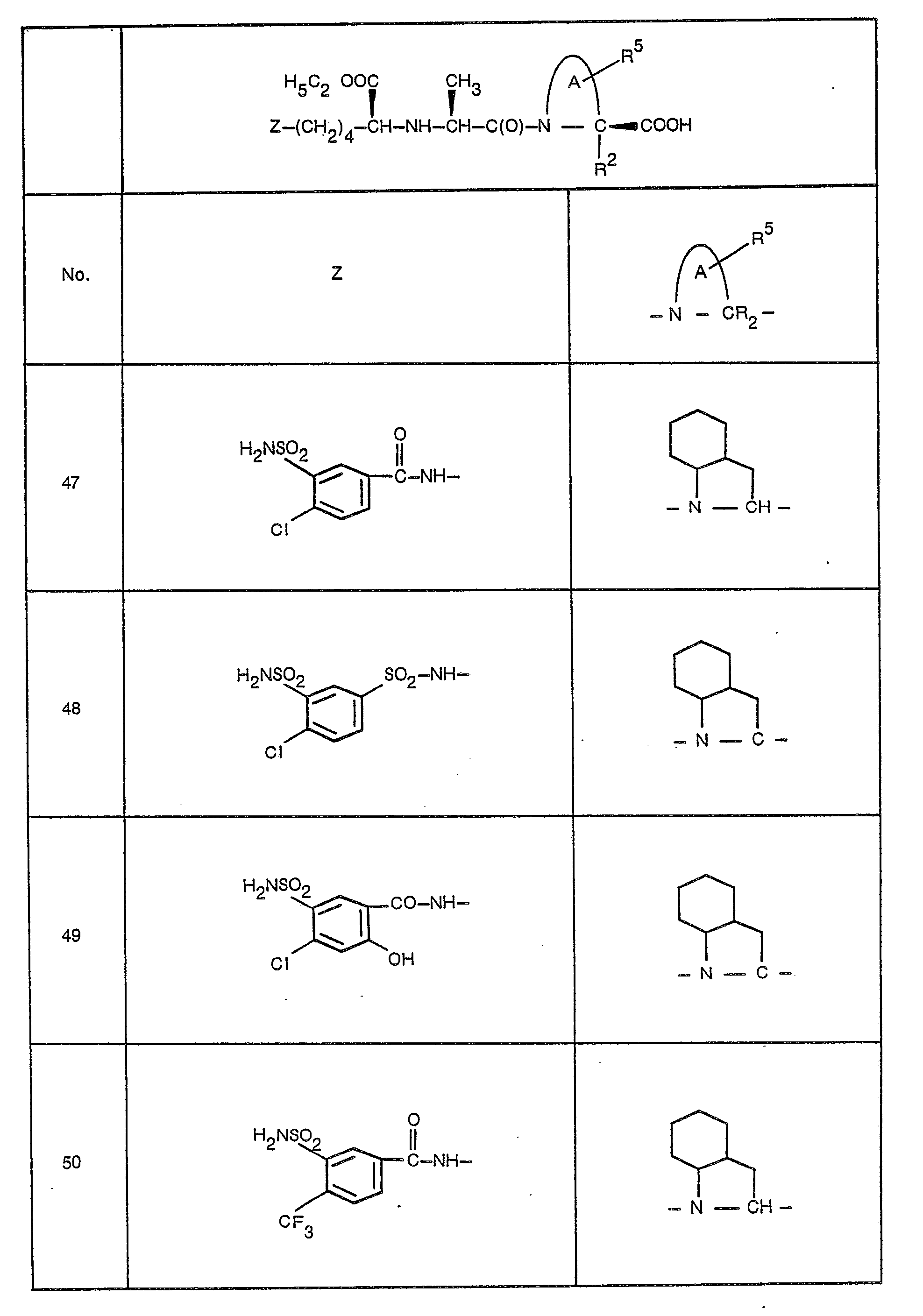

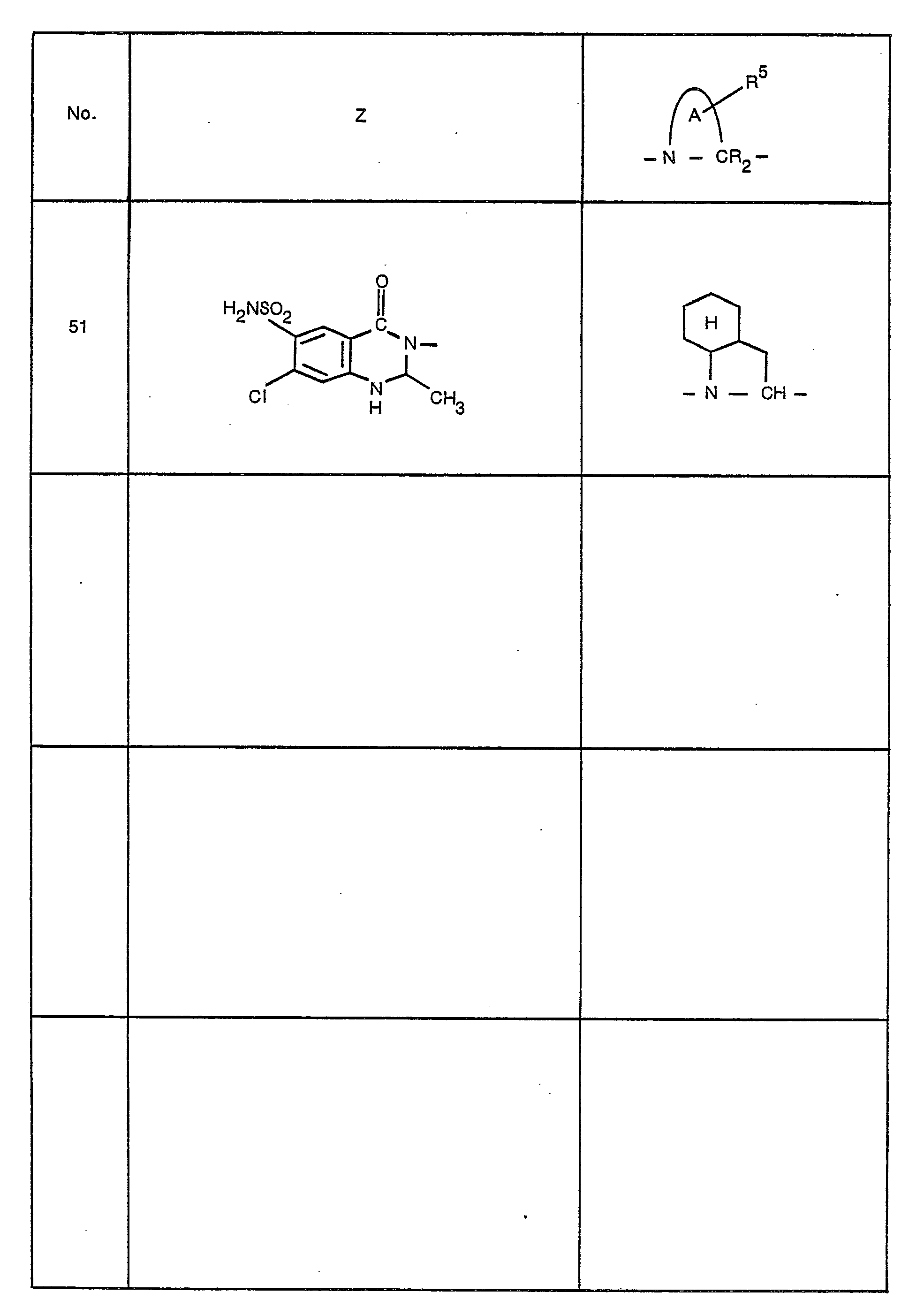

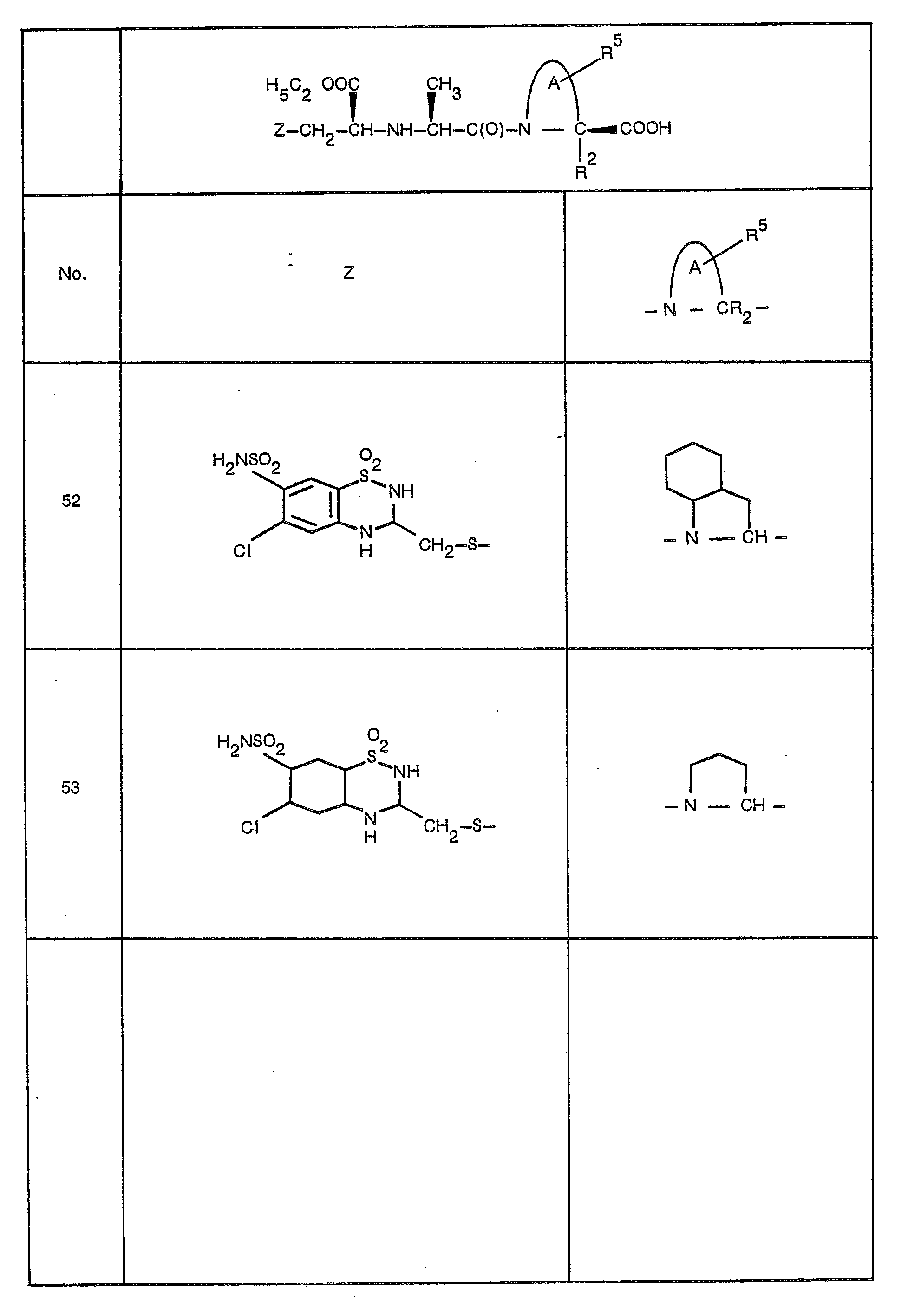

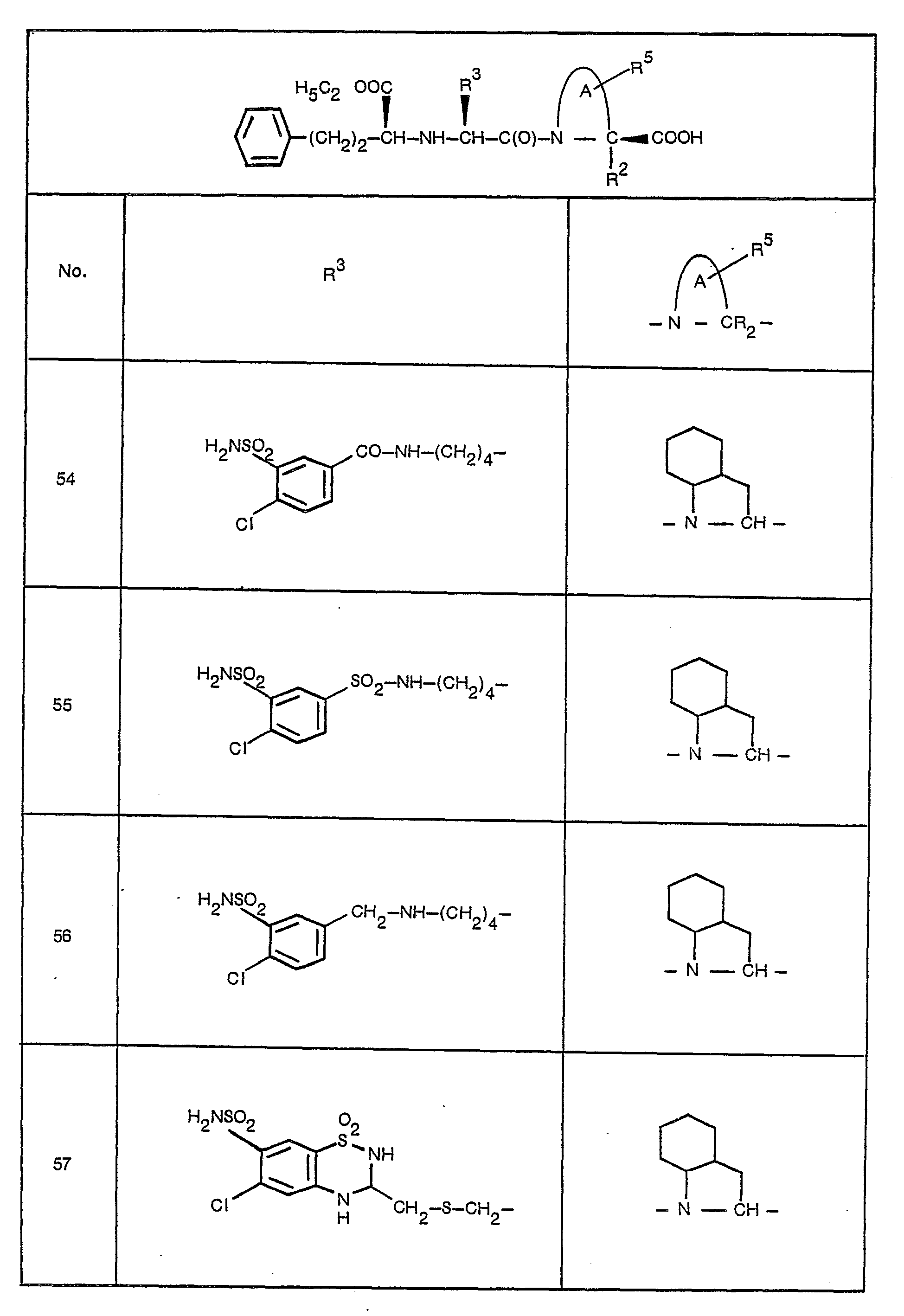

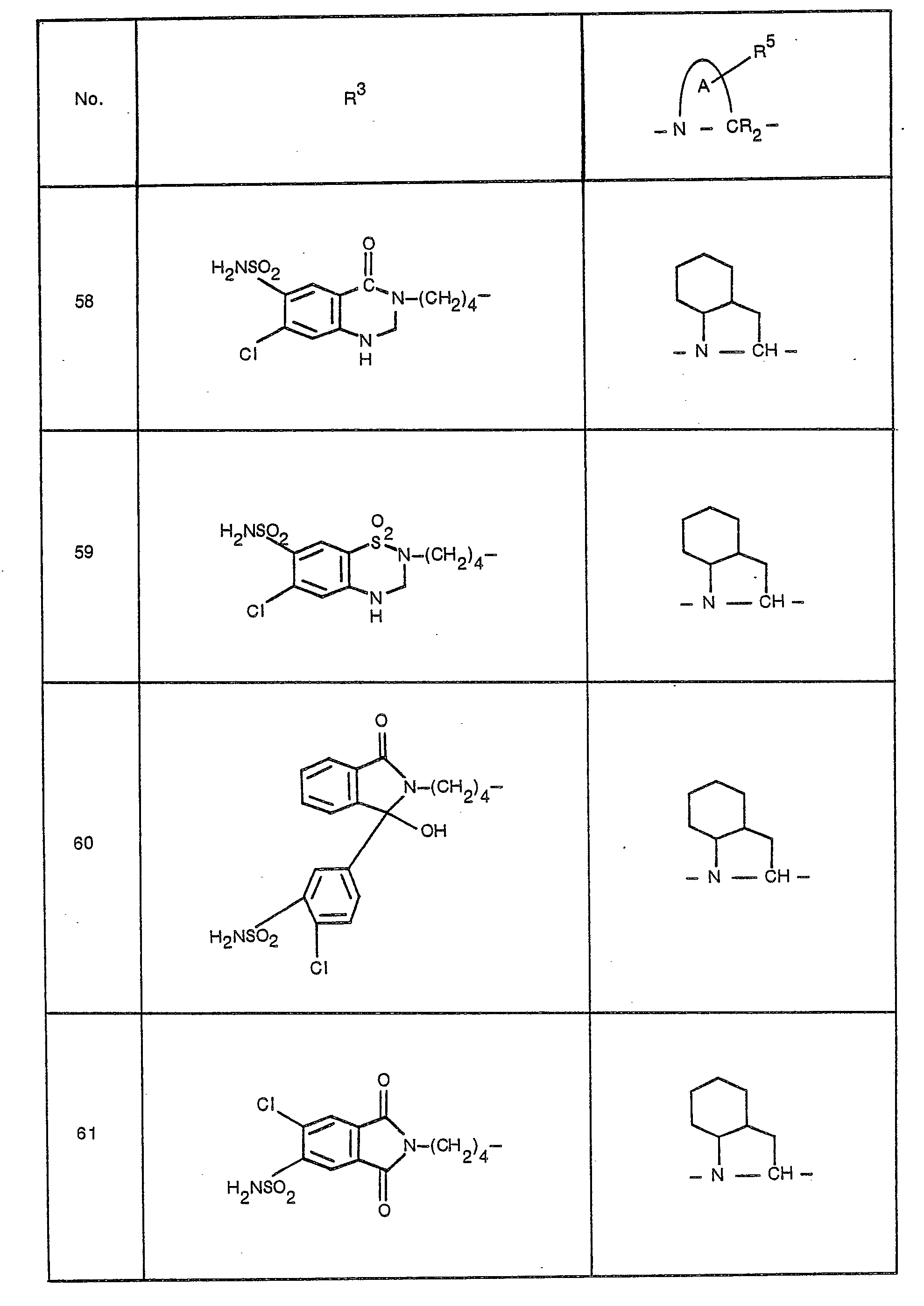

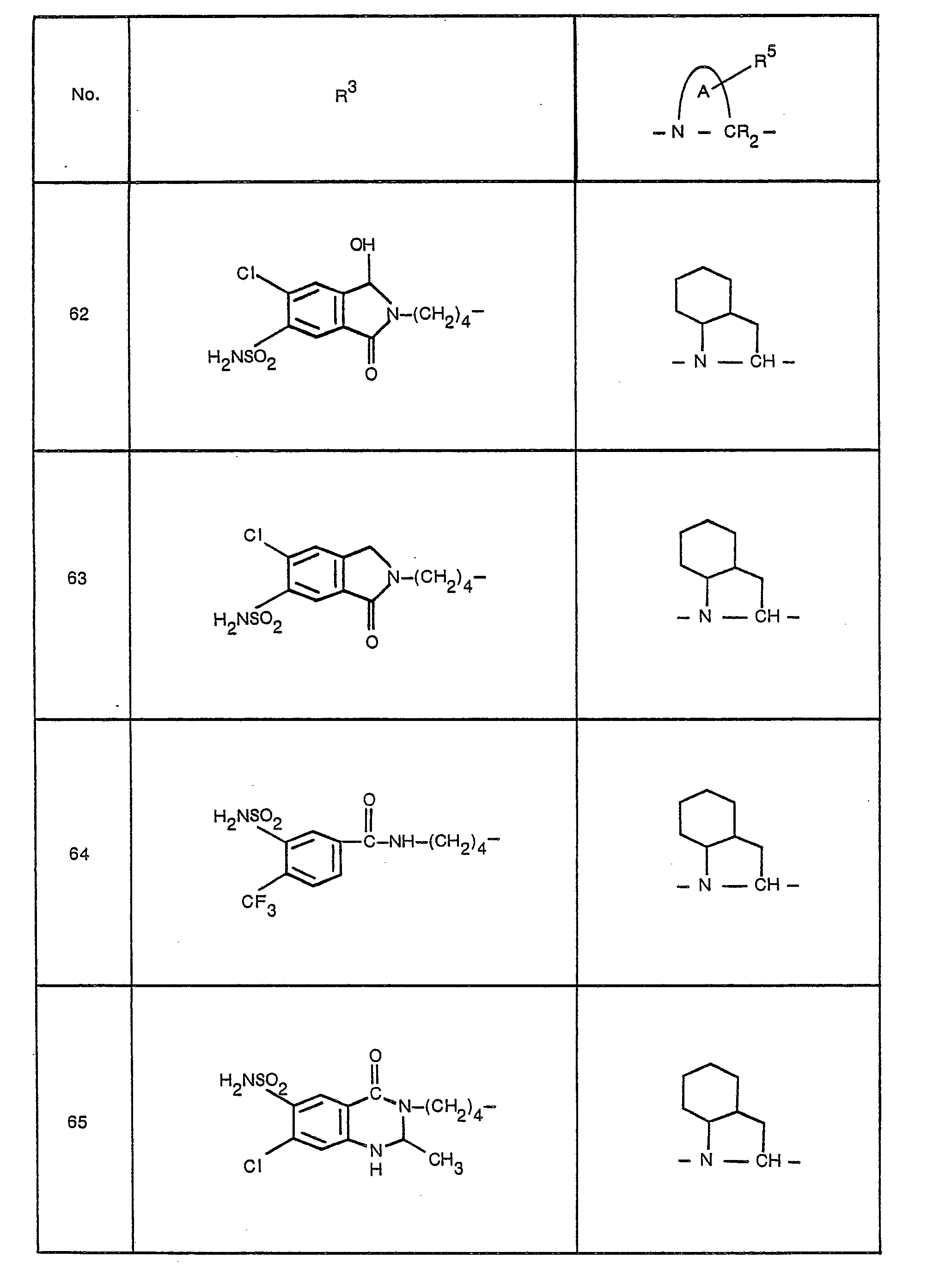

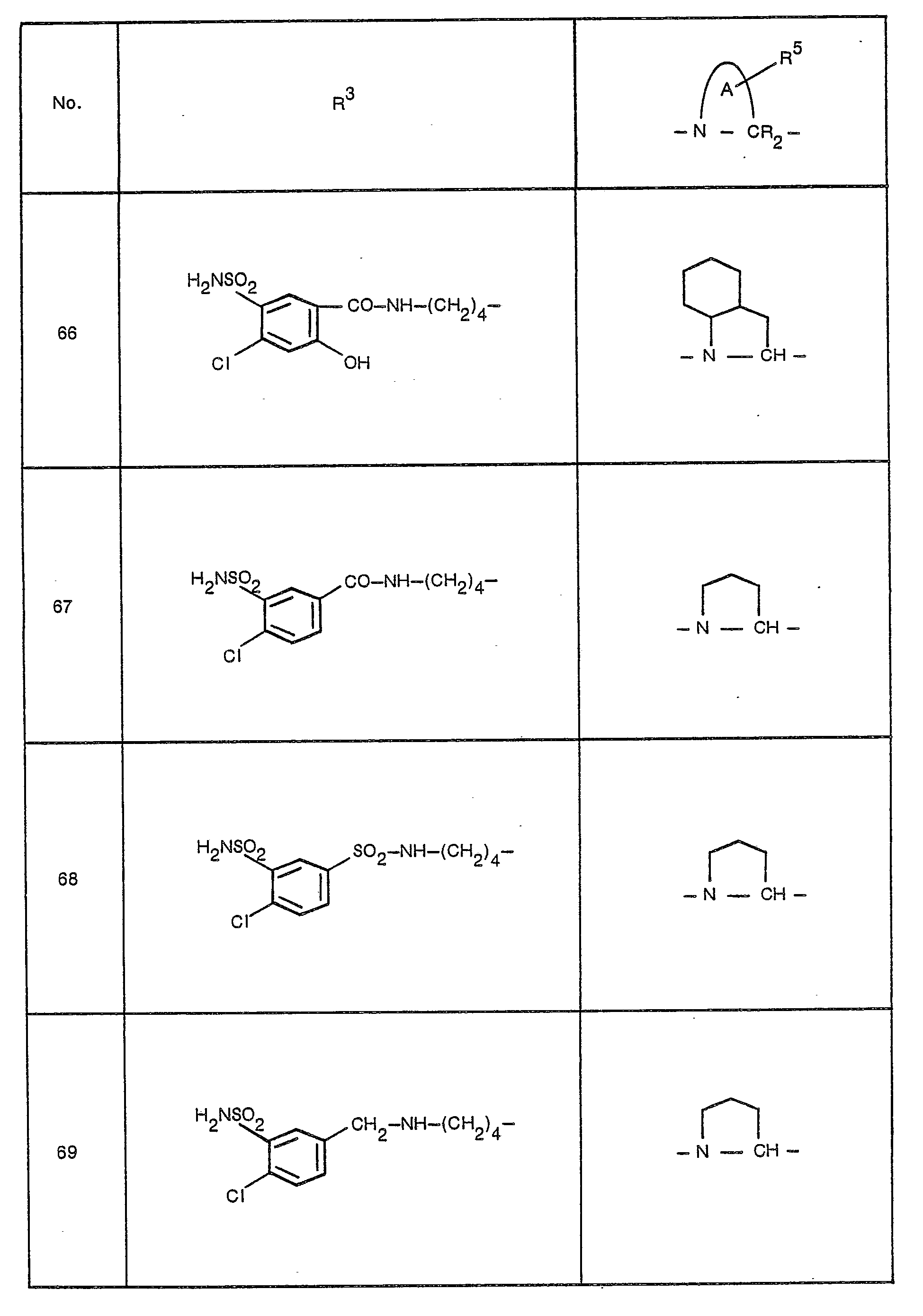

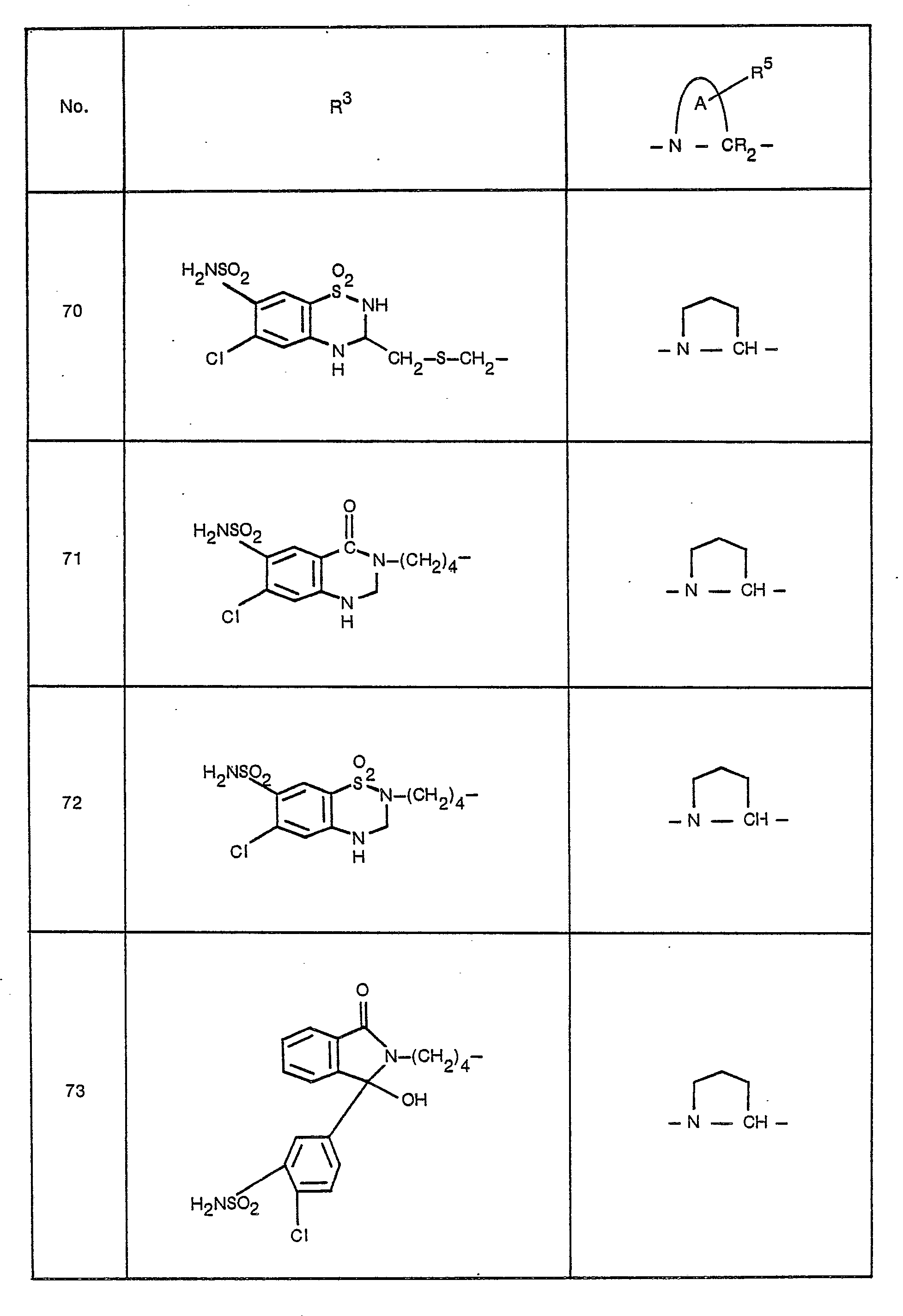

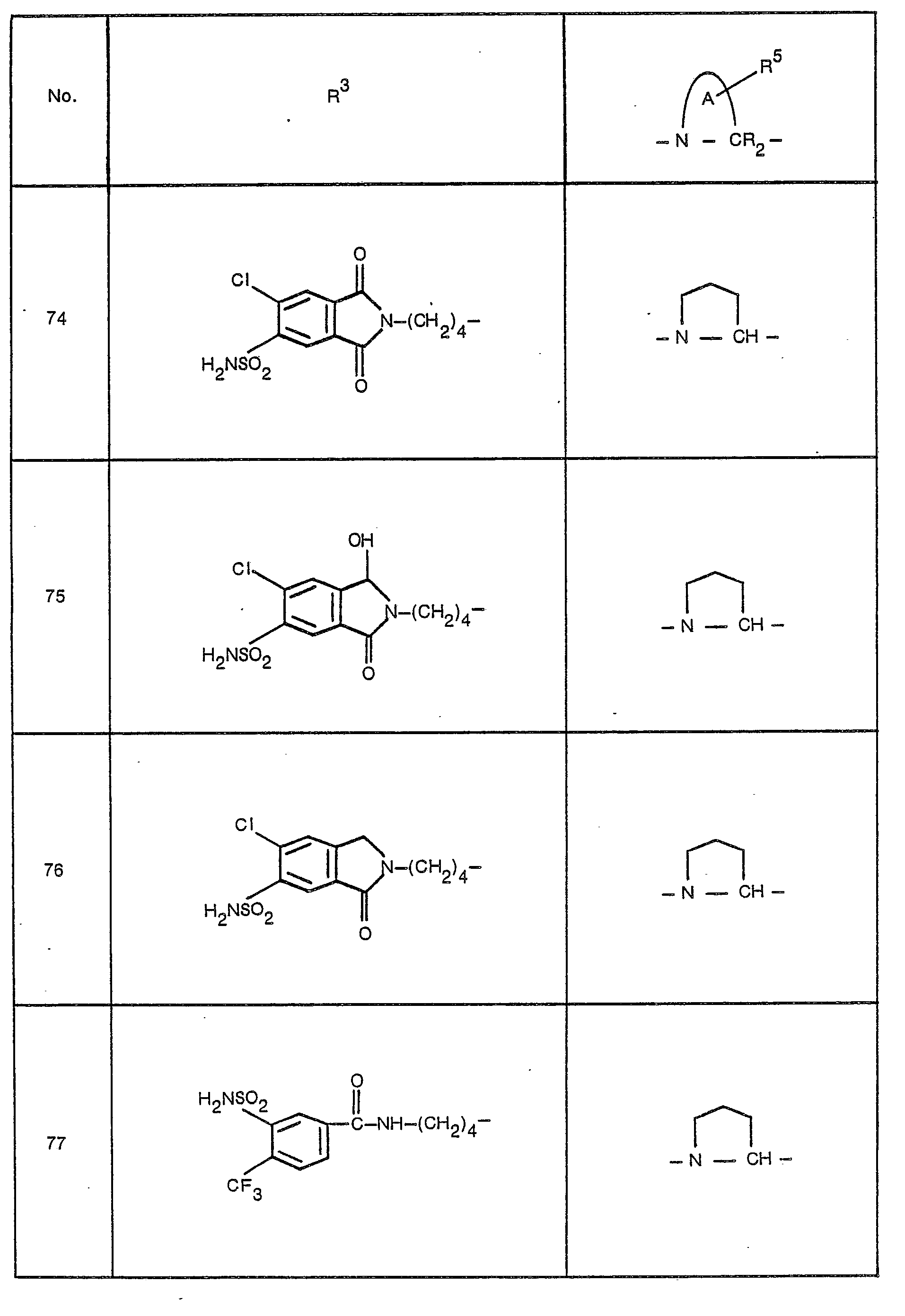

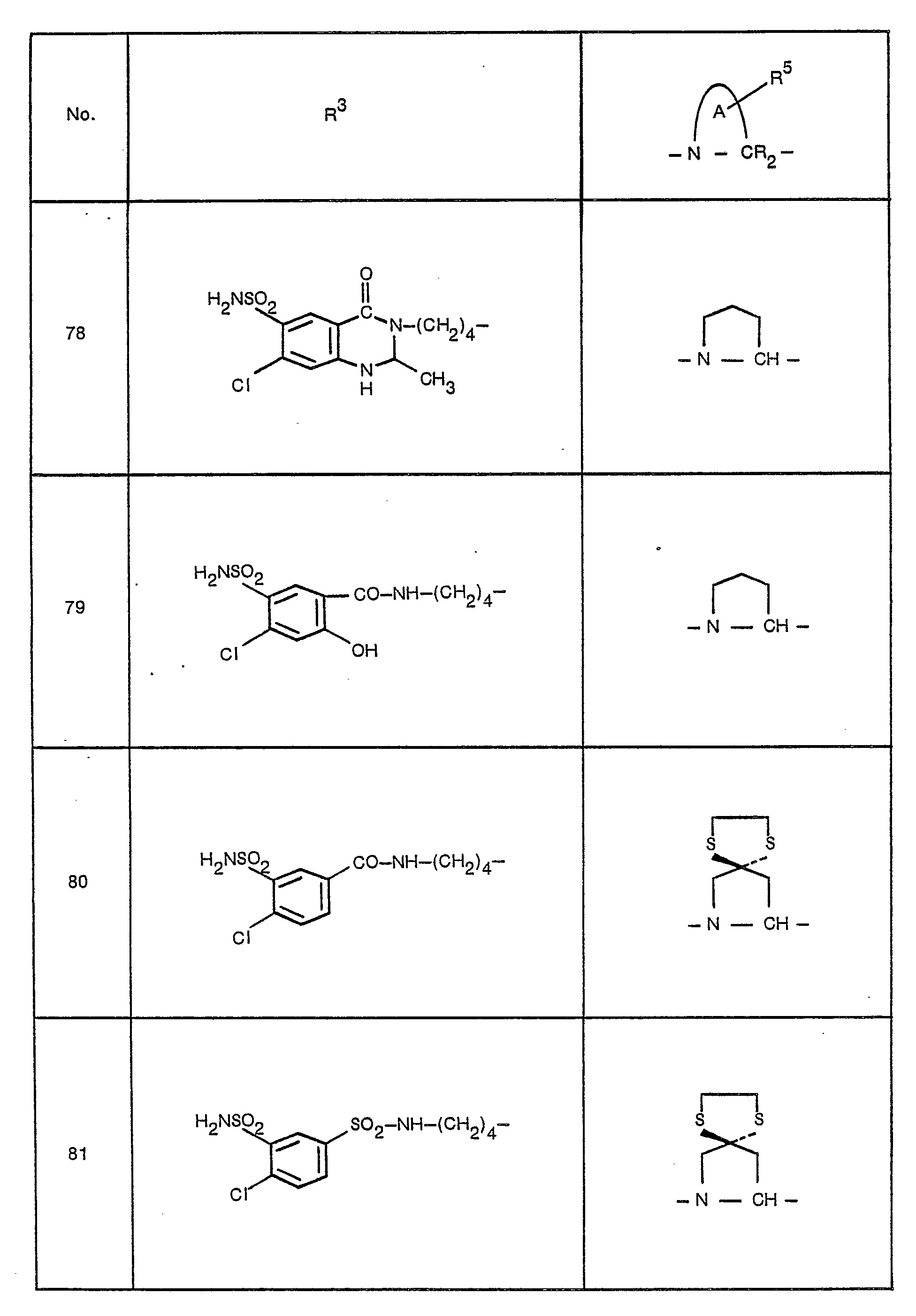

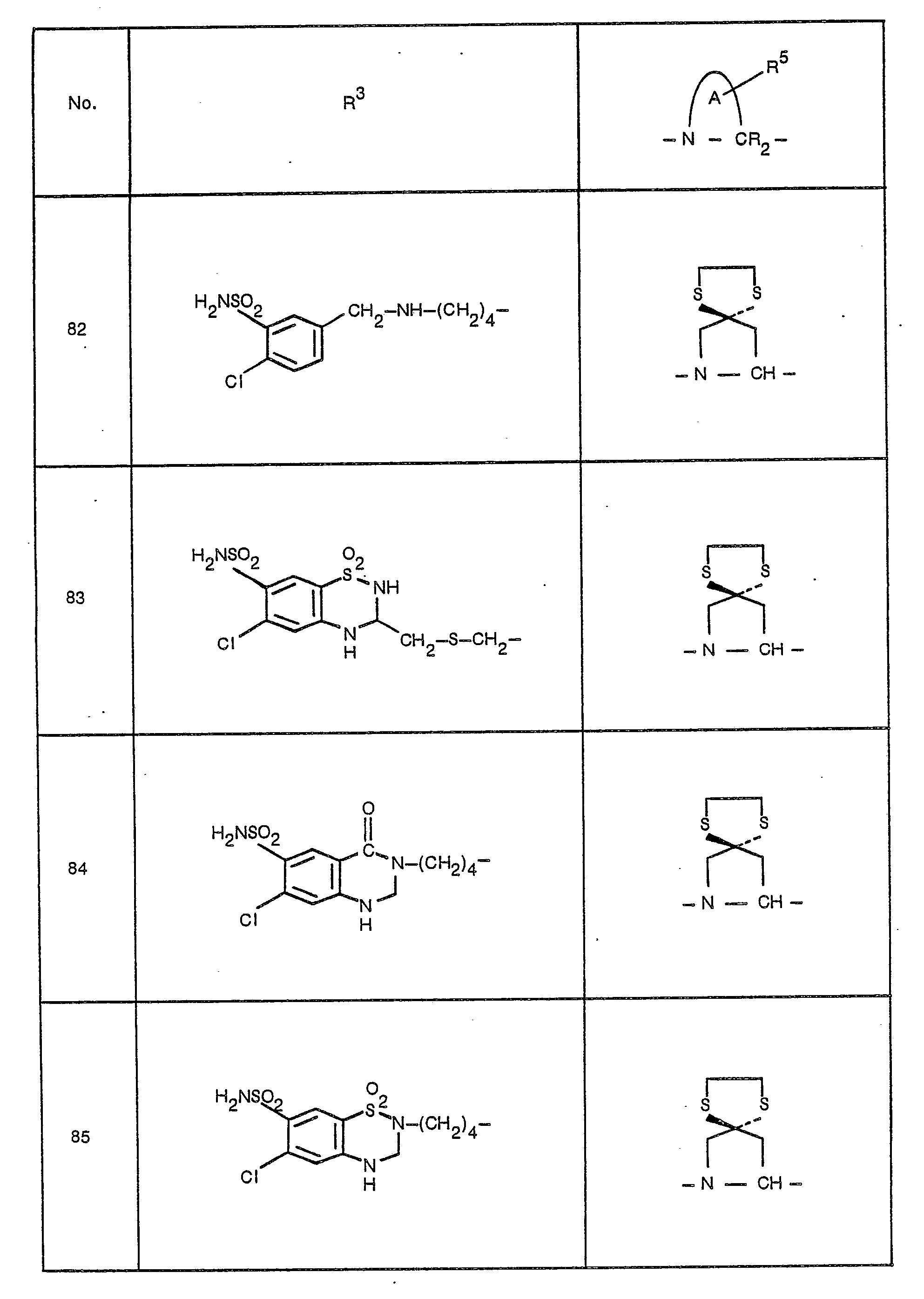

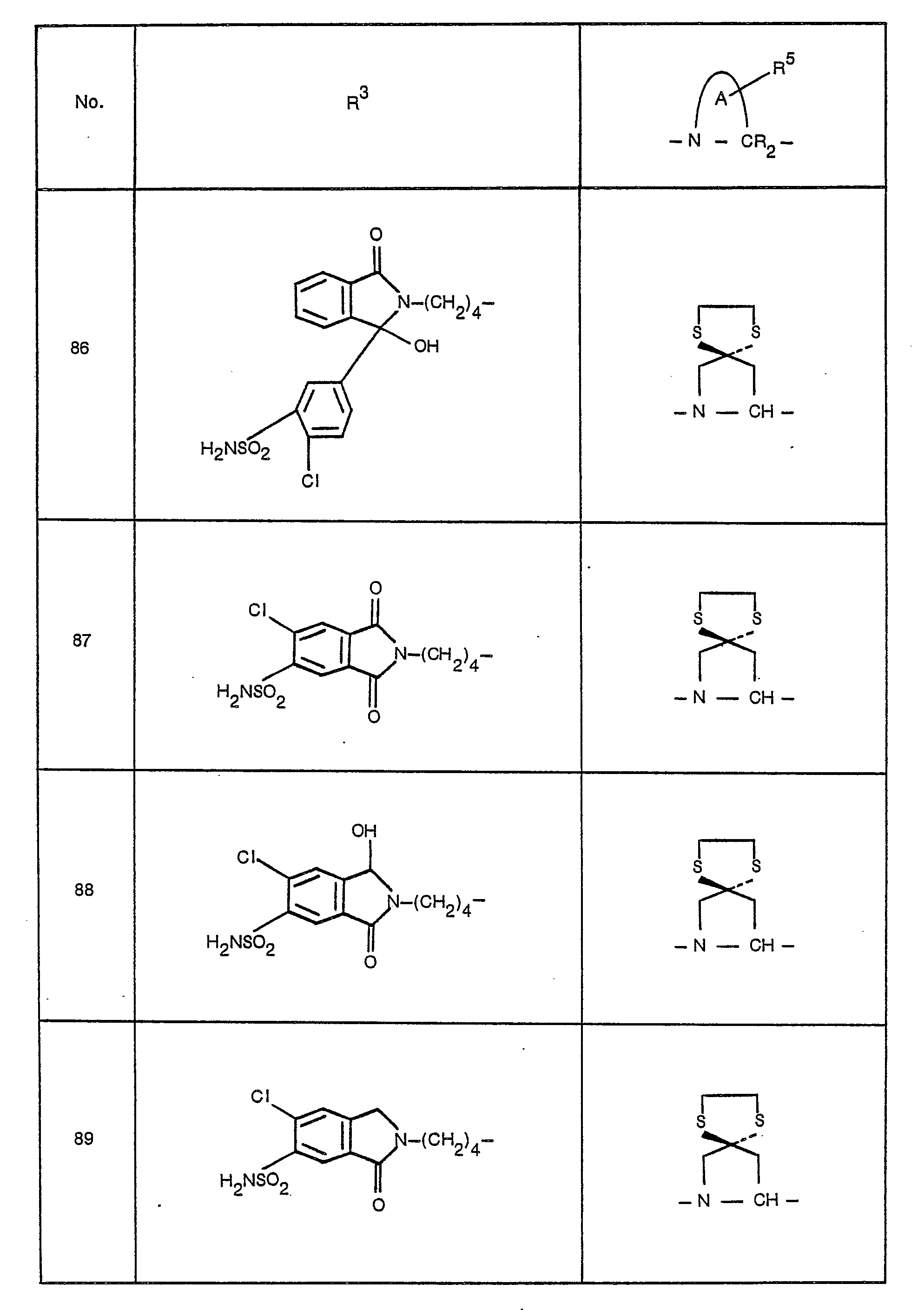

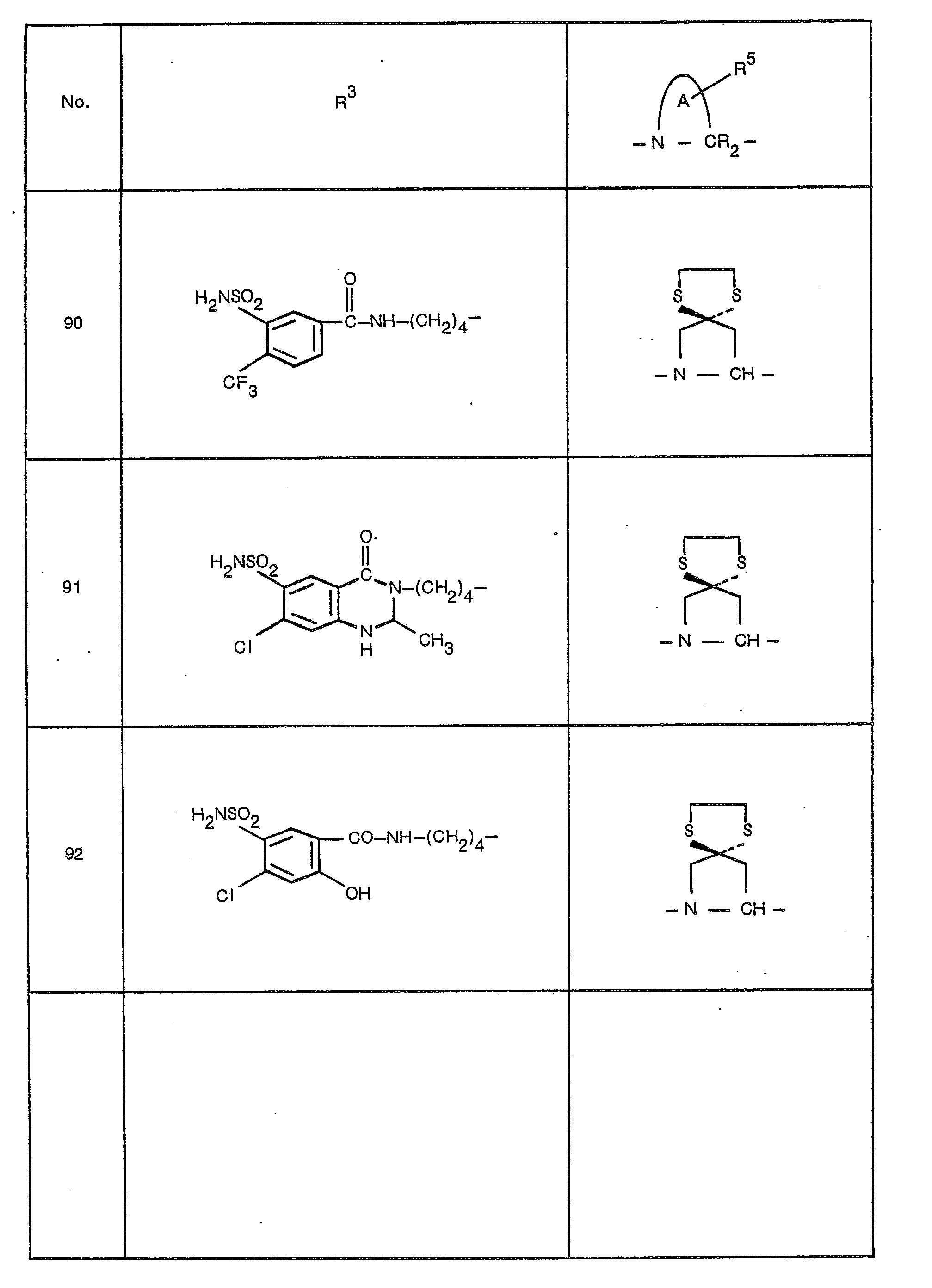

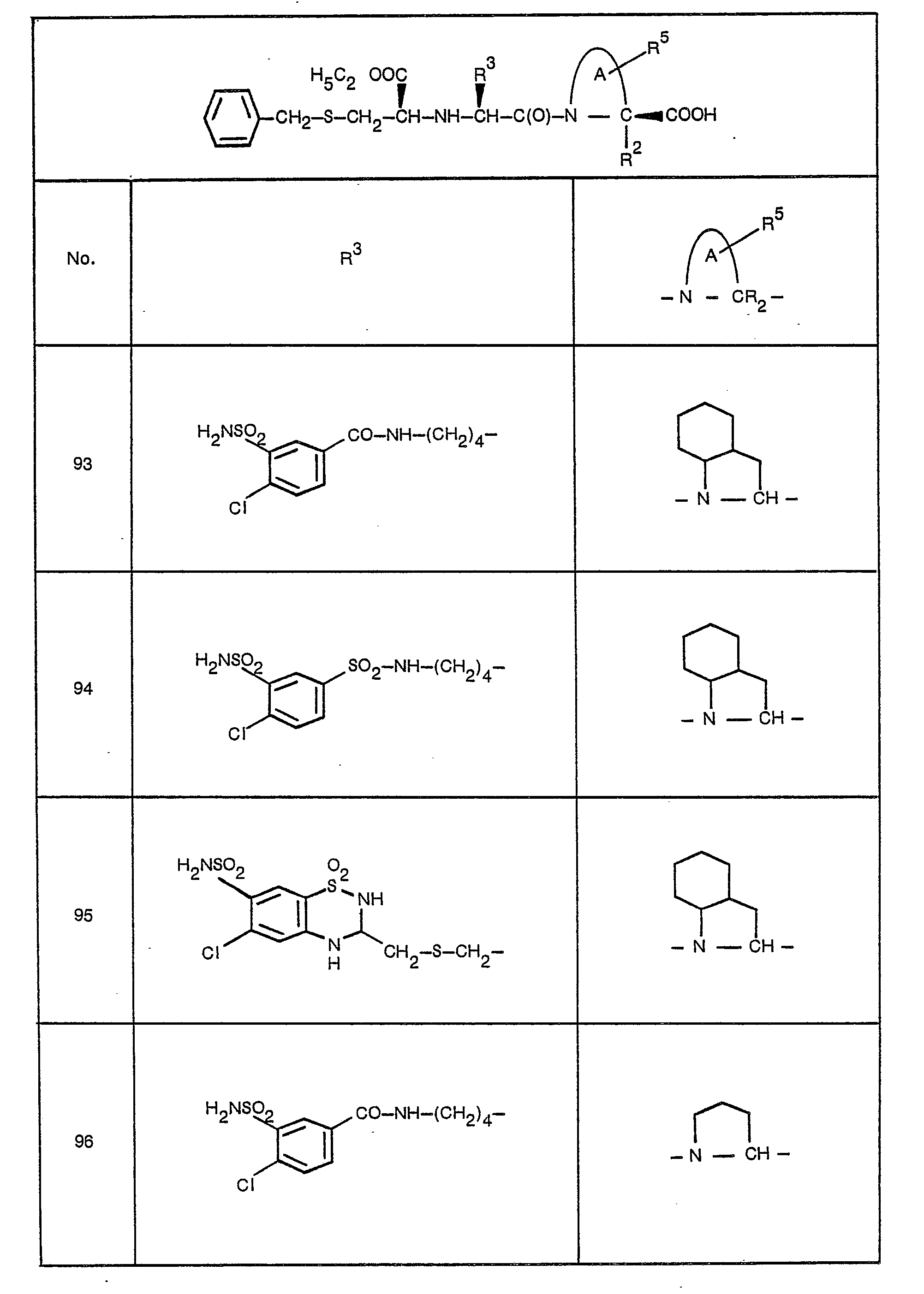

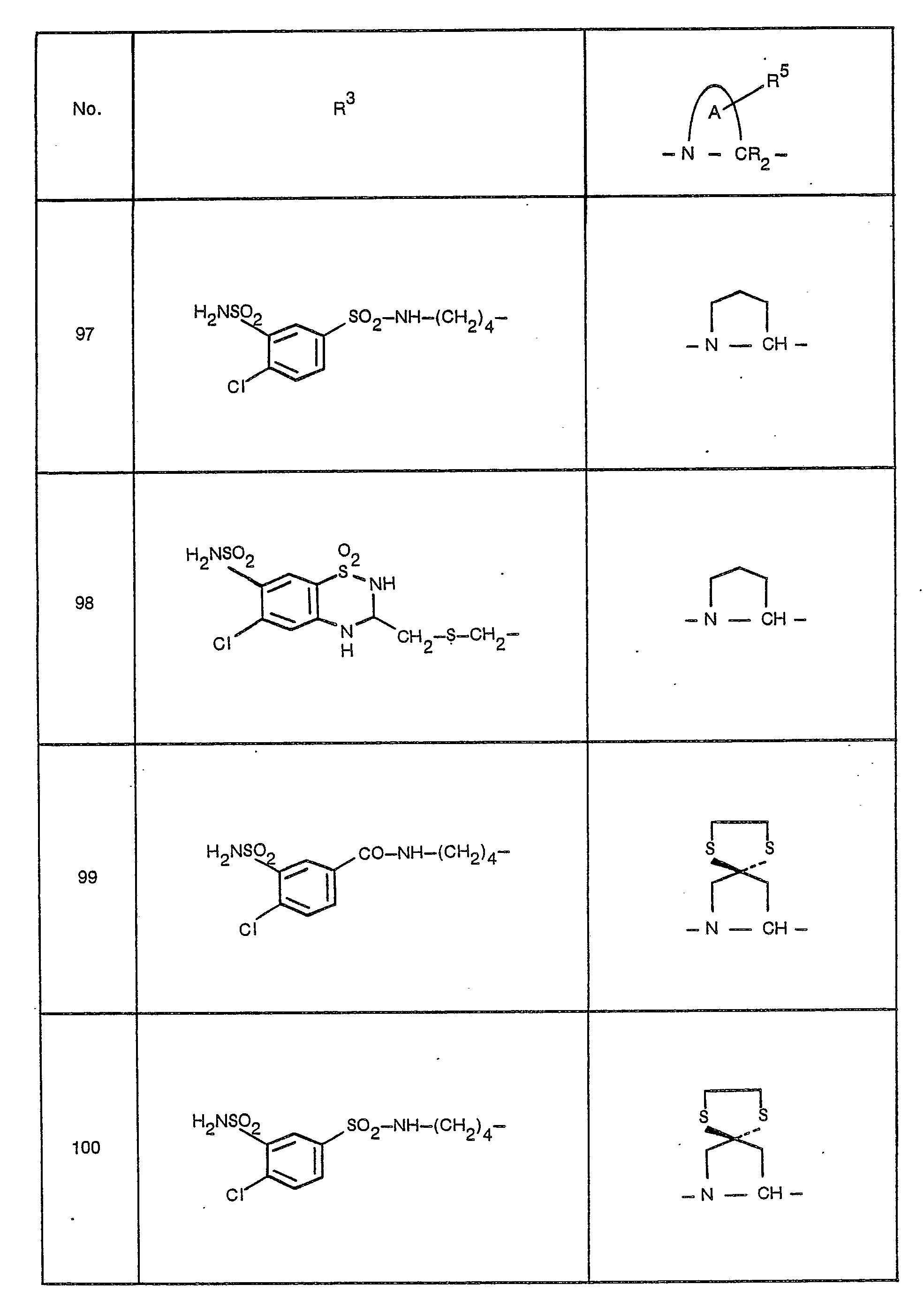

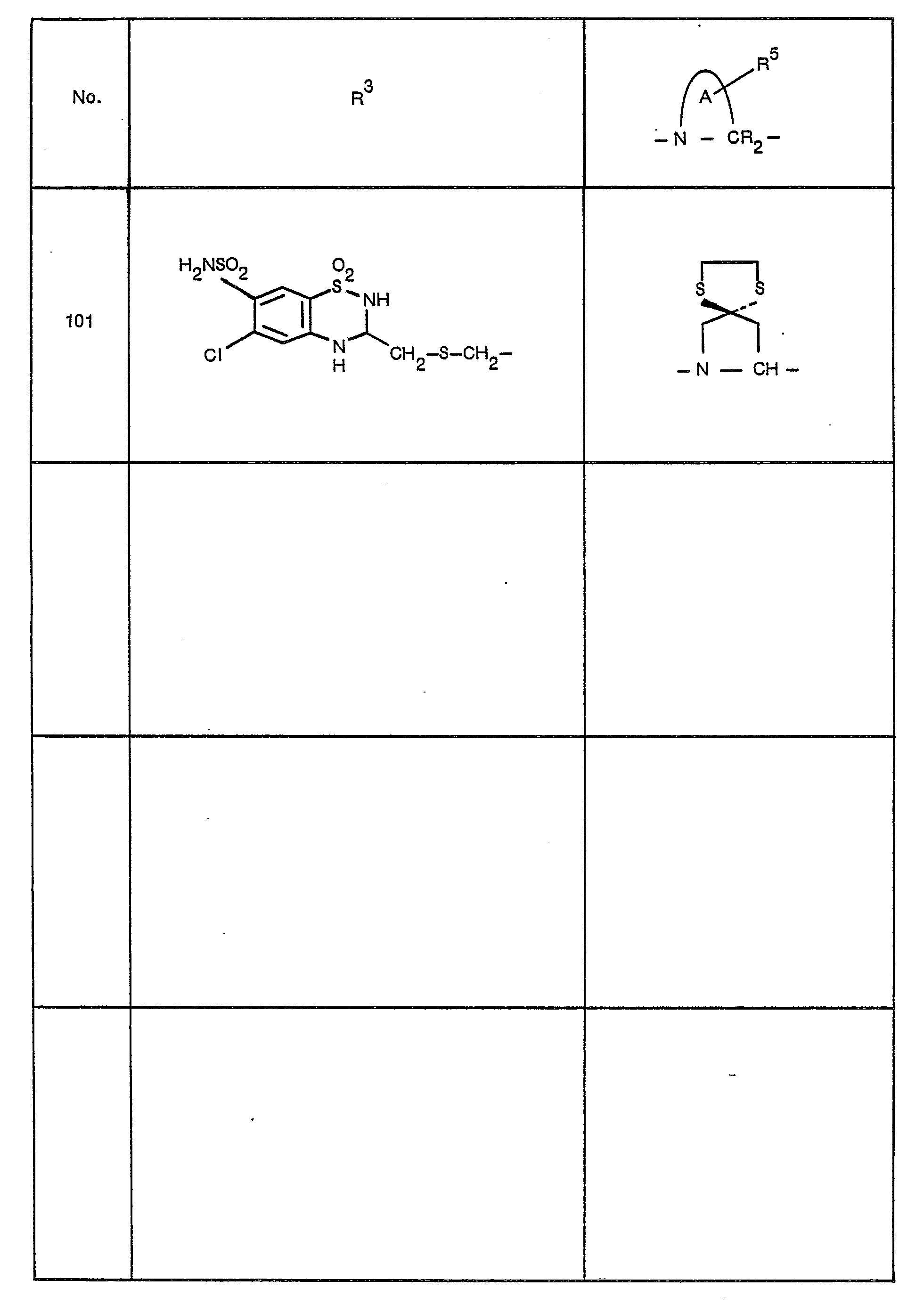

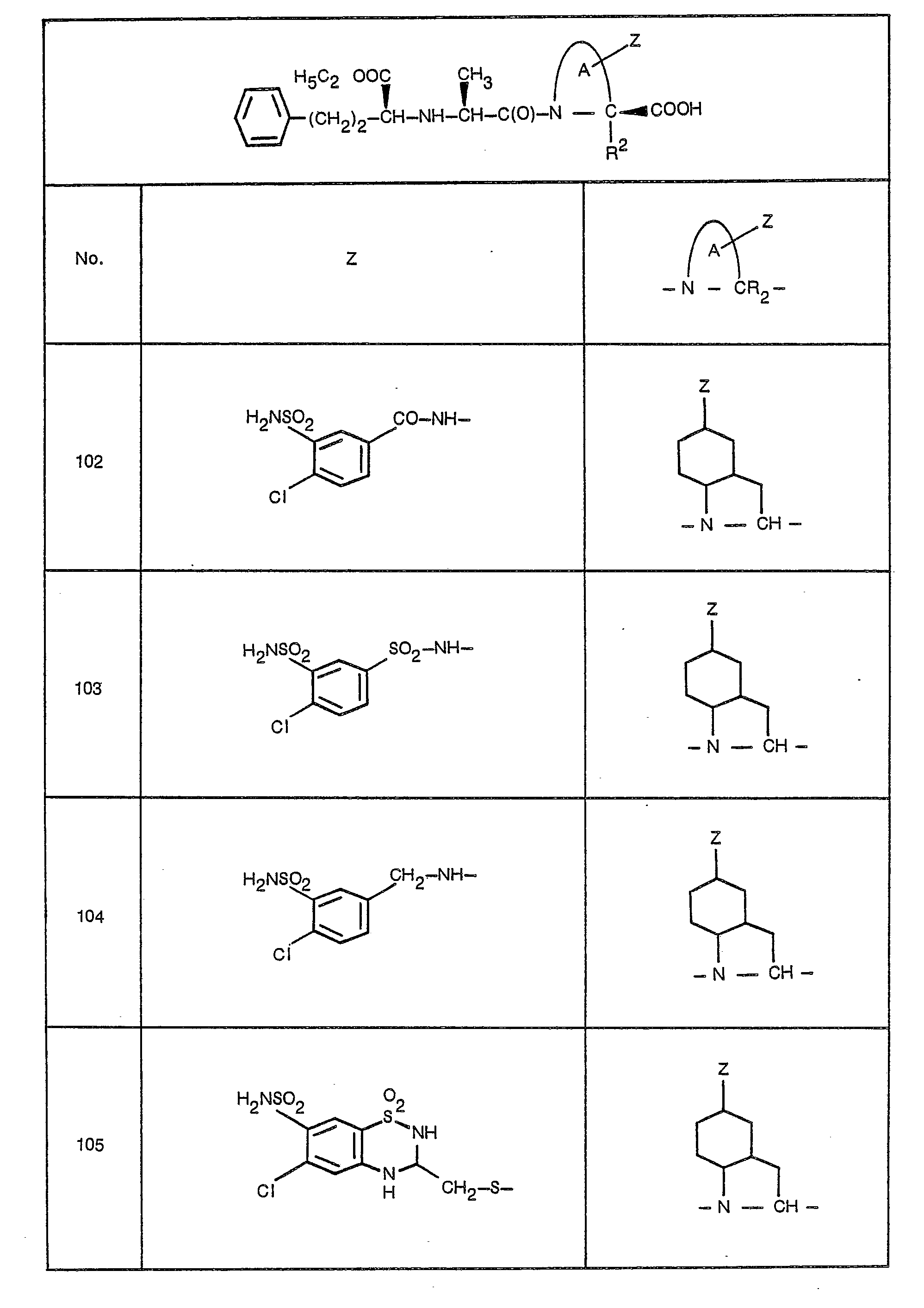

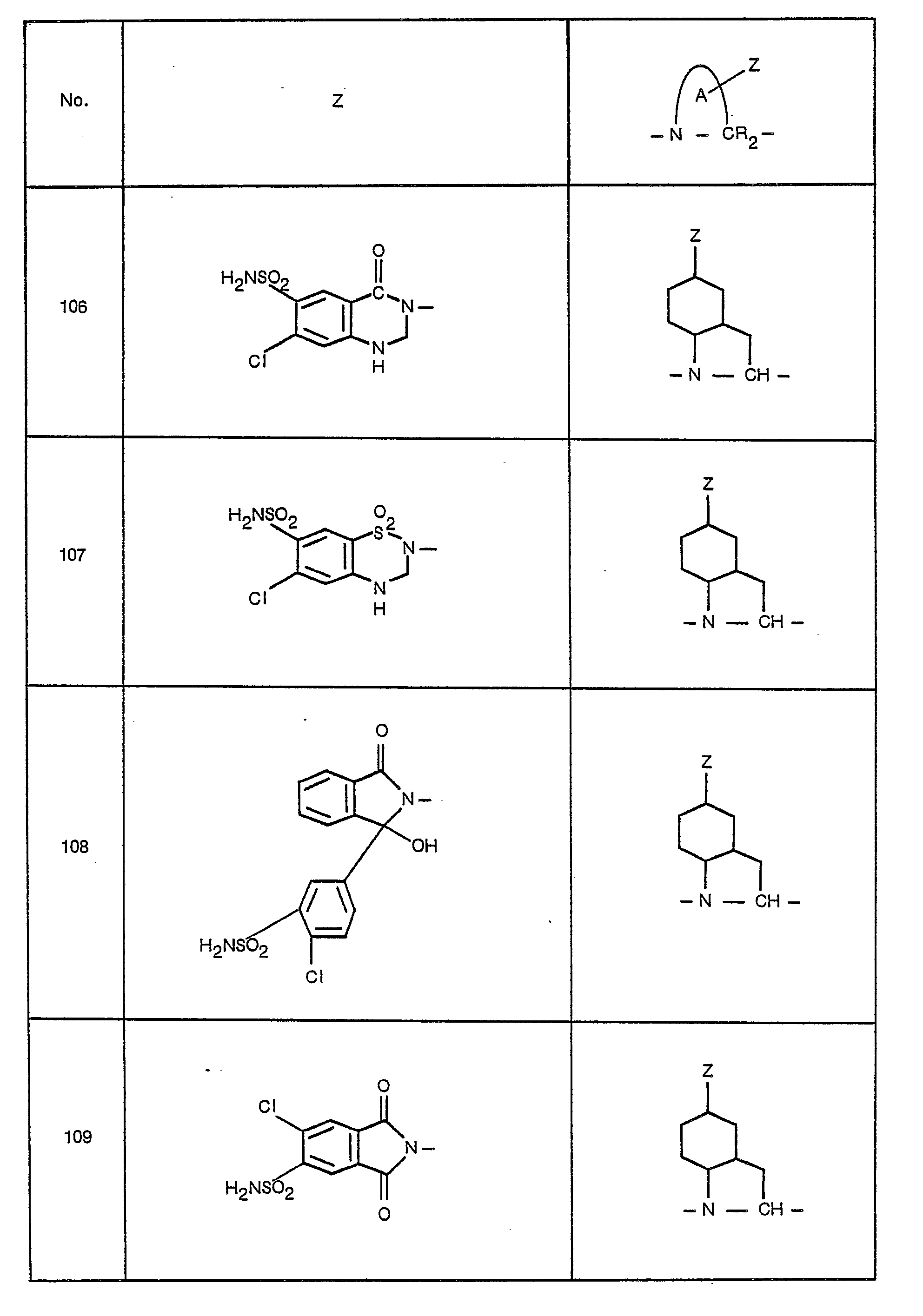

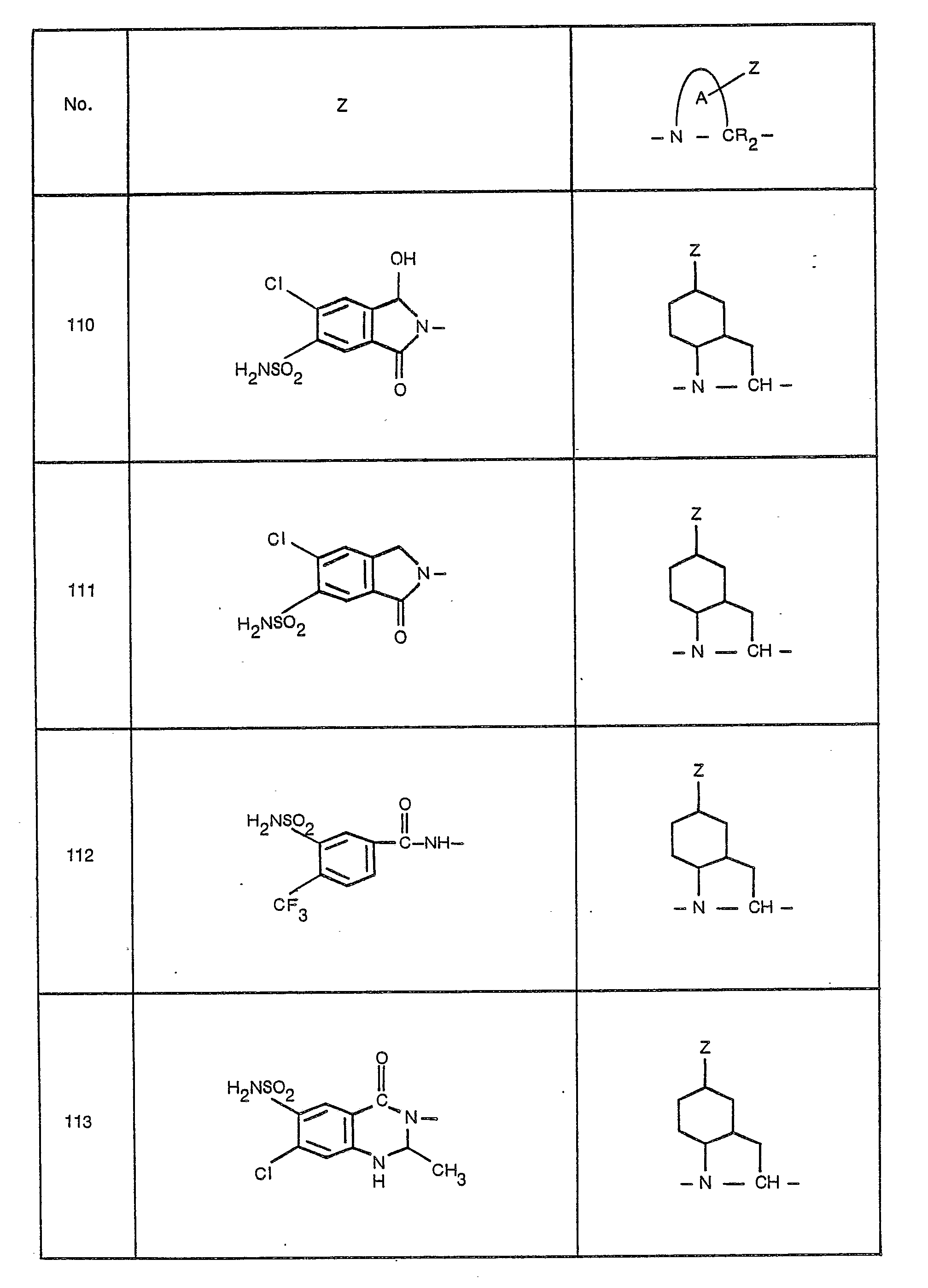

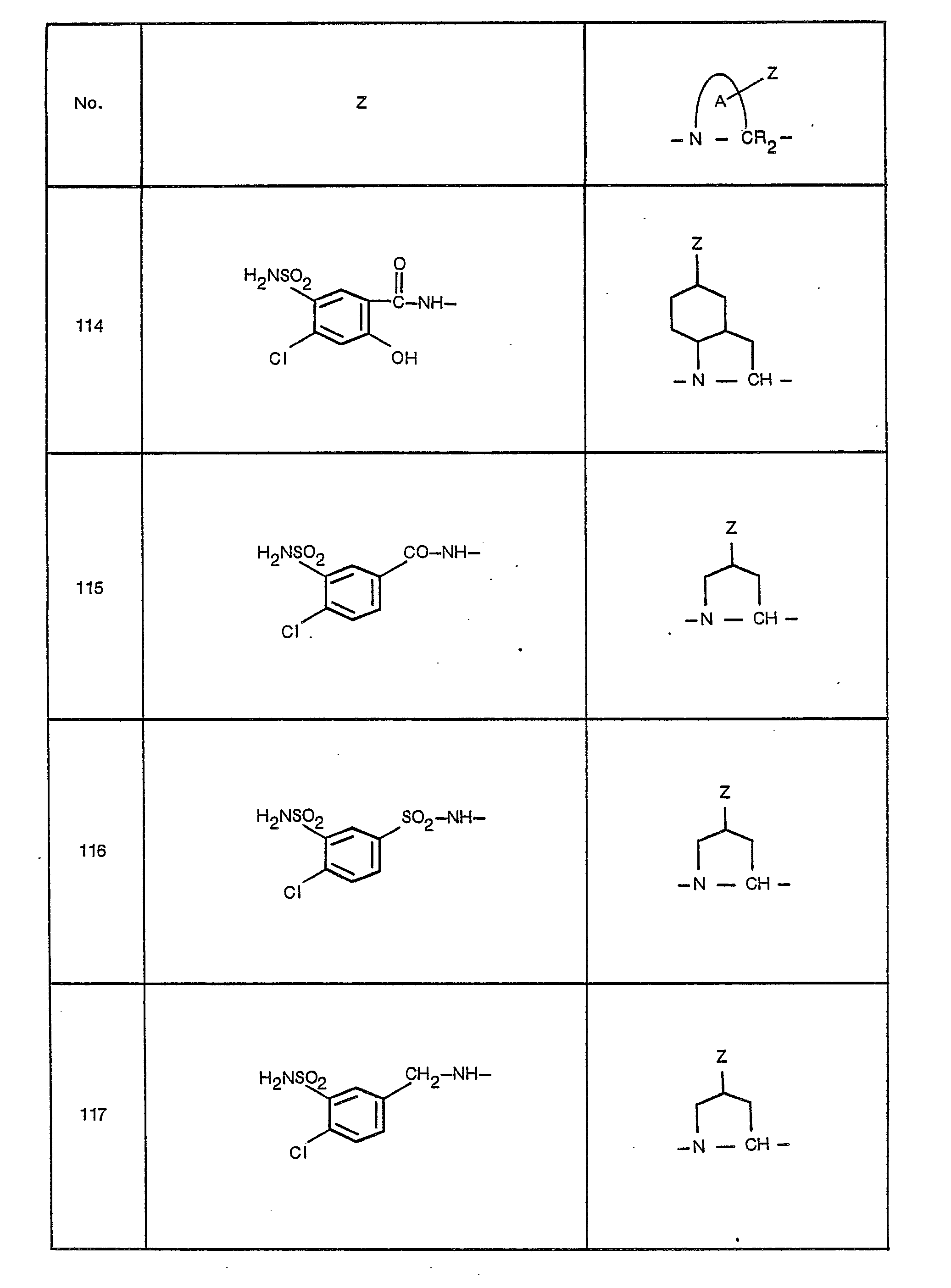

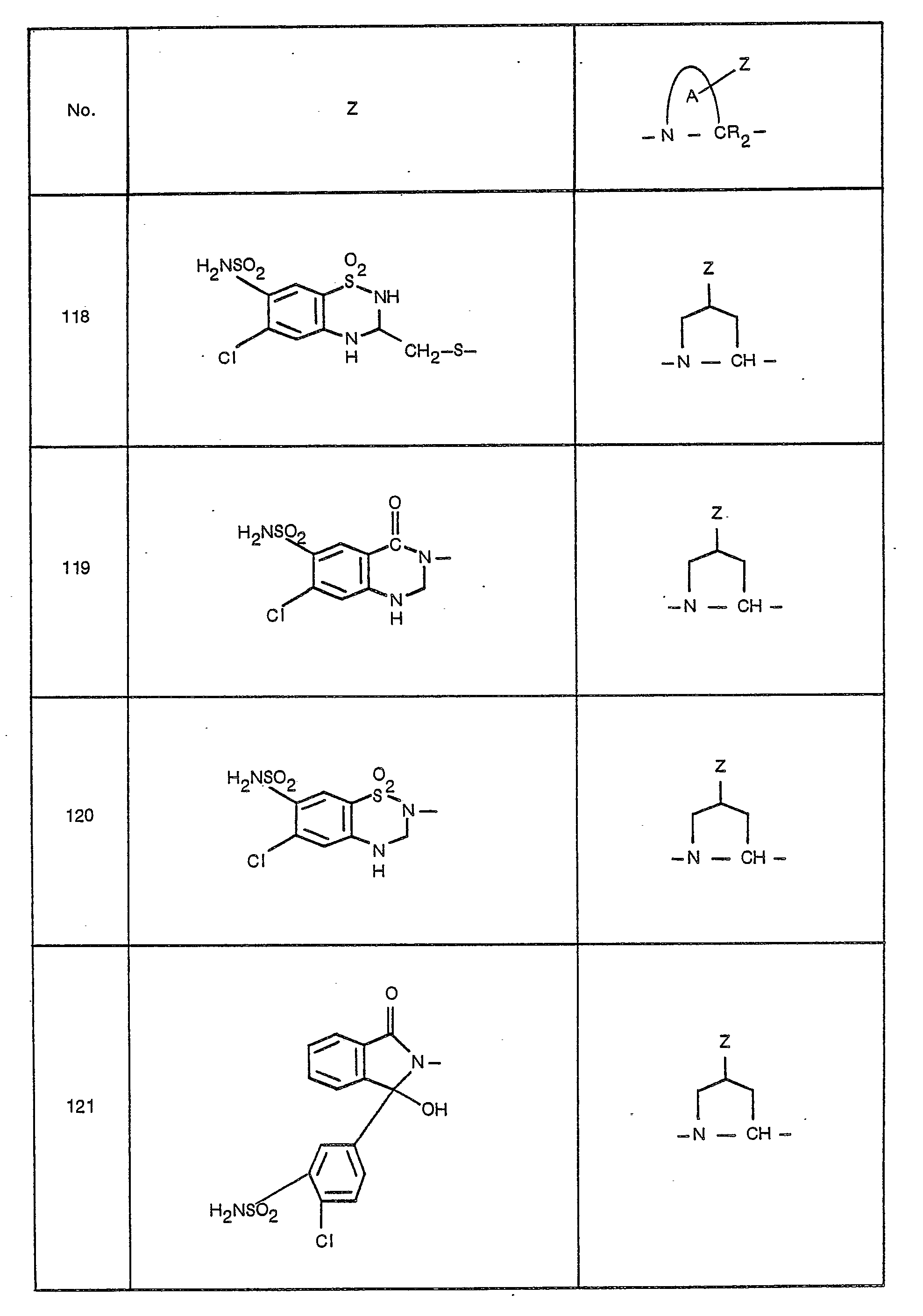

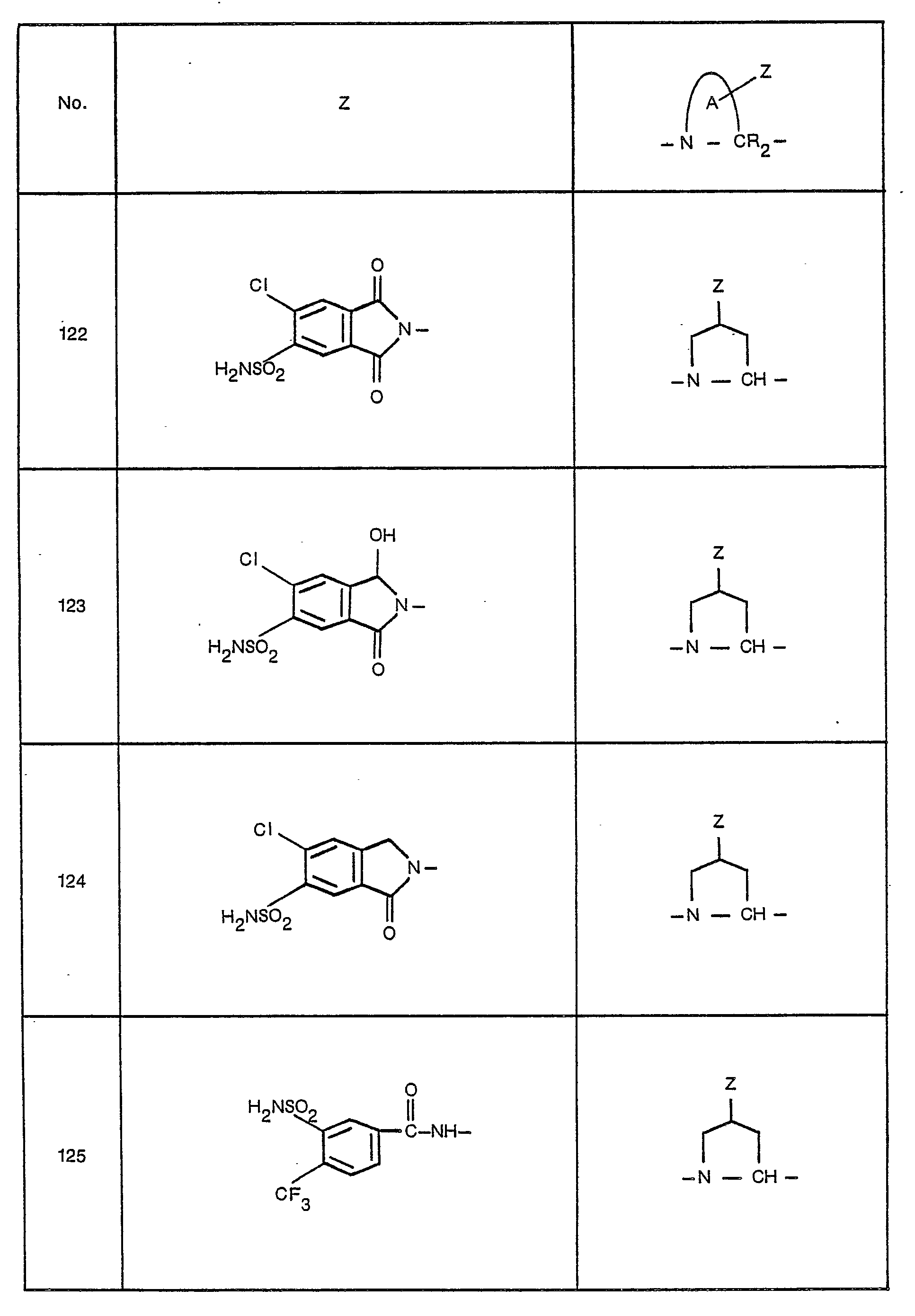

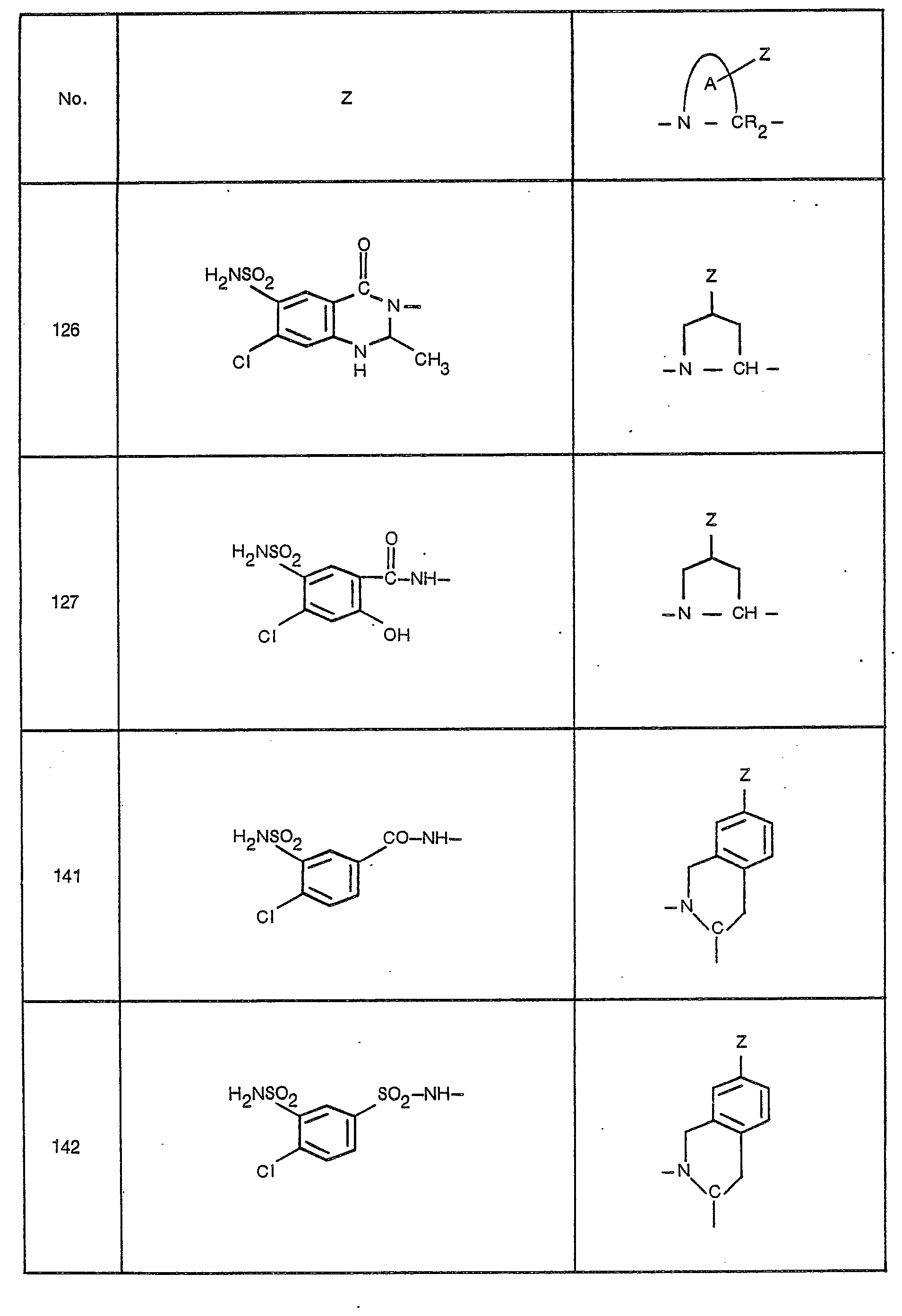

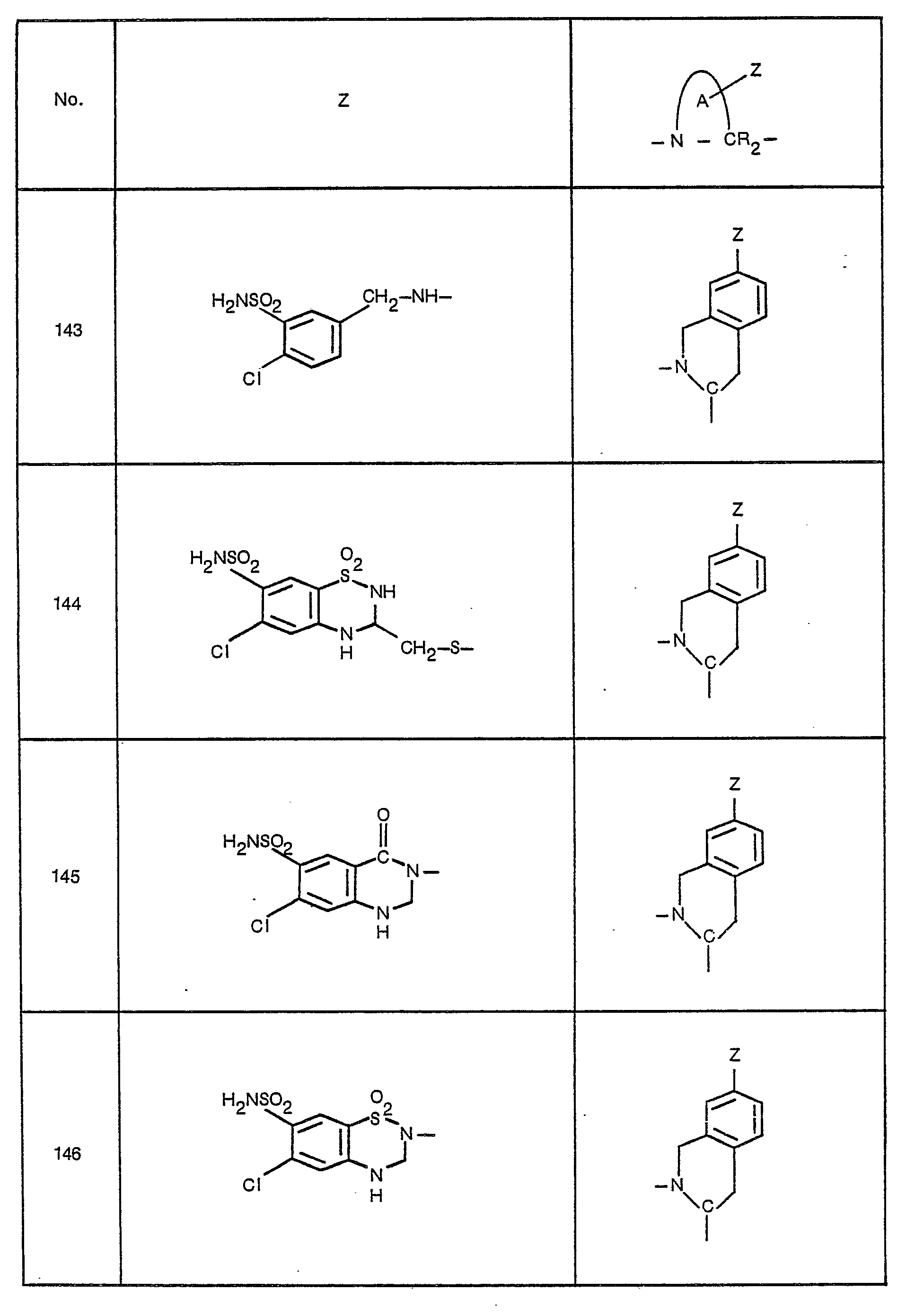

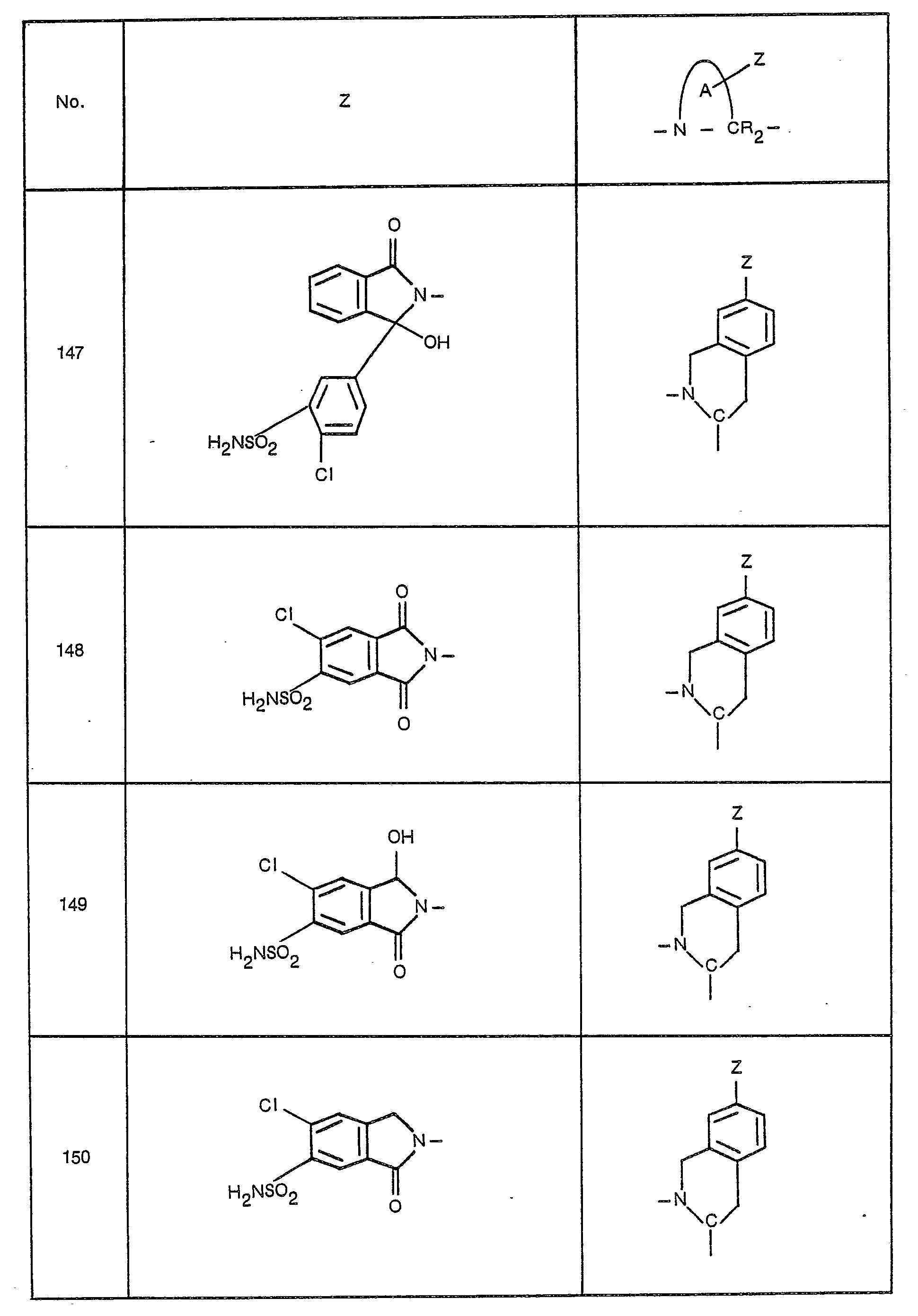

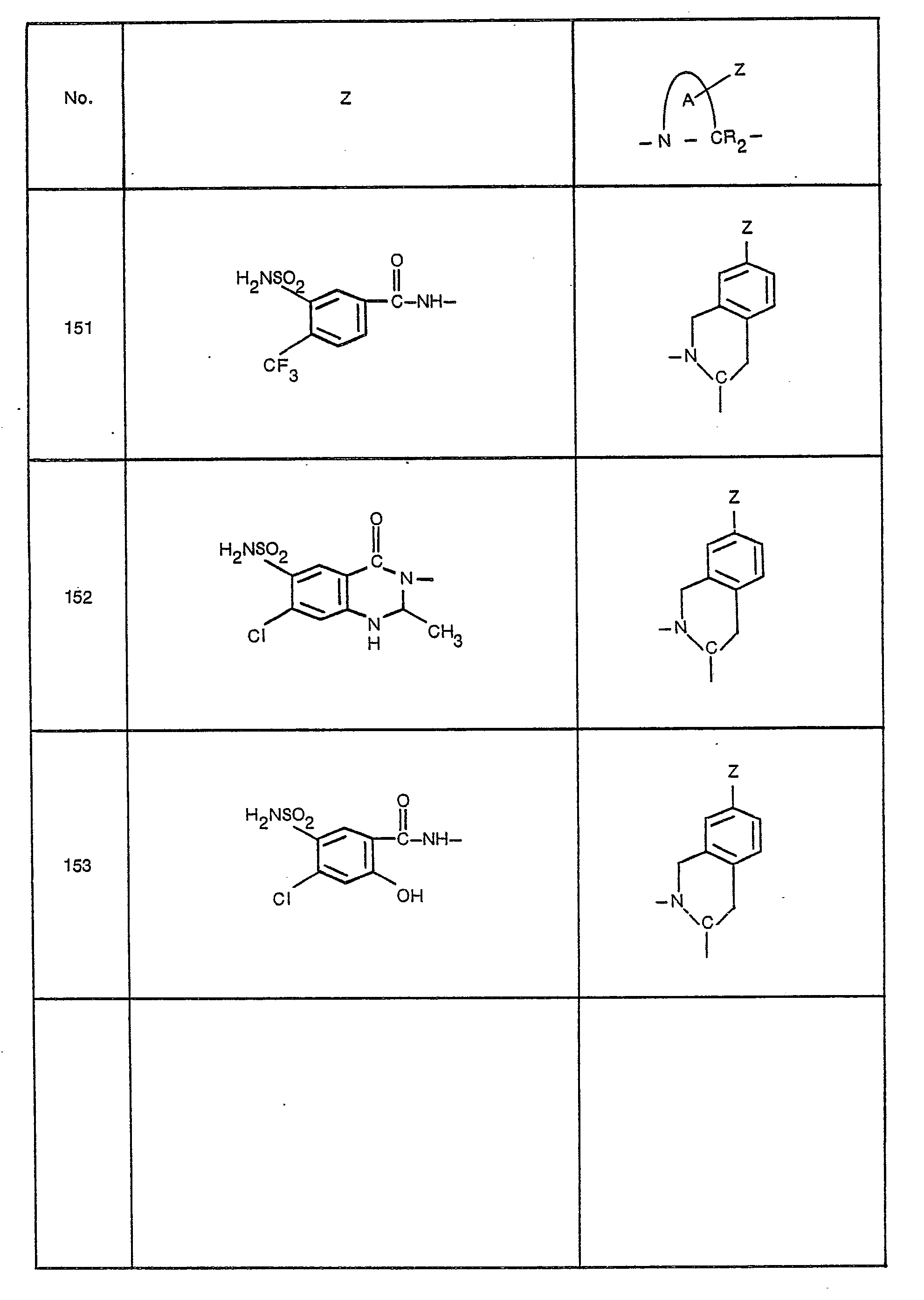

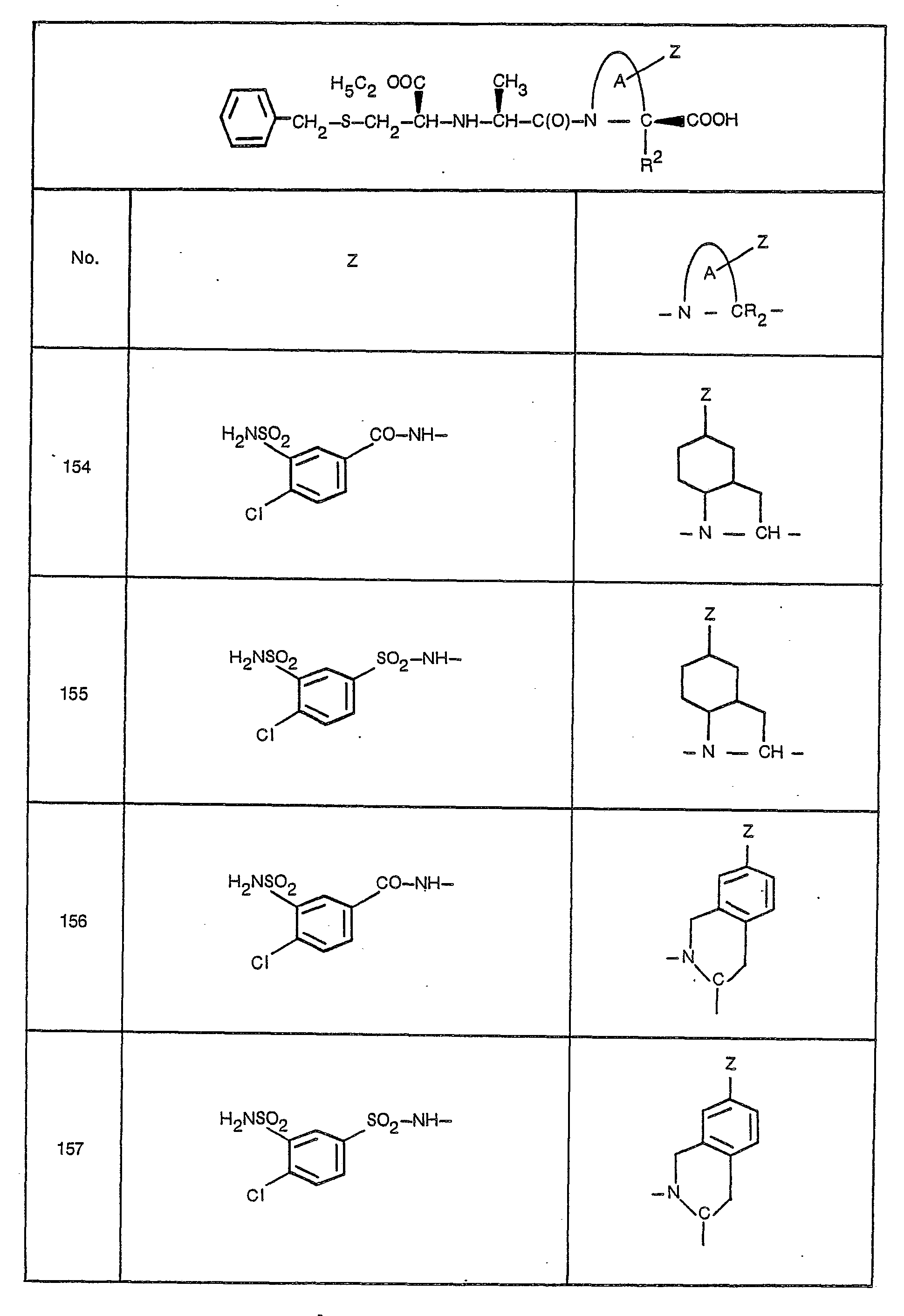

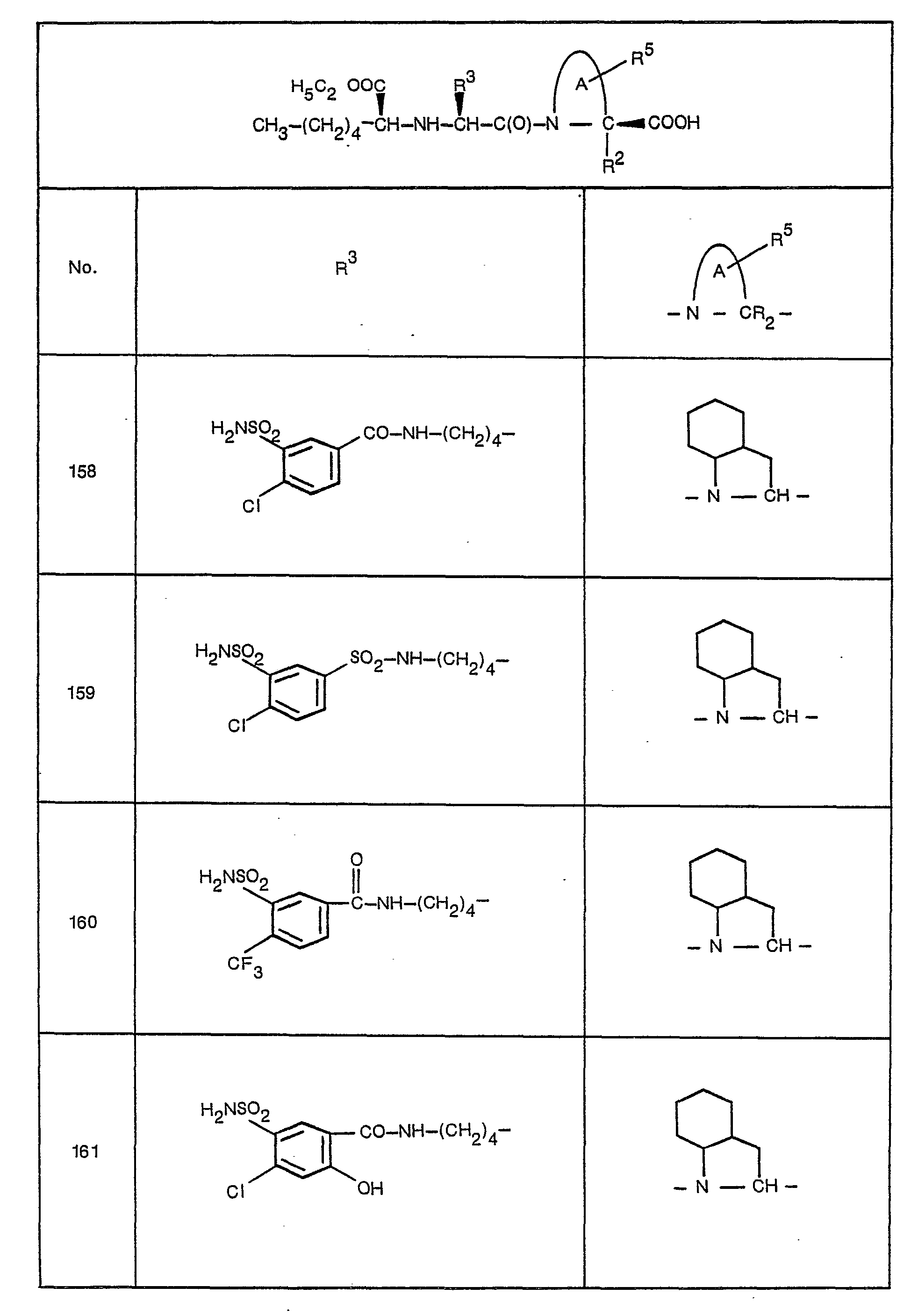

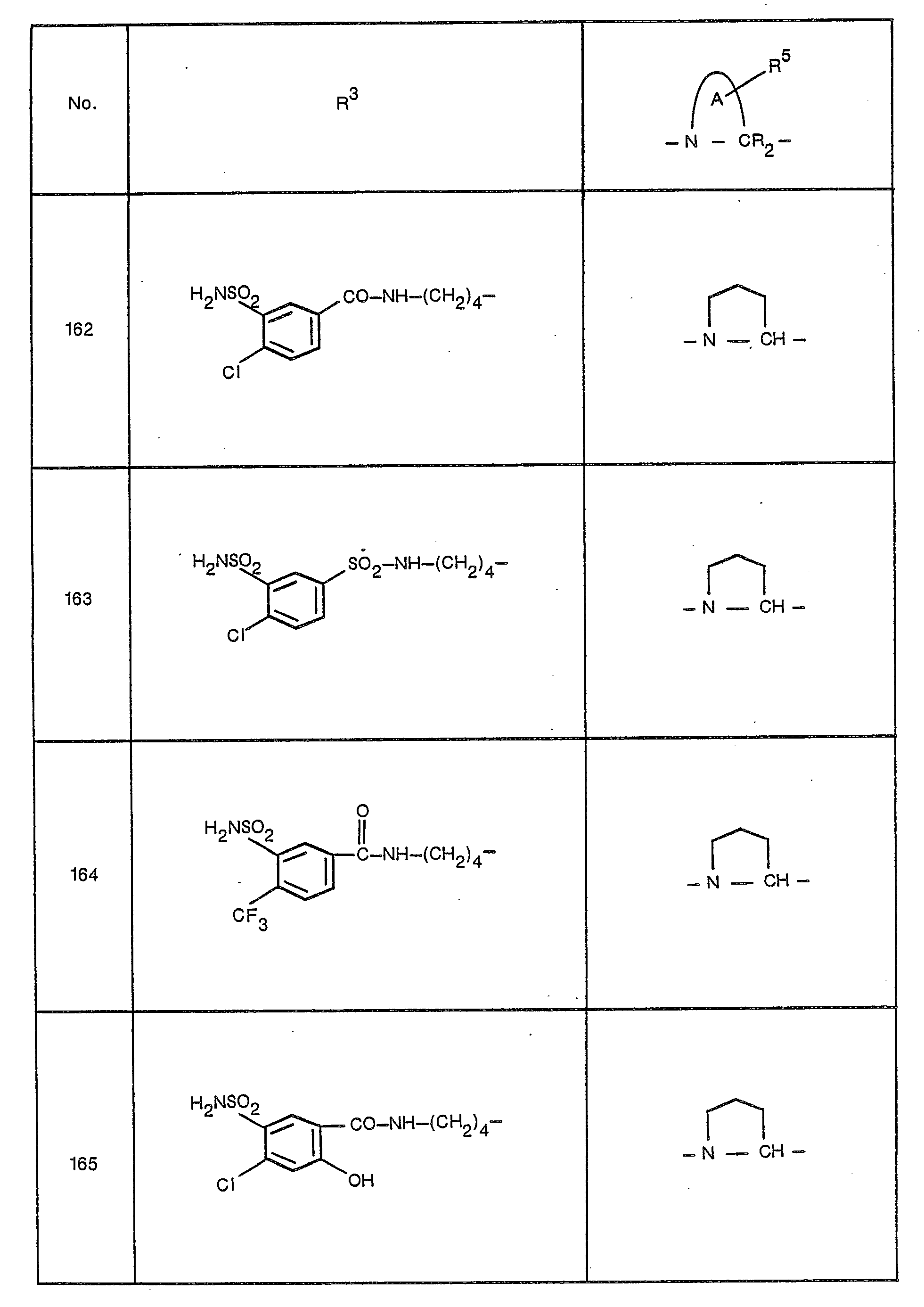

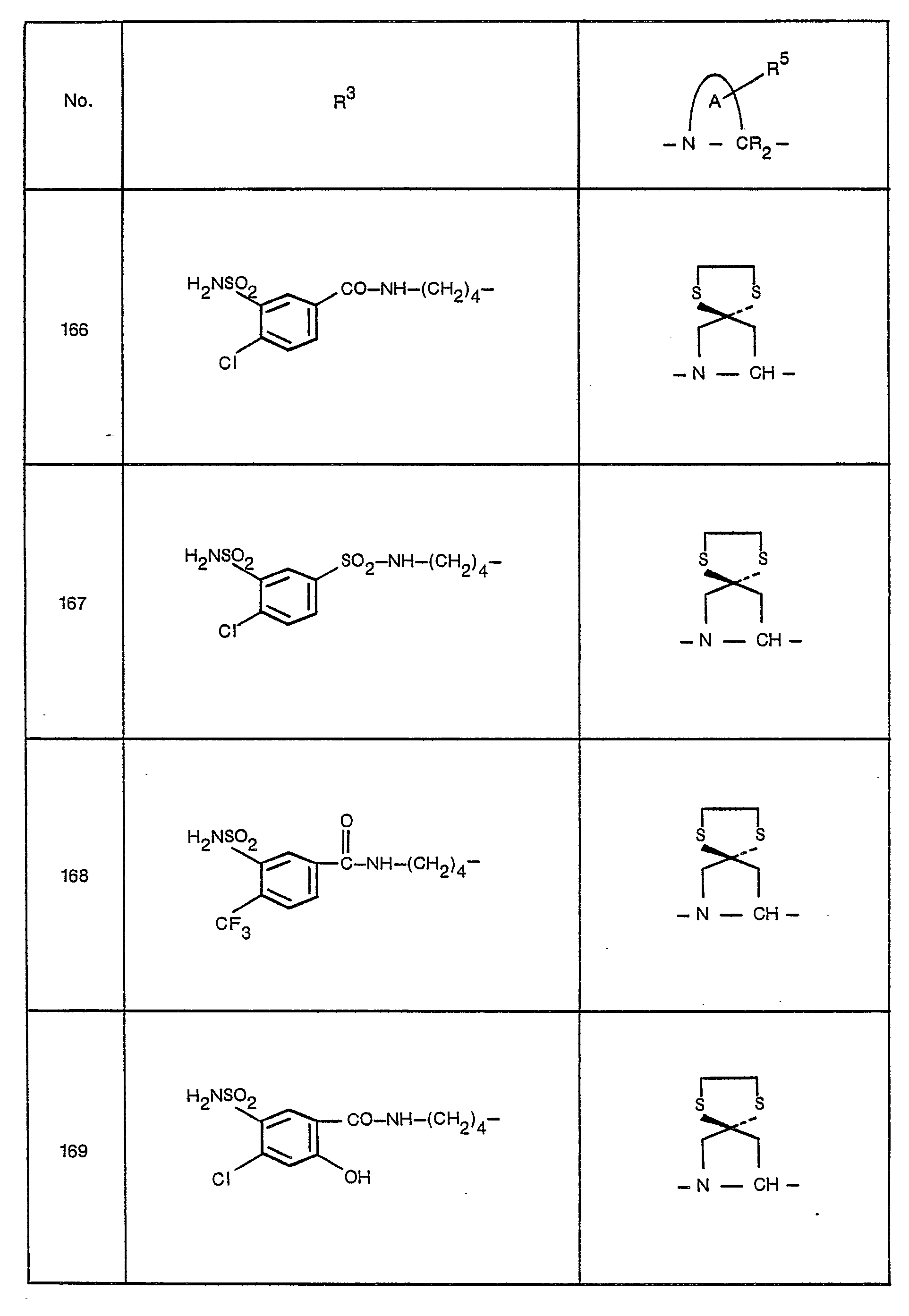

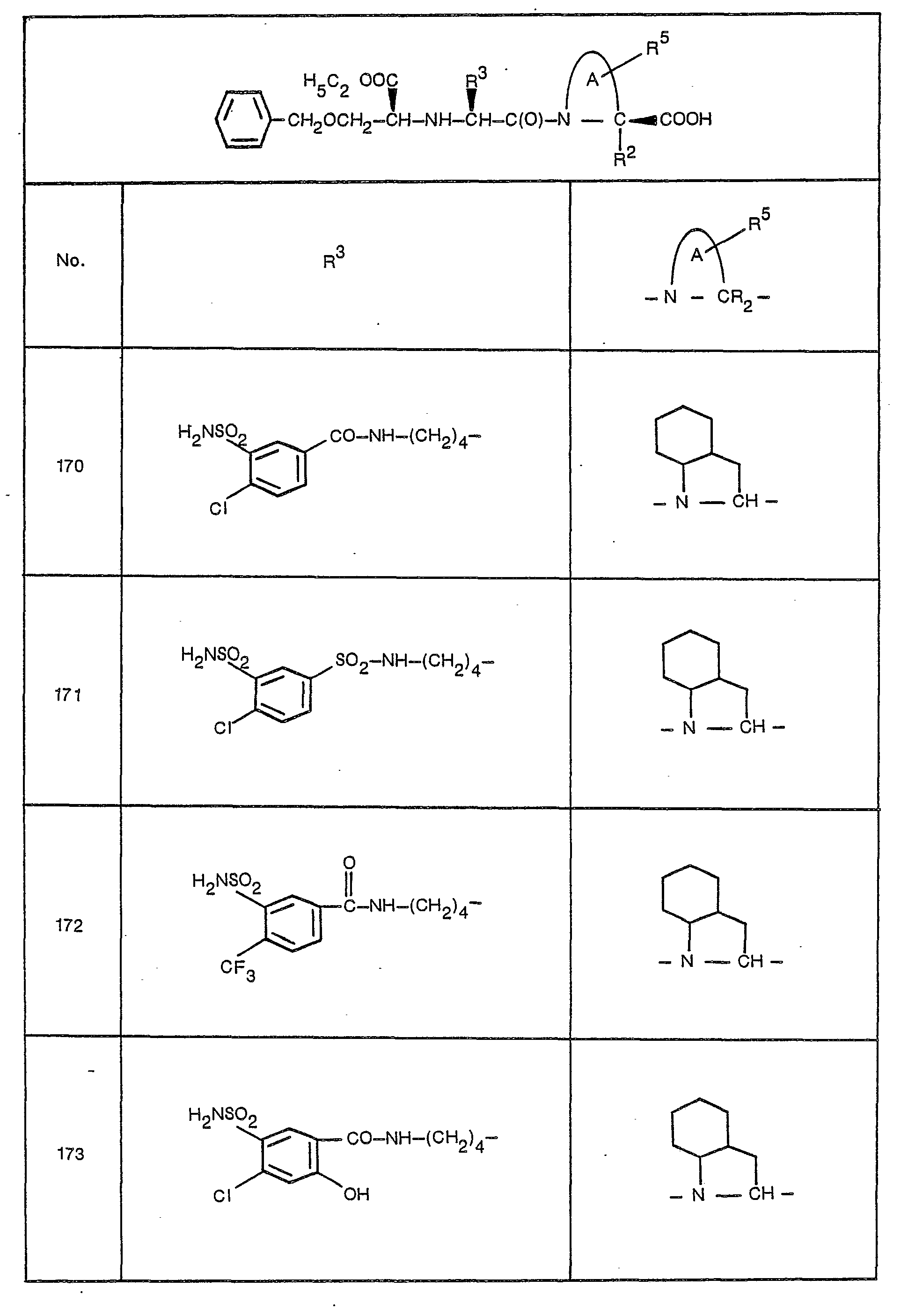

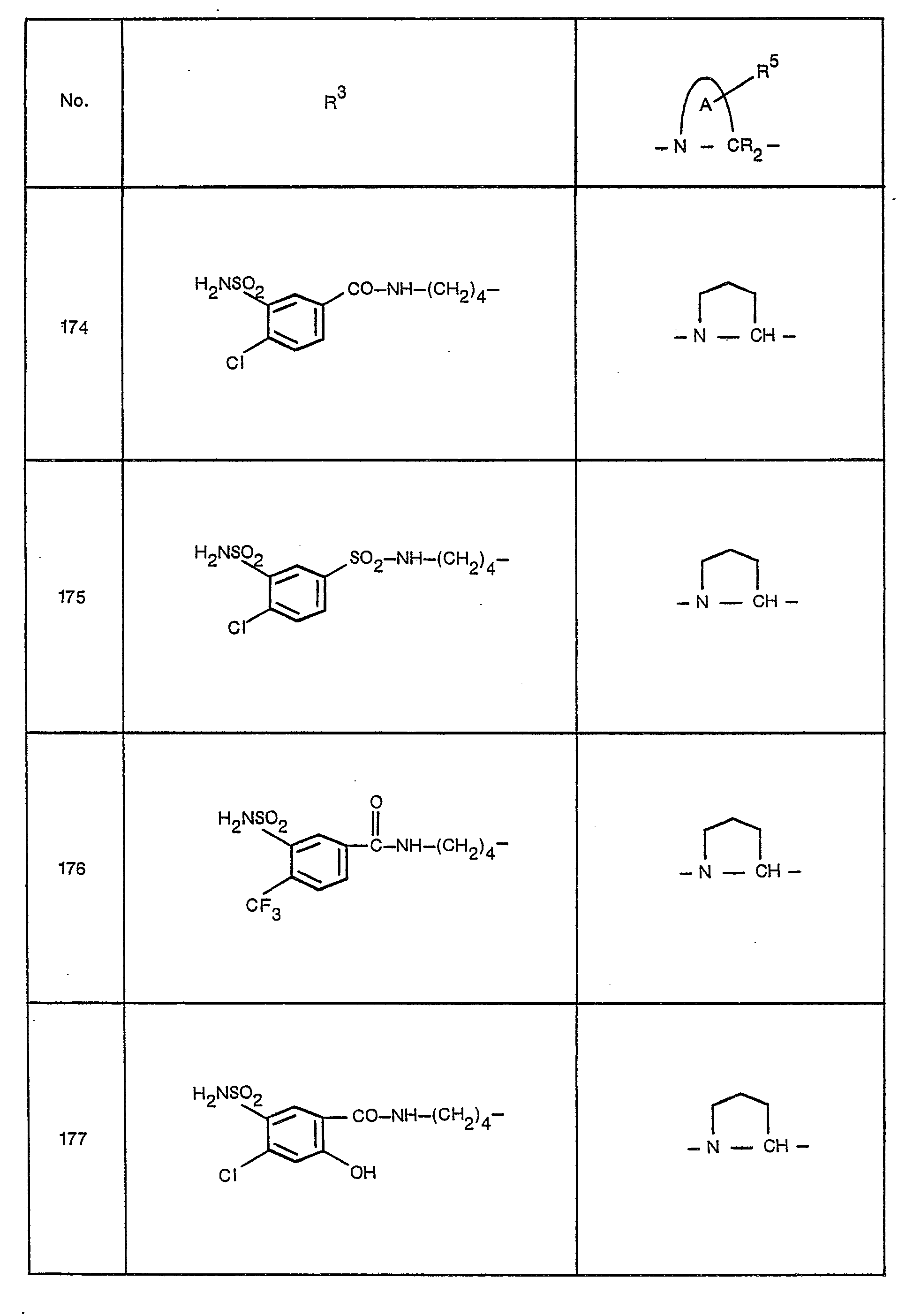

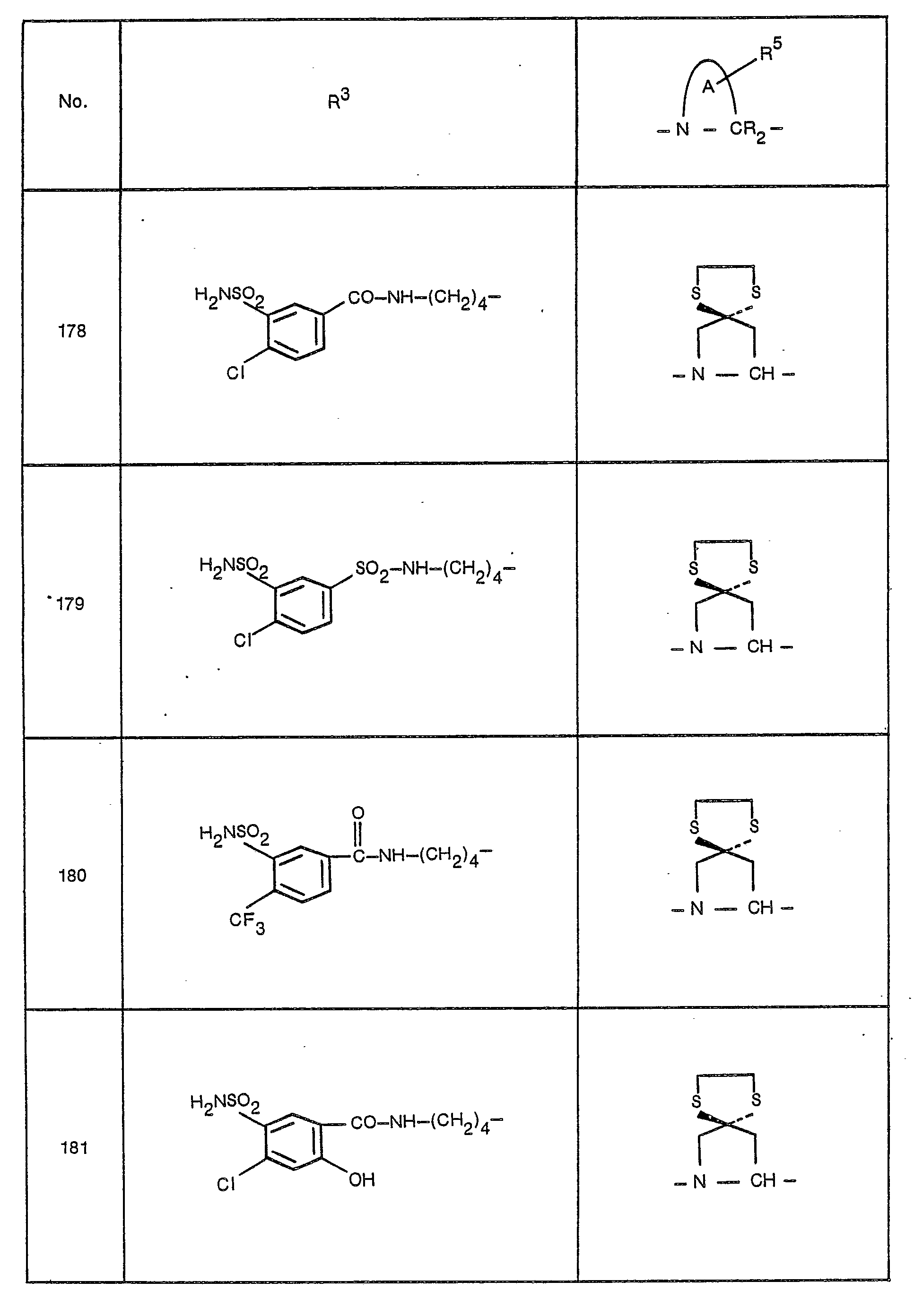

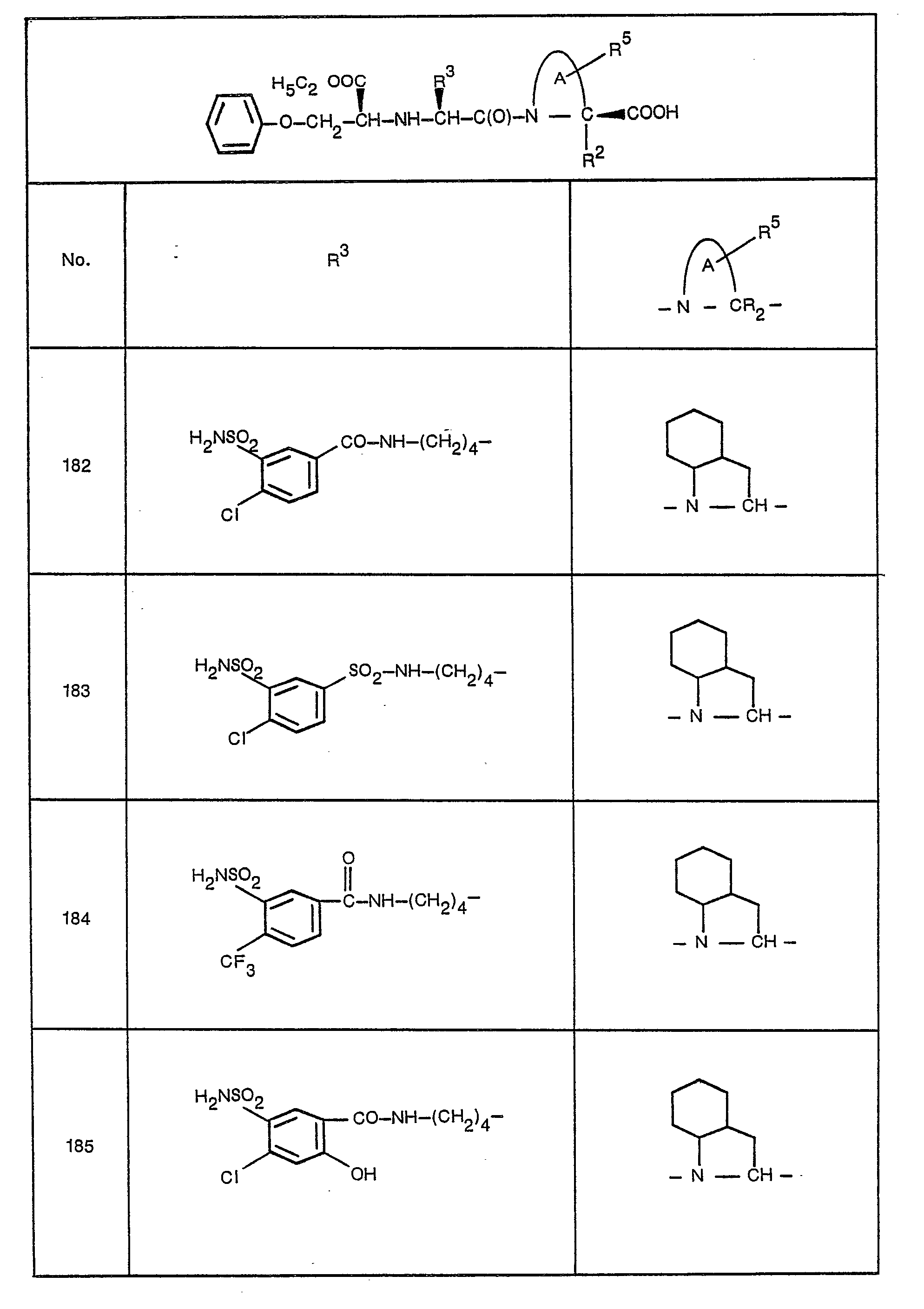

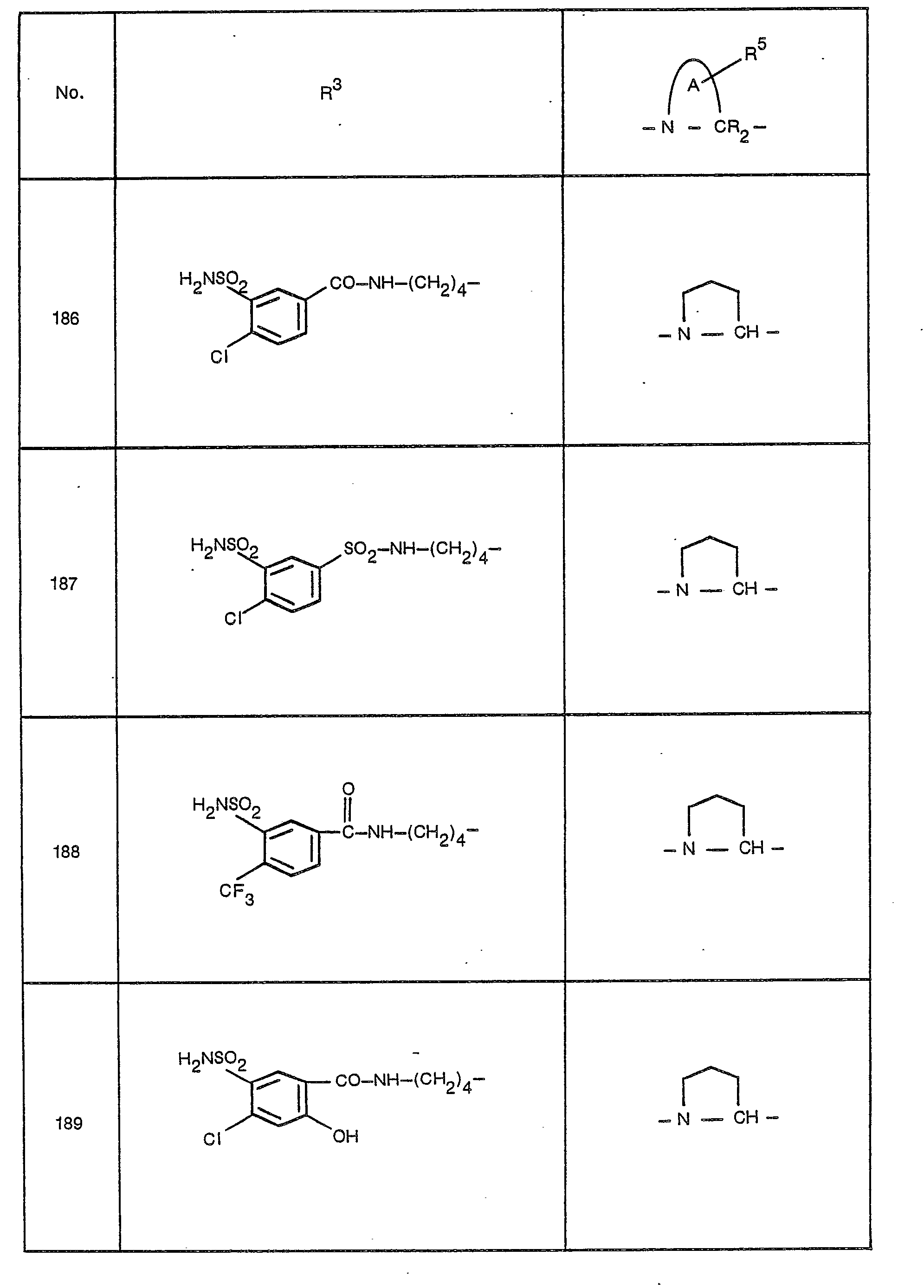

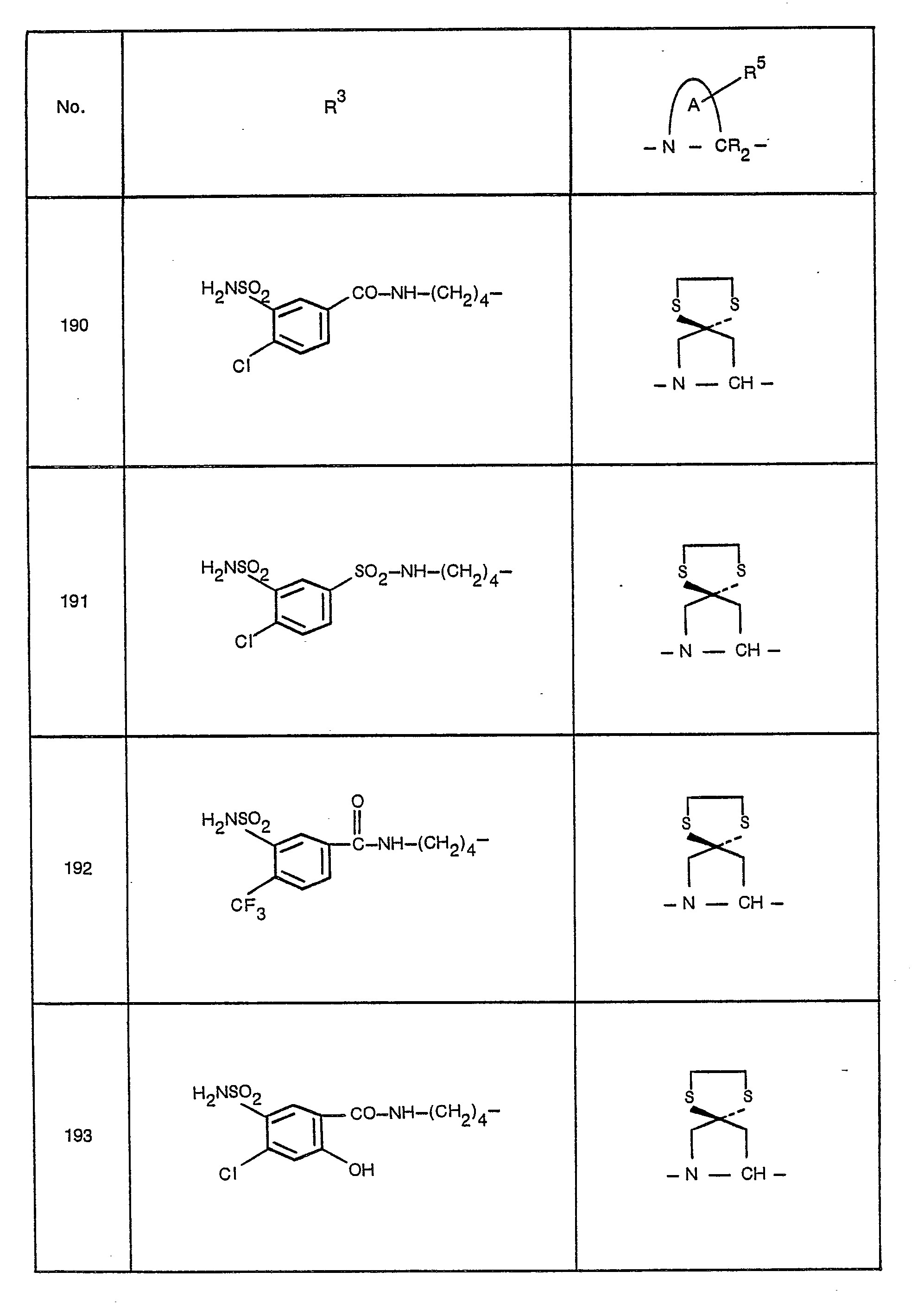

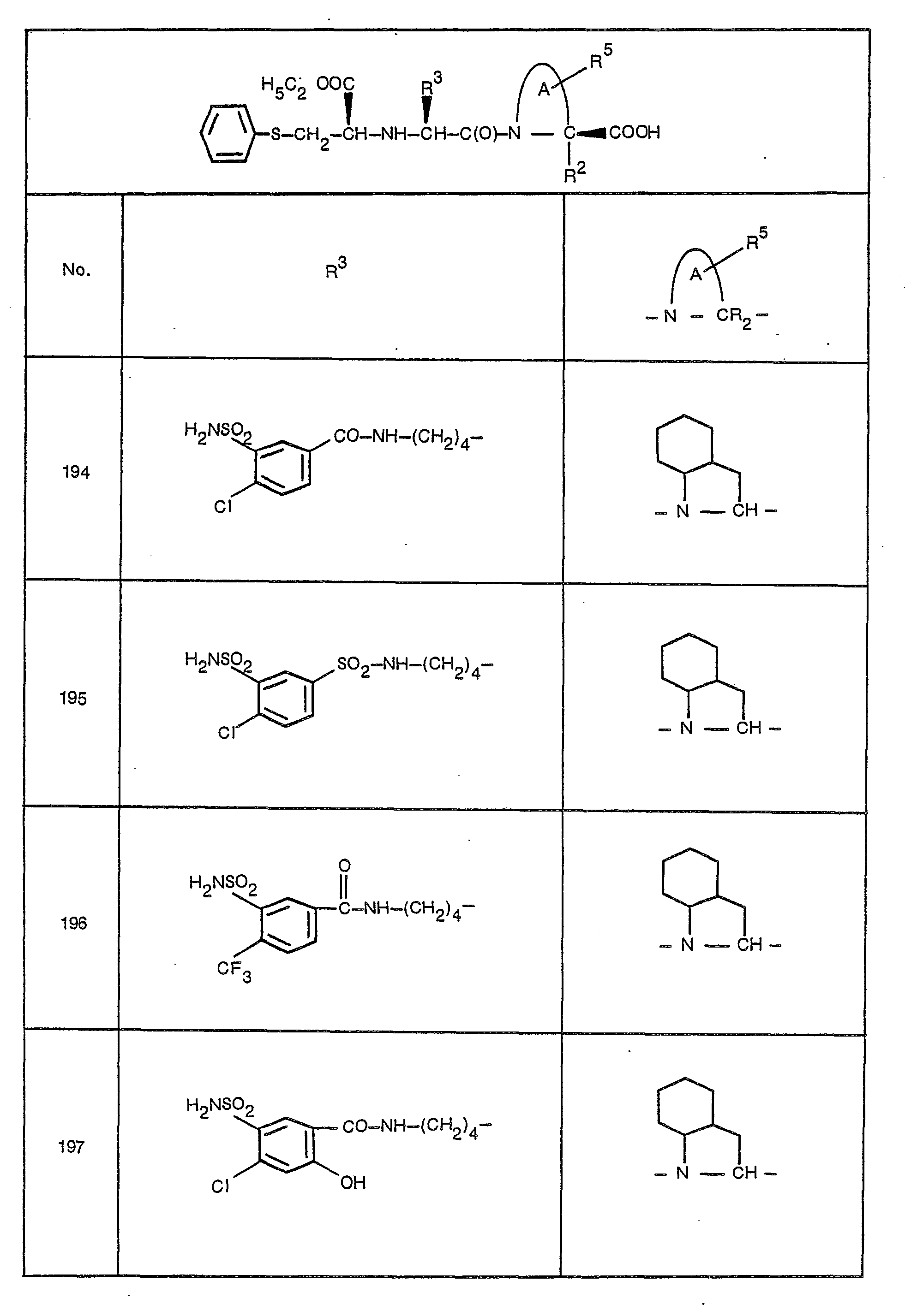

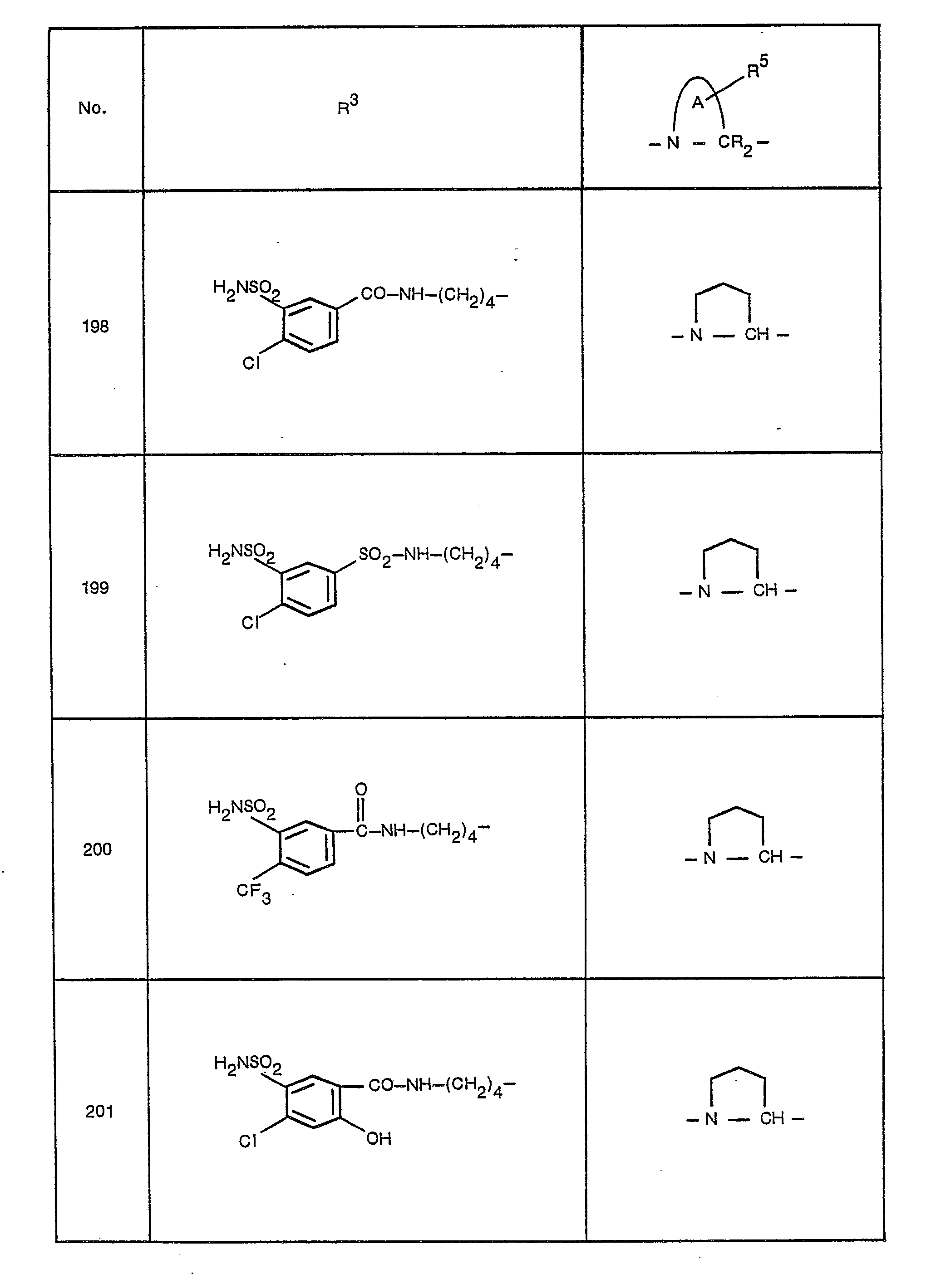

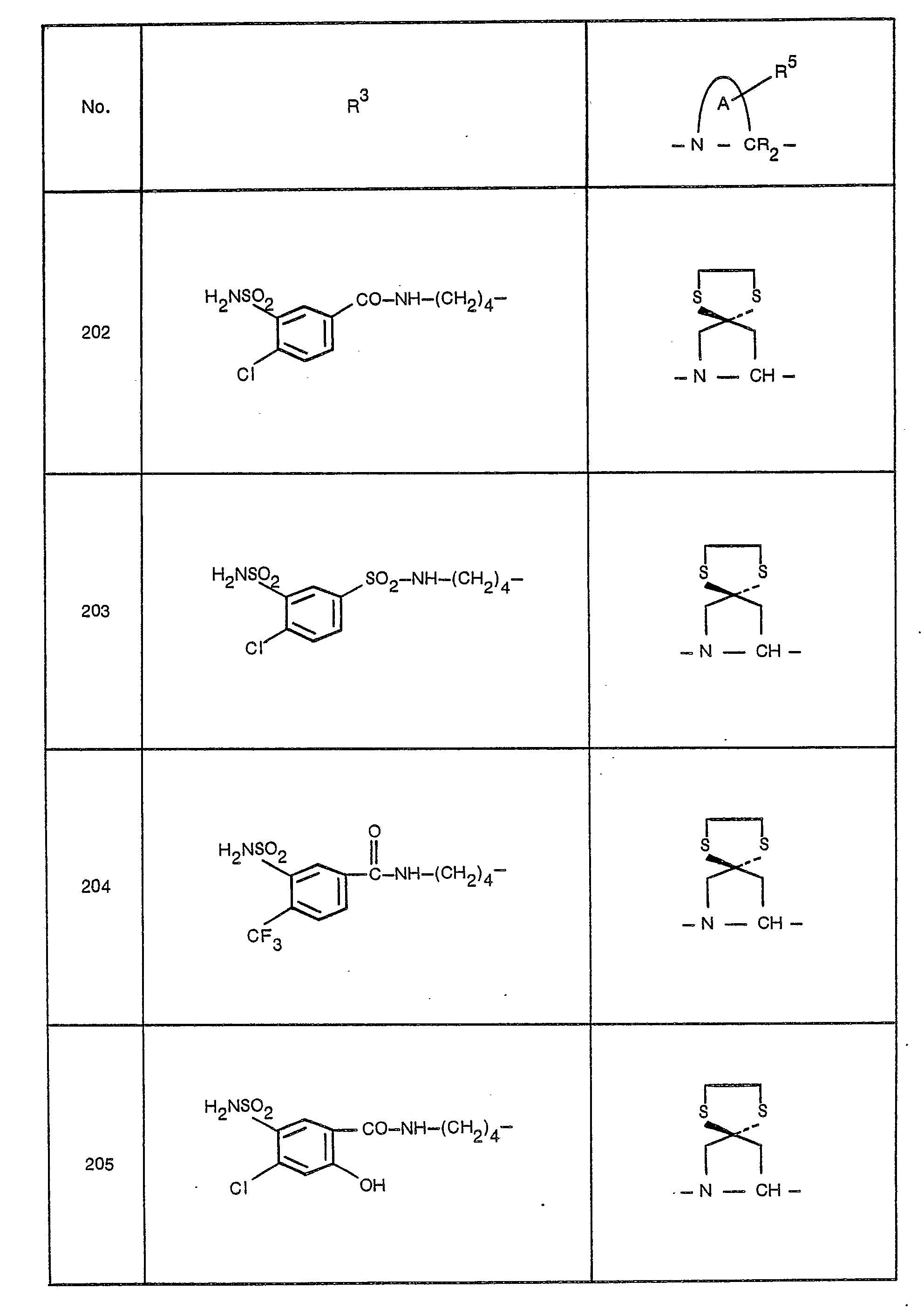

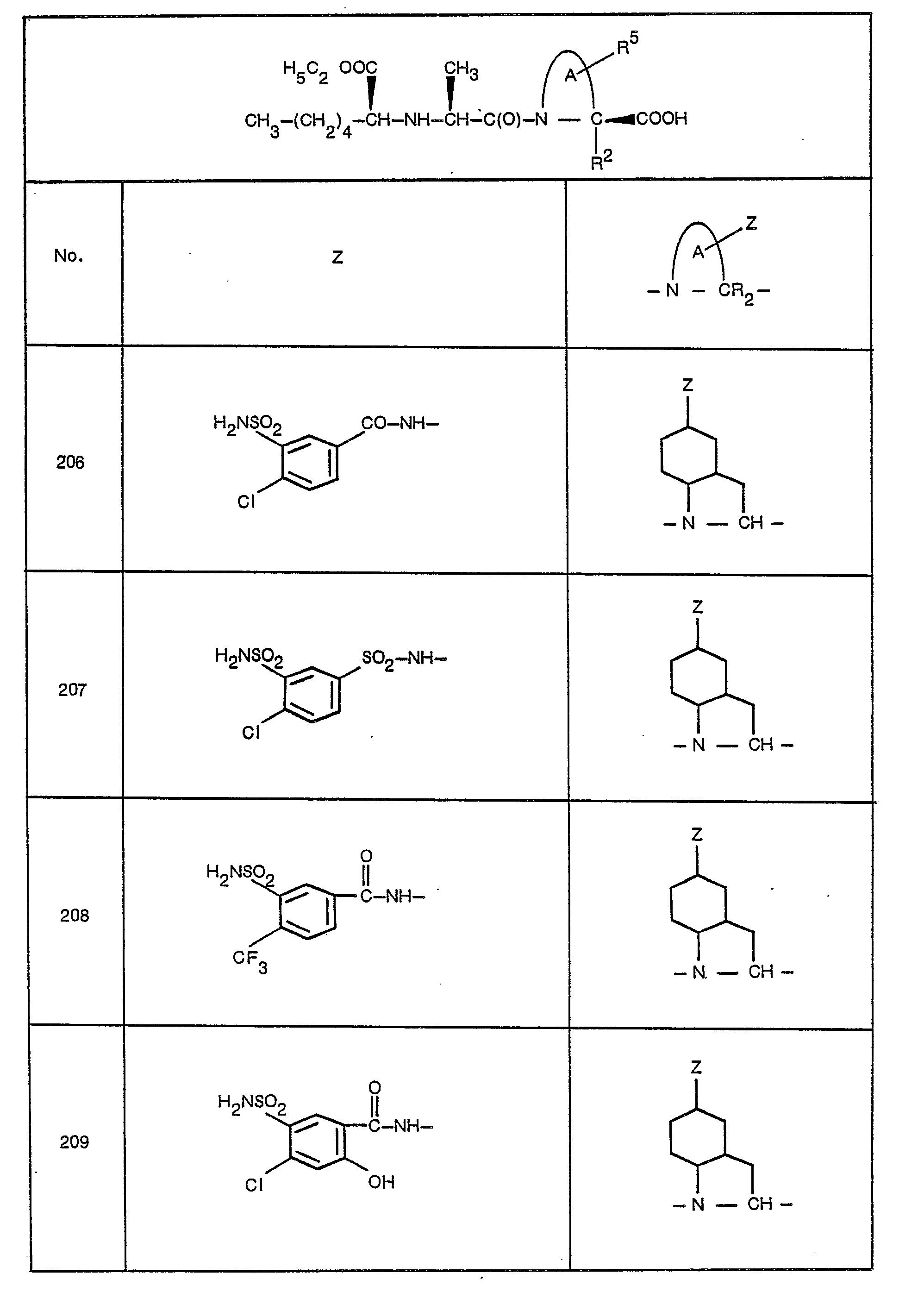

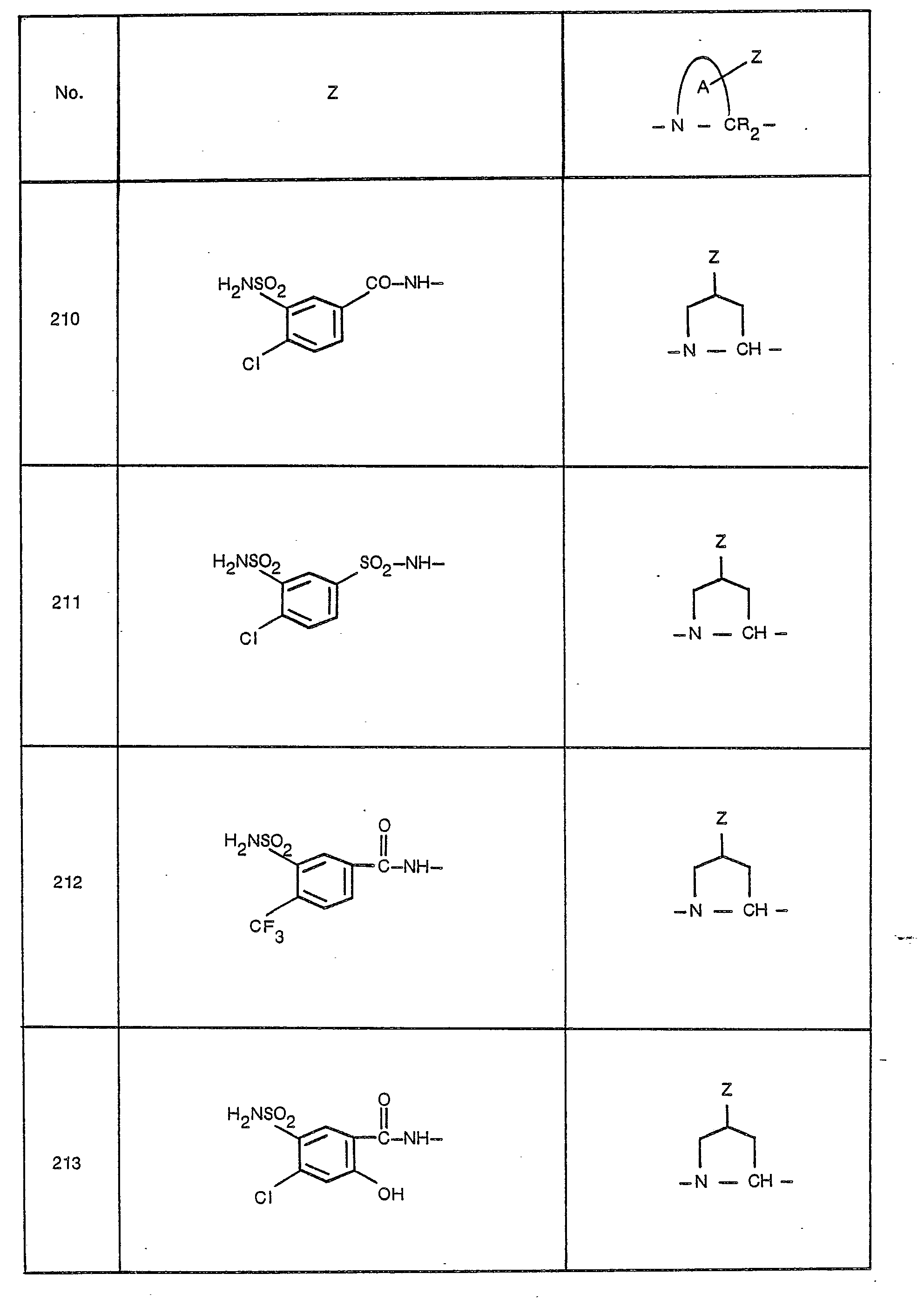

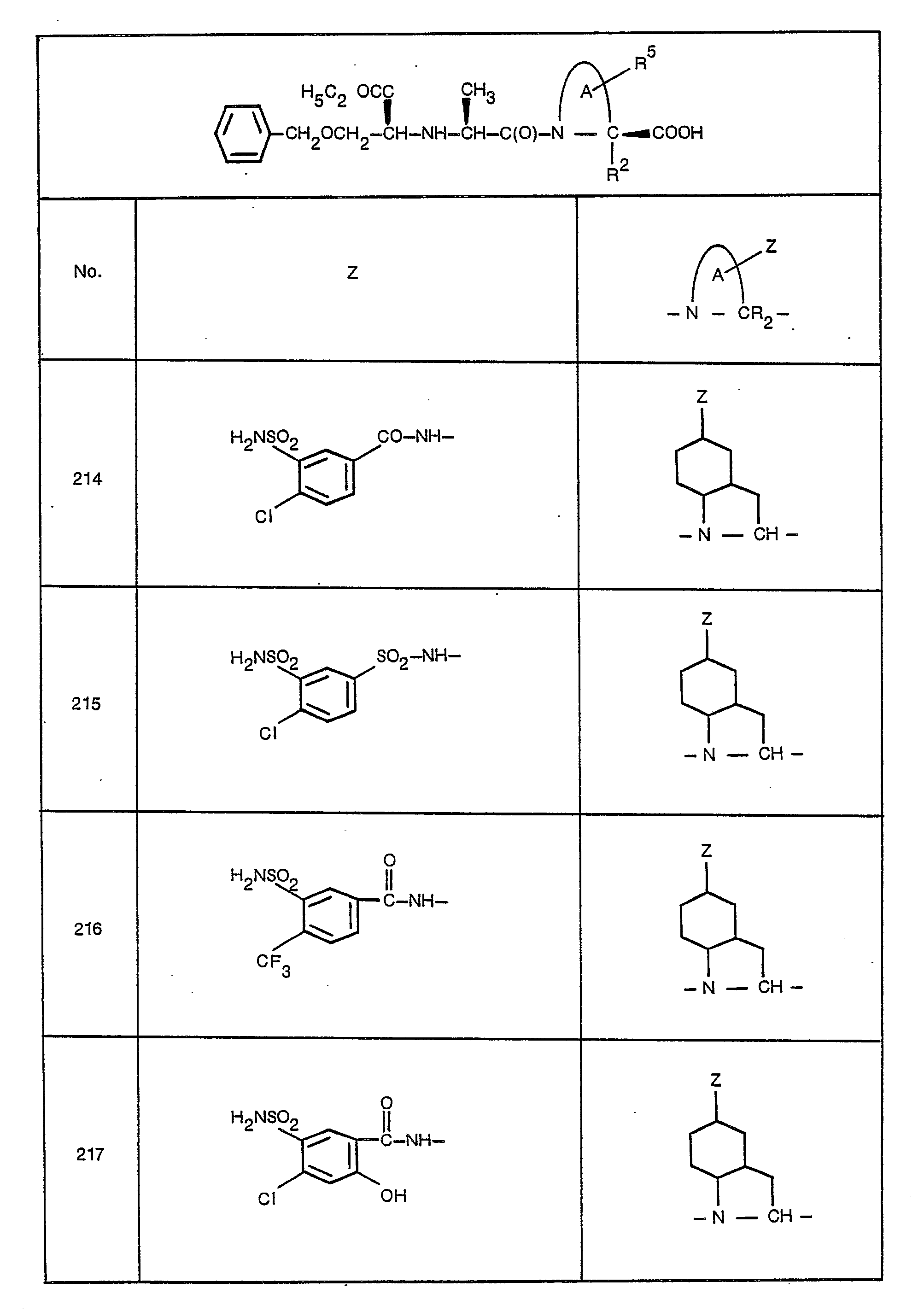

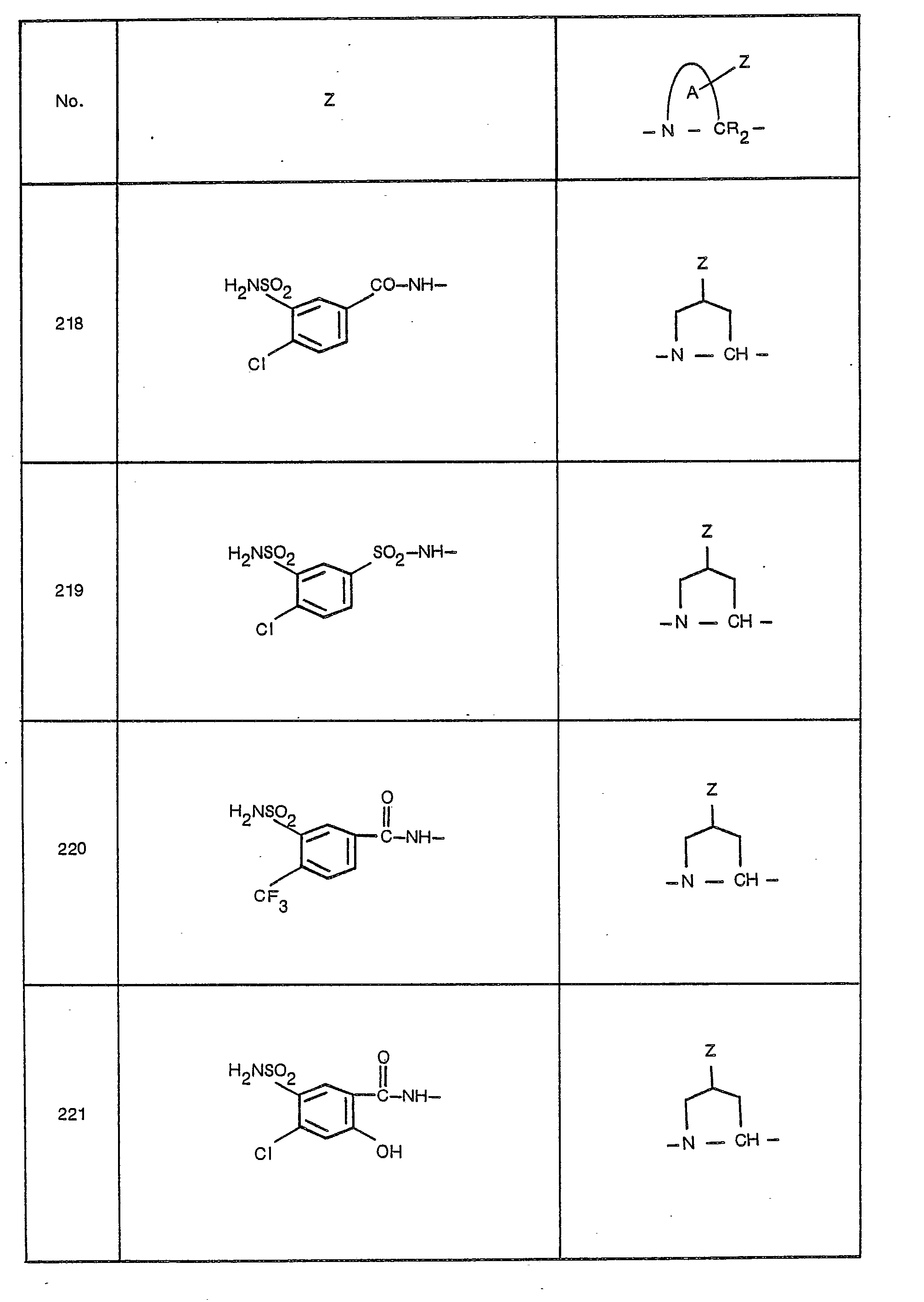

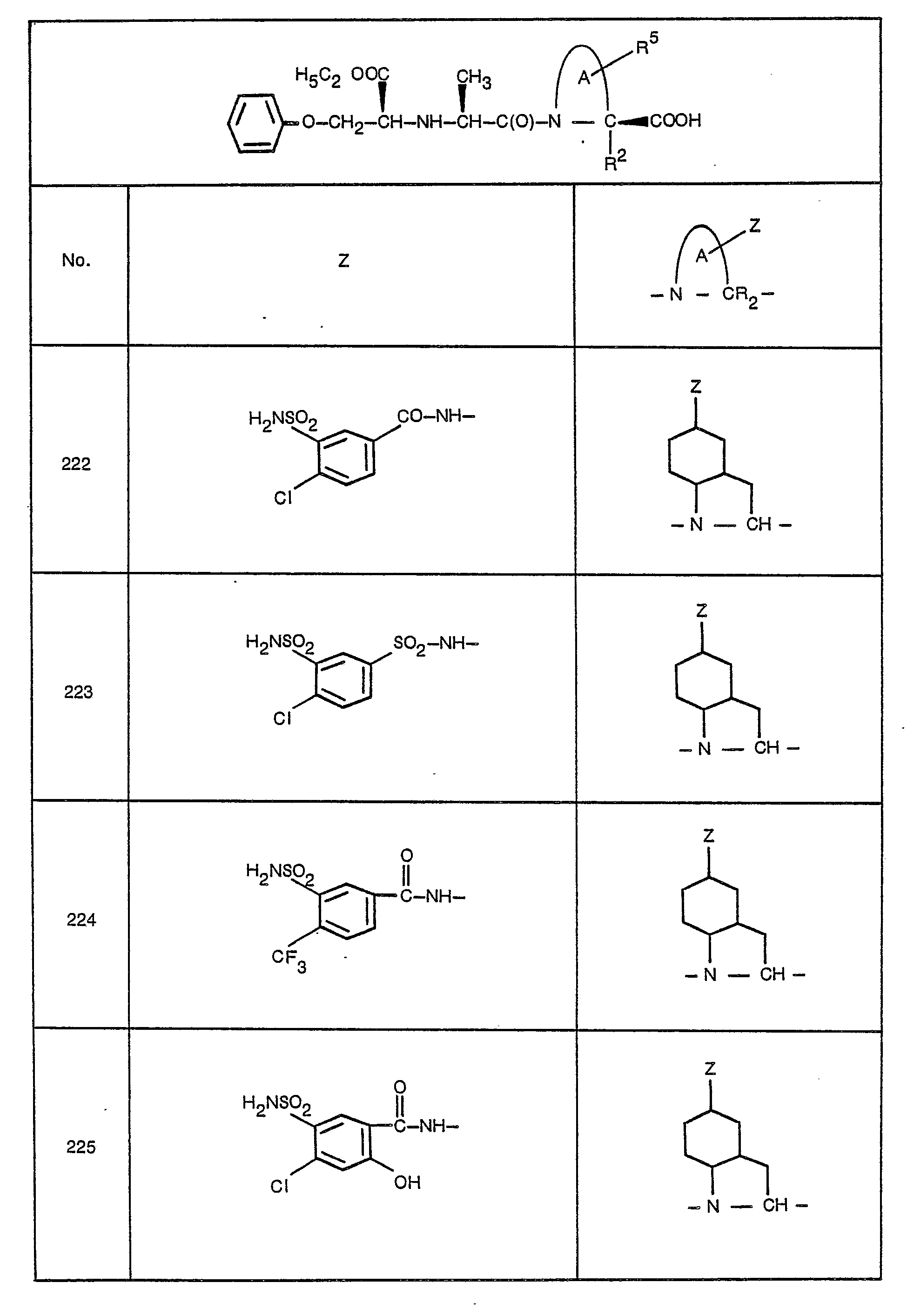

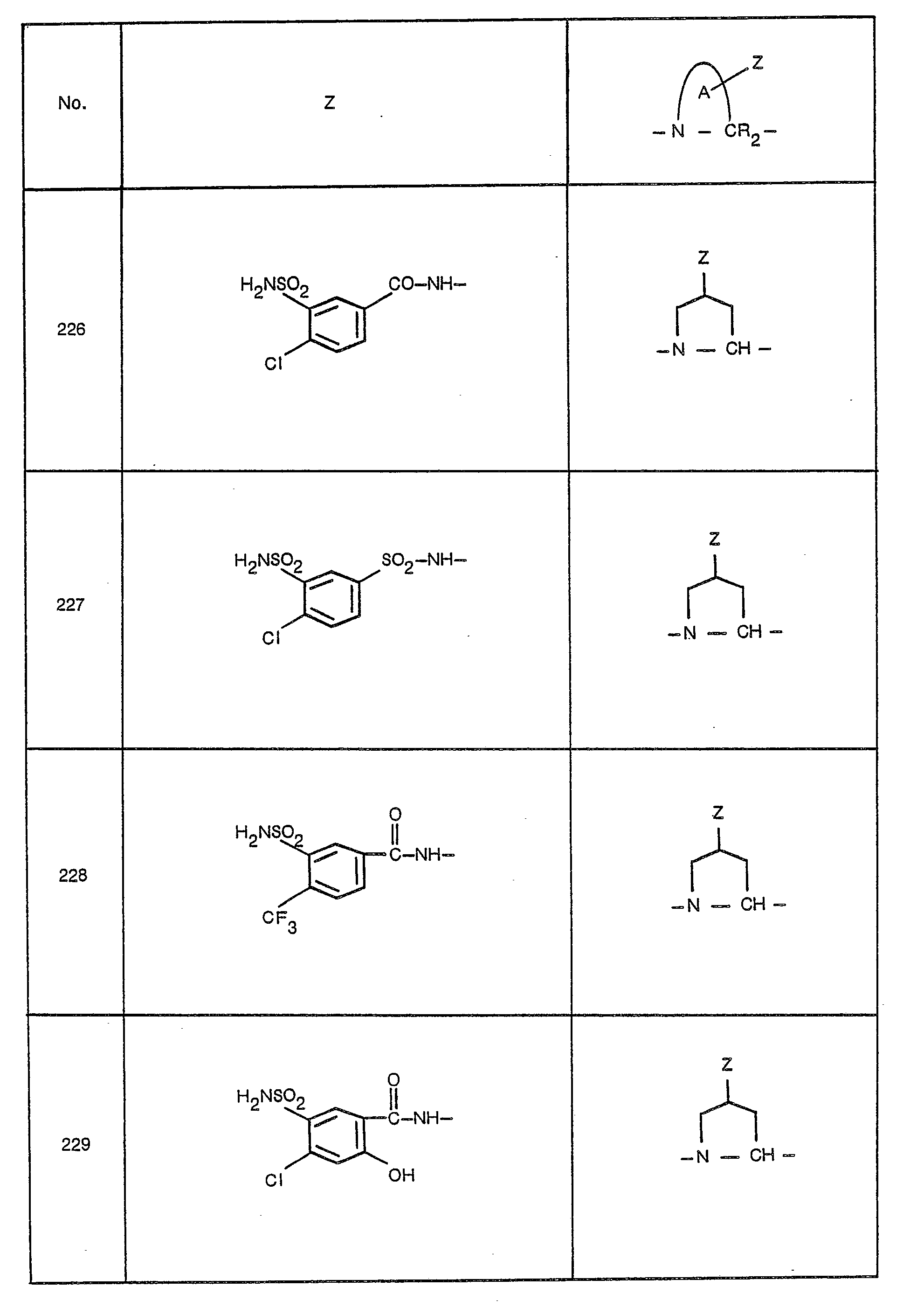

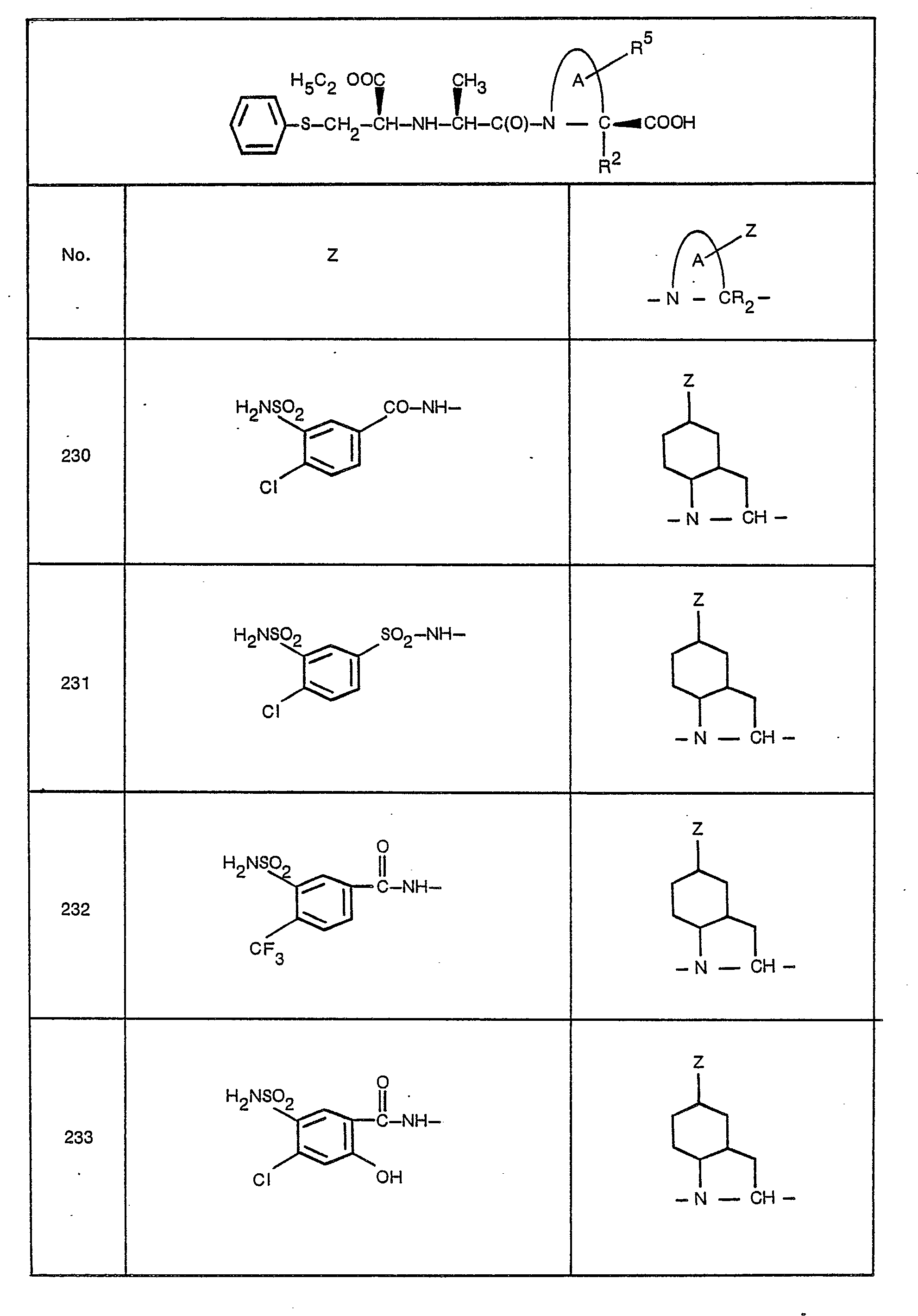

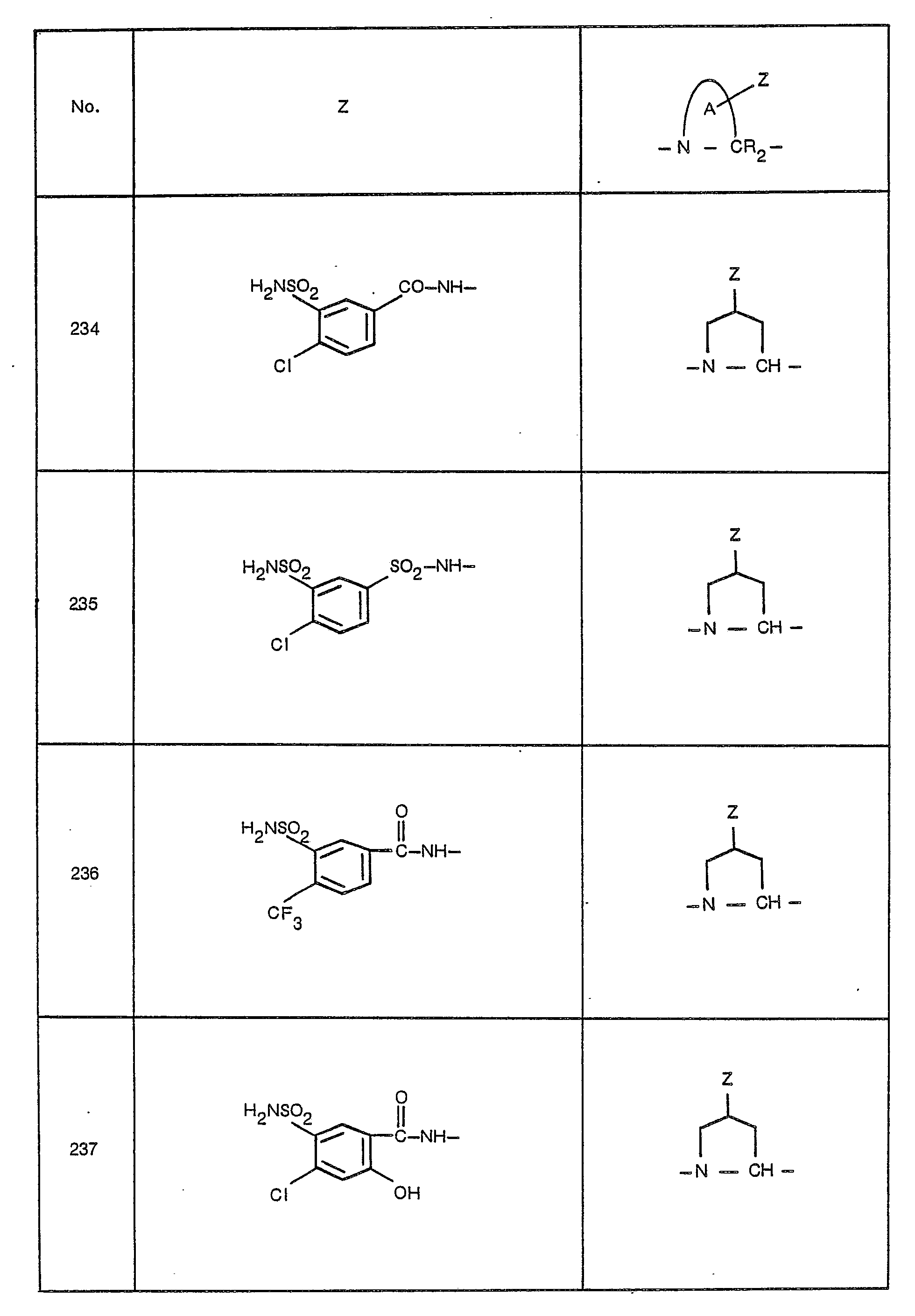

The following examples of formulation describe in detail compositions that are illustrative of the present invention. It will be apparent to those skilled in the art that many modifications, both of materials and methods, may be practised without departing from the purpose and intent of this disclosure.

In the formulation - examples the active ingredients are as follows:
Active ingredient A:
1 - {Na - [1(S) - ethoxycarbonyl - 3 - phenylpropyl] - Ne - [(4 - chloro - 3 - sulfamoyl)benzenesulfonyl] - (S) - lysyl} - cis,syn - octahydro - 1H - indole - 2(S) carboxylic acid.

Active ingredient B:
1 - {N - [1 (R) - carboxy - 2 - [S - ((3 - (6 - chloro - 3,4 - dihydro - 7 - sulfamoyl - -1,2,4 - benzothiadiazinyl - 1,1 - dioxide)methyl))thio]ethyl]] - (S) - alanyl} - cissyn - octahydro - 1H- indole - 2(S) - carboxylic acid.

Active ingredient C:
7 - (4 - chloro - 3 - sulfamoylbenzamido) - 2 - {N - [1(S) - carboethoxy - 3 - phenylpropyl] - (S) - alanyl} - 1,2,3,4 - tetrahydroisoquinoline - 3(S) - carboxylic acid.

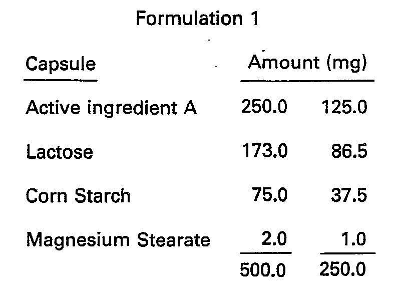

Blend the active ingredient, lactose and corn starch until uniform; then blend the magnesium stearate into the resulting powder. Encapsulate the mixture into suitably sized two-piece hard gelatin capsules.

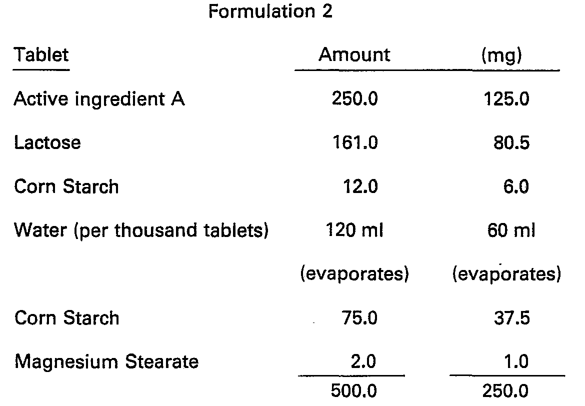

Blend the active ingredient with the lactose until uniform. Blend the smaller quantity of corn starch with the water and add the resulting corn starch paste, then mix until a uniform wet mass is formed. Add the remaining corn starch to the remaining wet mass and mix until uniform granules are obtained. Screen the granules through a suitable milling machine, using a 3/4 inch stainless steel screen. Dry the milled granules in a suitable drying oven until the desired moisture content is obtained. Mill the dried granules through a suitable milling machine using a 16 mesh stainless steel screen. Blend in the magnesium stearate and compress the resulting mixture into tablets of desired shape, thickness, hardness and disintegration.

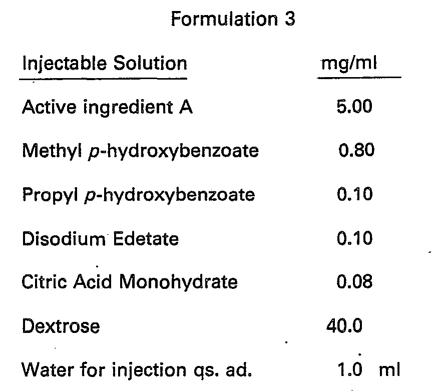

Dissolve the p-hydroxybenzoates in a portion of water for injection at 60-70°C and cool the solution to 25-35°C. Charge and dissolve all other excipients and the active ingredient. Bring the solution to final volume, filter it through a sterilizing membrane and fill into sterile containers.

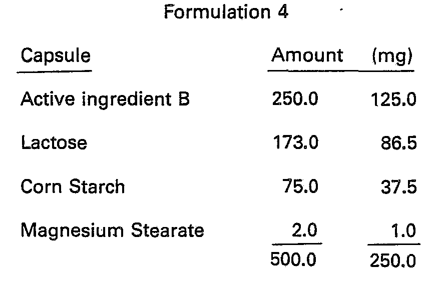

Blend the active ingredient, lactose and corn starch until uniform; then blend the magnesium stearate into the resulting powder. Encapsulate the mixture into suitably sized two-piece hard gelatin capsules.

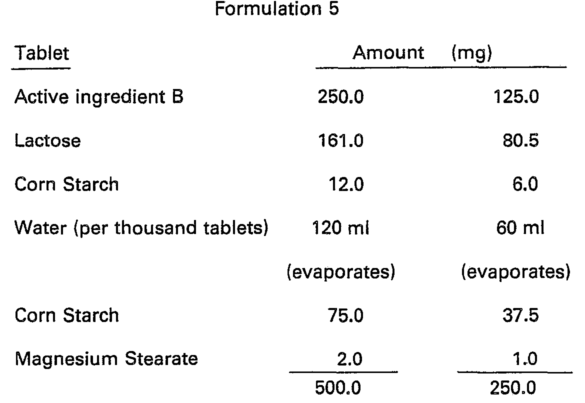

Blend the active ingredient with the lactose until uniform. Blend the smaller quantity of corn starch with the water and add the resulting corn starch paste, then mix until a uniform wet mass is formed. Add the remaining corn starch to the remaining wet mass and mix until uniform granules are obtained. Screen the granules through a suitable milling machine, using a 3/4 inch stainless steel screen. Dry the milled granules in a suitable drying oven until the desired moisture content is obtained. Mill the dried granules through a suitable milling machine using a 16 mesh stainless steel screen. Blend in the magnesium stearate and compress the resulting mixture into tablets of desired shape, thickness, hardness and disintegration.

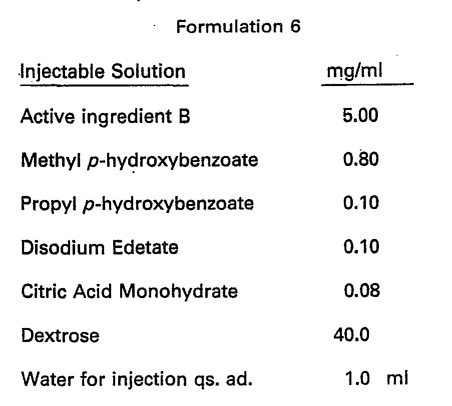

Dissolve the p-hydroxybenzoates in a portion of water for injection at 60―70°C and cool the solution to 25―35°C. Charge and dissolve all other excipients and the active ingredient. Bring the solution to final volume, filter it through a sterilizing membrane and fill into sterile containers.

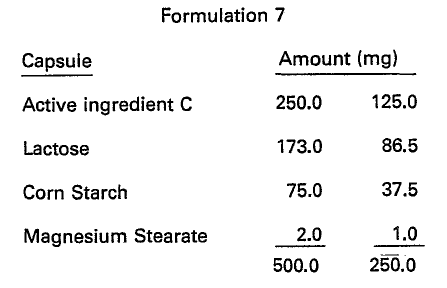

Blend the active ingredient, lactose and corn starch until uniform; then blend the magnesium stearate into the resulting powder. Encapsulate the mixture into suitably sized two-piece hard gelatin capsules.

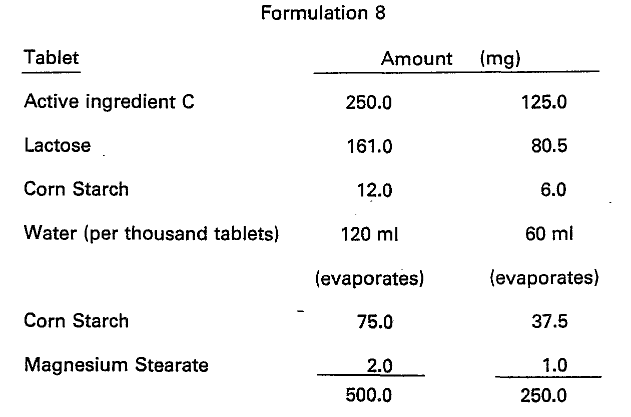

Blend the active ingredient with the lactose until uniform. Blend the smaller quantity of corn starch with the water and add the resulting corn starch paste, then mix until a uniform wet mass is formed. Add the remaining corn starch to the remaining wet mass and mix until uniform granules are obtained. Screen the granules through a suitable milling machine, using a 3/4 inch stainless steel screen. Dry the milled granules in a suitable drying oven until the desired moisture content is obtained. Mill the dried granules through. a suitable milling machine using a 16 mesh stainless steel screen..Blend in the magnesium stearate and compress the resulting mixture into tablets of desired shape, thickness, hardness and disintegration.

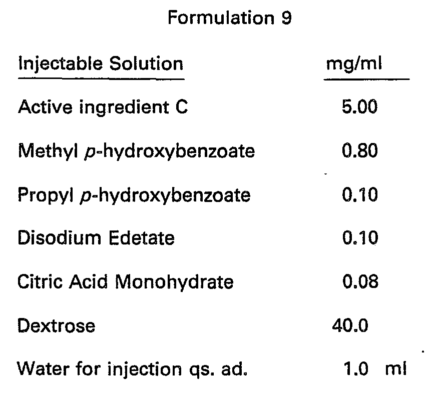

Dissolve the p-hydroxybenzoates in a portion of water for injection at 60―70°C and cool the solution to 25-35°C. Charge and dissolve all other excipients and the active ingredient. Bring the solution to final volume, filter it through a sterilizing membrane and fill into sterile containers.

## Claims (Claims for the following Contracting State(s) : s: BE CH DE FR IT LI LU NL SE)

1. A compound of the formula 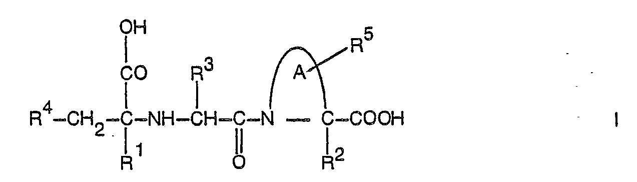 the pharmaceutically acceptable esters thereof and the pharmaceutically acceptable salts of the foregoing, wherein
R¹ and R² independently are hydrogen or lower alkyl; the group 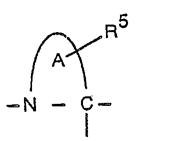 is one of the structures II to VIII 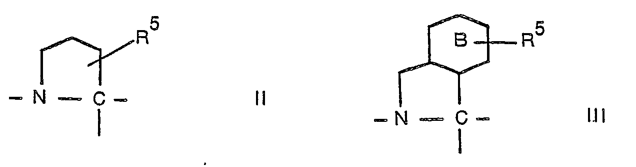 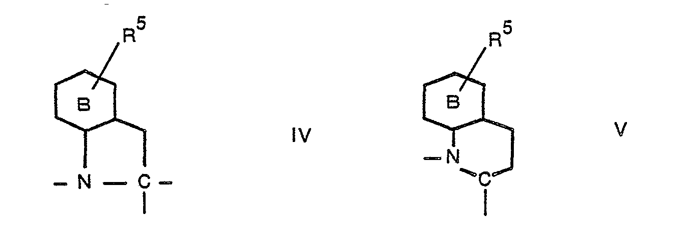 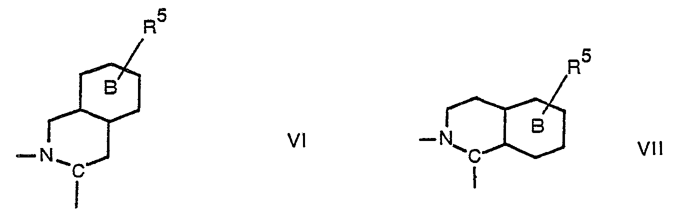 (wherein B is a saturated or aromatic ring) or 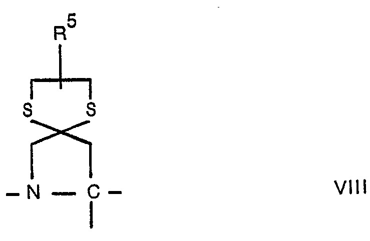 one of R³, R⁴ and R⁵ is a group Z―(CH₂)₀₋₆―, wherein Z has one of'the following values Z¹ to Z¹⁰: 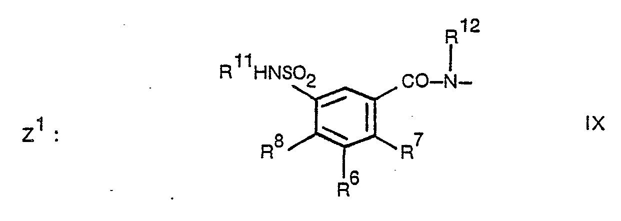 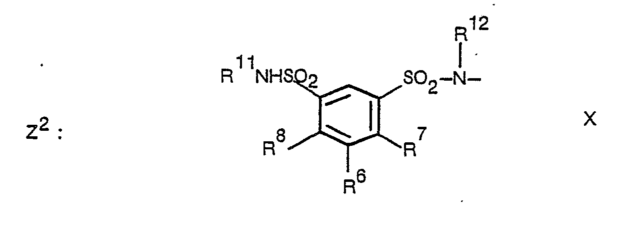 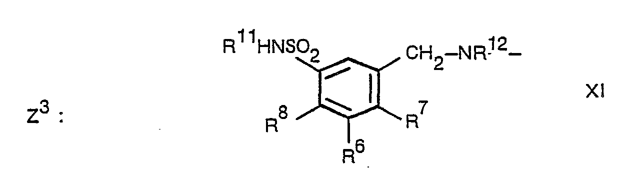 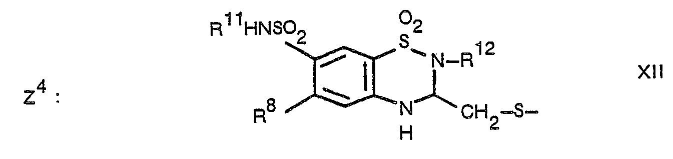 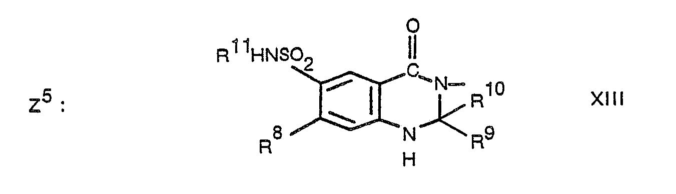 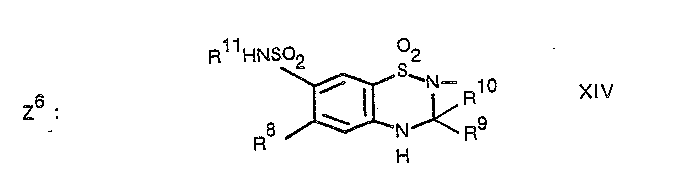 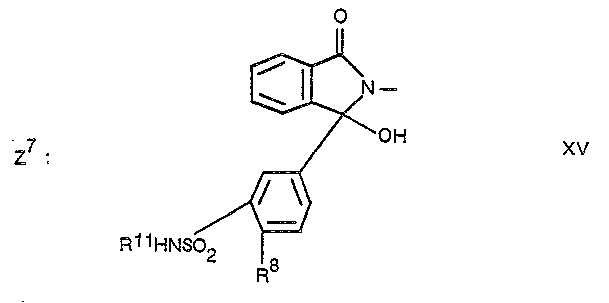 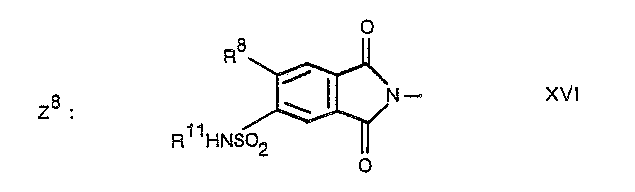 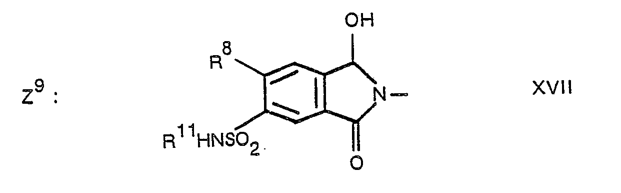 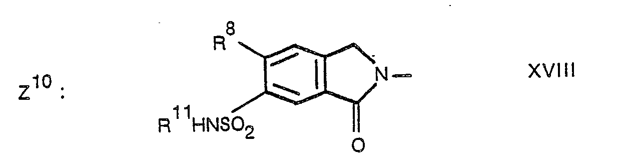 wherein
R⁸ is Cl or CF₃;
R⁶ is hydrogen or halogen;
R⁷ is hydrogen, halogen, carboxy, hydroxy or amino;
R⁹ and R¹⁰ are independently hydrogen, lower alkyl or halo-lower alkyl and R⁹ can also be phenyl or phenyl lower alkyl;
R¹¹ is hydrogen or lower alkyl;
R¹² is hydrogen, lower alkyl or phenyl lower alkyl; whereby when R³ is the group Z―(CH₂)₀₋₆―, then R³ is Z¹―(CH₂)₁₋₆―, Z²―(CH₂)₁₋₆―, Z³―(CH₂)₁₋₆―. Z⁴―CH₂―, Z⁵―(CH₂)₁₋₆―, Z⁶―(CH₂)₁₋₆―, Z⁷―(CH₂)₁₋₆―, Z⁸―(CH₂)₁₋₆― Z⁹―(CH₂)₁₋₆―, or Z¹⁰―(CH₂)₁₋₆―,
R⁴ is lower alkyl, benzyl, benzyloxy, benzylthio, phenoxy, or phenylthio, R⁵ is hydrogen; and the group 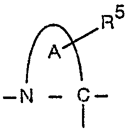 is one of the structures II to VIII;
and when R⁴ is the group Z―(CH₂)₀₋₆―, then R⁴ is Z¹―(CH₂)₀₋₆―, Z²―(CH₂)₀₋₆― Z³―(CH₂)₀₋₆―, Z⁴―(CH₂)₀₋₆―, Z⁵―(CH₂)₀₋₆―, Z⁶―(CH₂)₀₋₆―, Z⁷―(CH₂)₀₋₆―, Z⁸―(CH₂)₀₋₆―, Z⁹―(CH₂)₀₋₆― or Z¹⁰―(CH₂)₀₋₆― and
R³ is hydrogen, lower alkyl or amino lower alkyl and
R⁵ is hydrogen; and the group 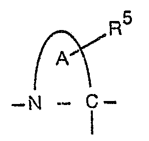 is one of the structures II to VIII;
and when R⁶ is the group Z―(CH₂)₀₋₆―, then R⁵ is Z¹, Z², Z³, Z⁴; Z⁵, Z⁶, Z⁷, Z⁸, Z⁹ or Z¹⁰, R³ is hydrogen, lower alkyl or amino lower alkyl and R⁴ is lower alkyl, benzyl, benzyloxy, benzylthio, phenoxy or phenylthio; and the group 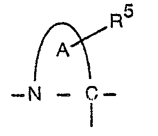 is one of the structures II to VII, preferably being in the form of the free di-carbonic acid or in the form of its alkyl ester, the alkyl group containing 1 to 6 carbon atoms, especially in the form of its monoester wherein the carboxy group attached to the group 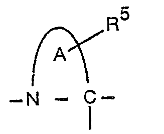 is in the free form, preferably all former compounds being the stereoisomer in which the absolute configurations at each of the three carbon atoms bonded to both a nitrogen and a carbonyl group corresponds most closely to the absolute configuration of L-aminoacids.

2. A compound according to claim 1, wherein R⁴ is a group Z―(CH₂)₀₋₆― as defined in claim 1, wherein Z preferably is Z¹, Z², Z³, Z⁵, Z⁷, Z⁸, Z⁹ or Z¹⁰;
R⁴ preferably being Z¹―(CH₂)₂ or 3―, Z²―(CH₂)₂ or 3―. Z³―(CH₂)₂ or 3―, Z⁵―(CH₂)₂ or 3―, Z⁷―(CH₂)₂ or 3―, Z⁸―(CH₂)₂ or 3―. Z⁹―(CH₂)₂ or ₃― or Z¹⁰―(CH₂)₂ or ₃―, and wherein preferably the group 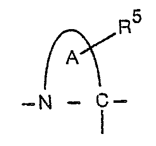 is the group of formula II. IV (wherein B is a saturated ring) or VIII, preferably R⁵ being hydrogen.

3. A compound according to claim 1 or 2, wherein R¹ and R² are hydrogen, and/or when Z is of the formula IX, X or XI R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety Z is chloro, and/ or R³ is methyl.

4. A compound according to claim 1 or 2, wherein R¹ and R² are hydrogen, the group 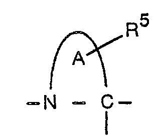 is the group of formula IV, wherein B is a saturated ring and R⁵ is hydrogen, R⁴ is Z¹―(CH₂)₃― or Z²―(CH₂)₃―, wherein R⁶ is hydrogen, R⁷ is hydrogen or hydroxy, and R⁸ is chloro; and R³ is methyl, preferably being
1 - {N - [1 (S) - ethoxycaronyl - 5 - (4 - chloro - 3 - sulfamoyl) - benzenesulfonaminopentyl] - (S) - alanyl} - cis,syn - octahydro - 1H - indole - 2(S) - carboxylic acid,
1 - {N - [1(S) - ethoxycarbonyl - 5 - (4 - chloro - 3 - sulfamoyl) - benzamidopentyl] - (S) - alanyl} - cis,syn - octahydro - 1H - indole - 2(S) - carboxylic acid, or
1 - {N - [1(S) - ethoxycarbonyl - 5 - (4 - chloro - 2 - hydroxy - 5 - sulfamoyl) - benzamidopentyl] - (S) - alanyl} - cis,syn - octahydro - 1H - indole - 2(S) - carboxylic acid,
1 - {N - [1(S) - carboxy - 5 - [[(4- chloro - 2 hydroxy - 5- sulfamoylphenyl) carbonyl]amino]pentyl] - (S) - alanyl} - cis,syn - octahydro - 1H - indole - 2(S) - carboxylic acid,
1 - {N - [1 (S) - carboxy - 5 - [[(4 - chloro - 3 - (N - methyl sulfamoyl)phenyl] carbonyl]aminolpentyl] - (S) - alanyl} - cis,syn - octahydro - 1H - indole - 2(S) - carboxylic acid,
1 - {N - [1 (S) - carboxy - 5 - [[(4 - chloro - 3 - sulfamoyl phenyl)carbonyl]amino]pentyl] - (S) - alanyl} - cis,syn - octahydro - 1H - indole - 2(S) - carboxylic acid,
in the free form or in the form of its ester, preferably in the form of its mono-or-di-ethyl ester.

5. A compound according to claim 1, wherein R³ is a group Z―(CH₂)₀₋₆― as defined in claim 1, preferably Z¹―(CH₂)₄―, Z²―(CH₂)₄―, Z³―(CH₂)₄―, Z⁴―CH₂―, Z⁵―(CH₂)₄―, Z⁶―(CH₂)₄―, Z⁷―(CH₂)₄, Z⁸―(CH₂)₄―, Z⁹―(CH₂)4― or Z¹⁰―(CH₂)₄―, and wherein the group 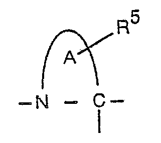 is preferably the group of formula II, IV (wherein B is a saturated ring) or VIII.

6. A compound according to claim 5, wherein R¹ and R² are hydrogen, and/or Z is of the formula IX, X or XI, R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety Z is chloro, and/or R⁴ is benzyl or ethyl.

7. A compound according to claim 5, wherein R¹ and R² are hydrogen, the group 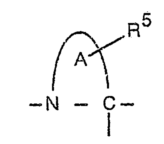 is the group of formula IV, wherein B is a saturated ring and R⁵ is hydrogen, R³ is Z¹―(CH₂)₄― or Z²―(CH₂)₄―, wherein R⁶ and R⁷ are hydrogen, and R⁸ is chloro, and R⁴ is benzyl, preferably being
1 - {Na - [1 (S) - ethoxycarbonyl - 3 - phenylpropyl] - Nε - [(4 - chloro - 3 - sulfamoyl)benzenesulfonyl] - (S) - lysyl} - cis,syn - octahydro - 1H - indole - 2(S) - carboxylic acid or
1 - {Na - [1 (S) - ethoxycarbonyl - 3 - phenylpropyl] - Nε - [(4 - chloro - 3 - sulfamoyl)benzoyl - (S) - lysyl} - cis,syn - octahydro - 1H - indole - 2(S) - carboxylic acid
in the free form or in the form of its ester, preferably in the form of its mono-or-di-ethyl ester.

8. A compound according to claim 1, wherein R⁵ is a group Z―(CH₂)₀₋₆― as defined in claim 1, preferably being Z¹, Z², Z³, Z⁵, Z⁷, Z⁸, Z⁹ or Z¹⁰; wherein the group 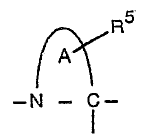 is preferably the group of formula II, VI (wherein B is an aromatic ring), or IV (wherein B is a saturated ring), preferably

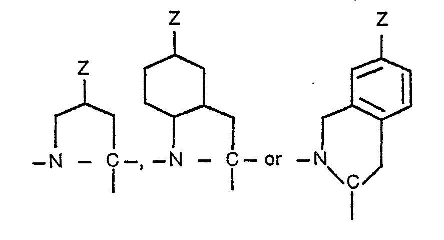

9. A compound according to claim 8, wherein R¹ and R² are hydrogen, and/or when Z is of the formula IX, X or XI R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety Z is chloro, and/or R³ is methyl and/or R⁴ is benzyl or ethyl, the compound preferably being
7 - (4 - chloro - 3 - sulfamoylbenzamido) - 2 - {N - [1(S) - ethoxycarbonyl - 3 - phenylpropyl] - (S) - alanyl} - 1,2,3,4 - tetrahydroisoquinoline - 3(S) - carboxylic acid or the corresponding 1 - S - carboxy - compound.

10. Process for the preparation of a compound of formula I as defined in any one of claims 1 to 9, characterized in that the compound is prepared by an appropriate process selected from the following processes a to i:
a) for the preparation of a compound of formula I, wherein R¹ is hydrogen: condensation of a ketocompound (XIX) with a depeptide (XX) under reduction 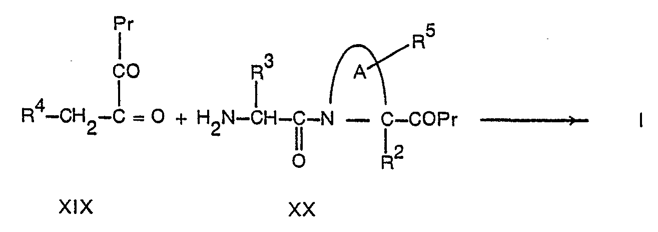 wherein A, R², R³, R⁴ and R⁵ are as define above and Pr stands for a free or a protected hydroxy group;
b) alkylation of a dipeptide (XX) by means of a compound of formula (XXI) under basic conditions 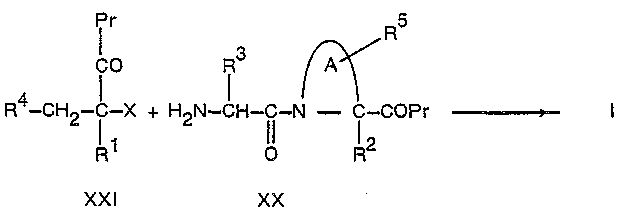 wherein X is chloro, bromo, iodo, alkanesulfonyloxy or arenesulfonyloxy, A, R¹, R², R³, R⁴, R⁵ are as defined above for compounds of formula I and Pr stands for a free or protected hydroxy group;
c) condensation of an aminoacid (XXII) with an aminoacid (XXIII) in the presence of a condensing agent 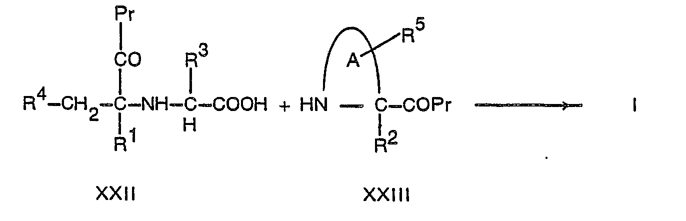 wherein A, R¹, R², R³, R⁴, R⁵ are as defined above for compounds of formula I, and Pr stands for a free or protected (e.g. by esterification) hydroxy group;
d) condensation of an amino compound (XXIV) with a ketocompound (XXV) 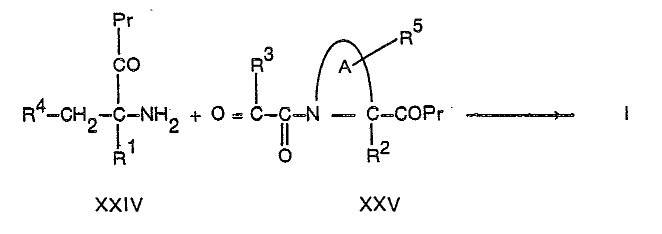 under the conditions described for process a wherein A, R¹, R², R³, R⁴, R⁵ are as defined above for compounds of formula I and Pr stands for a free or protected (e.g. by esterification) hydroxy group;
e) alkylation of an amino compound (XXIV) by means of a compound (XXVI) 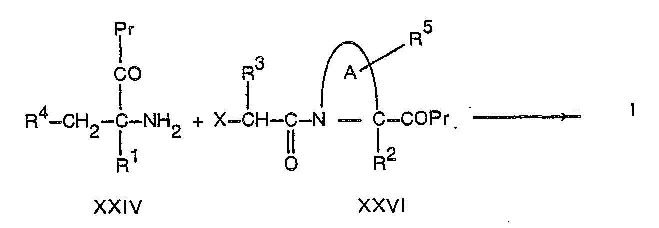 wherein X is chloro, bromo, iodo, alkanesulfonyloxy or arenesulfonyloxy, A, R¹, R², R³, R⁴, R⁵ are as defined above for compounds of formula I and Pr stands for a free or protected (e.g. by esterification) hydroxy group, under the conditions described for process b;
f) for the preparation of a compound of formula I, wherein one of R³, R⁴ and R⁵ is a group Z―(CH₂)₀₋₆―, wherein Z is Z⁵, Z⁶, Z⁷, Z⁸, Z⁹ or Z¹⁰, preferably Z⁷, Z⁸ or Z⁹: condensation of a peptide of the general formula (XXX) with a compound containing the desired group (XXXI) 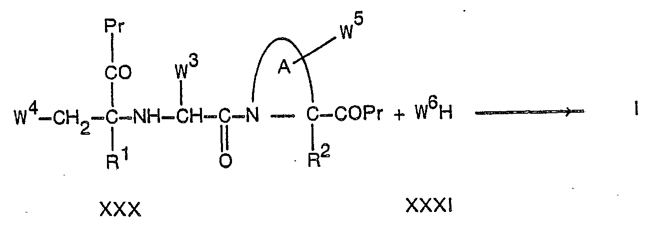 wherein R¹, R² and A are as defined for formula I, Pr is a protected hydroxy group W³, W⁴ and W⁵ are defined like R³, R⁴ and R⁵ respectively with the difference that one of W³, W⁴ and W⁵ contains an NH₂-group instead of the respective Z⁵ to Z¹⁰-group; and W⁶ is Z⁵, Z⁶, Z⁷, Z⁸, Z⁹ or Z¹⁰;
g) for the preparation of a compound of formula I, wherein one of R³, R⁴ and R⁵ is a group Z―(CH₂)ₒ₋₆―, wherein Z is Z¹, Z² or Z³: condensation of a peptide of formula XXXII with an appropriately substituted compound of formula XXXIII 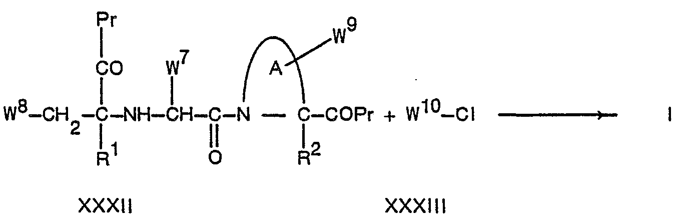 wherein R¹, R² and A are as defined for formula I, Pr is a protected hydroxy group, W⁷, W⁸ and W⁹ are defined like R³, R⁴ and R⁵ respectively, with the difference that one of W⁷, W⁸ and W⁹ contains an NH₂-group instead of the respective Z¹, Z² or Z³ group, and W¹⁰ is 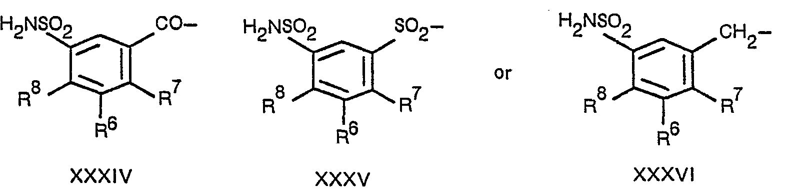
h) for the preparation of a compound of formula I, wherein one of R³, R⁴ and R⁵ is a group Z―(CH₂)₀₋₆―, wherein Z is Z⁴: condensation of a peptide of formula (XXXVII) with a 3 halomethylbenzothiadiazine (XXXVIII) 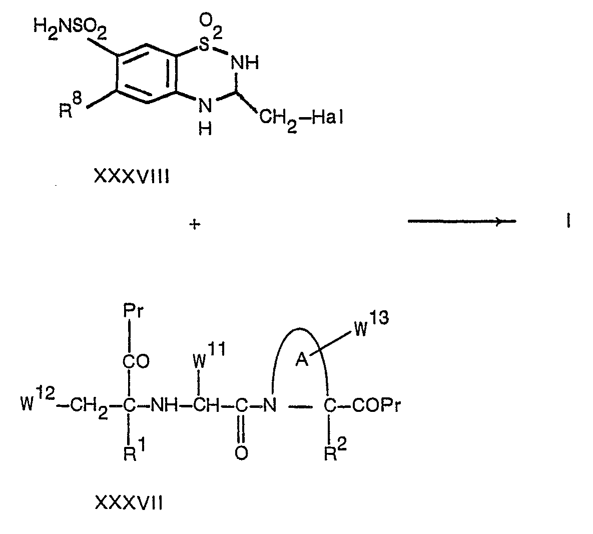 wherein R¹, R² and A are as defined for formula I, Pr is a protected hydroxy group, W¹¹, W¹² and W¹³ are defined like R³, R⁴ and R⁵ respectively with the difference that one of W¹¹, W¹² and W¹³ contains a―SH- group instead of of the respective Z⁴⁻group, and Hal is halogen, preferably chloro;
i) for the preparation of a compound of formula I, wherein one of R³, R⁴ and R⁵ is a group Z―(CH₂)₀₋₆―, wherein Z is Z⁵ or Z⁶: condensation of a peptide of formula XXXIX with a compound of formula XXXX 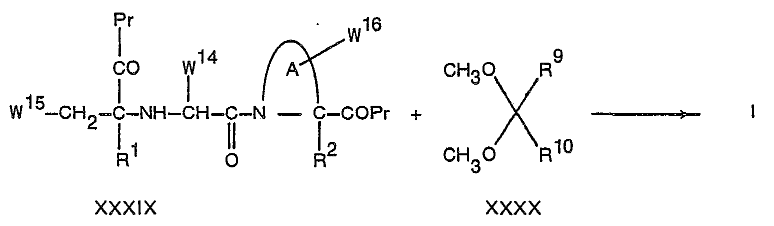 wherein R¹, R², R⁹, R¹⁰ and A are as defined for formula I, Pr is a protected hydroxy group, W¹⁴, W¹⁵ and W¹⁶ are defined like R³, R⁴ and R⁵ respectively with the difference that one of W¹⁴, W¹⁵ and W¹⁶ contains the group 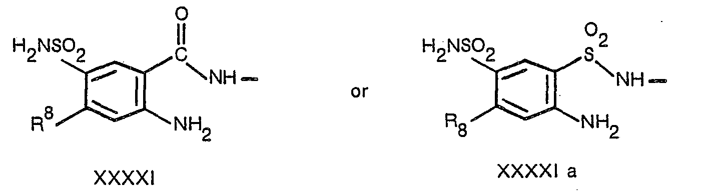 respectively instead of the group Z⁵ or Z⁶ respectively;
followed by removal of the protecting groups; if necessary, to yield the desired product, and if desired, converting a so obtained compound of formula I into its ester and/or setting free the compound of formula I from its ester or preparing a salt thereof and, if desired, isolating the preferred isomer.

11. A pharmaceutical composition comprising a compound of the general formula I or pharmaceutically acceptable salt or ester thereof as defined in any one of claims 1 to 9 or obtained according to a process of claim 10.

## Claims (Claims for the following Contracting State(s) : AT)

1. Process for the preparation of compound of the formula 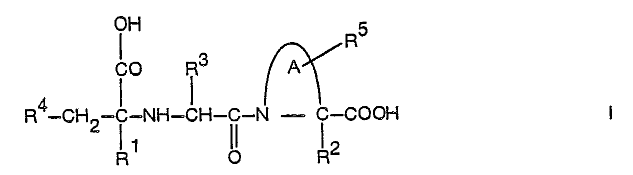 the pharmaceutically acceptable esters thereof and the pharmaceutically acceptable salts of the foregoing, wherein
R¹ and R² independently are hydrogen or lower alkyl; the group 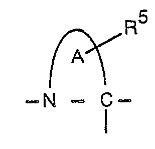 is one of the structures II to VIII 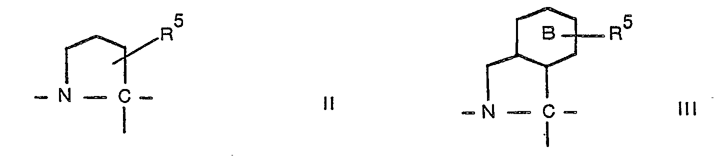 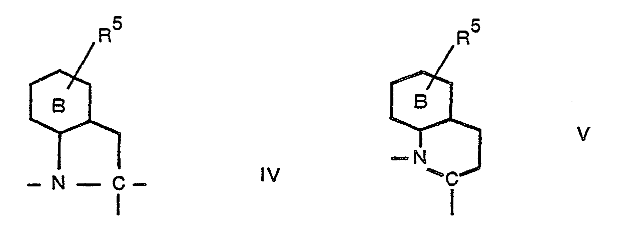 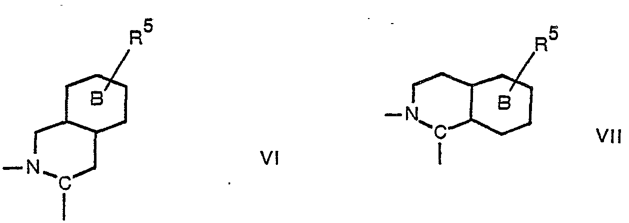 (wherein B is a saturated or aromatic ring) or 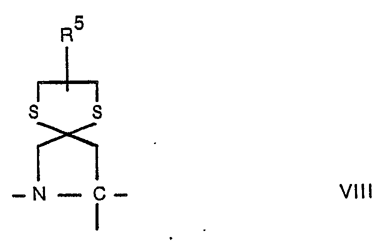 one of R³, R⁴ and R⁵ is a group Z―(CH₂)₀₋₆―, wherein Z has one of the following values Z¹ to Z¹⁰: 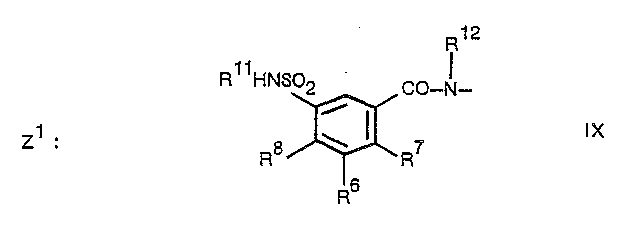 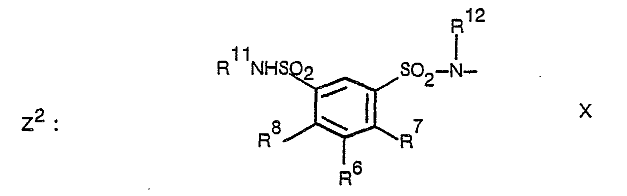 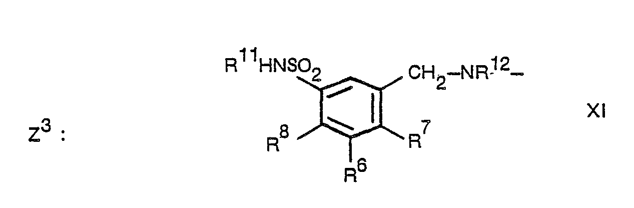 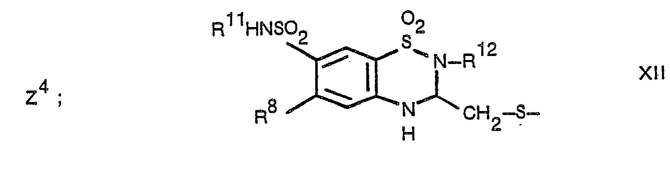 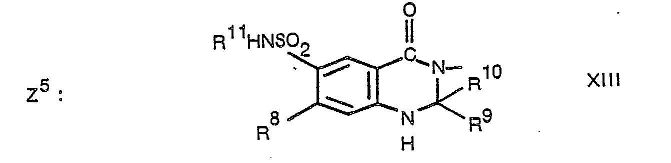 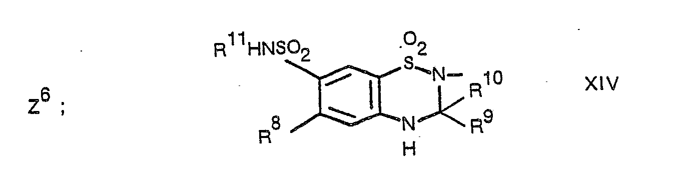 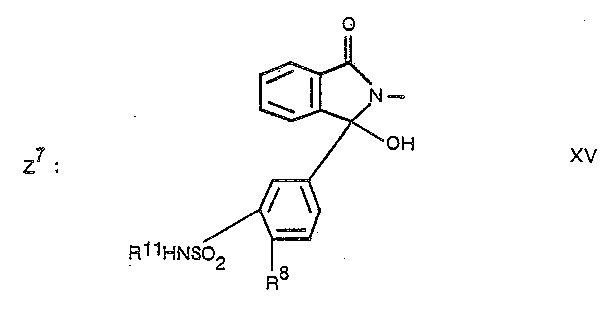 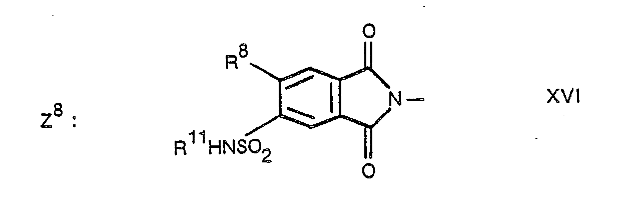 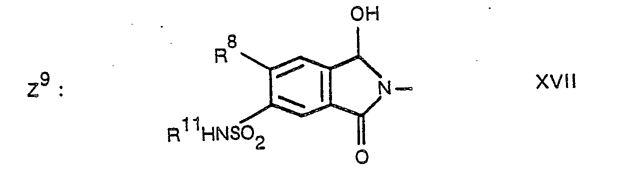 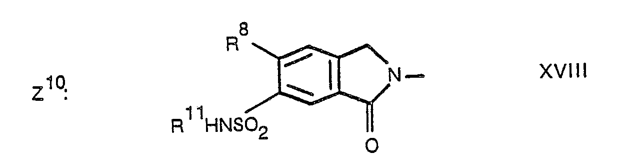 wherein R⁸ is Cl or CF₃;
R⁶ is hydrogen or halogen;
R⁷ is hydrogen, halogen, carboxy, hydroxy or amino;
R⁹ and R¹⁰ are independently hydrogen, lower alkyl or halo-lower alkyl and R⁹ can also be phenyl or phenyl lower alkyl;
R" is hydrogen or lower alkyl;
R¹² is hydrogen, lower alkyl or phenyl lower alkyl; whereby when R³ is the group Z―(CH₂)₀₋₆―, then R³ is Z¹―(CH₂)₁₋₆―, Z²―(CH₂)₁₋₆―, Z³―(CH₂)₁₋₆―, Z⁴―CH₂―, Z⁵―(CH₂)₁₋₆―, Z⁶―(CH₂)₁₋₆―, Z⁷―(CH₂)₁₋₆―, Z⁸―CH₂)₁₋₆―, Z⁹―(CH₂)₁₋₆―, or Z¹⁰―(CH₂)₁₋₆―,
R⁴ is lower alkyl, benzyl, benzyloxy, benzylthio, phenoxy, or phenylthio,
R⁵ is hydrogen; and the group 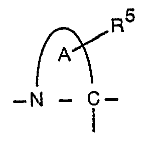 is one of the structures II to VIII;
and when R⁴ is the group Z―(CH₂)₀₋₆― then R⁴ is Z¹―(CH₂)₀₋₆― Z²―(CH₂)₀₋₆―, Z³―(CH₂)₀₋₆―, Z⁴―(CH₂)₀₋₆―, Z⁵―(CH₂)₀₋₆―, Z⁶―(CH₂)₀₋₆―, Z⁷―(CH₂)₀₋₆―, Z⁸―(CH₂)₀₋₆―, Z⁹―(CH₂)₀₋₆― or Z¹⁰―(CH₂)₀₋₆― and R³ is hydrogen, lower alkyl or amino lower alkyl and R⁵ is hydrogen; and the group 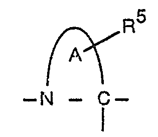 is one of the structures II to VIII;
and when R⁵ is the group Z―(CH₂)₀₋₆―, then R⁵ is Z¹, Z², Z³, Z⁴, Z⁵, Z⁶, Z⁷, Z⁸, Z⁹ or Z¹⁰, R³ is hydrogen, lower alkyl or amino lower alkyl and R⁴ is lower alkyl, benzyl, benzyloxy, benzylthio, phenoxy or phenylthio; and 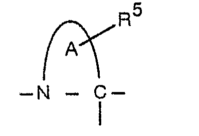 is one of the structures II to VII, preferably being in the form of the free di-carbonic acid or in the form of its alkyl ester, the alkyl group containing 1 to 6 carbon atoms, especially in the form of its monoester wherein the carboxy group attached to the group 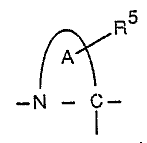 is in the free form, preferably all former compounds being the stereoisomer in which the absolute configurations at each of the three carbon atoms bonded to both a nitrogen and a carbonyl group corresponds most closely to the absolute configuration of L-aminoacids, characterized in that the compound is prepared by an appropriate process selected from the following processes a to i:
a) for the preparation of a compound of formula I, wherein R¹ is hydrogen: condensation of a ketocompound (XIX) with a depeptide (XX) under reduction 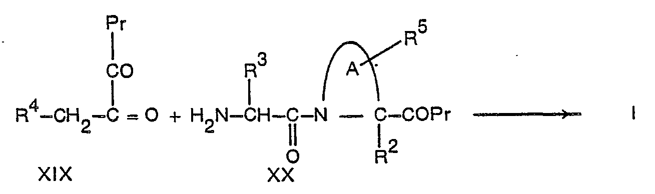
wherein A, R², R³, R⁴ and R⁵ are as defined above and Pr stands for a free or a protected hydroxy group;
b) alkylation of a dipeptide (XX) by means of a compound of formula (XXI) under basic conditions 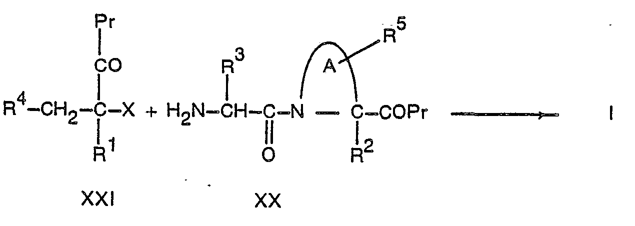 wherein X is chloro, bromo, iodo, alkanesulfonyloxy or arenesulfonyloxy, A, R¹, R², R³, R⁴, R⁵ are as defined above for compounds of formula I and Pr stands for a free or protected hydroxy group;
c) condensation of an aminoacid (XXII) with an aminoacid (XXIII) in the presence of a condensing agent 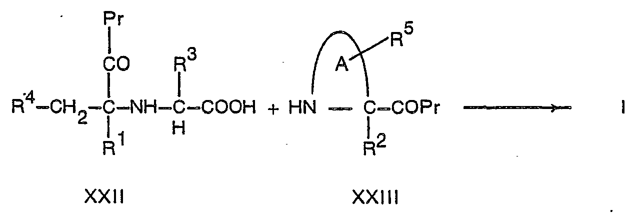 wherein A, R¹, R², R³, R⁴, R⁵ are as defined above for compounds of formula I, and Pr stands for a free or protected (e.g. by esterification) hydroxy group;
d) condensation of an amino compound (XXIV) with a ketocompound (XXV) 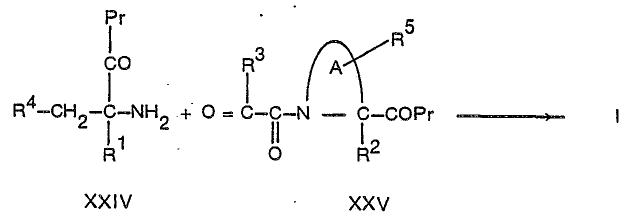 under the conditions described for process a wherein A, R¹, R², R³, R⁴, R⁵ are as defined above for compounds of formula I and Pr stands for a free or protected (e.g. by esterification) hydroxy group;
e) alkylation of an amino compound (XXIV) by means of a compound (XXVI) 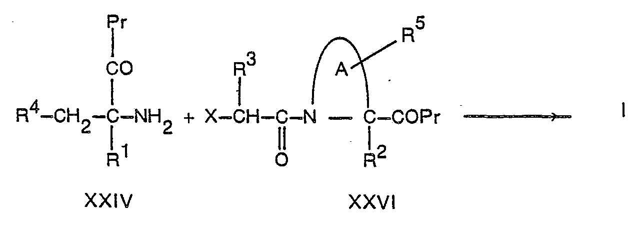 wherein X is chloro, bromo, iodo, alkanesulfonyloxy or arenesulfonyloxy, A, R¹, R², R³, R⁴, R⁵ are as defined above for compounds of formula I and Pr stands for a free or protected (e.g. by esterification) hydroxy group, under the conditions described for process b;
f) forthe preparation of a compound of formula I, wherein one of R³, R⁴ and R⁵ is a group Z―(CH₂)₀₋₆―, wherein Z is Z⁵, Z⁶, Z⁷, Z⁸, Z⁹ or Z¹⁰, preferably Z⁷, Z⁸ or Z⁹: condensation of a peptide of the general formula (XXX) with a compound containing the desired group (XXXI) 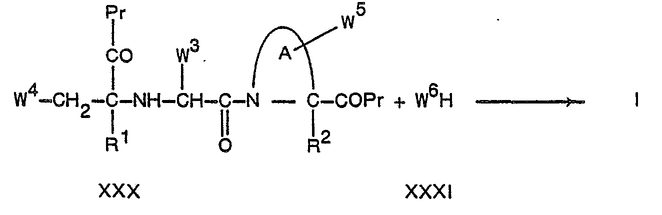 wherein R¹, R², and A are as defined for formula I, Pr is a protected hydroxy group W³, W⁴ and W⁵ are defined like R³, R⁴ and R⁵ respectively with the difference that one of W³, W⁴ and W⁵ contains an NH₂-group instead of the respective Z⁵ to Z¹⁰-group; and W⁶ is Z⁵, Z⁶, Z⁷, Z⁸, Z⁹ or Z¹⁰;
g) for the preparation of a compound of formula I, wherein one of R³, R⁴ and R⁵ is a group Z―(CH₂)₀₋₆―, wherein Z is Z¹, Z² or Z³: condensation of a peptide of formula XXXII with an appropriately substituted compound of formula XXXIII 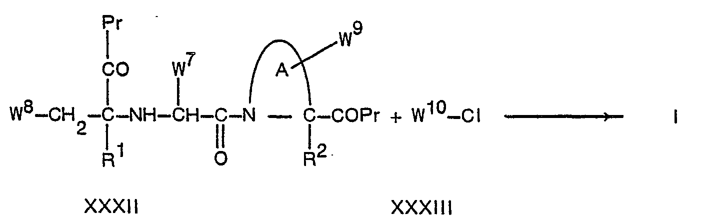 wherein R¹, R² and A are as defined for formula I, Pr is a protected hydroxy group, W⁷, W⁸ and W⁹ are defined like R³, R⁴ and R⁵ respectively, with the difference that one of W⁷, W⁸ and W⁹ contains an NH₂-group instead of the respective Z¹, Z² or Z³ group, and W¹⁰ is 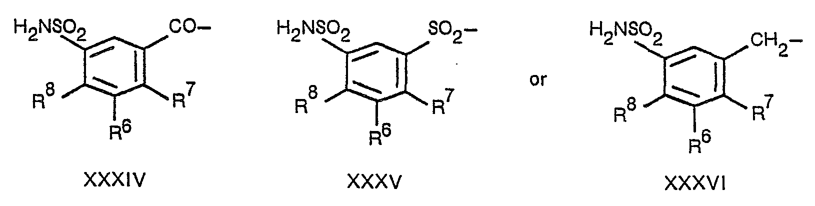
h) for the preparation of a compound of formula I, wherein one of R³, R⁴ and R⁵ is a group Z―(CH₂)₀₋₆―. wherein Z is Z⁴: condensation of a peptide of formula (XXXVII) with a 3 halomethylbenzothiadiazine (XXXVIII) 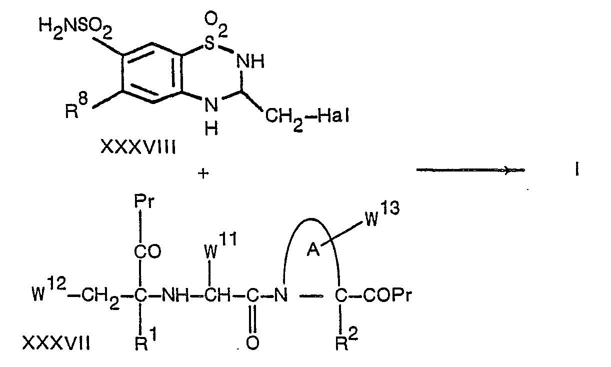 wherein R¹, R² and A are as defined for formula I, Pr is a protected hydroxy group, W¹¹, W¹² and W¹³ are defined like R³, R⁴ and R⁵ respectively with the difference that one of W¹¹, W¹² and W¹³ contains a ―SH- group instead of the respective Z⁴-group, and Hal is halogen, preferably chloro;
i) for the preparation of a compound of formula I, wherein one of R³, R⁴ and R⁵ is a group Z―(CH₂)₀₋₆―, wherein Z is Z⁵ or Z⁶; condensation of a peptide of formula XXXIX with a compound of formula XXXX 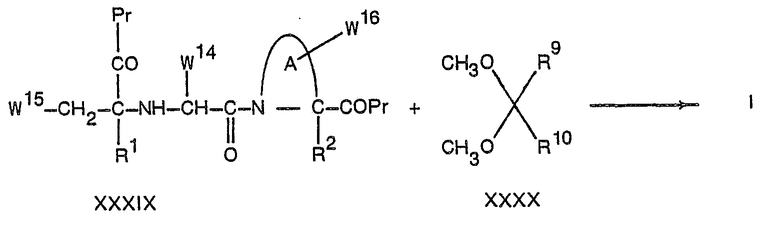 wherein R¹, R², R⁹, R¹⁰ and A are as defined for formula I, Pr is a protected hydroxy group, W¹⁴, W¹⁵ and W¹⁶ are defined like R³, R⁴ and R⁵ respectively with the difference that one of W¹⁴, W¹⁵ and W¹⁶ contains the group 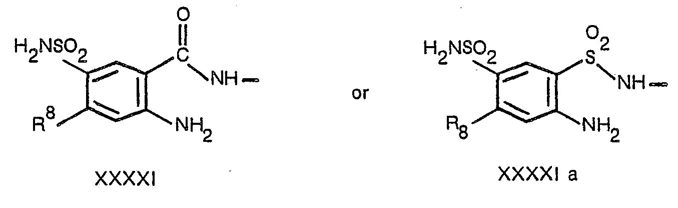 respectively instead of the group Z⁵ or Z⁶ respectively; followed by removal of the protecting groups, if necessary, to yield the desired product, and if desired, converting a so obtained compound of formula I into its ester and/ or setting free the compound of formula I from its ester or preparing a salt thereof and, if desired, isolating the preferred isomer,

2. Process according to claim 1, characterized in that a compound is prepared, wherein R⁴ is a group Z―(CH₂)₀₋₆― as defined in claim 1, wherein Z preferably is Z¹, Z², Z³, Z⁵, Z⁷, Z⁸, Z⁹ or Z¹⁰;
R⁴ preferably being Z¹―(CH₂)₂ or ₃― Z²―(CH₂)₂ or ₃―, Z³―(CH₂)₂ or ₃―, Z⁵―(CH₂)₂ or ₃―, Z⁷―(CH₂)₂ or ₃―, Z⁸―(CH₂)₂ or ₃―, Z⁹―(CH₂)₂ or ₃― or Z¹⁰―(CH₂)₂ or ₃―' and wherein preferably the group 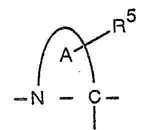 is the group of formula II, IV (wherein B is a saturated ring) or VIII, preferably R⁵ being hydrogen.

3. Process according to claim 1 or 2, characterized in that a compound is prepared, wherein R¹ and R² are hydrogen, and/or when Z is of the formula IX, X or XI R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety Z is chloro, and/or R³ is methyl.

4. Process according to claim 1 or 2, characterized in that a compound is prepared, wherein R¹ and R² are hydrogen, the group 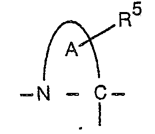 is the group of formula IV, wherein B is a saturated ring and R⁵ is hydrogen, R⁴ is Z¹―(CH₂)₃― or Z²―(CH₂)₃―, wherein R⁶ is hydrogen, R⁷ is hydrogen or hydroxy, and R⁸ is chloro; and R³ is methyl, preferably being
1-{N-[1(S)-ethoxycaronyl-5-(4-chloro-3-sulfamoyl)-benzenesulfonaminopentyl]-(S)-alanyl} - cis,syn - octahydro - 1H - indole - 2(S) - carboxylic acid,
1-{N-[1(S)-ethoxycarbonyl-5-(4-chloro-3-sulfamoyl)-benzamidopentyl]- (S)-alanyl}-cis,syn-octahydro - 1H - indole - 2(S) - carboxylic acid, or
1 - {N - [1(S) - ethoxycarbonyl - 5 - (4 - chloro - 2 - hydroxy - 5 - sulfamoyl) - benzamidopentyl) - (S) - alanyl} - cis,syn - octahydro - 1H- indole - 2(S) - carboxylic acid,
1 - {N - [1 (S) - carboxy - 5 - [[(4 - chloro - 2 - hydroxy - 5 - sulfamoylphenyl) carbonyl]amino]pentyl] - (S) - alanyl} - cis,syn - octahydro - 1H- indole - 2(S) - carboxylic acid,
1 - {N - [1 (S) - carboxy - 5 - [[(4 - chloro - 3 - (N - methyl sulfamoyl) phenyl] carbonyl]amino]pentyl] - (S) - alanyl} - cis,syn - octahydro - 1H- indole - 2(S) - carboxylic acid,
1 - {N - [1(S) - carboxy - 5 - [[(4 - chloro -.3 - sulfamoyl phenyl) carbonyl]amino]pentyl] - (S) - alanyl} - cis,syn - octahydro - 1H - indole - 2(S) - carboxylic acid, in the free form or in the form of its ester, preferably in the form of its mono-or-di-ethyl ester.

5. Process according to claim 1, characterized in that a compound is prepared, wherein R³ is a group Z―(CH₂)₀₋₆― as defined in claim 1, preferably Z¹―(CH₂)₄―, Z²―(CH₂)₄―, Z³―(CH₂)₄―, Z⁴CH₂―, Z⁵―(CH₂)₄―, Z⁶(CH₂)₄―, Z⁷―(CH₂)₄―, Z⁸―(CH₂)₄―, Z⁹―(CH₂)₄― or Z¹⁰―(CH₂)₄―, and wherein the group 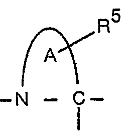 is preferably the group of formula II, IV (wherein B is a saturated ring) or VIII.

6. Process according to claim 5, characterized in that a compound is prepared, wherein R¹ and R² are hydrogen, and/or Z is of the formula IX, X or XI, R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety z is chloro, and/or R⁴ is benzyl or ethyl.

7. Process according to claim 5, characterized in that a compound is prepared, wherein R¹ and R² are hydrogen, the group 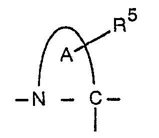 is the group of formula IV, wherein B is a saturated ring and R⁵ is hydrogen, R³ is Z¹―(CH₂)₄― or Z²―(CH₂)₄―, wherein R⁶ and R⁷ are hydrogen, and R⁸ is chloro, and R⁴ is benzyl, preferably being
1 - {Nα - [1(S) - ethoxycarbonyl - 3 - phenylpropyl] - Nε - [(4 - chloro - 3 - sulfamoyl)benzenesulfonyl] - (S) - lysyl} - cis,syn - octahydro - 1H - indole - 2(S) - carboxylic acid or
1 - {Nα - [1 (S) - ethoxycarbonyl - 3 - phenylpropyl] - Ne - [(4 - chloro - 3 - sulfamoyl)benzoyl - (S) - lysyl} - cis,syn - octahydro - 1H - indole - 2(S) - carboxylic acid in the free form or in the form of its ester, preferably in the form of its mono-or-di-ethyl ester.

8. Process according to claim 1, characterized in that a compound is prepared, wherein R⁵ is a group Z―(CH₂)₀₋₆― as defined in claim 1, preferably being Z¹, Z², Z³, Z⁵, Z⁷, Z⁸, Z⁹ or Z¹⁰; wherein the group 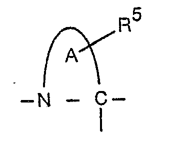 is preferably the group of formula II, VI (wherein B is an aromatic ring), or IV (wherein B is a saturated ring), preferably

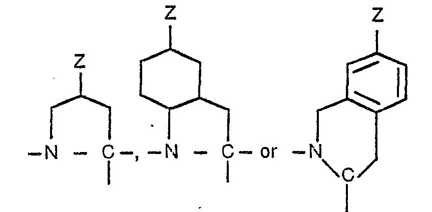

9. Process according to claim 8, characterised in that a compound is prepared, wherein R¹ and R² are hydrogen, and/or when Z is of the formula IX, X or XI R⁶ is hydrogen and R⁷ is hydrogen or hydroxy, and/or when Z is of the formula XIII or XIV R⁹ and R¹⁰ are independently hydrogen or methyl, and/or R⁸ in the definition of the moiety Z is chloro, and/or R³ is methyl and/or R⁴ is benzyl or ethyl, the compound preferably being
7 - (4 - chloro - 3 - sulfamoylbenzamido) - 2 - {N - [1(S) - ethoxycarbonyl - 3 - phenylpropyl] - (S) - alanyl} - 1,2,3,4 - tetrahydroisoquinoline - 3(S) - carboxylic acid or the corresponding 1 - S - carboxy- compound.

10. Process for the preparation of a pharmaceutical composition comprising a compound of the general formula I or pharmaceutically acceptable salt or ester thereof as defined in any one of claims 1 to 9, characterized in that active ingredient is brought into a form suitable for therapeutic application.

## Patentansprüche (Patentansprüche für folgende(n) Vertragsstaat(en) : BE CH DE FR GB IT LI LU NL SE)

1. Verbindungen der Formel 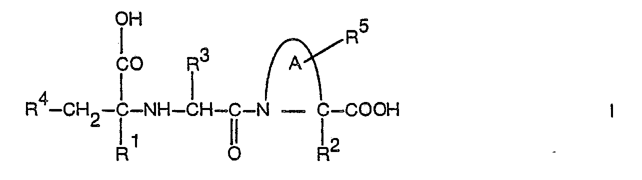 und deren pharmazeutisch unbedenkliche Ester sowie die pharmazeutisch unbedenklichen Salze der vorgenannten Verbindungen, worin
R¹ und R² unabhängig voneinander Wasserstoff oder Niederalkyl sind, die Gruppe 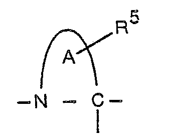 eine der Strukturen II bis VIII ist, 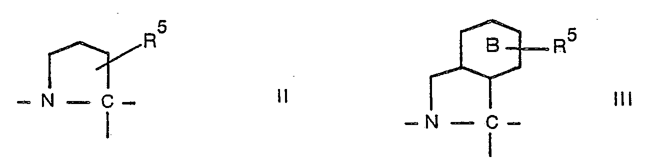 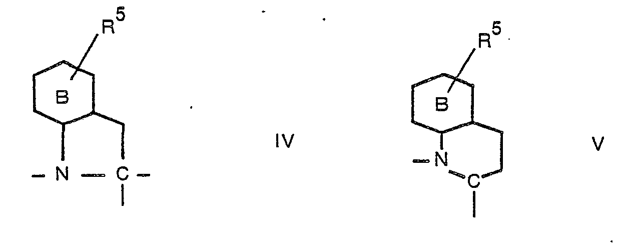 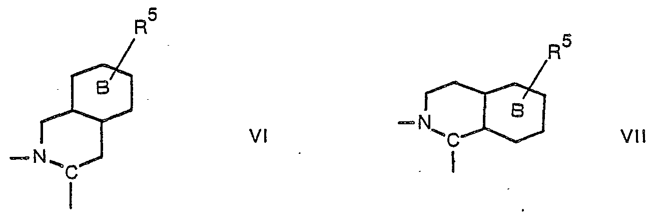 (worin B ein gesättigter oder aromatischer Ring ist) oder 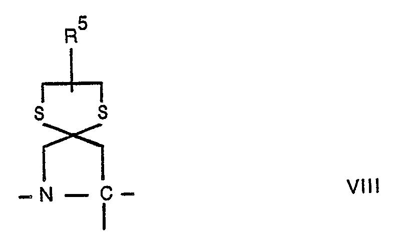 einer der Substituenten R³, R⁴ und R⁵ eine Gruppe Z―(CH₂)₀₋₆― ist, worin Z eine der nachstehenden Bedeutungen Z¹ bis Z¹⁰ besitzt, 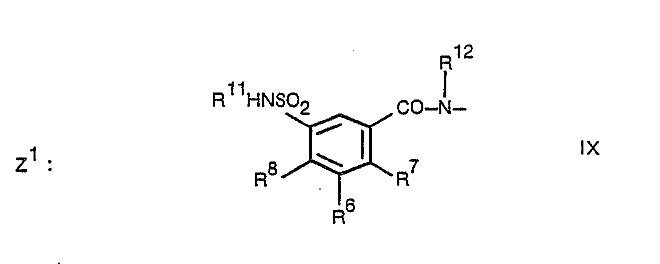 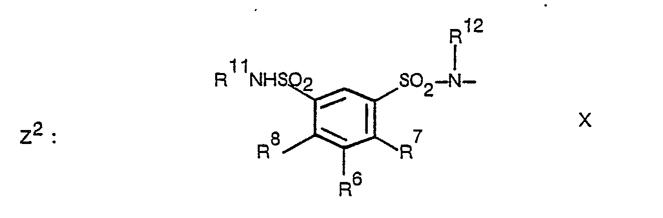 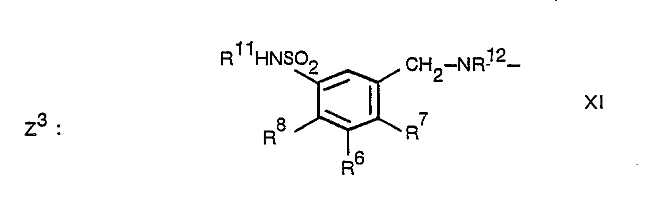 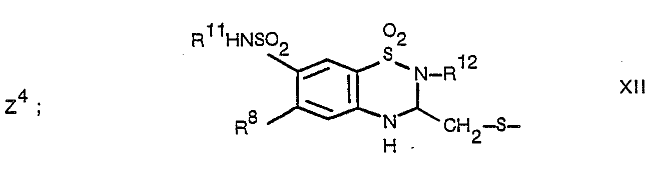 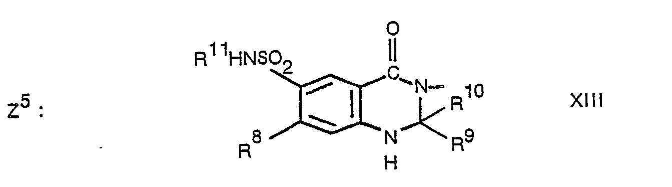 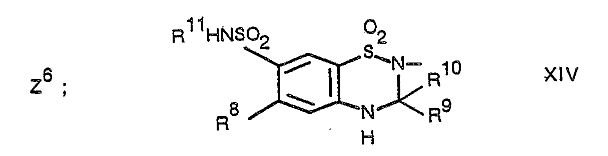 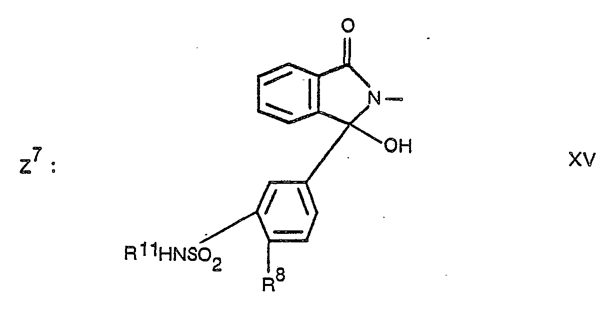 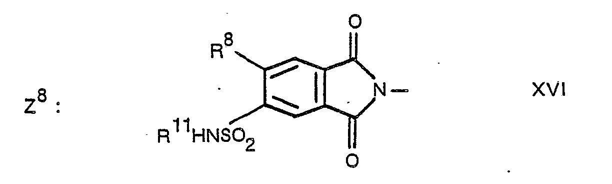 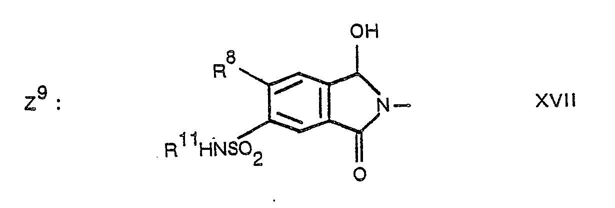 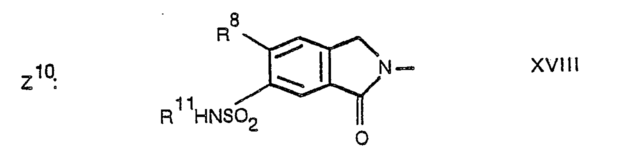 worin
R⁸ Cl oder CF₃ ist,
R⁶ Wasserstoff oder Halogen ist,
R⁷ Wasserstoff, Halogen, Carboxy, Hydroxy oder Amino ist,
R⁹ und R¹⁰ unabhängig voneinander Wasserstoff, Niederalkyl oder Halogeno-niederalkyl sind und R⁹ auch Phenyl oder Phenyl-niederalkyl sein kann,
R" Wasserstoff oder Niederalkyl ist,
R¹² Wasserstoff; Niederalkyl oder Phenyl-niederalkyl ist, wobei, wenn R³ die Gruppe Z―(CH₂)₀₋₆― ist, dann
R³ Z¹―(CH₂)₁₋₆―, Z²―(CH₂)₁₋₆― Z³(CH₂)₁₋₆―, Z⁴―(CH₂)―, Z⁵―(CH₂)₁₋₆―, Z⁶―(CH₂)₁₋₆. Z⁷―(CH₂)₁₋₆―, Z⁸―(CH₂)₁₋₆―, Z⁹―(CH₂)₁₋₆― oder Z¹⁰―(CH₂)₁₋₆― ist,
R⁴ Niederalkyl, Benzyl, Benzyloxy, Benzylthio, Phenoxy oder Phenylthio ist,
R⁵ Wasserstoff ist und die Gruppe eine der Strukturen 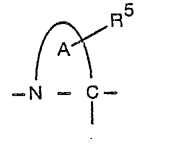 II bis VIII ist, und wenn R⁴ die Gruppe Z―(CH₂)₁₋₆― ist dann
R⁴ Z¹―(CH₂)₀₋₆―, Z²―(CH₂)₀₋₆― Z³(CH₂)₀₋₆― Z⁴―(CH₂)₀₋₆―, Z⁵―(CH₂)₀₋₆― Z⁶―(CH₂)₀₋₆―, Z⁷―(CH₂)₀₋₆―, Z⁸―(CH₂)₀₋₆―, Z⁹―(CH₂)₀₋₆― oder Z¹⁰(CH₂)₀₋₆― ist und
R³ Wasserstoff, Niederalkyl oder Amino-niederalkyl ist und
R⁵ Wasserstoff ist und die Gruppe eine der Strukturen 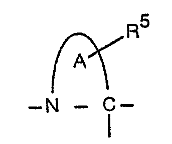 II bis VIII ist, und wenn R⁵ die Gruppe Z―(CH₂)₀₋₆― ist, dann
R⁵ Z¹, Z², Z³, Z⁴, Z⁵, Z⁶, Z⁷, Z⁸, Z⁹ oder Z¹⁰ ist,
R³ Wasserstoff, Niederalkyl oder Amino-niederalkyl ist und
R⁴ Niederalkyl, Benzyl, Benzyloxy, Benzylthio, Phenoxy oder Phenylthio ist, und die Gruppe eine der Strukturen 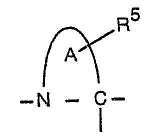 II bis VII ist, vorzugsweise in der Form der freien Dikohlensäure oder in Form ihres Alkylesters, wobei die Alkyl-Gruppe 1 bis 6 Kohlenstoff-Atome enthält, insbesondere in Form ihres Monoesters, in der die an die Gruppe 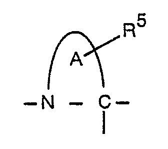 gebundene Carboxy-Gruppe in freier Form vorliegt, wobei vorzugsweise alle vorgenannten Verbindungen in der Form desjenigen Stereoisomeres vorliegen, in dem die Absolutkonfigurationen jedes der drei sowohl an einen Stickstoff als auch an eine Carbonyl-Gruppe gebundenen Kohlenstoff-Atome in der nächstmöglichen Weise der Konfiguration von L-Aminosäuren entspricht.

2. Verbindung nach Anspruch 1, dadurch gekennzeichnet, daß R⁴ eine Gruppe Z―(CH₂)₀₋₆― nach Anspruch 1 ist, wobei Z vorzugsweise Z¹, Z², Z³, Z⁵, Z⁷, Z⁸, Z⁹ oder Z¹⁰ ist, wobei R⁴ vorzugsweise Z¹―(CH₂)₂ _{oder 3}―, Z²―(CH₂)_{2 oder 3}― Z³―(CH₂)_{2 oder 3}―, Z⁵―(CH₂)_{2 oder 3}―, Z⁷―(CH₂)_{2 oder 3}―, Z⁸―(CH₂)_{2 oder 3}―, Z⁹(CH₂)_{2 oder 3}―, Z¹⁰―(CH₂)_{2 oder 3}― ist, und worin vorzugsweise die Gruppe 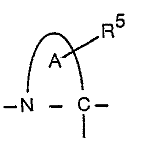 die Gruppe der Formel II, IV (worin B ein gesättigter Ring ist) oder VIII ist, wobei R⁵ vorzugsweise Wasserstoff ist.

3. Verbindung nach Anspruch 1 oder 2, dadurch gekennzeichnet, daß R¹ und R² Wasserstoff sind und/ oder, wenn Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in der Definition der Struktureinheit Z Chloro ist und/oder R³ Methyl ist.

4. Verbindung nach Anspruch 1 oder 2, dadurch gekennzeichnet, daß R¹ und R² Wasserstoff sind, die Gruppe 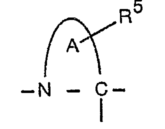 die Gruppe der Formel IV ist, worin B ein gesättigter Ring ist und R⁵ Wasserstoff ist, R⁴ Z¹―(CH₂)₃― oder Z₂―(CH₂)₃― ist, worin R⁶ Wasserstoff ist, R⁷ Wasserstoff oder Hydroxy ist und R⁸ Chloro ist, und R³ Methyl ist, vorzugsweise
1 - {N - [1(S) - Ethoxycarbonyl - 5 - (4 - chloro - 3 - sulfamoyl) - benzolsulfonaminopentyl] - (S) - alanyl} - cis,syn - octahydro - 1 H - indol - 2(S) - carbonsäure,
1 - {N - [1 (S) - Ethoxycarbonyl - 5 - (4 - chloro - 3 - sulfamoyl) - benzamidopentyl] - (S) - alanyl} - cis,syn - octahydro - 1 H - indol - 2(S) - carbonsäure oder
1 - {N - [1(S) - Ethoxycarbonyl - 5 - (4 - chloro - 2 - hydroxy - 5 - sulfamoyl) - benzamidopentyl] - (S) - alanyl} - cis,syn - octahydro - 1 H - indol - 2(S) - carbonsäure,
1 - {N - [1(S) - Carboxy - 5 - [[(4 - chloro - 2 - hydroxy - 5 - sulfamoyl - phenyl)carbonyl]amino]pentyl] - (S) - alanyl} - cis,syn - octahydro - 1 H - indol - 2(S) - carbonsäure,
1 - {N - [1 (S) - Carboxy - 5 - [[(4 - chloro - 3 - (N - methylsulfamoyl) - phenyl)carbonyl]amino]pentyl] - (S) - alanyl} - cis,syn - octahydro - 1 H - indol - 2(S) - carbonsäure,
1 - {N - [1(S) - Carboxy - 5 - [[(4 - chloro - 3 - sulfamoylphenyl)carbonyl]amino]pentyl] - (S) - alanyl} - cis,syn - octahydro - 1H - indol - 2(S) - carbonsäure, in freier Form oder in Form ihrer Ester, vorzugsweise in Form ihres Mono- oder Diethylesters.

5. Verbindung nach Anspruch 1, dadurch gekennzeichnet, daß R³ eine Gruppe Z―(CH₂)₀₋₆― nach Anspruch 1 ist, vorzugsweise Z¹―(CH₂)₄―, Z²―(CH₂)₄―, Z³―(CH₂)₄―, Z⁴―CH₂―, Z⁵―(CH₂)₄―, Z⁶―(CH₂)₄―; Z⁷―(CH₂)₄―, Z⁸(CH₂)₄―, Z⁹―(CH₂)₄ oder Z¹⁰―(CH₂)₄―, und worin vorzugsweise die Gruppe 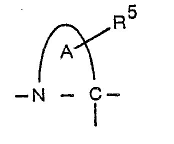 die Gruppe der Formel II, IV (worin B ein gesättigter Ring ist) oder VIII ist.

6. Verbindung nach Anspruch 5, dadurch gekennzeichnet, daß R¹ und R² Wasserstoff sind und/oder Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in .der Definition der Struktureinheit Z Chloro ist und/oder R⁴ Benzyl oder Ethyl ist.

7. Verbindung nach Anspruch 5, dadurch gekennzeichnet, daß R¹ und R² Wasserstoff sind, die Gruppe vorzugsweise die Gruppe 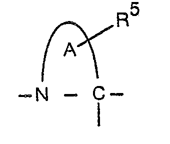 der Formel IV ist, worin B ein gesättigter Ring ist und R⁵ Wasserstoff ist, R³ Z¹―(CH₂)₄― oder Z²―(CH₂)₄― ist, worin R⁶ und R⁷ Wasserstoff sind, und R⁸ Chloro ist, und R⁴ Benzyl ist, vorzugsweise
1 - {Nα - [1 (S) - Ethoxycarbonyl - 3 - phenylpropyl] - Ne - [(4 - chloro - 3 - sulfamoyl)benzolsulfonyl - (S) - lysyl} - cis,syn - octahydro - 1H - indol - 2(S) - carbonsäure oder
1 - {Nα - [1 (S) - Ethoxycarbonyl - 3 - phenylpropyl] - Ne - [(4- chloro - 3 - sulfamoyl)benzoyl - (S) - lysyl} - cis,syn - octahydro - 1H - indol - 2(S) - carbonsäure, in freier Form oder in Form ihres Esters, vorzugsweise in Form ihres Mono- oder Diethylesters.

8. Verbindung nach Anspruch 1, dadurch gekennzeichnet, daß R⁵ eine Gruppe Z―(CH₂)₀₋₆― nach Anspruch 1 ist, vorzugsweise Z¹, Z², Z³, Z⁵, Z⁷, Z⁸, Z⁹ oder Z¹⁰, worin die Gruppe vorzugsweise die Gruppe 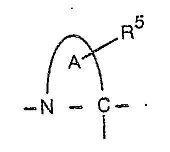 der Formel II, VI (worin B eine aromatischer Ring ist) oder IV (worin B ein gesättigter Ring ist) ist, vorzugsweise

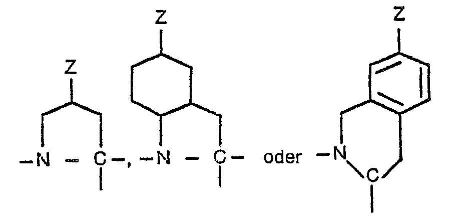

9. Verbindung nach Anspruch 8, dadurch gekennzeichnet, daß R¹ und R² Wasserstoff sind und/oder, wenn Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in der Definition der Struktureinheit Z Chloro ist und/oder R³ Methyl ist und/oder R⁴ Benzyl oder Ethyl ist, wobei die Verbindung vorzugsweise
7 - (4 - Chloro - 3 - sulfamoylbenzamido) - 2 - {N - [1 (S) - ethoxycarbonyl - 3 - phenylpropyl] - (S) - alanyl} - 1,2,3,4 - tetrahydroisochinolin - 3 - (S) - carbonsäure oder die entsprechende 1-S-Carboxy-Verbindung ist.

10. Verfahren zur Herstellung einer Verbindung der allgemeinen Formel I nach Anspruch 1 bis 9, dadurch gekennzeichnet, daß die Verbindung hergestellt wird durch ein geeignetes Verfahren ausgewählt aus den nachstehenden Verfahren a bis i:
a) zur Herstellung einer Verbindung der Formel I, in der R¹ Wasserstoff ist:
Kondensation einer Keto-Verbindung (XIX) mit einem Dipeptid (XX) unter Reduktion 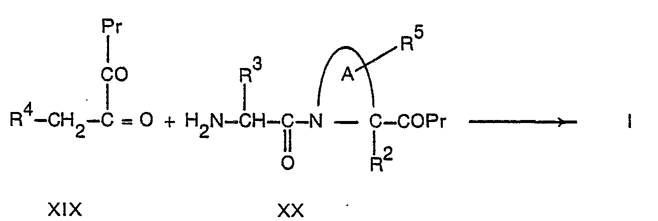 worin A, R², R³, R⁴ und R⁶ die oben angegebenen Bedeutungen haben und Prfür eine freie oder geschützte Hydroxy-Gruppe steht;
b) Alkylierung eines Dipeptids (XX) mittels einer Verbindung der Formel (XXI) unter basischen Bedingungen 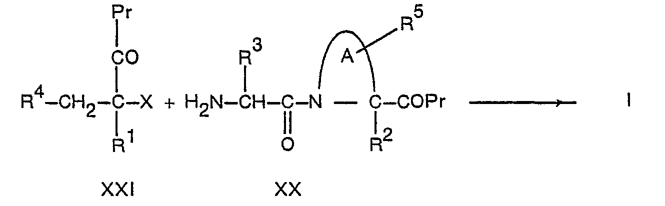 worin X Chloro, Bromo, lodo, Alkansulfonyloxy oder Arensulfonyloxy ist, A, R¹, R², R³, R⁴ und R⁵ die oben für Verbindungen der Formel I angegebenen Bedeutungen haben und Pr für eine freie oder geschützte Hydroxy-Gruppe steht;
c) Kondensation einer Aminosäure (XXII) mit einer Aminosäure (XXIII) in Gegenwart eines kondensierenden Mittels 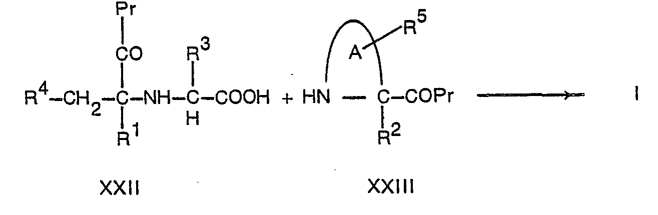 worin A, R¹, R², R³, R⁴ und R⁵ die oben für Verbindungen der Formel angegebenen Bedeutungen haben und Pr für eine freie oder geschützte (z.B. durch Veresterung) Hydroxy-Gruppe steht;
d) Kondensation einer Amino-Verbindung (XXIV) mit einer Keto-Verbindung (XXV) 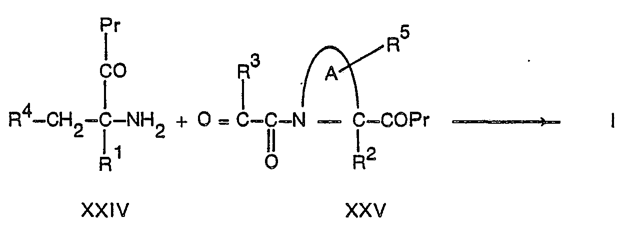 unter den für das Verfahren A beschriebenen Bedingungen, wobei A, R¹, R², R³, R⁴ und R⁵ die oben für Verbindungen der Formel I angegebenen Bedeutungen haben und Pr für eine freie oder geschützte (z.B. durch Veresterung) Hydroxy-Gruppe steht;
e) Alkylierung einer Amino-Verbindung (XXIV) mittels einer Verbindung (XXVI) 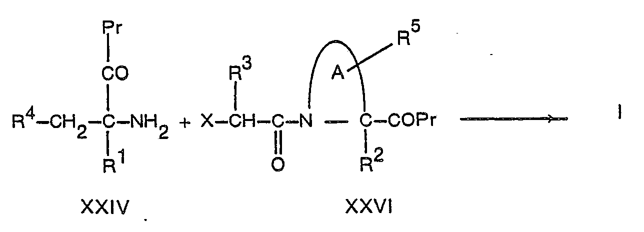 worin X Chloro, Bromo, lodo, Alkansulfonyloxy oder Arensulfonyloxy ist, A, R¹, R², R³, R⁴, R⁵ die oben für Verbindungen der Formel I angegebenen Bedeutungen haben und Pr für eine freie oder geschützte (z.B. durch Veresterung) Hydroxy-Gruppe steht, unter den für Verfahren b beschriebenen Bedingungen;
f) zur Herstellung von Verbindungen der Formel in denen einer der Substituenten R³, R⁴ und R⁵ eine Gruppe Z―(CH₂)₀₋₆― ist, worin Z Z⁵, Z⁶, Z⁷, Z⁸, Z⁹ oder Z¹⁰ ist, vorzugsweise Z⁷, Z⁸ oder Z⁹:
Kondensation eines Peptids der allgemeinen Formel (XXX) mit einer die gewünschte Gruppe enthaltenden Verbindung (XXXI) 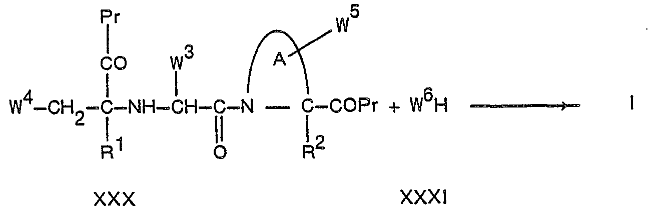 worin R¹, R² und A die für Formel angegebenen Bedeutungen haben, Pr eine geschützte Hydroxy-Gruppe ist, W³, W⁴ und W⁵ den Definitionen für R³, R⁴ bzw. R⁵ entsprechen, jedoch mit dem Unterschied, daß einer der Substituenten W³, W⁴ und W⁵ eine NH₂-Gruppe anstelle der betreffenden Gruppe Z⁵ bis Z¹⁰ enthält, und W⁶ Z⁵, Z⁶, Z⁷, Z⁸, Z⁹ oder Z¹⁰ ist;
g) zur Herstellung von Verbindungen der Formel in denen einer der Substituenten R³, R⁴ und R⁵ eine Gruppe Z―(CH₂)₀₋₆― ist, worin Z Z¹, Z² oder Z³ ist:
Kondensation eines Peptids der Formel XXXII mit einer passend substituierten Verbindung XXXIII 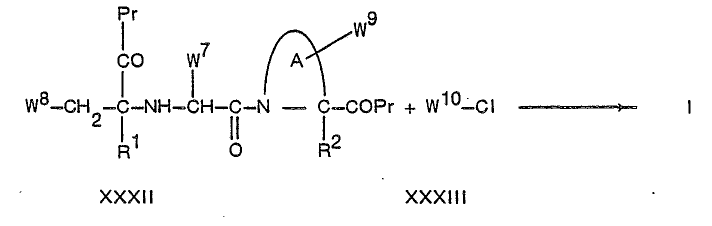 worin R¹, R² und A die für Formel I angegebenen Bedeutungen haben, Pr eine geschützte Hydroxy-Gruppe ist, W⁷, W⁸ und W⁹ den Definitionen für R³, R⁴ bzw. R⁵ entsprechen, jedoch mit dem Unterschied, daß einer der Substituenten W⁷, W⁸ und W⁹ eine NH₂-Gruppe anstelle der betreffenden Gruppe Z¹, Z² enthält und W¹⁰ 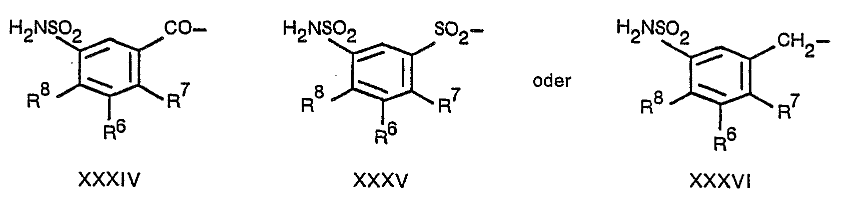 ist;
h) zur Herstellung von Verbindungen der Formel in denen einer der Substituenten R³, R⁴ und R⁵ eine Gruppe Z―(CH₂)₀₋₆―ist, worin Z Z⁴ ist:
Kondensation eines Peptids der Formel (XXXVII) mit einem 3-Halogenomethyl-benzothiadiazin (XXXVIII) 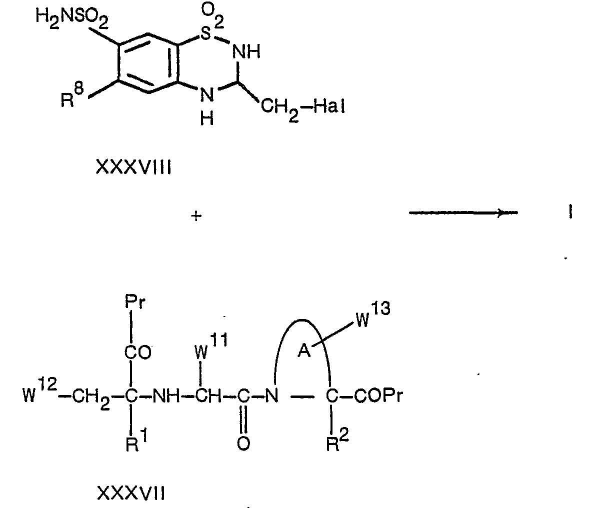 worin R¹, R² und A die für Formel I angegebenen Bedeutungen haben, Pr eine geschützte Hydroxy-Gruppe ist, W", W¹² und W¹³ den Definitionen für R³, R⁴ bzw. R⁵ entsprechen, jedoch mit dem Unterschied, daß einer der Substituenten W¹¹, W¹² und W¹³ eine SH-Gruppe anstelle der betreffenden Gruppe Z⁴ enthält, und Hal Halogen, vorzugsweise Chloro ist;
i) zur Herstellung von Verbindungen der Formel I, in denen einer der Substituenten R³, R⁴ und R⁵ eine Gruppe Z―(CH₂)₀₋₆― ist, worin Z Z⁵ oder Z⁶ ist:
Kondensation eines Peptids der Formel XXXIX mit einer Verbindung der Formel XXXX 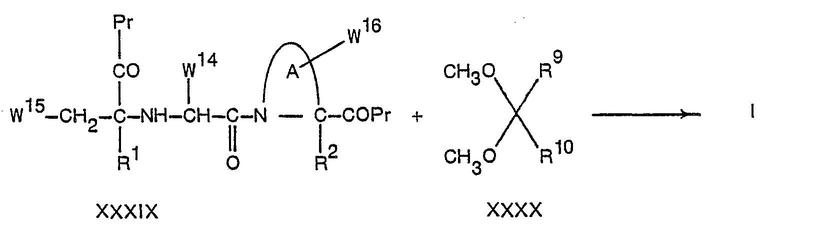 worin R¹, R², R⁹, R¹⁰ und A die für Formel angegebenen Bedeutungen haben, Pr eine geschützte Hydroxy-Gruppe ist, W¹⁴, W¹⁵ und W¹⁶ den Definitionen für R³, R⁴ bzw. R⁵ entsprechen, jedoch mit dem Unterschied, daß einer der Substituenten W¹⁴, W¹⁵ und W¹⁶ die Gruppe 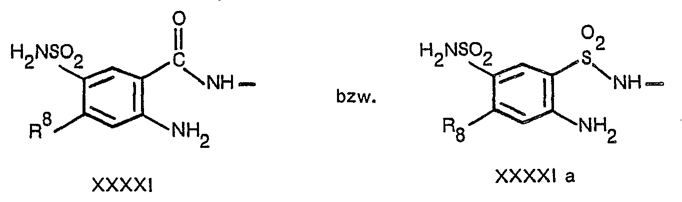 anstelle der betreffenden Gruppe Z⁵ bzw. Z⁶ enthält,
gefolgt von, sofern zur Gewinnung des gewünschten Produkts erforderlich, der Entfernung der Schutzgruppen und, gewünschtenfalls, der Umwandlung der so erhaltenen Verbindung der Formel I in ihren Ester und/oder Freisetzen der Verbindung der Formel aus ihrem Ester oder Herstellung eines Salzes derselben und, gewünschtenfalls, Isolierung des bevorzugten Isomers.

11. Pharmazeutische Zusammensetzung, enthaltend eine Verbindung der Allgemeinen Formel I oder ein pharmazeutisch unbedenkliches Salz derselben oder einen Ester derselben nach einem der Ansprüche 1 bis 9 oder hergestellt mittels des Verfahrens nach Anspruch 10.

## Patentansprüche (Patentansprüche für folgende(n) Vertragsstaat(en) : AT)

1. Verfahren zur Herstellung von Verbindungen der Formel 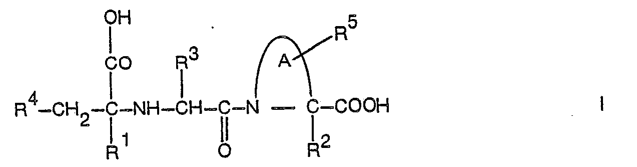 und deren pharmazeutisch unbedenklicher Ester sowie der pharmazeutisch unbedenklichen Salze der vorgenannten Verbindungen, worin
R¹ und R² unabhängig voneinander Wasserstoff oder Niederalkyl sind, die Gruppe 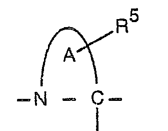 eine der Strukturen II bis VIII ist, 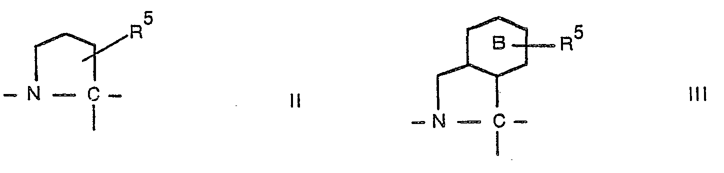 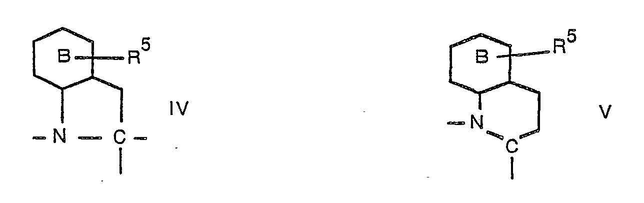 (worin B ein gesättigter oder aromatischer Ring ist) oder 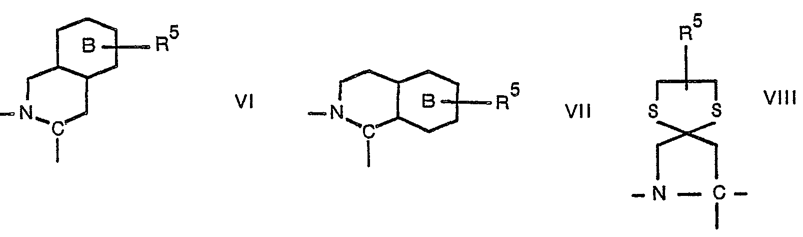 einer der Substituenten R³, R⁴ und R⁵ eine Gruppe Z―(CH₂)₀₋₆― ist, worin Z eine der nachstehenden Bedeutungen Z¹ bis Z¹⁰ besitzt, 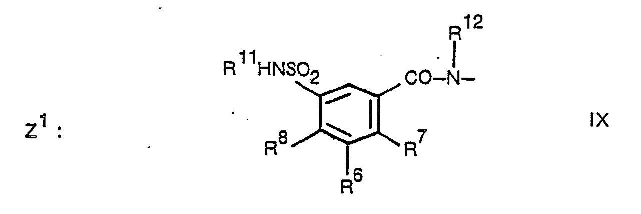 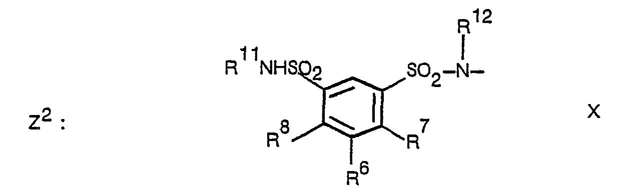 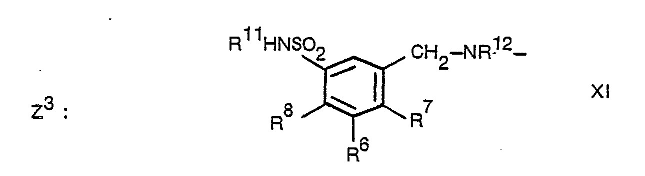 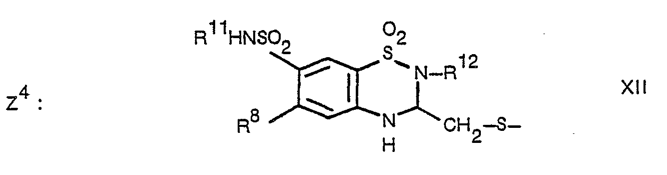 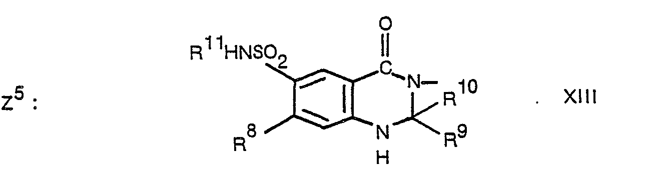 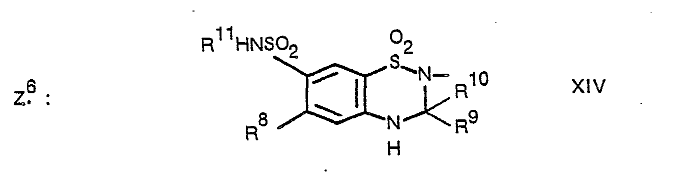 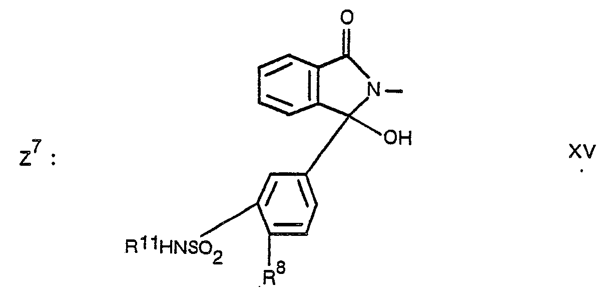 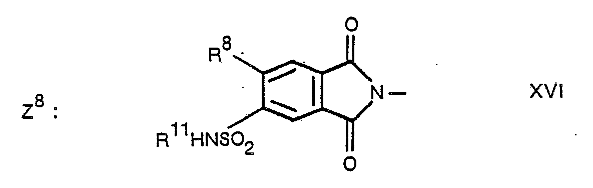 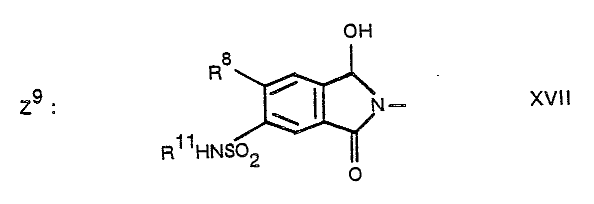 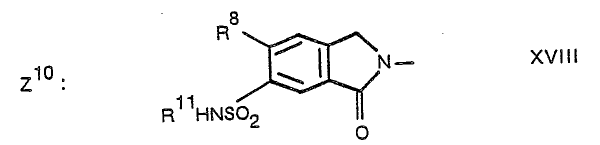 worin
R⁸ Cl oder CF₃ ist,
R⁶ Wasserstoff oder Halogen ist,
R⁷ Wasserstoff, Halogen, Carboxy, Hydroxy oder Amino ist,
R⁹ und R¹⁰ unabhängig voneinander Wasserstoff, Niederalkyl oder Halogeno-niederalkyl sind und R⁹ auch Phenyl oder Phenyl-niederalkyl sein kann,
R¹¹ Wasserstoff oder Niederalkyl ist,
R¹² Wasserstoff; Niederalkyl oder Phenyl-niederalkyl ist, wobei, wenn R³ die Gruppe Z―(CH₂)₀₋₆― ist, dann
R³ Z¹―(CH₂)₁₋₆―, Z₂―(CH₂)₁₋₆―, Z³―(CH₂)₁₋₆―, Z⁴―(CH₂)―, Z⁵―(CH₂)₁₋₆―, Z⁶―(CH₂)₁₋₆―, Z⁷―(CH₂)₁₋₆―, Z⁸―(CH₂)₁₋₆―, Z⁹―(CH₂)₁₋₆― oder Z¹⁰―(CH₂)₁₋₆ ―ist,
R⁴ Niederalkyl, Benzyl, Benzyloxy, Benzylthio, Phenoxy oder Phenylthio ist,
R⁵ Wasserstoff ist und die Gruppe eine der Strukturen 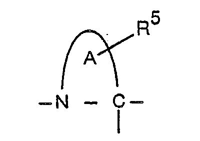 II bis VIII ist, und wenn R⁴ die Gruppe Z―(CH₂)₀₋₆― ist dann
R⁴ Z¹―(CH₂)₀₋₆―, Z²―(CH₂)₀₋₆―, Z³―(CH₂)₀₋₆― Z⁴―(CH₂)₀₋₆―, Z⁵―(CH₂)₀₋₆―. Z⁶―(CH₂)₀₋₆―. Z⁷―(CH₂)₀₋₆―, Z⁸―(CH₂)_{0-6.}―, Z⁹―(CH₂)₀₋₆― oder Z¹⁰―(CH₂)₀₋₆― ist und
R³ Wasserstoff, Niederalkyl oder Amino-niederalkyl ist und R⁵ Wasserstoff ist und die Gruppe eine der Strukturen 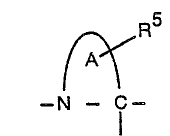 II bis VIII ist, und wenn R⁵ die Gruppe Z―(CH₂)₀₋₆― ist, dann
R⁵ Z¹, Z², Z³, Z⁴, Z⁵, Z⁶, Z⁷, Z⁸, Z⁹ oder Z¹⁰ ist,
R³ Wasserstoff, Niederalkyl oder Amino-niederalkyl ist und
R⁴ Niederalkyl, Benzyl, Benzyloxy, Benzylthio, Phenoxy oder Phenylthio ist, und die Gruppe eine der Strukturen 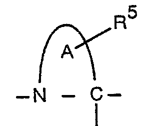 II bis VII ist, vorzugsweise in der Form der freien Dikohlensäure oder in Form ihres Alkylesters, wobei die Alkyl-Gruppe 1 bis 6 Kohlenstoff-Atome enthält, insbesondere in Form ihres Monoesters, in der die an die Gruppe 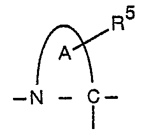 gebundene Carboxy-Gruppe in freier Form vorliegt, wobei vorzugsweise alle vorgenannten Verbindungen in der Form desjenigen Stereoisomeres vorliegen, in dem die Absolutkonfigurationen jedes der drei sowohl an einen Stickstoff als auch an eine Carbonyl-Gruppe .gebundenen Kohlenstoff-Atome in der nächstmöglichen Weise der Konfiguration von L-Aminosäuren entspricht, dadurch gekennzeichnet, daß die Verbindung hergestellt wird durch ein geeignetes Verfahren ausgewählt aus den nachstehenden Verfahren a bis i:
a) zur Herstellung einer Verbindung der Formel I, in der R¹ Wasserstoff ist:
Kondensation einer Keto-Verbindung (XIX) mit einem Dipeptid (XX) unter Reduktion 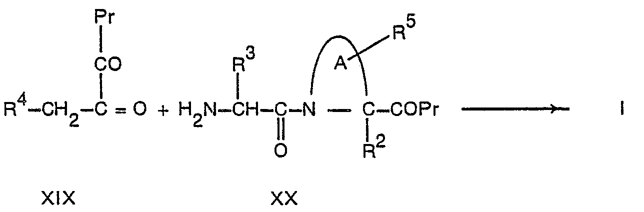 worin A, R², R³, R⁴ und R⁵ die oben angegebenen Bedeutungen haben und Pr für eine freie oder geschützte Hydroxy-Gruppe steht;
b) Alkylierung eines Dipeptids (XX) mittels einer Verbindung der Formel (XXI) unter basischen Bedingungen 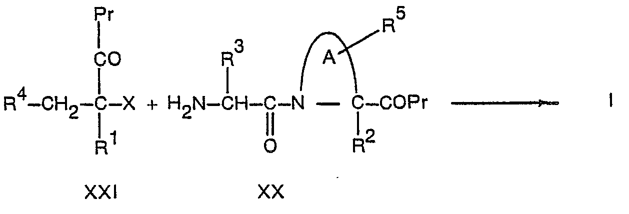 worin X Chloro, Bromo, lodo, Alkansulfonyloxy oder Arensulfonyloxy ist, A, R¹, R², R³, R⁴ und R⁵ die oben für Verbindungen der Formel I angegebenen Bedeutungen haben und Pr für eine freie oder geschützte Hydroxy-Gruppe steht;
c) Kondensation einer Aminosäure (XXII) mit einer Aminosäure (XXIII) in Gegenwart eines kondensierenden Mittels 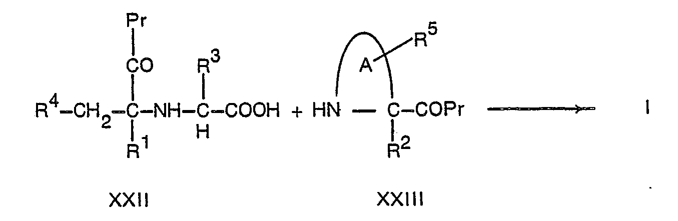 worin A, R¹, R², R³, R⁴ und R⁵ die oben für Verbindungen der Formel I angegebenen Bedeutungen haben und Pr für eine freie oder geschützte (z.B. durch Veresterung) Hydroxy-Gruppe steht;
d) Kondensation einer Amino-Verbindung (XXIV) mit einer Keto-Verbindung (XXV) 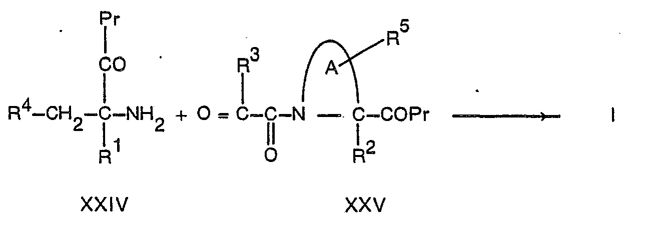 unter den für das Verfahren A beschriebenen Bedingungen, wobei A, R¹, R², R³, R⁴ und R⁵ die oben für Verbindungen der Formel I angegebenen Bedeutungen haben und Pr für eine freie oder geschützte (z.B. durch Veresterung) Hydroxy-Gruppe steht;
e) Alkylierung einer Amino-Verbindung (XXIV) mittels einer Verbindung (XXVI) 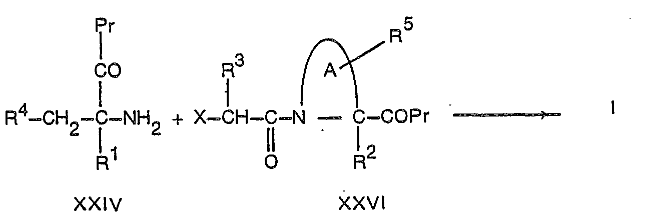 worin X Chloro, Bromo, lodo, Alkansulfonyloxy oder Arensulfonyloxy ist, A, R¹, R², R³, R⁴, R⁵ die oben für Verbindungen der Formel I angegebenen Bedeutungen haben und Pr für eine freie oder geschützte (z.B. durch Veresterung) Hydroxy-Gruppe steht, unter den für Verfahren b beschriebenen Bedingungen;
f) zur Herstellung von Verbindungen der Formel in denen einer der Substituenten R³, R⁴ und R⁵ eine Gruppe Z―(CH₂)₀₋₆― ist, worin Z Z⁵, Z⁶, Z⁷, Z⁸, Z⁹ oder Z¹⁰ ist, vorzugsweise Z⁷, Z⁸ oder Z⁹:
Kondensation eines Peptids der allgemeinen Formel (XXX) mit einer die gewünschte Gruppe enthaltenden Verbindung (XXXI) 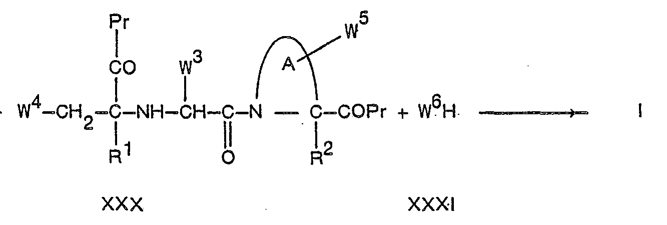 worin R¹, R² und A die für Formel angegebenen Bedeutungen haben, Pr eine geschützte Hydroxy-Gruppe ist, W³, W⁴ und W⁵ den Definitionen für R³, R⁴ bzw. R⁵ entsprechen, jedoch mit dem Unterschied, daß einer der Substituenten W³, W⁴ und W⁵ eine NH₂-Gruppe anstelle der betreffenden Gruppe Z5 bis Z¹⁰ enthält, und W⁶ Z⁵, Z⁶, Z⁷, Z⁸, Z⁹ oder Z¹⁰ ist;
g) zur Herstellung von Verbindungen der Formel I, in denen einer der Substituenten R³, R⁴ und R⁵ eine Gruppe Z―(CH₂)₀₋₆― ist, worin Z Z¹, Z² oder Z³ ist:
Kondensation eines Peptids der Formel XXXII mit einer passend substituierten Verbindung XXXIII 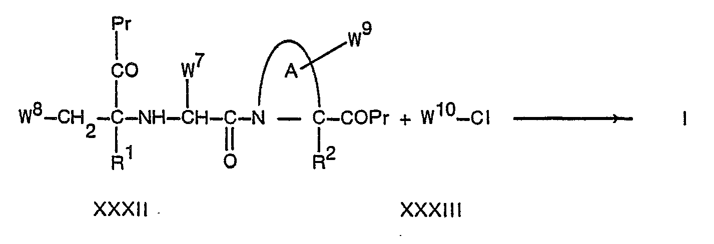 worin R¹, R² und A die für Formel angegebenen Bedeutungen haben, Pr eine geschützte Hydroxy-Gruppe ist, W⁷, W⁸ und W⁹ den Definitionen für R³, R⁴ bzw. R⁵ entsprechen, jedoch mit dem Unterschied, daß einer der Substituenten W⁷, W⁸ und W⁹ eine NH₂-Gruppe anstelle der betreffenden Gruppe Z¹, Z² enthält und W¹⁰ 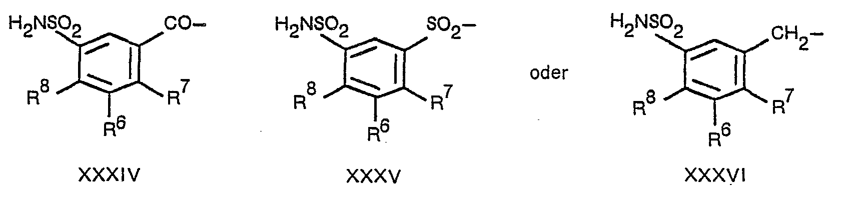 ist;
h) zur Herstellung von Verbindungen der Formel I, in denen einer der Substituenten R³, R⁴ und R⁵ eine Gruppe Z―(CH₂)₀₋₆― ist, worin Z Z⁴ ist:
Kondensation eines Peptids der Formel (XXXVII) mit einem 3-Halogenomethyl-benzothiadiazin (XXXVIII) 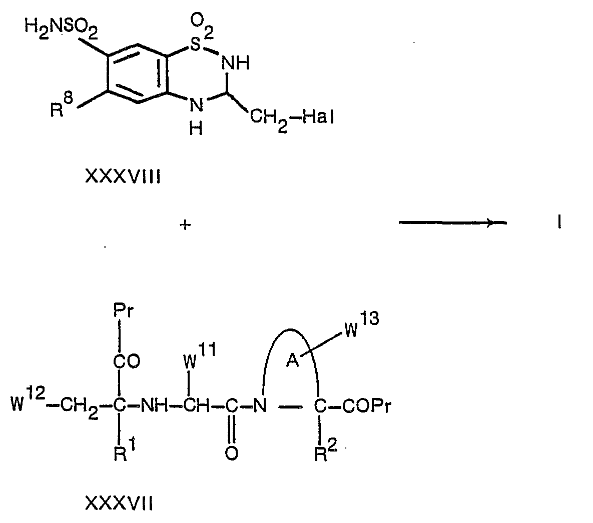 worin R¹, R² und A die für Formel angegebenen Bedeutungen haben, Pr eine geschützte Hydroxy-Gruppe ist, W¹¹, W¹² und W¹³ den Definitionen für R³, R⁴ bzw. R⁵ entsprechen, jedoch mit dem Unterschied, daß einer der Substituenten W¹¹, W¹² und W¹³ eine SH-Gruppe anstelle der betreffenden Gruppe Z⁴ enthält, und Hal Halogen, vorzugsweise Chloro ist;
i) zur Herstellung von Verbindungen der Formel I, in denen einer der Substituenten R³, R⁴ und R⁵ eine Gruppe Z―(CH₂)₀₋₆― ist, worin Z Z⁵ oder Z⁶ ist:
Kondensation eines Peptids der Formel XXXIX mit einer Verbindung der Formel XXXX 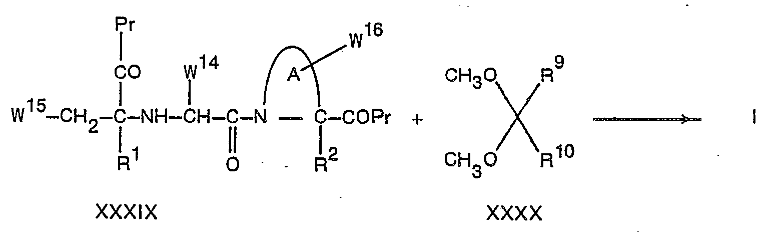 worin R¹, R², R⁹, R¹⁰ und A die für Formel angegebenen Bedeutungen haben, Pr eine geschützte Hydroxy-Gruppe ist, W¹⁴, W¹⁵ und W¹⁶ den Definitionen für R³, R⁴ bzw. R⁵ entsprechen, jedoch mit dem Unterschied, daß einer der Substituenten W¹⁴, W¹⁵ und W¹⁶ die Gruppe 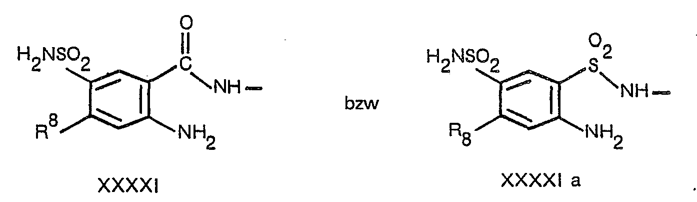 anstelle der betreffenden Gruppe Z⁵ bzw. Z⁶ enthält, gefolgt von, sofern zur Gewinnung des gewünschten Produkts erforderlich, der Entfernung der Schutzgruppen und, gewünschtenfalls, der Umwandlung der so erhaltenen Verbindung der Formel I in ihren Ester und/oder Freisetzen der Verbindung der Formel I aus ihrem Ester oder Herstellung eines Salzes derselben und, gewünschtenfalls, Isolierung des bevorzugten Isomers.

2. Verfahren nach Anspruch 1, dadurch gekennzeichnet, daß eine Verbindung hergestellt wird, in der R⁴ eine Gruppe Z―(CH₂)₀₋₆― nach Anspruch 1 ist, wobei Z vorzugsweise Z¹, Z², Z³, Z⁵, Z⁷, Z⁸, Z⁹ oder Z¹⁰ ist, wobei R⁴ vorzugsweise Z¹(CH₂)_{2 oder 3}―, Z²―(CH₂)_{2 oder 3}―, Z³―(CH₂)_{2 oder 3}―, Z⁵―(CH₂)_{2 oder 3}―, Z⁷―(CH₂)_{2 oder 3}―, Z⁸―(CH₂)_{2 oder 3}―, Z⁹―(CH₂)_{2 oder 3}―, Z¹⁰―(CH₂)_{2 oder 3}―,ist, und worin vorzugsweise die Gruppe die Gruppe 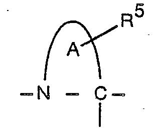 der Formel II, IV (worin B ein gesättigter Ring ist) oder VIII ist, wobei R⁵ vorzugsweise Wasserstoff ist.

3. Verfahren nach Anspruch 1 oder 2, dadurch gekennzeichnet, daß eine Verbindung hergestellt wird, in der R¹ und R² Wasserstoff sind und/oder, wenn Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in der Definition der Struktureinheit Z chloro ist und/oder R³ Methyl ist.

4. Verbindung nach Anspruch 1 oder 2, dadurch gekennzeichnet, daß eine Verbindung hergestellt wird, in der R¹ und R² Wasserstoff sind, die Gruppe 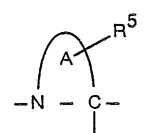 vorzugsweise die Gruppe der Formel IV ist, worin B ein gesättigter Ring ist und R⁵ Wasserstoff ist, R⁴ Z¹―(CH₂)₃― oder Z²―(CH₂)₃® ist, worin R⁶ Wasserstoff ist, R⁷ Wasserstoff oder Hydroxy ist und R⁸ Choro ist, und R³ Methyl ist, vorzugsweise
1 - {N - [1 (S) - Ethoxycarbonyl - 5 - (4- chloro - 3 - sulfamoyl) - benzolsulfonaminopentyl] - (S) - alanyl} - cis,syn - octahydro - 1H - indol - 2(S) - carbonsäure,
1 - {N - [1 (S) - Ethoxycarbonyl - 5 - (4 - chloro - 3 - sulfamoyl) - benzamidopentyl] - (S) - alanyl} - cis,syn - octahydro - 1 H - indol - 2(S) - carbonsäure oder
1 - {N - [1(S) - Ethoxycarbonyl - 5 - (4 - chloro - 2 - hydroxy - 5 - sulfamoyl) - benzamidopentyl] - (S) - alanyl} - cis,syn - octahydro - 1 H - indol - 2(S) - carbonsäure,
1 - {N - [1(S) - Carboxy - 5 - [[(4 - chloro - 2 - hydroxy - 5 - sulfamoyl - phenyl)carbonyl]amino]pentyl] - (S) - alanyl} - cis,syn - octahydro - 1H - indol - 2(S) - carbonsäure,
1 - {N - [1 (S) - Carboxy - 5 - [[(4 - chloro - 3 - (N - methylsulfamoyl) - phenyl)carbonyl]amino]pentyl] - (S) - alanyl} - cis,syn - octahydro - 1H - indol - 2(S) - carbonsäure,
1 - {N - [1(S) - Carboxy - 5 - [[(4 - chloro - 3 - sulfamoylphenyl)carbonyl]amino]pentyl] - (S) - alanyl} - cis,syn - octahydro - 1 H - indol - 2(S) - carbonsäure, in freier Form oder in Form ihrer Ester, vorzugsweise in Form ihres Mono- oder Diethylesters.

5. Verfahren nach Anspruch 1, dadurch gekennzeichnet, daß eine Verbindung hergestellt wird, in der R³ eine Gruppe Z―(CH₂)₀₋₆― nach Anspruch 1 ist, vorzugsweise Z¹―(CH₂)₄―, Z²―(CH₂)₄, Z³―(CH₂)₄―, Z⁴―CH₂―, Z⁵―(CH₂)₄―, Z⁶―(CH₂)₄―, Z⁷―(CH₂)₄―, Z⁸―(CH₂)₄―, Z⁹―(CH₂)₄― oder Z¹⁰―(CH₂)₄―, und worin vorzugsweise die Gruppe die Gruppe

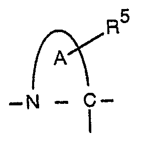

der Formel II, IV (worin B ein gesättigter Ring ist) oder VIII ist.

6. Verfahren nach Anspruch dadurch gekennzeichnet, daß eine Verbindung hergestellt wird, in der R¹ und R² Wasserstoff sind und/oder Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in der Definition der Struktureinheit Z Chloro ist und/oder R⁴ Benzyl oder Ethyl ist.

7. Verbindung nach Anspruch 5, dadurch gekennzeichnet, daß eine Verbindung hergestellt wird, in der R¹ und R² Wasserstoff sind, die Gruppe die Gruppe 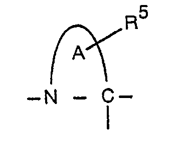 der Formel IV ist, worin B ein gesättigter Ring ist und R⁵ Wasserstoff ist, R³ Z¹―(CH₂)₄― oder Z²―(CH₂)₄― ist, worin R⁶ und R⁷ Wasserstoff sind, und R⁸ Chloro ist, und R⁴ Benzyl ist, vorzugsweise
1 - {Na - [1 (S) - Ethoxycarbonyl - 3 - phenylpropyl] - Nε - [(4 - chloro - 3 - sulfamoyl)benzolsulfonyl - (S) - lysyl} - cis,syn - octahydro - 1H - indol - 2(S) - carbonsäure oder
1 - {Na - [1 (S) - Ethoxycarbonyl - 3 - phenylpropyl] - Nε - [(4 - chloro - 3 - sulfamoyl)benzoyl - (S) - lysyl} - cis,syn - octahydro - 1H - indol - 2(S) - carbonsäure, in freier Form oder in Form ihres Ester, vorzugsweise in Form ihres Mono- oder Diethylesters.

8. Verfahren nach Anspruch 1, dadurch gekennzeichnet, daß eine Verbindung Hergestellt wird, in der R⁵ eine Gruppe Z―(CH₂)₀₋₆― nach Anspruch 1 ist, vorzugsweise Z¹, Z², Z³, Z5, Z⁷, Z⁸, Z⁹ oder Z¹⁰, worin die Gruppe vorzugsweise die Gruppe 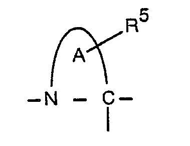 der Formel II, VI (worin B ein aromatischer Ring ist) oder IV (worin B ein gesättigter Ring ist) ist, vorzugsweise

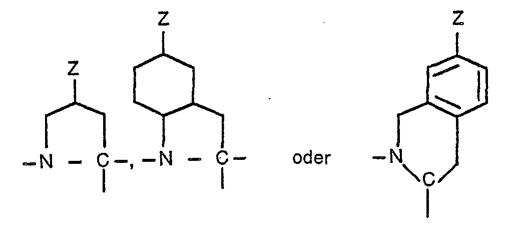

9. Verfahren nach Anspruch 8, dadurch gekennzeichnet, daß eine Verbindung hergestellt wird, in der R¹ und R² Wasserstoff sind und/oder, wenn Z die Formel IX, X oder XI hat, R⁶ Wasserstoff ist und R⁷ Wasserstoff oder Hydroxy ist, und/oder wenn Z die Formel XIII oder XIV hat, R⁹ und R¹⁰ unabhängig voneinander Wasserstoff oder Methyl sind und/oder R⁸ in der Definition der Struktureinheit Z Chloro ist und/oder R³ Methyl ist und/oder R⁴ Benzyl oder Ethyl ist, wobei die Verbindung vorzugsweise
7 - (4 - Chloro - 3 - sulfamoylbenzamido) - 2 - {N - [1 (S) - ethoxycarbonyl - 3 - phenylpropyl] - (S) - alanyl} - 1,2,3,4 - tetrahydroisochinolin - 3 - (S) - carbonsäure oder die entsprechende 1-S-Carboxy-Verbindung ist.

10. Verfahren zur Herstellung einer eine Verbindung der allgemeinen Formel I oder ein pharmazeutisch unbedenkliches Salz derselben oder einen Ester derselben nach einem der Ansprüche 1 bis 9 enthaltenden pharmazeutischen Zusammensetzung, dadurch gekennzeichnet, daß der Wirkstoff in eine für die therapeutische Anwendung geeignete Form gebracht wird.

## Revendications (Revendications pour l'(les) Etat(s) contractant(s) suivant(s) : BE CH DE FR GB IT LI LU NL SE)

1. Composé caractérisé en ce qu'il a pour formule 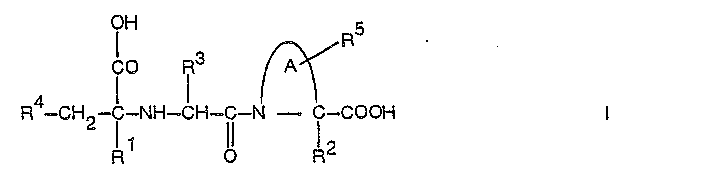 ses esters acceptables en pharmacie et les sels acceptables en pharmacie, où
R¹ et R² sont indépendamment de l'hydrogène ou un alcoyle inférieur; le groupe 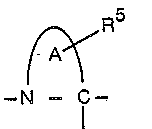 a l'une des structures II à VIII 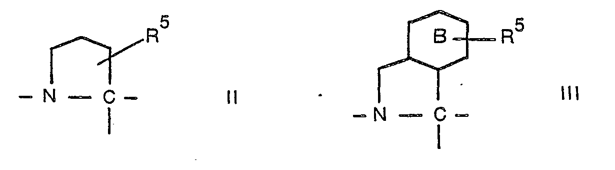 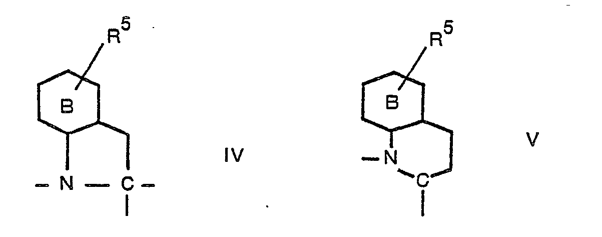 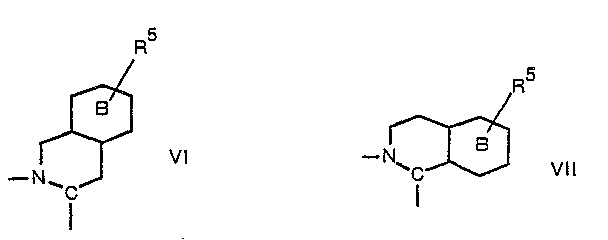 (où B est un noyau saturé ou aromatique) ou 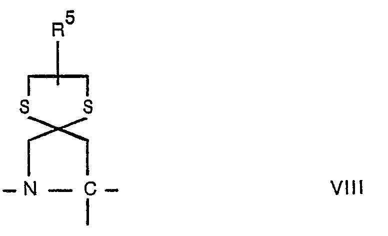 l'un de R³, R⁴ et R⁵ est un groupe Z―(CH₂)₀₋₆―, où Z a l'une des valeurs Z¹ à Z¹⁰ qui suivent: 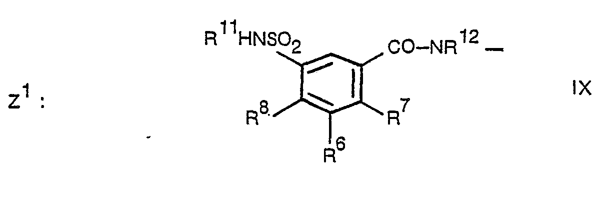 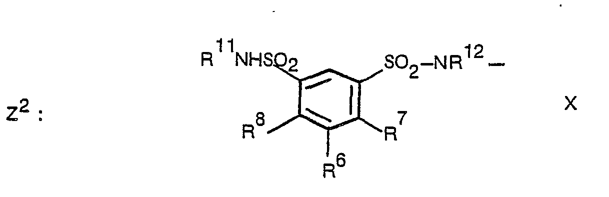 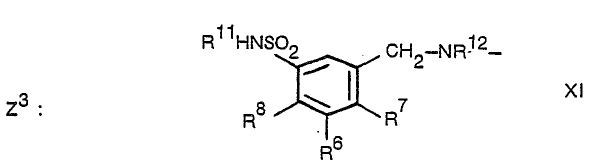 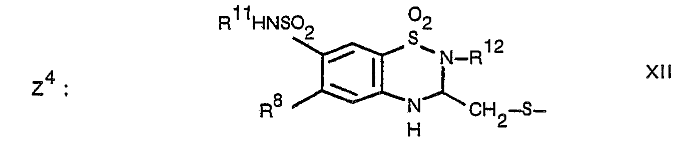 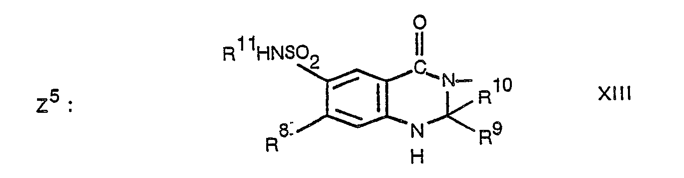 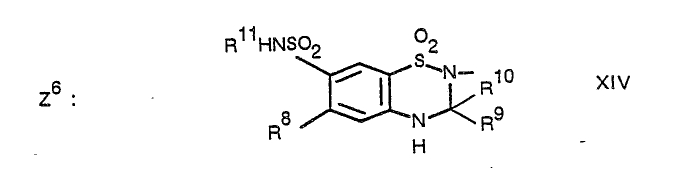 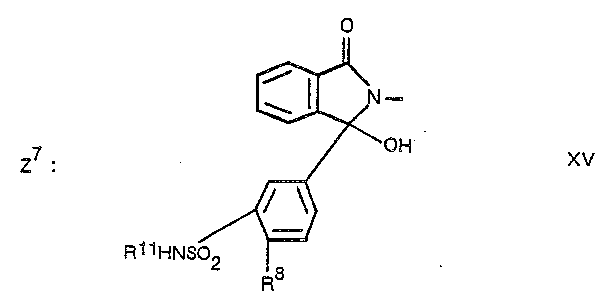 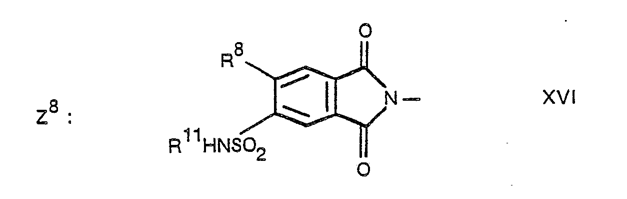 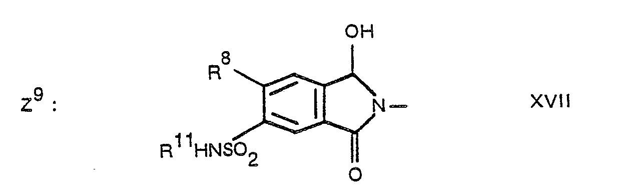 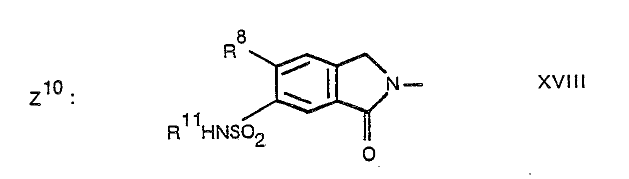 .où R⁸ est Cl ou CF₃;
R⁶ est hydrogène ou halogène;
R⁷ est hydrogène, halogène, carboxy, hydroxy ou amino;
R⁹ et R¹⁰ sont indépendamment de l'hydrogène, un alcoyle inférieur ou un halo alcoyle inférieur; et R⁹ peut également être phényle ou phényl-alcoyle inférieur;
R¹¹ est de l'hydrogène ou un alcoyle inférieur;
R¹² est de l'hydrogène, un alcoyle inférieur ou un phénylalcoyle inférieur; quand R³ est le groupe Z―(CH₂)₀₋₆―, alors
R³ est Z¹―(CH₂)₁₋₆―, Z²―(CH₂)₁₋₆―, Z³―(CH₂)₁₋₆―, Z⁴―CH₂―, Z⁵―(CH₂)₁₋₆―, Z⁶―(CH₂)₁₋₆―, Z⁷―(CH₂)₁₋₆―, Z⁸―(CH₂)₁₋₆―, Z⁹―(CH₂)₁₋₆―, ou Z¹⁰―(CH₂)₁₋₆―,
R⁴ est un alcoyle inférieur, benzyle, benzyloxy, benzylthio, phénoxy ou phénylthio,
R⁵ est de l'hydrogène; et le groupe 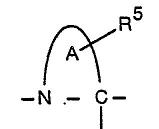 a l'une des structures II à VIII;
et quand R⁴ est le groupe Z―(CH₂)₁₋₆―. alors R⁴ est Z¹―(CH₂)₁₋₆―, Z²―(CH₂)₀₋₆―, Z³―(CH₂)₀₋₆―. Z⁴―(CH₂)₀₋₆―. Z⁵―(CH₂)₀₋₆―, Z⁶―(CH₂)₀₋₆―. Z⁷―(CH₂)₀₋₆―, Z⁸―(CH₂)₀₋₆―, Z⁹―(CH₂)₀₋₆― ou Z¹⁰―(CH₂)₀₋₆, et
R³ est hydrogène, alcoyle inférieur ou amino alcoyle inférieur et
R⁵ est de l'hydrogène; et le groupe 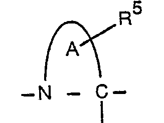 a l'une des structures II à VIII;
et quand R⁵ est le groupe Z―(CH₂)₀₋₆―, alors R⁵ est Z¹, Z², Z³, Z⁴, Z⁵, Z⁶, Z⁷, Z⁸, Z⁹ ou Z¹⁰
R³ est de l'hydrogène, un alcoyle inférieur ou un amino alcoyle inférieur et
R⁴ est un alcoyle inférieur, benzyle, benzyloxy, benzylthio, phénoxy ou phénylthio; et le groupe 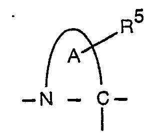 a l'une des structures II à VII, étant préférentiellement sous la forme de l'acide dicarbonique libre ou sous la forme de son ester alcoyle, le groupe alcoyle contenant 1 à 6 atomes de carbone, spécialement sous la forme de son monoester où le groupe carboxy attaché au groupe 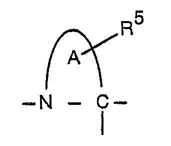 est sous forme libre, tous les composés précédents étant préférentiellement le stéréoisomère pour lequel la configuration absolue à chacun des trois atomes de carbone liés à la fois à un azote et un groupe carbonyle correspond de très près à la configuration absolue des L-aminoacides.

2. Composé selon la revendication 1, caractérisé en ce que R⁴ est un groupe Z―(CH₂)₀₋₆― précité, et où Z est préférentiellement Z¹, Z², Z³, Z⁵, Z⁷, Z⁸ Z⁹ ou Z¹⁰;
R⁴ étant préférentiellement Z¹―(CH₂)_{2 ou 3}―, Z²―(CH₂)_{2 ou 3}―, Z³―(CH₂)_{2 ou 3}―, Z⁵―(CH₂)_{2 ou 3}―, Z⁷―(CH₂)_{2 ou 3}―, Z⁸―(CH₂)_{2 ou 3}―, Z⁹―(CH₂)_{2 ou 3}― ou Z¹⁰―(CH₂)₂ ou 3―, et où préférentiellement le groupe 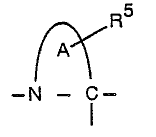 est le groupe de formule II, IV (où B est un noyau saturé) ou VIII, R⁵ étant préférentiellement hydrogène.

3. Composé selon la revendication 1 ou 2, caractérisé en ce que R¹ et R² sont hydrogène, et/ou quand Z a l'une des formules IX, X ou XI R⁶ est hydrogène et R⁷ est hydrogène ou hydroxy, et/ou quand Z a l'une des formules XIII ou XIV R⁹ et R¹⁰ sont indépendamment hydrogène ou méthyle, et/ou R⁸ dans la définition de la moitié Z, est chloro, et/ou R³ est méthyle.

4. Composé selon l'une des revendications 1 ou 2, caractérisé en ce que R¹ et R² sont hydrogène, le groupe 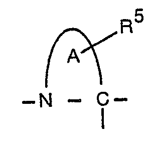 est un groupe de formule IV, où B est un noyau saturé et R⁵ est hydrogène, R⁴ est Z¹―(CH₂)₃― ou Z²―(CH₂)₃―, où R⁶ est hydrogène, R⁷ est hydrogène ou hydroxy, et R⁸ est chlore; et R³ est méthyle, étant de préférence
de l'acide 1-{N-[1(S)-éthoxycarbonyl-5-(4-chloro-3-sulfamoyl)-benzènesulfonaminopentyl]-(S)-alanyl}-cis, syn-octahydro-1 H-indole-2(S)-carboxylique,
de l'acide 1-{N-[1(S)-éthoxycarbonyl-5-(4-chloro-3-sulfamoyl)-benzamidopentyl]-(S)-alanyl}-cis, yn- octahydro-1H-indole-2(S)-carboxylique, ou
de l'acide 1-{N-[1(S)-éthoxycarbonyl-5-(4-chloro-2-hydroxy-5-sulfamoyl)-benzamidopentyl]-(S)-aianyl}-cis, syn-octahydro-1 H-indole-2(S)-carboxylique,
de l'acide 1-{N-[1(S)-carboxy-5-[[(4-chloro-2-hydroxy-5-sulfamoyl-phényl)carbonyl]amino]pentyl]-(S)-alanyl}-cis, syn-octahydro-1 H-indole-2(S)-carboxylique,
de l'acide 1-{N-[1(S)-carboxy-5-[[(4-chloro-3-(N-méthylsulfamoyl)phényl]carbonyl]amino]pentyl]-(S1- alanyl}-cis, syn-octahydro-1H-indole-2(S)-carboxylique,
de l'acide 1-{N-[1(S)-carboxy-5-[[(4-chloro-3-sulfamoyl phényl)carbonyl]amino]pentyl]-(S)-alanyl}-cis, syn-octahydro-1 H-indole-2(S)-carboxylique,
sous forme libre ou sous la forme de son ester, de préférence ester mono-ou-di-éthyle.

5. Composé selon la revendication 1, caractérisé en ce que R³ est un groupe Z―(CH₂)₀₋₆― précité, de préférence Z¹―(CH₂)₄―, Z²―(CH₂)₄―, Z³―(CH₂)₄―, Z⁴―CH₂―, Z⁵―(CH₂)₄―, Z⁶―(CH₂)₄―, Z⁷―(CH²)₄―, Z⁸―(CH₂)₄―, Z⁹―(CH₂)₄―, ou Z¹⁰―(CH₂)₄― et où le groupe 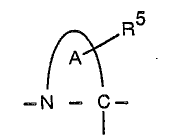 est de préférence le groupe de formule II, IV (où B est un noyau saturé) ou VIII.

6. Composé selon la revendication 5, caractérisé en ce que R¹ et R² sont hydrogène, et/ou Z a l'une des formules IX, X ou XI, R⁶ est hydrogène et R⁷ est hydrogène ou hydroxy, et/ou quand Z a la formule XIII ou XIV R⁹ et R¹⁰ sont indépendamment hydrogène ou méthyle, et/ou R⁸ dans la définition de la moitié de Z est chloro, et/ou R⁴ est benzyle ou éthyle.

7. Composé selon la revendication 5, caractérisé en ce que R¹ et R² sont hydrogène, le groupe 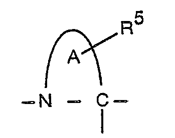 est le groupe de formule IV, où B est un noyau saturé est R⁵ est hydrogène, R³ et Z¹―(CH₂)₄― ou Z²―(CH₂)₄―, où R⁶ et R⁷ sont hydrogène, et R³ est chloro, et R⁴ est benzyle, étant préférentiellement de l'acide
1-{Nα-[1(S)-éthoxycarbonyl-3-phénylpropyl]-Nε-[(4-chloro-3-sulfamoyl)benzènesulfonyl]-(S)-lysyl}-cis, syn-octahydro-1H-indole-2(S)-carboxylique ou
1-{-Nα-[1(S)-éthoxycarbonyl-3-phénylpropyl]-Nε-[(4-chloro-3-sulfamoyl)benzoyl-(S)-lysyl}-cis, syn-octahydro-1 H-indole-2(S)-carboxylique
sous forme libre ou sous forme de son ester, de préférence sous forme de l'ester mono-ou-di-éthyle.

8. Composé selon la revendication 1, caractérisé en ce que R⁵ est un groupe Z―(CH₂)₀₋₆― précité, étant de préférence Z¹, Z², Z³, Z⁵, Z⁷, Z⁸, Z⁹ ou Z¹⁰; où le groupe 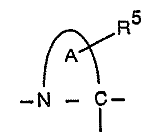 est de préférence le groupe de formule II, VI (où B est un noyau aromatique), ou IV (où B est un noyau saturé), de préférence

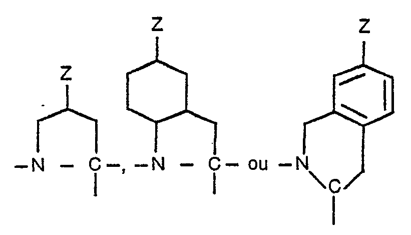

9. Composé selon la revendication 8, caractérisé en ce que R¹ et R² sont hydrogène, et/ou quand Z a la formule IX, X ou XI R⁶ est hydrogène et R⁷ est hydrogène ou hydroxy, et/ou quand Z a la formule XIII ou XIV R⁹ et R¹⁰ sont indépendamment hydrogène ou méthyle, et/ou R⁸ dans la définition de la moitié de Z est chloro, et/ou R³ est méthyle et/ou R⁴ est benzyle ou éthyle, le composé étant de préférence (7-(4-chloro-3-sulfamoylbenzamido)-2-{N-[1(S)-éthoxycarbonyl-3-phénylpropyl]-(S)-alanyl}-1,2,3,4-tétrahydroisoquinoline-3(S)-carboxylique, ou le composé 1-S-carboxy correspondant.

10. Procédé de préparation d'un composé de la formule I tel que défini selon l'une quelconque des revendications 1 à 9, caractérisé en ce que le composé est préparé par un procédé approprié choisi dans les procédés qui suivant a à i:
a) pour la préparation d'un composé de formule I, où R¹ est de l'hydrogène: condensation d'un composé céto (XIX) avec un dipeptide (XX) sous réduction 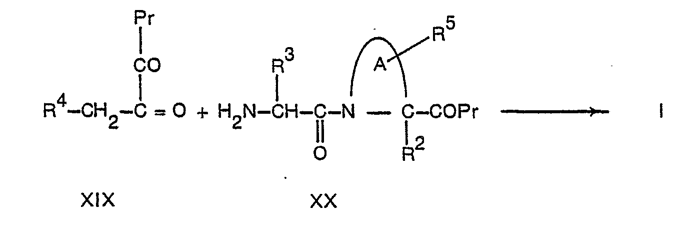 où A, R², R³, R⁴ et R⁵ sont tels que définis ci-dessus et Pr indique un groupe hydroxy libre ou protégé;
b) alcoylation d'un peptide (XX) au moyen d'un composé de formule (XXI) en conditions basiques 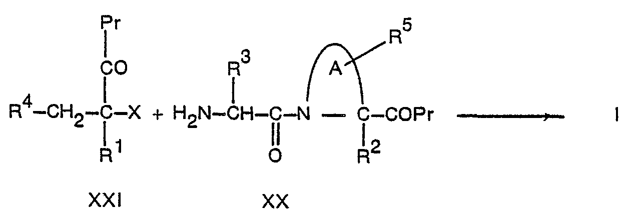 où X est chloro, bromo, iodo, alcanesulfonyloxy ou arènesulfonyloxy, A, R¹, R² R³, R⁴, R⁵ sont tels que définis ci-dessus pour les composés de formule I et Pr indique un groupe hydroxy libre ou protégé;
c) condensation d'un aminoacide (XXII) avec un aminoacide (XXIII) en présence d'un agent de condensation 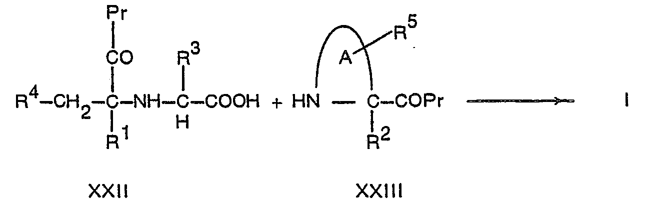 où A, R¹, R² R³, R⁴, R⁵ sont tels que définis ci-dessus pour les composés de formule I et Pr indique un groupe hydroxy libre ou protégé (par exemple par estérification);
d) condensation d'un composé amino (XXIV) avec un composé céto (XXV) 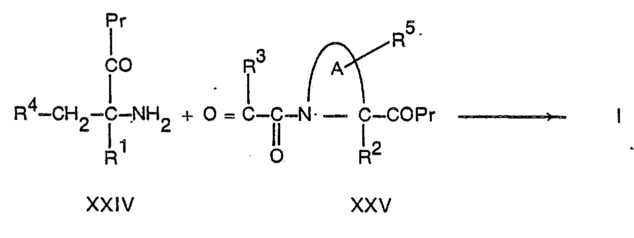 aux conditions décrites par le procédé a où A, R¹, R² R³, R⁴, R⁵ sont tels que définis ci-dessus pour les composés de formule I et Pr indique un groupe hydroxy libre ou protégé (par exemple par estérification);
e) alcoylation d'un composé amino (XXIV) au moyen d'un composé (XXVI) 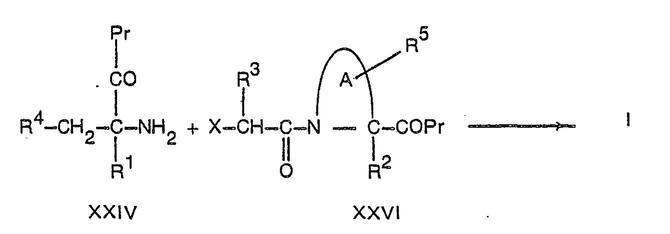 où X est chloro, bromo, iodo, alcanesulfonyloxy ou arènesulfonyloxy, A, R¹, R² R3, R⁴, R⁵ sont tels que définis ci-dessus pour les composés de formule I et Pr indique un groupe hydroxy libre ou protégé (par exemple par estérification), aux conditions décrites pour le procédé b;
f) pour la préparation d'un composé de formule I, où l'un de R³, R⁴ et R⁵ est un groupe Z―(CH₂)₀₋₆―, où Z est Z⁵, Z⁶, Z⁷, Z⁸, Z⁹ ou Z¹⁰, de préférence Z⁷, Z⁸ ou Z⁹: la condensation d'un peptide de formule générale (XXX) avec un composé contenant le groupe souhaité (XXXI) 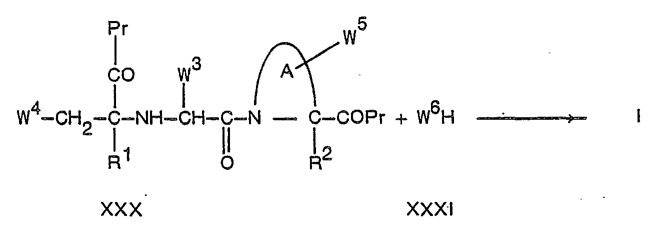 où R¹, R² et A sont tels que définis pour la formule I, Pr est un groupe hydroxy protégé, W³, W⁴ et W⁵ sont définis comme R³, R⁴ et R⁵ respectivement à la différence que l'une de W³, W⁴ et W⁵ contient un groupe NH₂― au lieu du groupe respectif Z⁵ à Z¹⁰; et W⁶ est Z⁵, Z⁶, Z⁷, Z⁸, Z⁹ ou Z¹⁰;
g) pour la préparation d'un composé de formule I, où l'un de R³, R⁴ et R⁵ est un groupe Z―(CH₂)₀₋₆―, où Z est Z¹, Z² ou Z³: la condensation d'un peptide de formule XXXII avec un composé substitué de façon appropriée de formule XXXIII 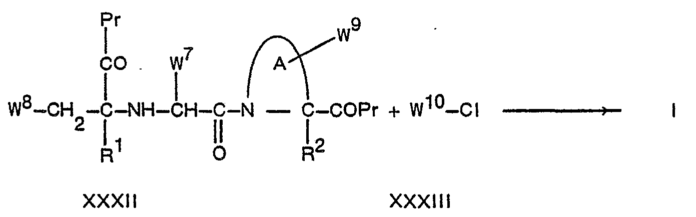 où R¹, R² et A sont tels que définis pour la formule I, Pr est un groupe hydroxy protégé, W⁷, W⁸ et W⁹ sont définis comme R³, R⁴ et R⁵ respectivement, à la différence que l'un de W⁷, W⁸ et W⁹ contient un groupe NH₂― au lieu du groupe respectif Z¹, Z² ou Z³, et W¹⁰ est 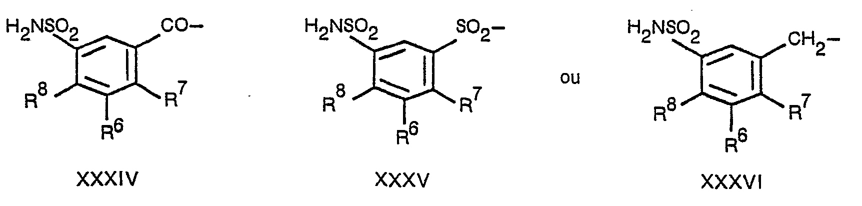
h) pour la préparation d'un composé de formule I, où l'un de R³, R⁴ et R⁵ est un groupe Z―(CH₂)₀₋₆―, où Z est Z⁴: la condensation d'un peptide de formule (XXXVII) avec une 3-halométhylbenzothiadiazine (XXXVIII) 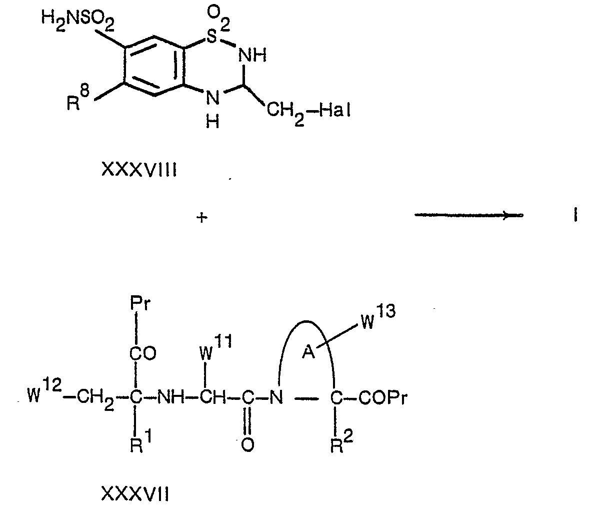 où R¹, R² et A sont tels que définis pour la formule I, Pr est un groupe hydroxy protégé, W¹¹, W¹² et W¹³ sont définis comme R³, R⁴ et R⁵ respectivement à la différence que l'un de W", W¹² et W¹³ contient un groupe ―SH― au lieu du groupe Z⁴ respectif et Hal est un halogène, de préférence chloro;
i) pour la préparation d'un composé de formule I, où l'un de R³, R⁴ et R⁵ est un groupe Z―(CH₂)₀₋₆―, où Z est Z⁵ ou Z⁶: la condensation d'un peptide de formule XXXIX avec un composé de formule XXXX 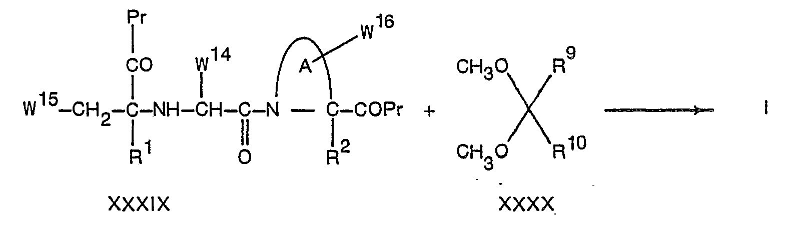 où R¹, R², R⁹, R¹⁰ et A sont tels que définis pour la formule I, Pr est un groupe hydroxy protégé, W¹⁴, W¹⁵ et W¹⁶ sont définis comme R³, R⁴ et R⁵ respectivement à la différence que l'un de W¹⁴, W¹⁵ et W¹⁶ contient le groupe 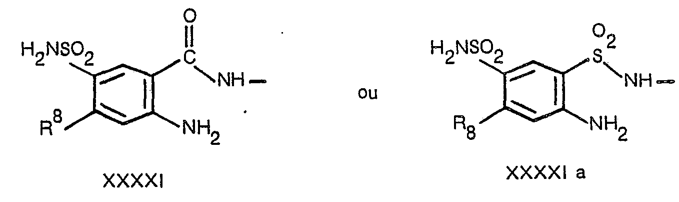 au lieu du groupe Z⁵; avec ensuite enlèvement des groupes protecteurs, si nécessaire pour donner le produit souhaité et si on le souhaite, la conversion d'un composé ainsi obtenu de formule l en son ester et/ou la libération du composé de formule I de son ester ou la préparation de son sel et, si on le souhaite, l'isolement de l'isomère préféré.

11. Composition pharmaceutique caractérisée en ce qu'elle comprend un composé de formule générale I ou son sel ou ester accéptable en pharmacie défini selon l'une des revendications 1 à 9 et obtenu selon le procédé de la revendication 10.

## Revendications (Revendications pour l'(les) Etat(s) contractant(s) suivant(s) : AT)

1. Procédé pour la fabrication d'un composé de formule 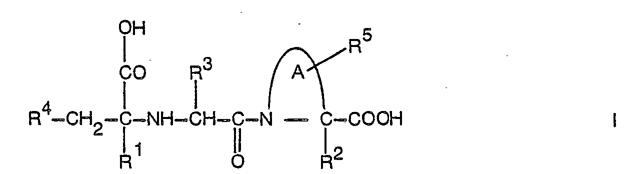 ses esters acceptables en pharmacie et les sels acceptables en pharmacie, où
R¹ et R² sont indépendamment de l'hydrogène ou un alcoyle inférieur; le groupe 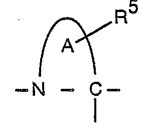 a l'une des structures II à VIII 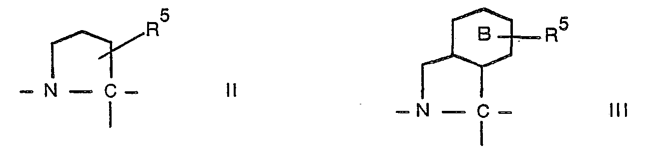 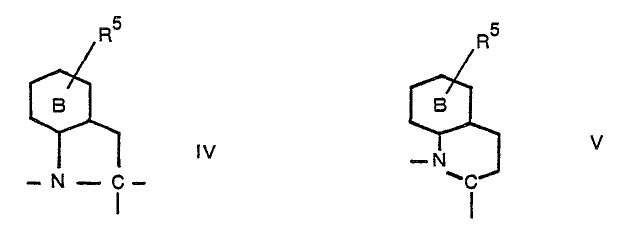 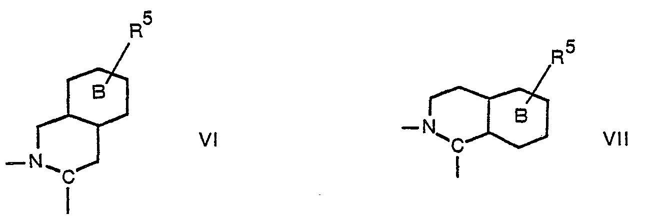 (où B est un noyau saturé ou aromatique) ou 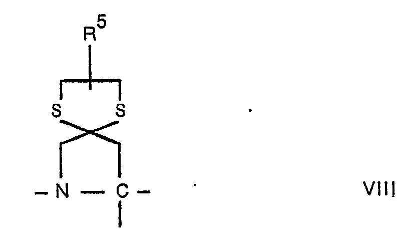 l'un de R³, R⁴ et R⁵ est un groupe Z―(CH₂)₀₋₆―, où Z a l'une des valeurs Z¹ à Z¹⁰ qui suivent: 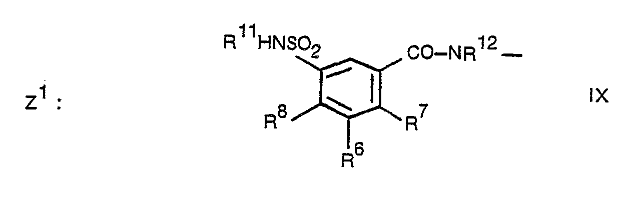 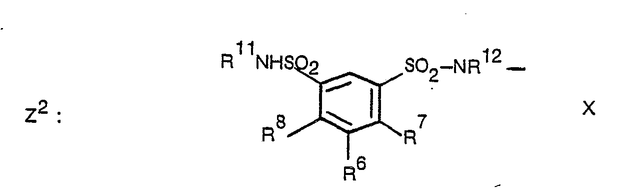 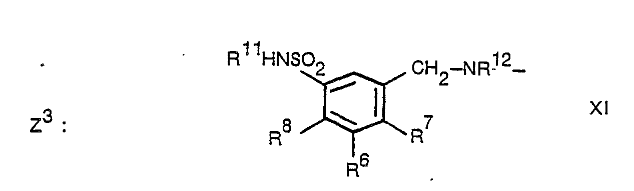 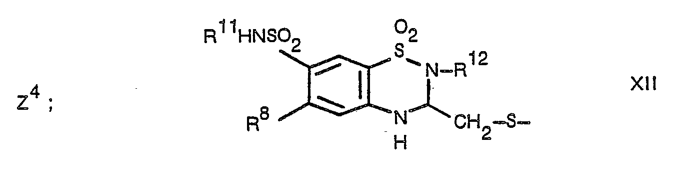 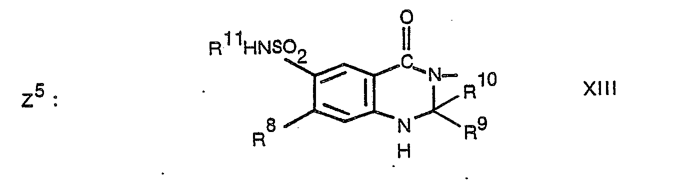 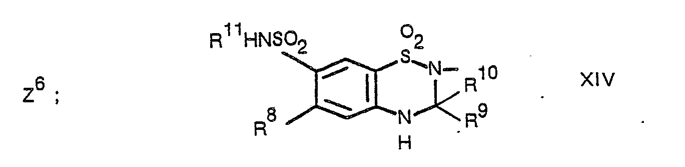 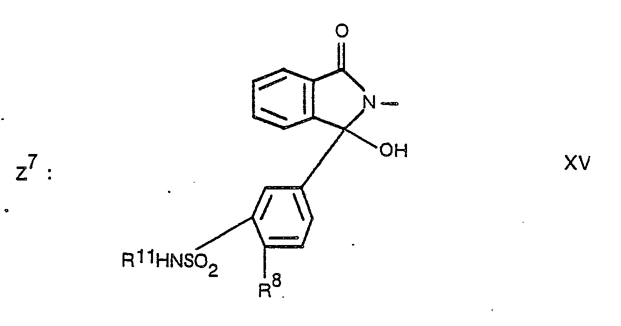 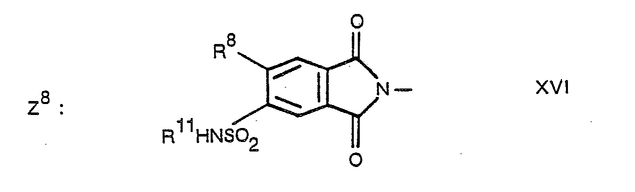 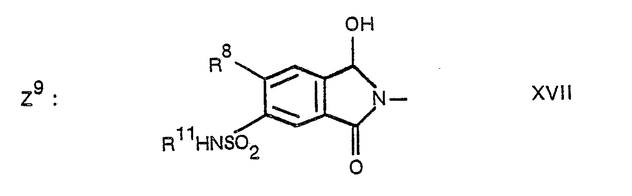 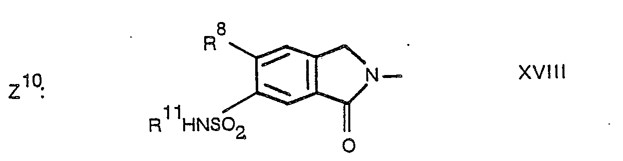 où R⁸ est CI ou CF₃;
R⁶ est hydrogène ou halogène;
R⁷ est hydrogène, halogène, carboxy, hydroxy ou amino;
R⁹ et R¹⁰ sont indépendamment de l'hydrogène, un alcoyle inférieur ou un halo alcoyle inférieur; et R⁹ peut également être phényle ou phényl-alcoyle inférieur;
R¹¹ est de l'hydrogène ou un alcoyle inférieur;
R¹² est de l'hydrogène, un alcoyle inférieur ou un phénylalcoyle inférieur; quand R³ est le groupe Z―(CH₂)₀₋₆―, alors
R³ est Z¹―(CH₂)₁₋₆―, Z²―(CH₂)₁₋₆―, Z³―(CH₂)₁₋₆―, Z⁴―CH₂―, Z⁵―(CH₂)₁₋₆―, Z⁶―(CH₂)₁₋₆―, Z⁷―(CH₂)₁₋₆―, Z⁸―(CH₂)₁₋₆―, Z⁹―(CH₂)₁₋₆―. ou Z¹⁰―(CH₂)₁₋₆―,
R⁴ est un alcoyle inférieur, benzyle, benzyloxy, benzylthio, phénoxy ou phénylthio,
R⁵ est de l'hydrogène; et le groupe 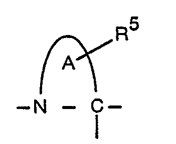 a l'une des structures II à VIII;
et quand R⁴ est le groupe Z―(CH₂)₀₋₆―, alors R⁴ est Z¹―(CH₂)₀₋₆―, Z²―(CH₂)₀₋₆―. Z³―(CH₂)₀₋₆―. Z⁴―(CH₂)₀₋₆―, Z⁵―(CH₂)₀₋₆―, Z⁶―(CH₂)₀₋₆―, Z⁷―(CH₂)₀₋₆―. Z⁸―(CH₂)₀₋₆―, Z⁹―(CH₂)₀₋₆― ou Z¹⁰―(CH₂)₀₋₆, et
R³ est hydrogène, alcoyle inférieur ou amino alcoyle inférieur et
R⁵ est de l'hydrogène; et le groupe 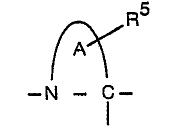 a l'une des structurer II à VIII;
et quand R⁵ est le groupe Z―(CH₂)₀₋₆―, alors R⁵ est Z¹, Z², Z³, Z⁴, Z⁵, Z⁶, Z⁷, Z⁸, Z⁹ ou Z¹⁰
R³ est de l'hydrogène, un alcoyle inférieur ou un amino alcoyle inférieur et
R⁴ est un alcoyle inférieur, benzyle, benzyloxy, benzylthio, phénoxy ou phénylthio; et le groupe 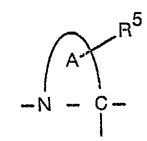 a l'une des structures II à VII, étant préférentiellement sous la forme de l'acide dicarbonique libre ou sous la forme de son ester alcoyle, le groupe alcoyle contenant 1 à 6 atomes de carbone, spécialement sous la forme de son monoester où le groupe carboxy attaché au groupe 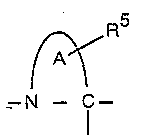 est sous forme libre, tous les composés précédents étant préférentiellement le stéréoisomère pour lequel la configuration absolue à chacun des trois atomes de carbone liés à la fois à un azote et un groupe carbonyle correspond de très près à la configuration absolue des L-aminoacides, caractérisé en ce que le composé est préparé par un procédé approprié choisi parmi les procédés qui suivent a à i:
a) pour la préparation d'un composé de formule I, où R¹ est de l'hydrogène: condensation d'un composé céto (XIX) avec un dipeptide (XX) sous réduction 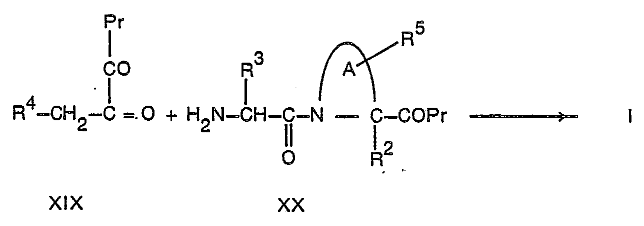 où A, R², R³, R⁴ et R⁵ sont tels que définis ci-dessus et Pr indique un groupe hydroxy libre ou protégé;
b) alcoylation d'un peptide (XX) au moyen d'un composé de formule (XXI) en conditions basiques 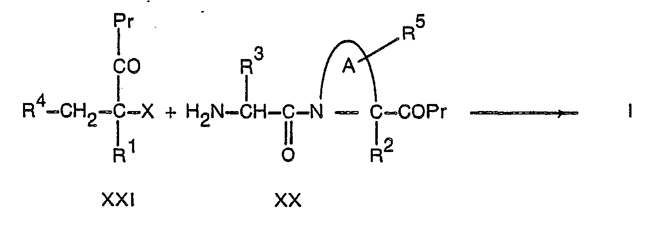 où X est chloro, bromo, iodo, alcanesulfonyloxy ou arènesulfonyloxy, A, R¹, R² R³, R⁴, R⁵ sont tels que définis ci-dessus pour les composés de formule I et Pr indique un groupe hydroxy libre ou protégé;
c) condensation d'un aminoacide (XXII) avec un aminoacide (XXIII) en présence d'un agent de condensation 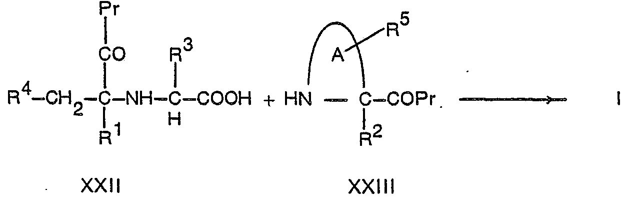 où A, R¹, R² R³, R⁴, R⁵ sont tels que définis ci-dessus pour les composés de formule I, et Pr indique un groupe hydroxy libre ou protégé (par exemple par estérification);
d) condensation d'un composé amino (XXIV) avec un composé céto (XXV) 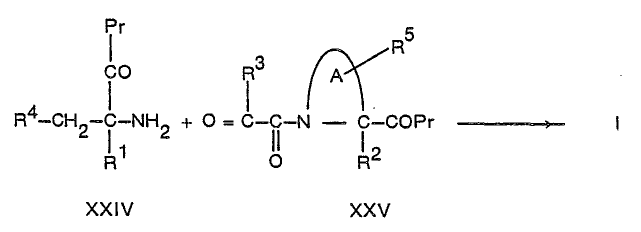 aux conditions décrites par le procédé a où A, R¹, R² R³, R⁴, R⁵ sont tels que définis ci-dessus pour les composés de formule 1 et Pr indique un groupe hydroxy libre ou protégé (par exemple par estérification);
e) alcoylation d'un composé amino (XXIV) au moyen d'un composé (XXVI) 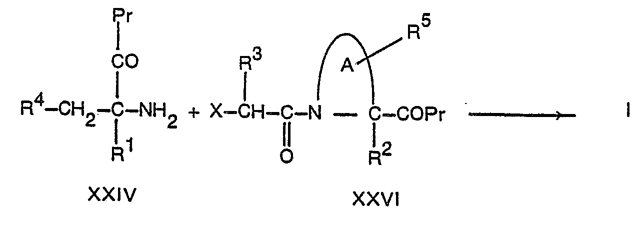 où X est chloro, bromo, iodo, alcanesulfonyloxy ou arènesulfonyloxy, A, R¹, R² R3, R⁴, R⁵ sont tels que définis ci-dessus pour les composés de formule I et Pr indique un groupe hydroxy libre ou protégé (par exemple par estérification), aux conditions décrites pour le procédé b;
f) pour la préparation d'un composé de formule I, où l'un de R³, R⁴ et R⁵ est un groupe Z―(CH₂)₀₋₆―. où Z est Z⁵, Z⁶, Z⁷, Z⁸, Z⁹ ou Z¹⁰, de préférence Z⁷, Z⁸ ou Z⁹: la condensation d'un peptide de formule générale (XXX) avec un composé contenant le groupe souhaité (XXXI) 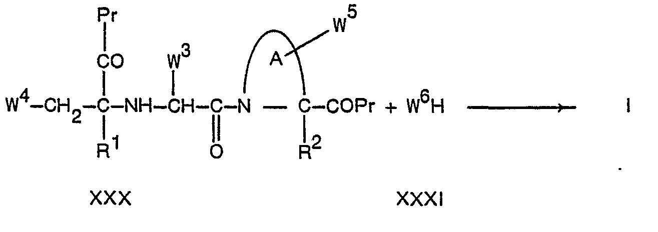 où R¹, R² et A sont tels que définis pour la formule I, Pr est un groupe hydroxy protégé, W3, W⁴ et W⁵ sont définis comme R³, R⁴ et R⁵ respectivement à la différence que l'une de W³, W⁴ et W⁵ contient un groupe NH₂― au lieu du groupe respectif Z⁵ à Z¹⁰; et W⁶ est Z⁵, Z⁶, Z⁷, Z⁸, Z⁹ ou Z¹⁰;
g) pour la préparation d'un composé de formule I, où l'un de R³, R⁴ et R⁵ est un groupe Z―(CN₂)₀₋₆―, où Z est Z¹, Z² ou Z³: la condensation d'un peptide de formule XXXII avec un composé substitué de façon appropriée de formule XXXIII 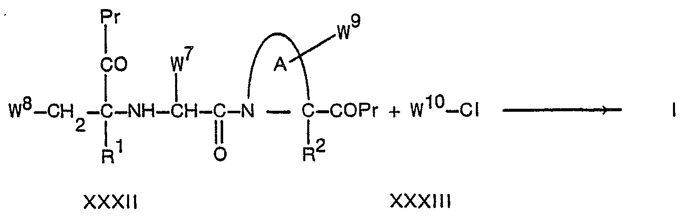 où R¹, R² et A sont tels que définis pour la formule I, Pr est un groupe hydroxy protégé, W⁷, W⁸ et W⁹ sont définis comme R³, R⁴ et R⁵ respectivement, à la différence que l'un de W⁷, W⁸ et W⁹ contient un groupe NH₂― au lieu du groupe respectif Z¹, Z² ou Z³, et W¹⁰ est 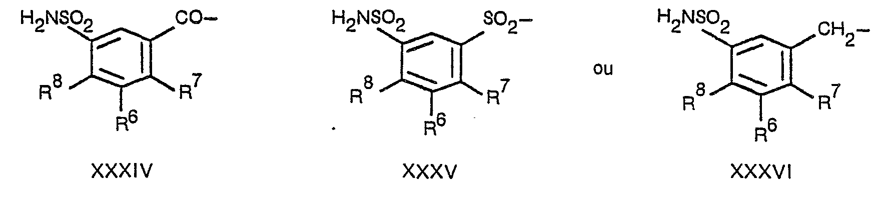
h) pour la préparation d'un composé de formule I, où l'un de R³, R⁴ et R⁵ est un groupe Z―(CH₂)₀₋₆―, où Z est Z⁴: la condensation d'un peptide de formule (XXXVII) avec une 3-halométhylbenzothiadiazine (XXXVIII) 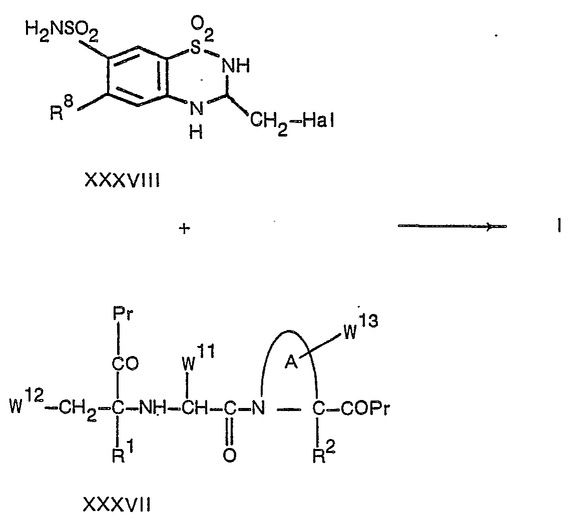 où R¹, R² et A sont tels que définis pour la formule I, Pr est un groupe hydroxy protégé, W¹¹, W¹² et W¹³ sont définis comme R³, R⁴ et R⁵ respectivement à la différence que l'un de W¹¹, W¹² et W¹³ contient un groupe -SH- au lieu du groupe Z⁴ respectif et Hal est un halogène, de préférence chloro;
i) pour la préparation d'un composé de formule I, où l'un de R³, R⁴ et R⁵ est un groupe Z―(CH₂)₀₋₆―, où Z est Z⁵ ou Z⁶: la condensation d'un peptide de formule XXXIX avec un composé de formule XXXX 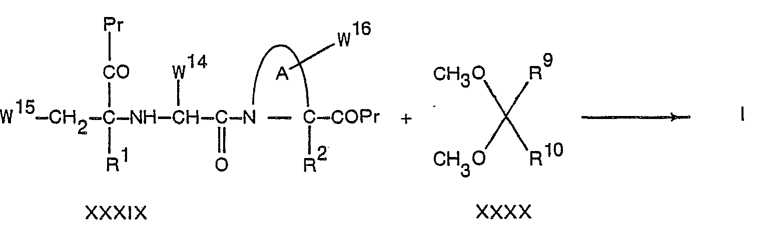 où R¹, R², R⁹, R¹⁰ et A sont tels que définis pour la formule I, Pr est un groupe hydroxy protégé, W¹⁴, W¹⁵ et W¹⁶ sont définis comme R³, R⁴ et R⁵ respectivement à la différence que l'un de W¹⁴, W¹⁵ et W¹⁶ contient le groupe 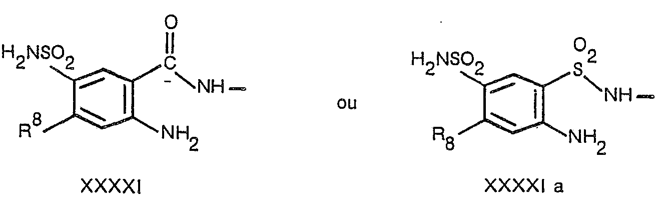 au lieu du groupe Z⁵; avec ensuite enlèvement des groupes protecteurs, si nécessaire pour donner le produit souhaité et si on le souhaite, la conversion d'un composé ainsi obtenu de formule I en son ester et/ou la libération du composé de formule I de son ester ou la préparation de son sel et, si on le souhaite, l'isolement de l'isomère préféré.

2. Procédé selon la revendication 1, caractérisé en ce qu'un composé est préparé, où R⁴ est un groupe Z―(CH₂)₀₋₆― précité, et où Z est préférentiellement Z¹, Z², Z³_{;} Z⁵, Z⁷, Z⁸ Z⁹ ou Z¹⁰;
R⁴ étant préférentiellement Z¹―(CH₂)₂ ou 3―, Z²―(CH₂)₂ ou 3―, Z³―(CH₂)₂ ou 3―, Z⁵―(CH2)₂ ou 3―, Z⁷―(CH₂)₂ ou 3―, Z⁸―(CH₂)₂ ou 3―, Z⁹―(CH₂)₂ ou 3― ou Z¹⁰―(CH₂)₂ ou 3―, et où préférentiellement le groupe 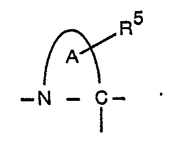 est le groupe de formule II, IV (où B est un noyau saturé) ou VIII, R⁵ étant préférentiellement hydrogène.

3. Procédé selon la revendication 1 ou 2, caractérisé en ce qu'un composé est préparé, où R¹ et R² sont hydrogène, et/ou quand Z a l'une des formules IX, X ou XI R⁶ est hydrogène et R⁷ est hydrogène ou hydroxy, et/ou quand Z a l'une des formules XIII ou XIV R⁹ et R¹⁰ sont indépendamment hydrogène ou méthyle, et/ou R⁸ dans la définition de la moitié Z, est chloro, et/ou R³ est méthyle.

4. Procédé selon la revendication 1 ou 2, caractérisé en ce qu'un composé est préparé, où R¹ et R² sont hydrogène, le groupé 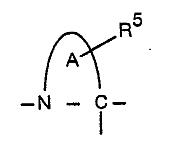 est un groupe de formule IV, où B est un noyau saturé et R⁵ est hydrogène, R⁴ est Z¹―(CH₂)₃― ou Z²―(CH₂)₃―, où R⁶ est hydrogène, R⁷ est hydrogène ou hydroxy, et R⁸ est chlore; et R³ est méthyle, étant de préférence
de l'acide 1-{N-[1(S)-éthoxycarbonyl-5-(4-chloro-3-sulfamoyl)-benzènesulfonaminopentyl]-(S)-alanyl}-cis, syn-octahydro-1 H-indole-2(S)-carboxylique,
de l'acide 1-{N-[1(S)-éthoxycarbonyl-5-(4-chloro-3-sulfamoyl)-benzamidopentyl]-(S)-alanyl}-cis, syn-octahydro-1H-indole-2(S)-carboxylique, ou
de l'acide 1-{N-[1(S)-éthoxycarbonyl-5-(4-chloro-2-hydroxy-5-sulfamoyl)-benzamidopentyl]-(S)-alanyl}-cis, syn-octahydro-1 H-indole-2(S)-carboxylique,
de l'acide 1-{N-[1(S)-carboxy-5-[[(4-chloro-2-hydroxy-5-sulfamoyl-phényl)carbonyl]amino]pentyl]-(S)-alanyl}-cis, syn-octahydro-1 H-indole-2(S)-carboxylique,
de l'acide 1-{N-[1(S)-carboxy-5-[[(4-chloro-3-(N-méthylsulfamoyl)phényl]carbonyl]amino]pentyl]-(S)-afanyt}-cis, syn-octahydro-1 H-indole-2(S)-carboxylique,
de l'acide 1-{N-[1(S)-carboxy-5-[[(4-chloro-3-sulfamoyl phényl)carbonyl]amino]pentyl]-(S1-alanyl}-cis, syn-octahydro-1 H-indole-2(S)-carboxylique,
sous forme libre ou sous la forme de son ester, de préférence ester mono-ou-di-éthyle.

5. Procédé selon la revendication 1, caractérisé en ce qu'un composé est préparé, où R³ est un groupe Z―(CH₂)₀₋₆― précité, de préférence Z¹―(CH₂)₄―, Z²―(CH₂)₄―, Z³―(CH₂)₄―, Z⁴―CH₂―, Z⁵―(CH₂)₄―, Z⁶―(CH₂)₄―, Z⁷―(CH₂)₄―, Z⁸―(CH₂)₄―, Z⁹―(CH₂)₄―, ou Z¹⁰―(CH₂)₄― et où le groupe 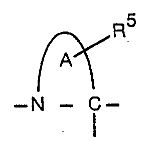 est de préférence le groupe de formule II, IV (où B est un noyau saturé) ou VIII.

6. Procédé selon la revendication 5, caractérisé en ce qu'un composé est préparé, où R¹ et R² sont hydrogène, et/ou Z a l'une des formules IX, X ou XI, R⁶ est hydrogène et R⁷ est hydrogène ou hydroxy, et/ou quand Z a la formule XIII ou XIV R⁹ et R¹⁰ sont indépendamment hydrogène ou méthyle, et/ou R⁸ dans la définition de la moitié de Z est chloro, et/ou R⁴ est benzyle ou éthyle.

7. Procédé selon la revendication 5, caractérisé en ce qu'un composé est préparé où R¹ et R² sont hydrogène, le groupe 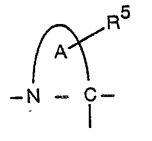 est le groupe de formule IV, où B est un noyau saturé et R⁵ est hydrogène, R³ et Z¹―(CH₂)₄― ou Z²―(CH₂)₄―, où R⁶ et R⁷ sont hydrogène, et R⁸ est chloro, et R⁴ est benzyle, étant préférentiellement de l'acide
1-{Nα-[1(S)-éthoxycarbonyl-3-phénylpropyl]-Nε-[(4-chloro-3-sulfamoyl)benzènesulfonyl]-(S)-lysyl}-cis, syn-octahydro-1H-indole-2(S)-carboxylique ou
1-{-Nα-[1(S)-éthoxycarbonyl-3-phénylpropyl]-Nε-[(4-chloro-3-sulfamoyl)benzoyl-(S)-lysyl}-cis,syn-octahydro-1H-indole-2(S)-carboxylique
sous forme libre ou sous forme de son ester, de préférence sous forme de l'ester mono-ou-di-éthyle.

8. Procédé selon la revendication 1, caractérisé en ce qu'un composé est préparé, où R⁵ est un groupe Z―(CH₂)₀₋₆― précité, étant de préférence Z¹, Z², Z³, Z⁵, Z⁷, Z⁸, Z⁹ ou Z¹⁰; où le groupe 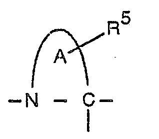 est de préférence le groupe de formule II, VI (où B est un noyau aromatique), ou IV (où B est un noyau saturé), de préférence

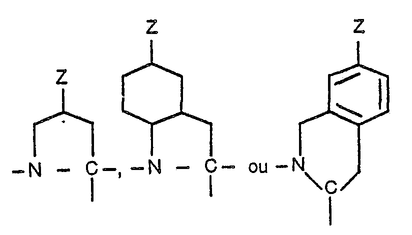

9. Procédé selon la revendication 8, caractérisé en ce qu'un composé est préparé, où R¹et R² sont hydrogène, et/ou quand Z a la formule IX, X ou XI R⁶ est hydrogène et R⁷ est hydrogène ou hydroxy, et/ou quand Z a la formule XIII ou XIV R⁹ et R¹⁰ sont indépendamment hydrogène ou méthyle, et/ou R⁸ dans la définition de la moitié de Z est chloro, et/ou R³ est méthyle et/ou R⁴ est benzyle ou éthyle, le composé étant de préférence (7-(4-chloro-3-sulfamoylbenzamido)-2-{N-[1(S)-éthoxycarbonyl-3-phénylpropyl]-(S)-alanyl}-1,2,3,4-tétrahydroisoquinoline-3(S)-carboxylique, ou le composé 1-S-carboxy correspondant.

10. Procédé pour. la préparation d'une composition pharmaceutique caractérisé en ce qu'elle comprend un composé de formule générale I où son sel ou ester acceptable en pharmacie défini selon l'une des revendications 1 à 9, caractérisé en ce que le composant actif est mis sous forme convenable pour son application thérapeutique.
